(19) Europäisches Patentamt
European Patent Office
Office européen des brevets

(11) Publication number: **0 251 827 B1**

(12) # EUROPEAN PATENT SPECIFICATION

(45) Date of publication of patent specification: **15.09.93**

(51) Int. Cl.[5]: **C07D 417/12**, A61K 31/335, C07D 405/12, C07D 413/12, C07D 413/04, C07D 307/20, C07D 307/18, C07D 313/04, C07D 309/12, A61K 31/34, A61K 31/35

(21) Application number: **87305972.9**

(22) Date of filing: **06.07.87**

The file contains technical information submitted after the application was filed and not included in this specification

(54) **New cyclic ether derivatives, their preparation and their use.**

(30) Priority: **04.07.86 JP 157319/86**
**26.01.87 JP 15484/86**

(43) Date of publication of application:
**07.01.88 Bulletin 88/01**

(45) Publication of the grant of the patent:
**15.09.93 Bulletin 93/37**

(84) Designated Contracting States:
**AT BE CH DE ES FR GB GR IT LI LU NL SE**

(56) References cited:
**EP-A- 157 609**
**EP-A- 188 384**
**EP-A- 210 804**
**DE-A- 2 629 599**
**DE-A- 2 630 947**
**US-A- 4 408 052**
**US-A- 4 675 390**

(73) Proprietor: **Sankyo Company Limited**
**5-1 Nihonbashi Honcho 3-chome Chuo-ku**
**Tokyo(JP)**

(72) Inventor: **Nakamura, Norio Sankyo Company Limited**
**Sankyo Chemical Research Labs. No. 2-58, 1-chome**
**Hiromachi Shinagawa-ku Tokyo 140(JP)**
Inventor: **Miyazaki, Hideki Sankyo Company Limited**
**Sankyo Chemical Research Labs. No. 2-58, 1-chome**
**Hiromachi Shinagawa-ku Tokyo 140(JP)**
Inventor: **Koike, Hiroyuki Sankyo Company Limited**
**Sankyo Biological Research Labs. No. 2-58, 1-chome**
**Hiromachi Shinagawa-ku Tokyo 140(JP)**
Inventor: **Oshima, Takeshi Sankyo Company Limited**
**Sankyo Biological Research Labs. No. 2-58, 1-chome**
**Hiromachi Shinagawa-ku Tokyo 140(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

(74) Representative: **Gibson, Christian John Robert et al**
**MARKS & CLERK 57/60 Lincoln's Inn Fields**
**London WC2A 3LS (GB)**

## Description

The present invention relates to a new class of cyclic ether derivatives and inner salts and pharmaceutically acceptable salts thereof which have excellent antagonism platelet activating factor (hereafter abbreviated, as is conventional, to "PAF").

Natural PAF, at least as isolated from mammalian tissues, is a mixture of from 2 to 5 phospholipids, the number upon the nature of the original tissue. The major constituents of PAF may be represented by the formula (A):

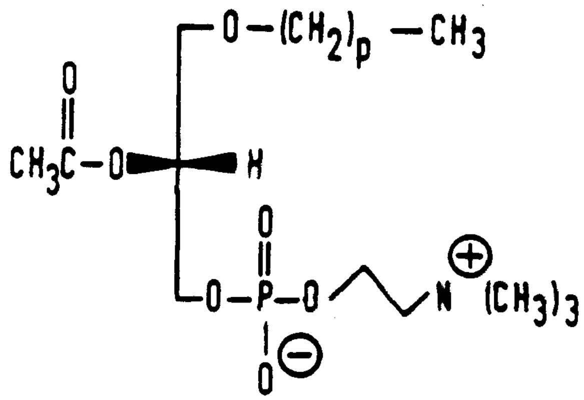

(A)

in which p is the integer 15 or 17. Natural PAF is levorotatory and the various components of natural PAF may be identified, for example as: l-$C_{16:0}$ = formula (A) where p is 15; and l-$C_{18:0}$ = formula (A) where p is 17.

PAF exhibits a strong platelet activating and aggregating effect. It also has a hypotensive effect and increases vasopermeability; it is believed to be an active agent in the induction of anaphylactic shock (for example endotoxin-induced shock), to act as a mediator of inflammatory disease and to act as an activator of neutrophiles. Accordingly, PAF antagonists have been investigated with a view to developing new types of anti-shock agent and of anti-inflammatory agent. In particular, analogues of natural PAF's have been investigated in an attempt to find such PAF antagonists. Currently, several compounds are known as PAF antagonists. For example, the compound of formula (B):

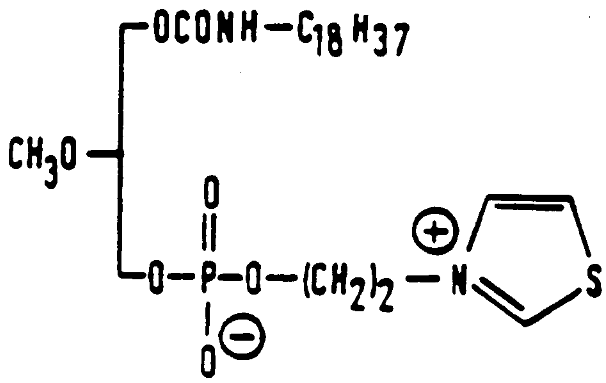

(B)

(also known as CV-3988) is disclosed in U.S. Patent No. 4,408,052, and CV3988 was subsequently found to have PAF antagonist activity, see for example, Life Science, Vol. 32, 1983, pages 1975 - 1982 and Advances in Inflammation Research, Vol. 12, 1988, pages 135 - 157, whilst the compound of formula (C):

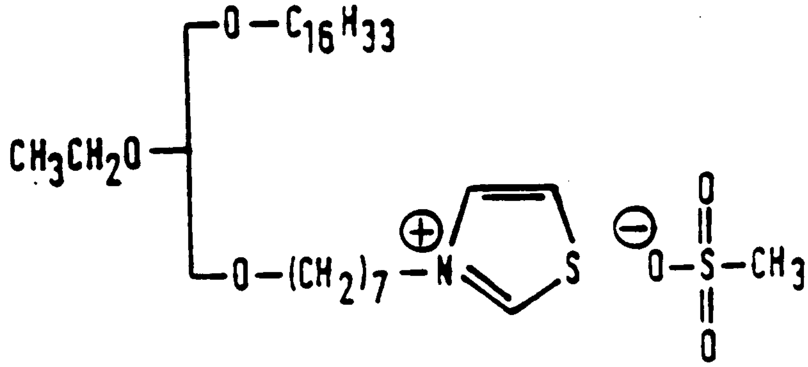

(C)

(known as ONO-6240) is disclosed in European Patent Publication No. 146258. These compounds, however, are unsatisfactory for one or more of the following reasons: they lack sufficient intensity of antagonism towards PAF; the duration of their effect is insufficient; biological utilization is inadequate.

The closest prior art, from the structural point of view, to the compounds of the present invention is believed to be the compounds disclosed in European Patent Publication No. 210 804, but the compounds of that prior art differ from the compounds of the present invention in the nature of one of the groups represented by $R^1$ or $R^2$ in the compounds of the invention and also represented by the same symbols in the prior art compounds. Other structurally similar compounds are disclosed in European Patent Publication No. 188 384. However, these prior compounds are useful as anti-tumor agents, and appear to be substantially free from PAF-like activity and from PAF antagonistic activity. On the contrary, the compounds of the present invention have been found to be excellent PAF antagonists, resulting in anti-asthmatic, anti-inflammatory and anti-shock activities which have excellent duration biological utilization and level of activity. Other PAF antagonists are described in European Patent Application No. 87301283.5, but those compounds, unlike those of the present invention, lack a cyclic ether structure.

Other glycerol derivatives known to have PAF inhibitory activity are disclosed in EP Patent Publication No. 157 609.

The compounds of the invention are those cyclic ethers of formula (I):

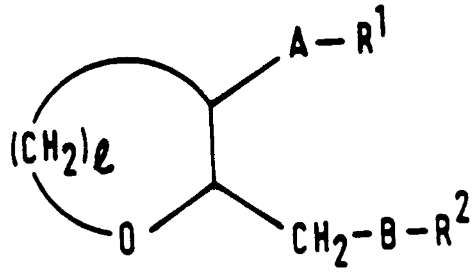

(I)

in which:
l is an integer of from 2 to 4;
A and B are the same or different and each represents an oxygen atom or a sulphur atom;
one of $R^1$ and $R^2$ represents an alkyl group containing from 8 to 22 carbon atoms, an aliphatic carboxylic acyl group containing from 8 to 22 carbon atoms or a group of formula (II):

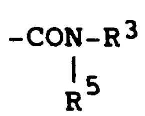

(II)

in which $R^3$ represents an alkyl group containing from 8 to 22 carbon atoms, and
$R^5$ represents a hydrogen atom, a $C_1$ - $C_4$ alkyl group, a $C_1$ - $C_4$ alkanoyl group or an aralkyl group in which the alkyl part is $C_1$ - $C_4$,

and the other of $R^1$ and $R^2$ represents a group of formula (III):

$$-E-(CH_2)_m-\underset{\displaystyle R^4}{\underset{|}{(CH)}}_q-(CH_2)_n-Q \qquad (III)$$

in which E represents a single bond, a bivalent heterocyclic group or a group of formula

$$-\underset{\displaystyle O}{\underset{\|}{C}}-, \quad -\underset{\displaystyle O}{\underset{\|}{C}}-O- \quad \text{or} \quad -\underset{\displaystyle O}{\underset{\|}{C}}-\underset{\displaystyle R^6}{\underset{|}{N}}-$$

in which $R^6$ represents a hydrogen atom or an imino-protecting group;
$\underline{m}$ is the cypher O or an integer from 1 to 3;
$\underline{n}$ is the cypher O or an integer from 1 to 10;
$\underline{q}$ is the cypher O or the integer 1;
$R^4$ represents a hydroxy group, a $C_1$ - $C_4$ alkanoyloxy group, a $C_1$ - $C_4$ alkoxy group, a $C_7$ - $C_9$ aralkyloxy group, a carbamoyloxy group, an alkylcarbamoyloxy group in which the alkyl part is $C_1$ - $C_4$, a dialkylcarbamoyloxy group in which each alkyl part is $C_1$ - $C_4$, a mercapto group, a $C_1$ - $C_4$ alkylthio group, a $C_7$ - $C_9$ aralkylthio group, a $C_1$ - $C_4$ alkanoylthio group, a carbamoylthio group, an alkylcarbamoylthio group in which the alkyl part is $C_1$ - $C_4$, a dialkylcarbamoylthio group in which each alkyl part is $C_1$ - $C_4$ or a carboxy group;
Q represents a group of formula (IV):

$$-N\begin{matrix} / R^7 \\ \backslash R^8 \end{matrix} \qquad (IV)$$

in which $R^7$ and $R^8$ are the same or different and each represents a hydrogen atom or a $C_1$ - $C_6$ alkyl group,
or a monovalent heterocyclic group;
said heterocyclic groups having from 5 to 14 ring atoms, of which from 1 to 4 atoms are nitrogen and/or oxygen and/or sulphur hetero-atoms, at least one of these being a nitrogen atom, and said heterocyclic group being unsubstituted or having at least one of substituents (a) and/or substituents (b);

substituents (a):

oxygen atoms and $C_6$ - $C_{14}$ aryl groups;

substituents (b):

halogen atoms, hydroxy groups, $C_1$ - $C_4$ alkyl groups, $C_1$ - $C_4$ haloalkyl groups, $C_1$ - $C_4$ hydroxyalkyl groups, $C_1$ - $C_4$ alkoxy groups, $C_1$ - $C_6$ alkanoyl groups, $C_3$ - $C_6$ alkenoyl groups, $C_7$ - $C_{15}$ aromatic carboxylic acyl groups, carbamoyl groups, $C_7$ - $C_{15}$ aralkyl groups, $C_2$ - $C_5$ alkoxycarbonyl groups, cyano groups, amino groups, alkylamino groups in which the alkyl part is $C_1$ - $C_4$, dialkylcarbamoyloxy groups in which each alkyl part is $C_1$ - $C_4$ and nitro groups;
said aryl groups and the aryl parts of aralkyl groups and aromatic acyl groups being substituted or having at least one of substituents (b);
and pharmaceutically acceptable salts (including quaternary and inner salts) and esters thereof.

The invention also provides a pharmaceutical composition for the treatment of inflammation or shock, comprising a PAF antagonist in combination with a pharmaceutically acceptable carrier or diluent, wherein the PAF antagonist is at least one compound of formula (I) or a pharmaceutically acceptable salt or ester thereof.

The invention still further provides the use for the preparation of a medicaments for the treatment or prophylaxis of asthma, inflammation or shock of at least one compound of formula (I) or a pharmaceutically acceptable salt or ester thereof.

The invention also prodies methods of preparing the compounds of the invention, which are described in more detail hereafter. In general terms, the compounds of the invention may be prepared by:

(i) reacting a compound of formula (D):

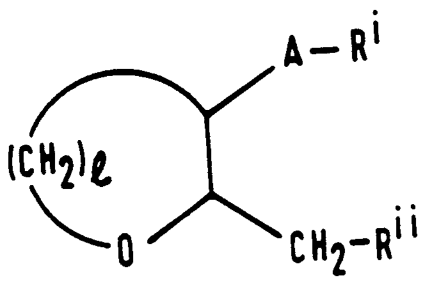

(D)

[in which:

(a) $R^i$ represents a group of formula -A-$R^{1x}$ and $R^{ii}$ represents a group of formula -B-H; or

(b) $R^i$ represents a group of formula -A-$R^{1x'}$ and $R^{ii}$ represents a group of formula -Y; or

(c) $R^i$ represents a group of formula -A-$R^{10}$ and $R^{ii}$ represents a group of formula -Y; or

(d) $R^i$ represents a group of formula -A-H and $R^{ii}$ represents a group of formula -B-$R^{1x}$; or

(e) $R^i$ represents a group of formula -Y and $R^{ii}$ represents a group of formula -B-$R^{1x'}$; or

(f) $R^i$ represents a group of formula -Y and $R^{ii}$ represents a group of formula -B-$R^{10}$; or

(g) $R^i$ represents a group of formula -A-$R^{10}$ and $R^{ii}$ represents a group of formula -B-H; or

(h) $R^i$ represents a group of formula -A-H and $R^{ii}$ represents a group of formula -B-$R^{10}$; or

(i) $R^i$ represents a double bond between the positions $\alpha$ and $\beta$ to the ether oxygen atom and $R^{ii}$ represents a group of formula -Y;

in which:

A and B are as defined above;

$R^{1x}$ represents an alkyl group containing from 8 to 22 carbon atoms, an aliphatic carboxylic acyl group containing from 8 to 22 carbon atoms or a group of formula (II):

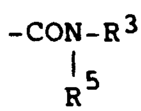

(II)

in which $R^3$ and $R^5$ are as defined above;

$R^{1x'}$ represents an alkyl group contain from 8 to 22 carbon atoms;

$R^{10}$ represents a hydroxy-protecting or mercapto-protecting group;

Y represents a halogen atom];

or a compound of formula (D) in which any active group is protected, with:

<u>in case (a) or (g)</u>

a compound of formula (VI):

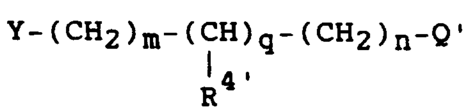

(VI)

or of formula (VII):

$$HB-E^{f}-(CH_2)_m-(\underset{R^{4'}}{\underset{|}{C}}H)_q-(CH_2)_n-Q' \qquad (VII)$$

or of formula (XIX):

$$W-\underset{O}{\underset{\|}{C}}-(CH_2)_m-(\underset{R^{4'}}{\underset{|}{C}}H)_q-(CH_2)_n-Q'' \qquad (XIX)$$

or of formula (XX):

$$O=C=N-(CH_2)_m-(\underset{R^{4'}}{\underset{|}{C}}H)_q-(CH_2)_n-Q'' \qquad (XX)$$

or with a compound of formula (XXII) followed by a compound of formula (XXIV):

$$Y-\underset{O}{\underset{\|}{C}}-Y'' \qquad (XXII)$$

$$H_2N-(CH_2)_m-(\underset{R^{4'}}{\underset{|}{C}}H)_q-(CH_2)_n-Q' \qquad (XXIV)$$

or with said compound of formula (XXII) and then with a compound of formula (XXX):

$$HO-(CH_2)_m-(\underset{R^{4'}}{\underset{|}{C}}H)_q-(CH_2)_n-Q' \qquad (XXX)$$

in case (b) or (c)

a compound of formula (XI):

$$HB-E-(CH_2)_m-(\underset{R^{4'}}{\underset{|}{C}}H)_q-(CH_2)_n-Q' \qquad (XI)$$

in case (d) or (h)

a compound of formula (VI), defined above, or a compound of formula (VII'):

$$HA-E^{f}-(CH_2)_m-(\underset{R^{4'}}{\underset{|}{C}}H)_q-(CH_2)_n-Q' \qquad (VII')$$

or said compound of formula (XIX) or said compound of formula (XX) or with said compound of formula (XXII) followed by either said compound of formula (XXIV) or (XXX);
in case (e) or (f)
a compound of formula (XI'):

$$HA-E-(CH_2)_m-\underset{R^{4'}}{\underset{|}{(CH)}}_q-(CH_2)_n-Q' \qquad (XI')$$

in case (g)
either with an allyl halide and then an oxidising agent or with an epihalohydrin and, in either case, then with a compound of formula $M-(CH_2)_{(n-1)}-Q'$,
and
in case (i)
with a compound of formula (XI), followed by hydroboration and alkylations, acylation or carbamation of the hydroxy group;
[in which:
$R^{4'}$ represents any of the groups defined above for $R^4$, but in which any reactive group is, if necessary, protected;
$E^f$ represents a heterocyclic group containing from 5 to 14 ring atoms, as defined above for E.
Q' represents a group having the formula $-O-R^{11}$, in which $R^{11}$ represents a hydroxy-protecting group;
Q" represents a group of formula Y, defined above, or any one of the heterocyclic groups represented by Q, in which Q is as defined above;
W represents a residue of a reactive carboxylic acid, a lower aliphatic acyloxy group or an aromatic acyloxy group;
Y" represents a leaving group; and
M represents a metal atom];

(ii) where the product of step (i) contains a group of formula Q', converting said group to a group of formula Q"; and

(iii) optionally subjecting the product of step (i) or (ii) to one or more of the reactions amination, protection, deprotection, salification and esterification, in any order.

The compounds of the invention exhibit a number of possibilities for salt formation. For example, the compounds may, depending on their exact nature, form acid addition salts (because of the presence of a nitrogen atom in the heterocyclic group represented by E or Q or in the group of formula $-NR^7R^8$ represented by Q), carboxylate salts (where $R^4$ represents a carboxy group), inner salts (where $R^4$ represents a carboxy group and there is a basic nitrogen atom in the molecule, e.g. from E or Q) and quaternary ammonium salts (where there is a basic nitrogen atom in the molecule, e.g. from E or Q), by the addition of a suitable compound, e.g. an alkyl halide. It is also possible for one compound to exist as a combination of two or more of these different forms of salt, for example, where the compound contains both a carboxy group (from $R^4$) and a basic nitrogen atom (from E and/or Q), the compound may be both a carboxylate salt (e.g. with an alkali metal atom such as sodium or potassium) and an acid addition salt of that nitrogen atom (e.g. with an acid such as hydrochloric acid). All of these possibilities form part of the present invention, as explained in more detail below.

Thus, the compounds of the invention can exist in the form of an inner salt, i.e. a compound of formula (I) as shown above in which one of $R^1$ and $R^2$ represents a group of formula (III'):

$$-E-(CH_2)_m-\underset{COO^-}{\underset{|}{CH}}-(CH_2)_n-Q^+ \qquad (III')$$

in which E, m and n are as defined above and $Q^+$ represents a quaternised form of any of the groups defined above for Q, e.g. a quaternised heterocyclic group or a group of formula (IV'):

```
   R7
   /
-N+-R9          (IV')
   \
    R8
```

in which $R^7$, $R^8$ and $R^9$ are the same or different and each represents a hydrogen atom or a $C_1$ - $C_6$ alkyl group.

Alternatively, the compounds may exist in the form of a quaternary ammonium salt or an acid addition salt, in which Q represents a group of formula (IV"):

```
   R7
   /
-N+-R9      Z-      (IV")
   \
    R8
```

in which $R^7$, $R^8$ and $R^9$ are as defined above and $Z^-$ represents a complementary pharmaceutically acceptable anion (preferably a halogen atom, an anionic residue of another mineral acid, a $C_1$ - $C_6$ alkylsulphonyloxy group, an arylsulphonyloxy group or an anion derived from an organic carboxylic acid or an amino acid),

or a quaternised heterocyclic group together with a complementary pharmaceutically acceptable anion.

Also, where $R^4$ represents a carboxy group, the resulting compounds may form salts with cations, particularly with metals (e.g. alkali metals such as sodium or potassium, alkaline earth metals such as calcium or magnesium or other metals such as tin), but also with ammonia and organic amines and amino acids, as is, of course, well known.

In addition, it is possible to have some combination of the above. In particular, it is possible under specific circumstances, e.g. during certain chromatographic procedures, to form a compound in which the carboxy group represented by $R^4$ has formed a salt with a cation and the nitrogen atom or atoms of Q and/or E has or have formed a quaternary ammonium salt.

Where $Z^-$ in the above formula (IV") represents a halide ion, this may be, for example, a fluoride, chloride, bromide or iodide ion. Where $Z^-$ represents a residue of another mineral acid, this may be any pharmaceutically acceptable acid, and the anion may be for example, a sulphate or phosphate anion. Where $Z^-$ represents an alkylsulphonyloxy group, the alkyl part is $C_1$ - $C_6$ and may be a straight or branched chain group, which may be substituted or unsubstituted; examples include the methanesulphonyloxy and ethanesulphonyloxy groups; substituents are preferably halogen atoms and a preferred substituted group is the trifluoromethanesulphonyloxy group. Where $Z^-$ represents an arylsulphonyloxy group, the aryl part is a $C_6$ - $C_{10}$ carbocyclic aryl group, which may be substituted or unsubstituted and, if substituted, may have from 1 to 3 substituents preferably selected from $C_1$ - $C_4$ alkyl (preferably methyl) groups, halogen atoms, $C_1$ - $C_4$ alkoxy groups and nitro groups. Examples of such arylsulphonyloxy groups include the benzenesulphonyloxy and p-toluenesulphonyloxy groups. Where $Z^-$ represents an anion derived from an organic carboxylic acid, this may be any pharmaceutically acceptable acid, preferably a lower alkanoic acid, and examples include oxalic acid and maleic acid. Where $Z^-$ represents an anion derived from an amino acid, this may be any known pharmaceutically acceptable amino acid, and examples include glutamic acid and aspartic acid.

In the compounds of the invention, one of $R^1$ and $R^2$ represents the above defined group of formula (III) [or (III')], whilst the other represents a $C_8$ - $C_{22}$, preferably $C_{10}$ - $C_{22}$, alkyl or aliphatic acyl group, which may be a straight or branched chain group, or a group of formula (II):

```
-CON-R3
   |          (II)
   R5
```

in which $R^3$ represents an alkyl group containing from 8 to 22, preferably from 10 to 22, carbon atoms, which also may be a straight or branched chain group, and $R^5$ represents a hydrogen atom, a $C_1$ - $C_4$ alkyl group, a $C_1$ - $C_4$ alkanoyl group or a $C_7$ - $C_9$ aralkyl group.

Examples of such $C_8$ - $C_{22}$ alkyl groups which may be represented by $R^1$, $R^2$ and $R^3$ include the octyl, 3-methylheptyl, 4-methylheptyl, 2-ethylhexyl, nonyl, 1-methyloctyl, 2-methyloctyl, 3-ethylheptyl, decyl, 3-methylnonyl, 8-methylnonyl, 3-ethyloctyl, 3,7-dimethyloctyl, undecyl, dodecyl, tridecyl, tetradecyl, pentadecyl, hexadecyl, 1-methylpentadecyl, 14-methylpentadecyl, 13,13-dimethyltetradecyl, heptadecyl, 15-methylhexadecyl, octadecyl, 1-methylheptadecyl, nonadecyl, icosyl, henicosyl and docosyl groups. Straight and branched chain alkyl groups having from 13 to 20 carbon atoms, for example the hexadecyl, 1-methylpentadecyl, 14-methylpentadecyl, 13,13-dimethyltetradecyl, heptadecyl, 15-methyl-hexadecyl, octadecyl and 1-methylheptadecyl groups are preferred.

Examples of such $C_8$ - $C_{22}$ alkanoyl groups which may be represented by $R^1$ and $R^2$ include the heptylcarbonyl, octylcarbonyl, 3-methylheptylcarbonyl, 4-methylheptylcarbonyl, 2-ethylhexylcarbonyl, nonylcarbonyl, decylcarbonyl, 3-methylnonylcarbonyl, 8-methylnonylcarbonyl, 3-ethyloctylcarbonyl, 3,7-dimethyloctylcarbonyl, undecylcarbonyl, dodecylcarbonyl, tridecylcarbonyl, tetradecylcarbonyl, pentadecylcarbonyl, hexadecylcarbonyl, 1-methylpentadecylcarbonyl, 14-methylpentadecylcarbonyl, 13,13-dimethyltetradecylcarbonyl, heptadecylcarbonyl, 15-methylhexadecylcarbonyl, octadecylcarbonyl, 1-methylheptadecyclcarbonyl, nonadecylcarbonyl, icosylcarbonyl and henicosylcarbonyl; preferably a straight or branched chain aliphatic acyl group containing from 13 to 20 carbon atoms.

Where $R^5$ represents an alkyl group, this is a lower alkyl group having from 1 to 4 carbon atoms and it may be a straight or branched chain group. The group more preferably has from 1 to 3 carbon atoms, and examples of such groups include the methyl, ethyl, propyl, isopropyl, butyl, isobutyl, sec-butyl and t-butyl groups, of which the methyl and ethyl groups are preferred.

Where $R^5$ represents an alkanoyl group, this is a lower carboxylic alkanoyl group having from 1 to 4 carbon atoms and it may be a straight or branched chain group. The group more preferably has from 2 to 4 carbon atoms and examples of such groups include the formyl, acetyl, propionyl, butyryl, and isobutyryl groups, of which the acetyl and propionyl groups are preferred.

Where $R^5$ represents an aralkyl group, the aryl part may be as defined above and may be substituted or unsubstituted. Where it is substituted, the substituents are preferably selected from the group consisting of substituents (b), defined above, more preferably halogen atoms and $C_1$ to $C_4$ alkyl, $C_1$ to $C_4$ haloalkyl, $C_1$ to $C_4$ alkoxy, $C_2$ to $C_5$ alkoxycarbonyl, aryl and nitro groups. The alkyl part is preferably a $C_1$ to $C_4$ alkyl group, for example selected from those alkyl groups defined above in relation to $R^5$, more preferably a methyl, ethyl or propyl group. Examples of such aralkyl groups include aralkyl groups having, in total, from 7 to 9 carbon atoms, for example the benzyl, phenethyl, phenylpropyl and 1- or 2-, preferably 1-, naphthylmethyl groups, of which the benzyl group is preferred. Such preferred groups may, if desired, be substituted as defined above.

Where E represents said group of formula

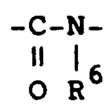

and $R^6$ represents an imino-protecting group, there is no particular limitation on the nature of such a group, and any group which is conventionally employed as an imino-protecting group may equally be employed in the present invention. In general, we prefer that $R^6$ should be a hydrogen atom, a $C_1$ - $C_4$ alkyl group, a $C_1$ - $C_4$ alkanoyl group or a $C_7$ - $C_9$ aralkyl group examples of which have been given in relation to the corresponding groups which may be represented by $R^5$. Examples of other imino-protecting groups include: haloalkanoyl groups and aromatic acyl groups, such as the chloroacetyl and benzoyl groups; alkoxycarbonyl groups, such as the t-butoxycarbonyl, 2,2,2-tribromoethoxycarbonyl and 2-trimethylsilylethoxycarbonyl groups; and the alkenyloxycarbonyl groups, such as the allyloxycarbonyl group.

Where E represents a bivalent heterocyclic group, this may have from 5 to 14, preferably from 5 to 7, ring atoms, of which from 1 to 4, preferably from 1 to 3, are sulphur and/or oxygen and/or nitrogen hetero-atoms, at least one being a nitrogen atom. Such groups may be fully unsaturated or partly or completely hydrogenated. Also included are analogues of such groups in which a phenyl or substituted phenyl group is fused to the heterocyclic ring. Examples of these heterocyclic groups include the furanediyl, thiophenediyl, pyrrolediyl, azepinediyl, morpholinediyl, thiomorpholinediyl, pyrazolediyl, imidazolediyl, oxazolediyl, isoxazolediyl, thiazolediyl, isothiazolediyl, oxadiazolediyl (e.g. 1,2,3-oxadiazolediyl), triazolediyl, tetrazolediyl, thiadiazolediyl (e.g. 1,2,3-thiadiazolediyl), pyranediyl, pyridinediyl, pyridazinediyl, pyrimidinediyl and pyrazinediyl groups, and partly or completely hydrogenated analogues thereof, of which those heterocyclic groups having 5 or 6, more preferably 5, ring atoms are preferred and the imidazolediyl, oxazolediyl, isoxazolediyl and thiazolediyl groups and partly or completely hydrogenated analogues thereof are more preferred.

The heterocyclic groups represented by E may be substituted or unsubstituted, and, if substituted, the substituents may be selected from substituents (a) and (b) defined above, more preferably those substituents exemplified hereafter in relation to the substituted heterocyclic groups which may be represented by Q. However, the heterocyclic groups represented by E are preferably unsubstituted.

Q may represent a group of formula (IV):

```
     R7
    /
 -N                          (IV)
    \
     R8
```

in which $R^7$ and $R^8$ are the same or different and each represents a hydrogen atom or a $C_1$ - $C_6$ alkyl group,

or a monovalent heterocyclic group having from 5 to 14, preferably from 5 to 10 and more preferably from 5 to 7, ring atoms; alternatively, of course, it may represent the corresponding quaternised group $Q^+$, which may be a heterocyclic group or a group of formula (IV'):

```
     R7
    /
 -N+-R9                      (IV')
    \
     R8
```

or (IV"):

```
     R7
    /
 -N+-R9          Z-          (IV")
    \
     R8
```

in which $R^7$, $R^8$, $R^9$ and $Z^-$ are as defined above.

Where Q represents a nitrogen-containing heterocyclic group, this has from 5 to 14, preferably from 5 to 10, ring atoms, of which from 1 to 4, preferably from 1 to 3, are sulphur and/or oxygen and/or nitrogen hetero-atoms, at least one being a nitrogen atom. Such groups may be fully unsaturated or partly or completely hydrogenated. Also included are analogues of such groups in which a phenyl or substituted phenyl group is fused to the heterocyclic ring. Examples of these heterocyclic groups include the pyrrolyl, pyrrolidinyl, piperidinyl, piperazinyl, azepinyl, morpholinyl (e.g. morpholino), thiomorpholinyl (e.g. thiomorpholino), pyridyl, thiazolyl, oxazolyl, oxadiazolyl, isoxazolyl, imidazolinyl, pyridazinyl, pyrimidinyl, pyrazinyl, quinolyl, isoquinolyl, imidazolyl, triazolyl and tetrazolyl groups, of which aromatic heterocyclic groups having 5 or 6 ring atoms and optionally having a phenyl group fused thereto are preferred, and the pyridyl, imidazolyl, thiazolyl, quinolyl and isoquinolyl groups are more preferred. Such groups may be quaternized, in which case the positive charge of the quaternary nitrogen atom is balanced by a negative charge from an anion $Z^-$, as defined above or from a carboxylate group represented by $R^4$.

These heterocyclic groups may be substituted or unsubstituted. Where they are substituted, the substituents are defined above as substituents (a) and (b), and preferred examples include: $C_1$ to $C_4$ alkyl groups, such as the alkyl groups exemplified above in relation to the alkyl groups, such as the $R^5$; $C_1$ to $C_4$

hydroxyalkyl groups, such as the hydroxymethyl, hydroxyethyl and hydroxypropyl groups; $C_1$ to $C_4$ alkoxy groups, such as the methoxy and ethoxy groups; and halogen atoms, such as the fluorine, chlorine and bromine atoms.

Where $R^7$, $R^8$, $R^9$ represents a lower alkyl group, this may be a straight or branched chain alkyl group containing from 1 to 6 carbon atoms, and examples include the $C_1$ - $C_4$ alkyl groups exemplified above in relation to $R^5$ as well as the pentyl, isopentyl, 2-methylbutyl, neopentyl, hexyl, 4-methylpentyl, 3-methylpentyl, 2-methylpentyl, 3,3-dimethylbutyl, 2,2-dimethylbutyl, 1,1-dimethylbutyl, 1,2-dimethylbutyl, 1,3-dimethylbutyl or 2,3-dimethylbutyl groups; of these, we particularly prefer those alkyl groups containing from 1 to 4 carbon atoms such as those exemplified above in relation to $R^5$.

$R^4$ may represent a hydroxy group, a $C_1$ - $C_4$ alkanoyloxy group, a $C_1$ - $C_4$ alkoxy group, a $C_7$ - $C_9$ aralkyloxy group, a carbamoyloxy group, a mono or di alkylcarbamoyloxy group in which the or each alkyl part is $C_1$ - $C_4$, a mercapto group, a $C_1$ - $C_4$ alkylthio group, a $C_7$ - $C_9$ aralkylthio group, a $C_1$ - $C_4$ alkanoylthio group, a carbamoylthio group, a mono or di alkylcarbamoylthio group in which the or each alkyl part is $C_1$ - $C_4$ or a carboxy group;

Where $R^4$ represents an alkoxy group, this may be a straight or branched chain group. The group more preferably has from 1 to 3 carbon atoms and examples of such groups include the methoxy, ethoxy, propoxy, isopropoxy, butoxy, isobutoxy, sec-butoxy and t-butoxy groups, of which the methoxy and ethoxy groups are preferred.

Where $R^4$ represents an aralkyloxy group, this may be the aralkyloxy group corresponding to any one of the aralkyl groups exemplified above in relation to the aralkyl groups which may be represented by $R^5$.

Where $R^4$ represents a alkanoyloxy group, this may be the alkanoyloxy group corresponding to any one of the alkanoyl groups exemplified above in relation to the alkanoyl groups which may be represented by $R^5$.

Where $R^4$ represents a mono or di alkylcarbamoyloxy group, the or each alkyl part may be any one of the alkyl groups exemplified above in relation to the alkyl groups which may be represented by $R^5$. Specific examples of such alkylcarbamoyloxy groups include the methylcarbamoyloxy, ethylcarbamoyloxy, propylcarbamoyloxy, isopropylcartbamoyloxy, butylcarbamoyloxy, isobutylcarbamoyloxy, sec-butylcarbamoyloxy, t-butylcarbamoyloxy, dimethylcarbamoyloxy, diethylcarbamoyloxy, dipropylcarbamoyloxy, dibutylcarbamoyloxy, methylethylcarbamoyloxy and methylpropylcarbamoyloxy groups.

Where $R^4$ represents an alkanoylthio group, this may be the alkanoylthio group corresponding to any one of the alkanoyl groups exemplified above in relation to the alkanoyl groups which may be represented by $R^5$.

Where $R^4$ represents a mono or di alkylcarbamoylthio group, the or each alkyl part may be any one of the alkyl groups exemplified above in relation to the alkyl groups which may be represented by $R^5$. Specific examples of such alkylcarbamoylthio groups include the methylcarbamoylthio, ethylcarbamoylthio, propylcarbamoylthio, isopropylcarbamoylthio, butylcarbamoylthio, isobutylcarbamoylthio, sec-butylcarbamoylthio, t-butylcarbamoylthio, dimethylcarbamoylthio, diethylcarbamoylthio, dipropylcarbamoylthio, dibutylcarbamoylthio, methylethylcarbamoylthio and methylpropylcarbamoylthio groups.

Where $R^4$ represents an alkythio group, this may be a straight or branched chain group. The group more preferably has from 1 to 3 carbon atoms and examples of such groups include the methylthio, ethylthio, propylthio, isopropylthio, butylthio, isobutylthio, sec-butylthio and t-butylthio groups, of which the methylthio and ethylthio groups are preferred.

Where $R^4$ represents an aralkylthio group, this may be the aralkylthio group corresponding to any one of the aralkyl groups exemplified above in relation to the aralkyl groups which may be represented by $R^5$.

Where $R^4$ represents a carboxy group, this may, if desired, be esterified to form the corresponding ester of the carboxylic acid of formula (I). There is no particular limitation on the nature of the ester to be formed, provided that, where the resulting compound is to be used for pharmaceutical purposes, it is pharmaceutically acceptable; where the compound is to be used for other purposes, e.g. as an intermediate, even this restriction does not apply. Examples of esters which may be formed included: esters with any one of the lower alkyl groups defined above in relation to the alkyl groups which may be represented by $R^5$; aralkyl esters, such as the benzyl, p-nitrobenzyl, o-nitrobenzyl, triphenylmethyl, diphenylmethyl, bis(o-nitrophenyl)methyl, 9-anthrylmethyl, 2,4,6-trimethylbenzyl, p-bromobenzyl, p-methoxybenzyl and piperonyl esters; aliphatic acyloxymethyl esters, such as the acetoxymethyl, propionyloxymethyl, butyryloxymethyl, isobutyryloxymethyl and pivaloyloxymethyl esters; 1-(alkoxycarbonyloxy)ethyl esters, in which the alkoxy part is $C_1$ - $C_6$, preferably $C_1$ - $C_4$, such as the 1-methoxycarbonyloxyethyl, 1-ethoxycarbonyloxyethyl, 1-propoxycarbonyloxyethyl, 1-isopropoxycarbonyloxyethyl, 1-butoxycarbonyloxyethyl and 1-isobutoxycarbonyloxyethyl esters; esters capable of being hydrolyzed in vivo, such as the phthalidyl, (2-oxo-5-methyl-1,3-dioxolen-4-yl)methyl and (2-oxo-5-phenyl-1,3-dioxolen-4-yl)methyl esters; alkoxymethyl esters, in which

the alkoxy part is $C_1$ - $C_6$, preferably $C_1$ - $C_4$, such as the methoxymethyl, ethoxymethyl, propoxymethyl, isopropoxymethyl, butoxymethyl and methoxyethoxymethyl esters; and halogenated $C_1$ - $C_6$, preferably $C_1$ - $C_4$, alkyl esters, such as the 2,2,2-trichloroethyl, 2-haloethyl (e.g. 2-chloroethyl, 2-fluoroethyl, 2-bromoethyl or 2-iodoethyl) and 2,2-dibromoethyl esters. Of these, the alkyl esters, the aralkyl esters and esters capable of being hydrolyzed in vivo are preferred and the $C_1$ - $C_4$ alkyl esters are most preferred.

More preferably, the compounds of the invention are represented by the formula (Ia):

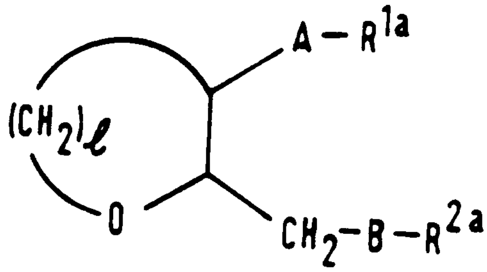

(Ia)

in which: l, A and B are as defined above, and

one of $R^{1a}$ and $R^{2a}$ represents an alkyl group containing from 8 to 22 carbon atoms, an aliphatic carboxylic acyl group containing from 8 to 22 carbon atoms or a group of formula (II), as defined above, and the other of $R^{1a}$ and $R^{2a}$ represent a group of formula (IIIa):

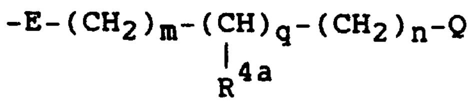

(IIIa)

in which m, n, q and Q are as defined above, and

E represents a single bond, a bivalent heterocyclic group or a group of formula

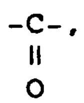

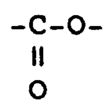

or

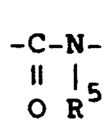

in which $R^5$ is as defined above;

$R^{4a}$ represents a hydroxy group, a $C_1$ - $C_4$ alkanoyloxy group, a $C_1$ - $C_4$ alkoxy group, a $C_7$ - $C_9$ aralkyloxy group, a carbamoyloxy group, an alkylcarbamoyloxy group in which the alkyl part is $C_1$ - $C_4$, a dialkylcarbamoyloxy group in which each alkyl part is $C_1$ - $C_4$, a mercapto group, a $C_1$ - $C_4$ alkythio group, a $C_7$ - $C_9$ aralkylthio group, a $C_1$ - $C_4$, a alkanoylthio group, a carbamoylthio group, an alkylcarbamoylthio group in which the alkyl part is $C_1$ - $C_4$, a dialkylcarbamoylthio group in which each alkyl part is $C_1$ - $C_4$, a carboxy group or an alkoxycarbonyl group in which the alkoxy part is $C_1$ - $C_4$;

said heterocyclic groups having from 5 to 10 ring atoms, of which from 1 to 4 are nitrogen and/or oxygen and/or sulphur hetero-atoms, the heterocyclic groups represented by E being unsubstituted and the

heterocyclic groups represented by Q being unsubstituted or having at least one $C_1$ - $C_4$ alkyl substituent.

Where $R^{4a}$ represents an alkanoyloxy group, an alkoxy group, an aralkyloxy group, an alkylcarbamoyloxy group, a dialkylcarbamoyloxy group, an alkythio group, an aralkylthio group, an alkanoylthio group, an alkylcarbamoylthio group or a dialkylcarbamoylthio group, these may be as exemplified above in relation to the corresponding groups which may be represented by $R^4$. Specific examples of alkoxycarbonyl groups which may be represented by $R^{4a}$ include the methoxycarbonyl, ethoxycarbonyl, propoxycarbonyl, isopropoxycarbonyl, butoxycarbonyl, isobutoxycarbonyl, sec-butoxycarbonyl and t-butoxycarbonyl groups, of which the methoxycarbonyl and ethoxycarbonyl groups are preferred.

Since the compounds of the present invention contain at least two asymmetric carbons at the α- and β-positions relative to the ethereal oxygen atom in the ether ring, and may, depending on the nature of the substituents, contain more asymmetric carbon atoms, there exist at least four stereoisomers due to the R and S configurations of each α- and β- carbon atoms. The present invention covers both the individual isolated stereoisomers and mixtures of any two or more thereof. In tests, it has been found that the 3S isomers surprisingly have even better activity than do the 3R isomers; thus, the (3S, 2R) isomers are better than the (3R, 2S) isomers and the (3S, 2S) isomers are better than the (3R, 2R) isomers.

One class of compounds of the present invention are those compounds of formula (I), defined above, in which:

l is an integer of from 2 to 4;

A and B are the same or different and each represents an oxygen atom or a sulphur atom;

one or $R^1$ or $R^2$ represents an alkyl group containing from 10 to 22 carbon atoms, an aliphatic carboxylic acyl group containing from 10 to 22 carbon atoms or a group of formula (IIa):

$-CONH-R^3$ (IIa)

in which $R^3$ represents an alkyl group containing from 10 to 22 carbon atoms,
and the other represents a group of formula (IIIb):

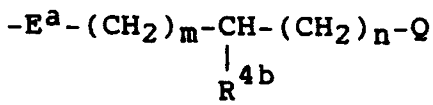 (IIIb)

in which $E^a$ represents a single bond or a group of formula

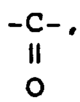 , 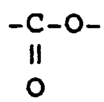 or 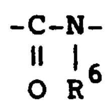

in which $R^6$ represents a hydrogen atom or an imino-protecting group;

m is the cypher O or an integer from 1 to 3;

n is the cypher O or an integer from 1 to 10;

$R^{4b}$ represents a hydrogen atom, a carboxy group or an alkoxycarbonyl group in which the alkoxy part is $C_1$ - $C_4$;

Q represents a group of formula (IV):

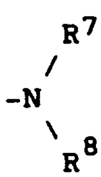 (IV)

in which $R^7$ and $R^8$ are the same or different and each represents a hydrogen atom or a $C_1$ - $C_6$ alkyl group,

or a monovalent heterocyclic group having from 5 to 7 ring atoms, of which from 1 to 4 atoms are nitrogen and/or oxygen and/or sulphur hetero-atoms, at least one of these being a nitrogen atom, and the heterocyclic groups represented by Q being unsubstituted or having at least one substituent selected from $C_1$ - $C_4$ alkyl groups, $C_1$ - $C_4$ hydroxyalkyl groups, $C_1$ - $C_4$ alkoxy groups and halogen atoms or such a heterocyclic group having another ring fused thereto;
and pharmaceutically acceptable salts thereof (including quaternary and inner salts).

Another class of compounds of the present invention are those compounds of formula (I), defined above, in which:
l is an integer of from 2 to 4;
A and B are the same or different and each represents an oxygen atom or a sulphur atom;
one or $R^1$ or $R^2$ represents an alkyl group containing from 10 to 22 carbon atoms, an aliphatic carboxylic acyl group containing from 10 to 22 carbon atoms or a group of formula (IIa):

$-CONH-R^3$ (IIa)

in which $R^3$ represents an alkyl group containing from 10 to 22 carbon atoms,
and the other represents a group of formula (IIIc):

```
-Eb-(CH)q-(CH2)n-Q          (IIIc)
     |
     R4c
```

in which $E^b$ represents a group of formula $-(CH_2)_{m'}-$ or a bivalent heterocyclic group;
m' is an integer from 1 to 3;
n is the cypher O or an integer from 1 to 10;
q is the cypher O or the integer 1;
$R^{4c}$ represents a hydrogen group, a $C_1$ - $C_4$ alkanoyloxy group, a $C_1$ - $C_4$ alkoxy group, a $C_7$ - $C_9$ aralkyloxy group, a carbamoyloxy group, an alkylcarbamoyloxy group in which the alkyl part is $C_1$ - $C_4$, a dialkylcarbamyloxy group in which each alkyl part is $C_1$ - $C_4$, a mercapto group, a $C_1$ - $C_4$ alkanoylthio group, a $C_1$ - $C_4$ alkylthio group, a $C_7$ - $C_9$ aralkylthio group, a carbamoylthio group, an alkylcarbamoylthio group in which the alkyl part is $C_1$ - $C_4$ or a dialkylcarbamoylthio group in which each alkyl part is $C_1$ - $C_4$;
Q represents a group of formula (IV):

```
     R7
    /
-N                (IV)
    \
     R8
```

in which $R^7$ and $R^8$ are the same or different and each represents a hydrogen atom or a $C_1$ - $C_6$ alkyl group,
or a monovalent heterocyclic group having from 5 to 7 ring atoms, of which from 1 to 4 atoms are nitrogen and/or oxygen and/or sulphur hetero-atoms, at least one of these being a nitrogen atom, and the heterocyclic groups represented by Q being unsubstituted or having at least one substituent selected from $C_1$ - $C_4$ alkyl groups, $C_1$ - $C_4$ hydroxyalkyl groups, $C_1$ - $C_4$ alkoxy groups, carbamoyl groups and halogen atoms or such a heterocyclic group having another ring fused thereto;
and pharmaceutically acceptable salts thereof (including quaternary and inner salts).

Preferred compounds of the invention are:

(1) Those compounds of formula (I), defined above, in which:
l is the integer 2 or 3, preferably 3.

(2) Those compounds of formula (I), defined above, in which:
A represents an oxygen or sulphur atom and B represents an oxygen atom.

(3) Those compounds of formula (I), defined above, in which:
$R^1$ represents $C_8$ - $C_{22}$ alkyl group or a group of formula (II):

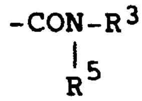 (II)

(in which $R^3$ and $R^5$ are as defined above), preferably said group of formula (II).

(4) Those compounds defined in (3) above in which $R^5$ represents a hydrogen atom or a $C_2$ - $C_4$ alkanoyl group.

(5) Those compounds defined in (3) above in which $R^3$ represents a $C_{13}$ - $C_{20}$ alkyl group.

(6) Those compounds of formula (I), defined above, in which:
n is an integer from 1 to 7.

(7) Those compounds of formula (I), defined above, in which:
Q represents a thiazolyl, pyridyl, quino or imidazolyl group or a thiazolyl, pyridy isoquinolyl or imidazolyl group containing $C_1$ - $C_4$ alkyl substituent.

(8) Those compounds of formula (I), defined above which:
Q represents a thiazolyl or pyridyl group.

(9) Those compounds of formula (I), defined above, in which:
E represents an isoxazolediyl or thiazolediyl group.

(10) Those compounds defined in (9) above, in which:
E represents a 3,5-isoxazolediyl group.

(11) Those compounds for formula (I), defined, in which:
$R^2$ represents a group of formula (III):

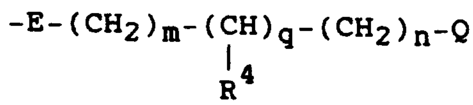 (III)

in which E, Q, $R^4$, m, n and q are as defined above.

(12) Those compounds of formula (I), defined above, in which:
in the group represented by $R^2$, the group of formula

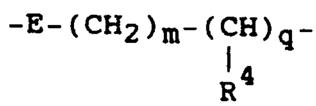

is a group of formula:

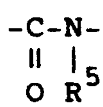 or 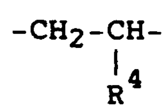

[in which $R^4$ is as defined above and $R^5$ is as defined above, but is more preferably a hydrogen atom or an acetyl group]
or an isoxazolediyl group.

(13) Those compounds of formula (I), defined above, in which:
$R^1$ represents a group of formula (II):

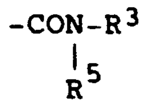

(II)

in which $R^3$ and $R^5$ are as defined above, and
$R^2$ represents a group of formula (III):

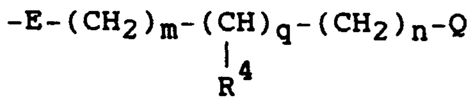

(III)

in which E, m, n, q $R^4$ and Q are as defined above.

(14) Those compounds defined in (13) above in which $R^5$ represents a hydrogen atom or a $C_2$ - $C_4$ alkanoyl group.

(15) Those compounds defined in (13) above, in which:
the group of formula

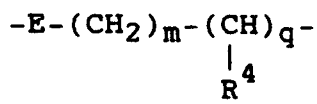

in the group represent by $R^2$ is a group of formula:

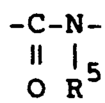

or

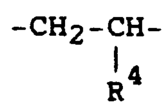

[in which $R^5$ as as defined in (10) above, but is more preferably a hydrogen atom or an acetyl group] or an isoxazolediyl group.

Examples of specific compounds of the invention are given in the following formulae (I-1) to (I-10), in which the substituents are as defined in the corresponding one of Tables 1 to 10 [i.e. Table 1 relates to formula (I-1), Table 2 relates to formula (I-2) and so on]. In the Tables, the following abbreviations are used:

| | |
|---|---|
| Ac | acetyl |
| All | allyl |
| Boz | benzoyl |
| Bz | benzyl |
| Bzc | benzyloxycarbonyl |
| Car | carbamoyl |
| Dc | decyl |
| Dco | decanoyl |
| Ddc | dodecyl |
| Doc | docosyl |
| Doco | docosanoyl |
| Ei | icosyl |
| Eio | icosanoyl |
| Et | ethyl |
| Etc | ethoxycarbonyl |
| Hdc | hexadecyl |
| Hen | henicosyl |
| Heno | henicosanoyl |
| Hpdc | heptadecyl |

| | |
|---|---|
| Hpdo | heptadecanoyl |
| Imd | imidazolediyl, e.g. 2,4-Imd is: 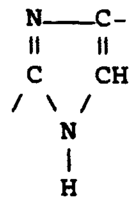 |
| $Imid^+$ | imidazolyl 3-cation |
| $Imin^+$ | imidazolinyl 1-cation |
| Isoxd | isoxazolediyl, e.g. 3,5-Isoxd is: 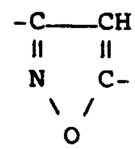 |
| Lau | lauroyl |
| Me | methyl |
| Mec | methoxycarbonyl |
| Mor | morpholino |
| $Mor^+$ | morpholino 4-cation |
| Myr | myristyl |
| Ndc | nonadecyl |
| Ndco | nonadecanoyl |
| Odc | octadecyl |
| $Oxa^+$ | oxazolyl 1-cation |
| Oxad | oxazolediyl, e.g. 2,4-Oxad is: 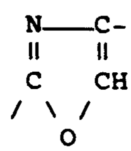 |
| Pal | palmitoyl |
| Pdc | pentadecyl |
| Pdco | pentadecanoyl |
| Pip | piperidyl |
| $Pip^+$ | piperidyl 1-cation |
| $Pym^+$ | pyrimidinyl 1-cation |
| Pyr | pyridyl |

| | |
|---|---|
| $Pyr^+$ | pyridyl 1-cation |
| Pyrd | pyrrolidinyl |
| $Pyrd^+$ | pyrrolidine 1-cation |
| $Pyz^+$ | pyrazinyl 1-cation |
| $Pyzn^+$ | pyridazinyl 1-cation |
| $Quin^+$ | quinolyl 1-cation |
| <u>i</u>$Quin^+$ | isoquinolyl 2-cation |
| Ste | stearoyl |
| Tco | tricosanoyl |
| Tdc | tridecyl |
| Tdco | tridecanoyl |
| Tedc | tetradecyl |
| $Tez_2$ | 2<u>H</u>-tetrazolyl |
| Thd | thiazolediyl, e.g. 2,4-Thd is: 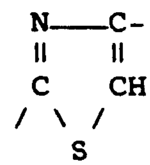 |
| Thi | thienyl |
| $Thi^+$ | thiazolyl 3-cation |
| Thp | tetrahydropyranyl |
| Thz | perhydro-1,4-thiazin-4-yl (= thiomorpholino) |
| Tms | trimethylsilyl |
| Udc | undecyl |
| Udco | undecanoyl |

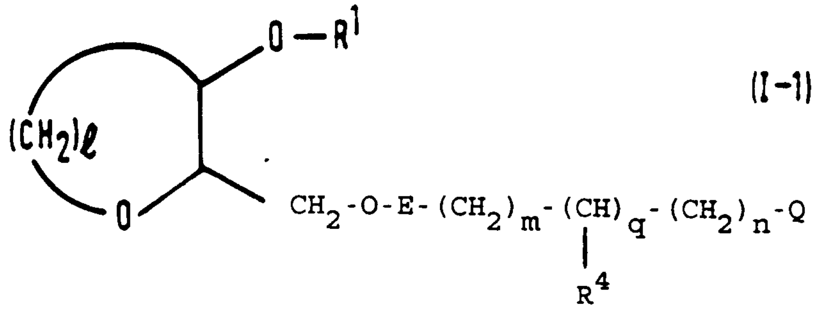

(I-1)

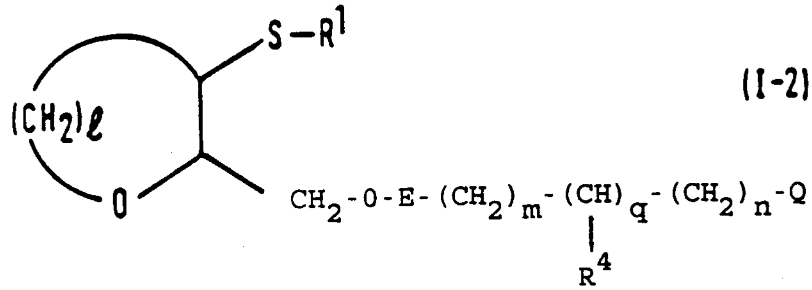

(I-2)

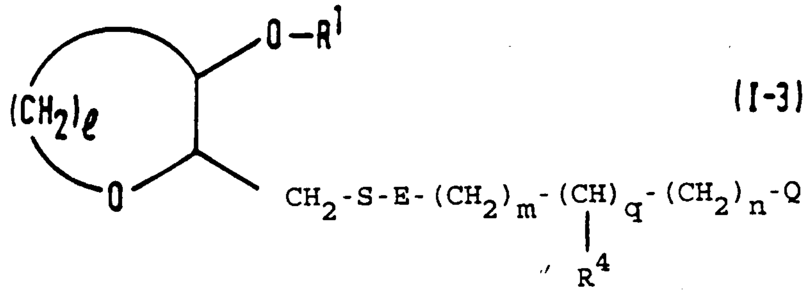

(I-3)

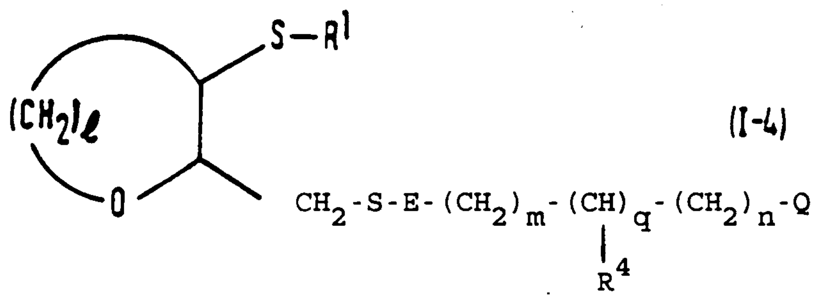

(I-4)

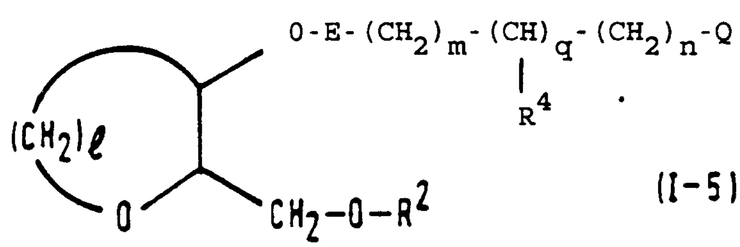

(I-5)

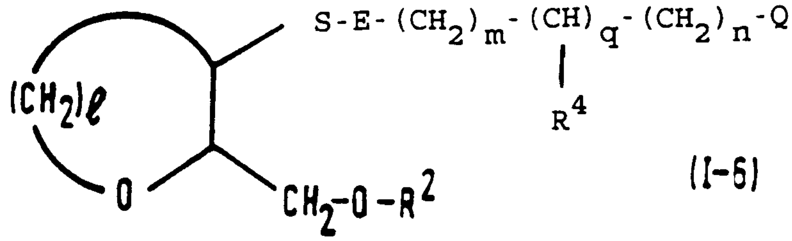

(I-6)

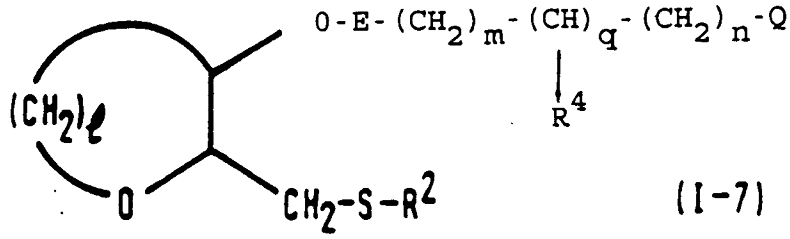

(I-7)

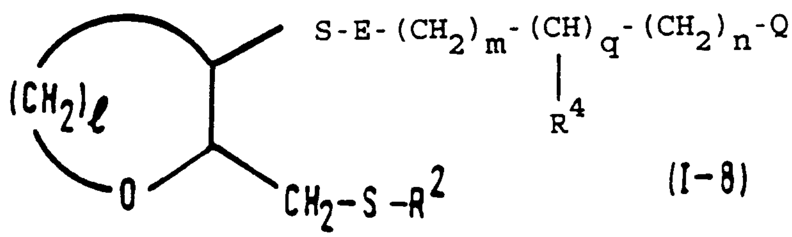

(I-8)

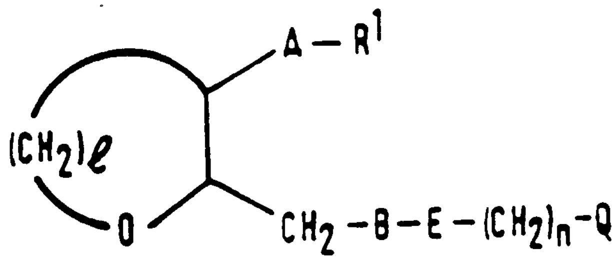

(I-9)

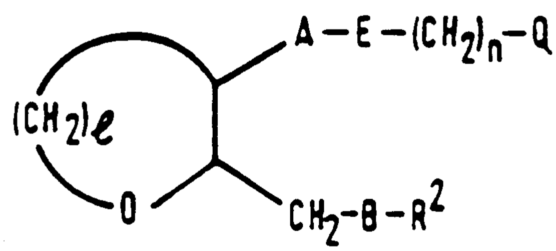

(I-10)

TABLE 1

| Cpd. No. | $R^1$ | E | l | m | n | $R^4$ | q | Q |
|---|---|---|---|---|---|---|---|---|
| 1-1 | Dc | -CO- | 2 | 0 | 1 | - | 0 | $3\text{-Thi}^+$ |
| 1-2 | Udc | -CONH- | 3 | 1 | 1 | Etc | 1 | $1\text{-Me-3-Imid}^+$ |
| 1-3 | Ddc | -CONAc- | 4 | 2 | 2 | COOH | 1 | $-N^+Me_3$ |
| 1-4 | Tdc | - | 2 | 3 | 3 | Mec | 1 | $-NMe_2$ |
| 1-5 | Tedc | -CO- | 3 | 1 | 4 | - | 0 | 1-Pip |
| 1-6 | Pdc | -CONH- | 4 | 1 | 5 | Etc | 1 | $1\text{-Me-1-Pip}^+$ |
| 1-7 | Hdc | -CONAc- | 2 | 2 | 6 | COOH | 1 | Mor |
| 1-8 | Hpdc | -COO- | 3 | 3 | 7 | Mec | 1 | $4\text{-Me-4-Mor}^+$ |
| 1-9 | Odc | -CO- | 4 | 1 | 8 | - | 0 | $1\text{-Me-2-Pyr}^+$ |
| 1-10 | Ndc | -CONH- | 2 | 2 | 9 | - | 0 | $1\text{-Et-2-Pyr}^+$ |
| 1-11 | Ei | -CONAc- | 3 | 2 | 10 | Etc | 1 | 3-Pyr |
| 1-12 | Hen | -COO- | 4 | 3 | 0 | COOH | 1 | $1\text{-Me-3-Pyr}^+$ |
| 1-13 | Doc | -CO- | 2 | 0 | 1 | Mec | 1 | 2-Pyr |
| 1-14 | Dco | -CONH- | 3 | 2 | 2 | - | 0 | $3\text{-Me-4-Thi}^+$ |
| 1-15 | Udco | -CONAc- | 4 | 2 | 3 | Etc | 1 | $3\text{-Et-4-Me-5-Thi}^+$ |
| 1-16 | Lau | - | 2 | 3 | 4 | COOH | 1 | 1-Et-2-Pyrd |
| 1-17 | Tdco | -CO- | 3 | 0 | 5 | Mec | 1 | $1,1\text{-diEt-2-Pyrd}^+$ |
| 1-18 | Myr | -CONH- | 4 | 2 | 6 | - | 0 | $1\text{-Me-1-Imid}^+$ |
| 1-19 | Pdco | -CONAc- | 2 | 2 | 7 | Etc | 1 | $1\text{-Me-1-Pyrd}^+$ |
| 1-20 | Pal | -COO- | 3 | 3 | 8 | COOH | 1 | $1\text{-Me-1-Pip}^+$ |
| 1-21 | Hpdo | -CO- | 4 | 0 | 9 | Mec | 1 | $3,4\text{-diMe-5-Thi}^+$ |
| 1-22 | Ste | -CONH- | 2 | 2 | 10 | - | 0 | $1\text{-Imin}^+$ |
| 1-23 | Ndco | -CONAc- | 3 | 2 | 0 | Etc | 1 | Thz |
| 1-24 | Eio | -COO- | 4 | 3 | 1 | COOH | 1 | $3\text{-Thi}^+$ |
| 1-25 | Heno | -CO- | 2 | 0 | 2 | Mec | 1 | $1\text{-Me-3-Imid}^+$ |

TABLE 1 (cont.)

| Cpd. No. | $R^1$ | E | l | m | n | $R^4$ | q | Q |
|---|---|---|---|---|---|---|---|---|
| 1-26 | Doco | -CONH- | 3 | 2 | 3 | - | 0 | $-N^+Me_3$ |
| 1-27 | -CONH-<br>-Dc | -CONAc- | 4 | 2 | 4 | Etc | 1 | $5\text{-}Tez_2$ |
| 1-28 | -CONH-<br>-Udc | - | 2 | 3 | 5 | COOH | 1 | $1\text{-}Pyz^+$ |
| 1-29 | -CONH-<br>-Ddc | -CO- | 3 | 0 | 6 | Mec | 1 | $1\text{-}Pym^+$ |
| 1-30 | -CONH-<br>-Tdc | -CONH- | 4 | 2 | 7 | - | 0 | $1\text{-}Quin^+$ |
| 1-31 | -CONH-<br>-Tedc | -CONAc- | 2 | 2 | 8 | Etc | 1 | $2\text{-}iQuin^+$ |
| 1-32 | -CONH-<br>-Pdc | -COO- | 3 | 3 | 9 | COOH | 1 | $1\text{-}Me\text{-}2\text{-}Pyr^+$ |
| 1-33 | -CONH-<br>-Hdc | -CO- | 4 | 0 | 10 | Mec | 1 | $1\text{-}Et\text{-}2\text{-}Pyr^+$ |
| 1-34 | -CONH-<br>-Hpdc | -CONH- | 3 | 0 | 5 | Etc | 1 | $3\text{-}Thi^+$ |
| 1-35 | -CONH-<br>-Odc | -CONAc- | 3 | 2 | 1 | Etc | 1 | $1\text{-}Me\text{-}3\text{-}Pyr^+$ |
| 1-36 | -CONH-<br>-Ndc | -COO- | 4 | 3 | 2 | COOH | 1 | $1\text{-}Pyzn^+$ |
| 1-37 | -CONH-<br>-Ei | -CO- | 2 | 0 | 3 | Mec | 1 | $3\text{-}Me\text{-}5\text{-}Thi^+$ |
| 1-38 | -CONH-<br>-Hen | -CONH- | 3 | 2 | 4 | - | 0 | 4-Me-5-Thi |
| 1-39 | -CONH-<br>-Doc | -CONAc- | 4 | 2 | 5 | Etc | 1 | $3\text{-}Et\text{-}4\text{-}Oxa^+$ |
| 1-40 | Dc | -CO- | 2 | 3 | 6 | COOH | 1 | $1,1\text{-}diEt\text{-}2\text{-}Pyrd^+$ |
| 1-41 | Ddc | -CONH- | 3 | 0 | 7 | Mec | 1 | $1\text{-}Me\text{-}1\text{-}Imid^+$ |
| 1-42 | Tedc | -CONAc- | 4 | 2 | 8 | - | 0 | $1\text{-}Me\text{-}1\text{-}Pyrd^+$ |

TABLE 1 (cont.)

| Cpd. No. | $R^1$ | E | l | m | n | $R^4$ | q | Q |
|---|---|---|---|---|---|---|---|---|
| 1-43 | Hdc | -CO- | 2 | 2 | 9 | Etc | 1 | $1-Me-1-Pip^+$ |
| 1-44 | Hdc | -CONH- | 3 | 1 | 4 | - | 0 | $3-Thi^+$ |
| 1-45 | Hdc | -CONAc- | 3 | 2 | 3 | - | 0 | $3-Me-2-Thi^+$ |
| 1-46 | Hdc | - | 3 | 3 | 4 | - | 0 | $3-Thi^+$ |
| 1-47 | Odc | -CONH- | 4 | 0 | 10 | COOH | 1 | $3,4-diMe-5-Thi^+$ |
| 1-48 | Ei | -CONAc- | 2 | 1 | 0 | Mec | 1 | $1-Imin^+$ |
| 1-49 | Doc | -CO- | 3 | 3 | 1 | - | 0 | $1-Me-3-Imid^+$ |
| 1-50 | -CONH- -Udc | -CONH- | 4 | 3 | 2 | Etc | 1 | $-N^+Me_3$ |
| 1-51 | -CONH- -Tdc | -CONAc- | 2 | 0 | 3 | COOH | 1 | $4-Me-4-Mor^+$ |
| 1-52 | -CONH- -Hdc | -CONH- | 3 | 1 | 4 | - | 0 | $3-Thi^+$ |
| 1-53 | -CONH- -Hpdc | -CONH- | 3 | 1 | 4 | - | 0 | $3-Thi^+$ |
| 1-54 | -CONH- -Odc | -CONH- | 3 | 1 | 4 | - | 0 | $3-Thi^+$ |
| 1-55 | -CONH- -Hpdc | -CONAc- | 3 | 1 | 4 | - | 0 | $3-Thi^+$ |
| 1-56 | -CONH- -Hpdc | -CONH- | 3 | 1 | 0 | - | 0 | $1-Me-2-Pyr^+$ |
| 1-57 | -CONH- -Hpdc | -CONAc- | 3 | 1 | 0 | - | 0 | $1-Et-2-Pyr^+$ |
| 1-58 | Dc | - | 3 | 2 | 4 | -SAc | 1 | $3-Thi^+$ |
| 1-59 | Tedc | 3,5- -Isoxd | 3 | 0 | 4 | -SAc | 1 | 1-Pip |
| 1-60 | Pdc | 2,4-Thd | 4 | 0 | 5 | OH | 1 | $1-Me-1-Pip^+$ |
| 1-61 | Hdc | - | 3 | 1 | 3 | -OAc | 1 | $3-Thi^+$ |
| 1-62 | Hpdc | - | 2 | 1 | 7 | SH | 1 | $4-Me-4-Mor^+$ |
| 1-63 | Odc | - | 3 | 1 | 8 | SH | 1 | $1-Me-2-Pyr^+$ |

TABLE 1 (cont.)

| Cpd. No. | $R^1$ | E | l | m | n | $R^4$ | q | Q |
|---|---|---|---|---|---|---|---|---|
| 1-64 | Ndc | - | 4 | 2 | 9 | MeCarO- | 1 | 1-Et-2-$Pyr^+$ |
| 1-65 | Ei | - | 2 | 3 | 10 | 2-ThpO- | 1 | 3-Pyr |
| 1-66 | Hen | - | 3 | 1 | 0 | TmsO- | 1 | 1-Me-3-$Pyr^+$ |
| 1-67 | Doc | - | 4 | 2 | 1 | MeOMeO- | 1 | 2-Pyr |
| 1-68 | Tdco | - | 2 | 1 | 5 | MecO | 1 | 1,1-diEt-2-$Pyrd^+$ |
| 1-69 | Hpdo | - | 3 | 1 | 9 | -SAc | 1 | Thz |
| 1-70 | Ndco | - | 2 | 1 | 0 | -SMe | 1 | 1-$Pyz^+$ |
| 1-71 | Eio | - | 3 | 2 | 1 | -SBz | 1 | 1-$Pym^+$ |
| 1-72 | Doco | - | 2 | 2 | 3 | SH | 1 | 2-iQuin$^+$ |
| 1-73 | -CONH- -Dc | - | 4 | 2 | 5 | OH | 1 | 1-$Pyzn^+$ |
| 1-74 | -CONH- -Udc | - | 2 | 1 | 6 | -OAc | 1 | 3-Et-4-$Oxa^+$ |
| 1-75 | -CONH- -Ddc | - | 3 | 2 | 7 | OH | 1 | 3-Me-2-$Thi^+$ |
| 1-76 | -CONH- -Tedc | - | 2 | 2 | 9 | diMe- CarO | 1 | 4-Mor |
| 1-77 | -CONH- -Hdc | - | 3 | 2 | 4 | -SAc | 1 | 3-$Thi^+$ |
| 1-78 | -CONH- -Hpdc | - | 2 | 2 | 1 | -OAc | 1 | 3-$Thi^+$ |
| 1-79 | -CONH- -Odc | - | 3 | 1 | 2 | -OAc | 1 | 3-$Thi^+$ |
| 1-80 | -CONH- -Doc | - | 3 | 2 | 6 | SH | 1 | 3-$Thi^+$ |
| 1-81 | -CONH- -Hdc | - | 2 | 1 | 1 | -OBz | 1 | 3-$Thi^+$ |
| 1-82 | -CONH- -Hdc | - | 2 | 1 | 2 | OH | 1 | 1-$Quin^+$ |

TABLE 1 (cont.)

| Cpd. No. | $R^1$ | E | l | m | n | $R^4$ | q | Q |
|---|---|---|---|---|---|---|---|---|
| 1-83 | -CONH- -Hdc | - | 4 | 1 | 3 | -OAc | 1 | 3-Thi$^+$ |
| 1-84 | -CONH- -Hdc | - | 3 | 1 | 4 | OH | 1 | 3-Thi$^+$ |
| 1-85 | -CONH- -Hdc | - | 3 | 1 | 5 | OH | 1 | 3-Thi$^+$ |
| 1-86 | -CONH- -Hdc | - | 3 | 1 | 6 | OH | 1 | 3-Thi$^+$ |
| 1-87 | -CONH- -Hdc | - | 3 | 1 | 6 | -OAc | 1 | 3-Thi$^+$ |
| 1-88 | -CONH- -Hpdc | - | 2 | 1 | 2 | -OAc | 1 | 3-Thi$^+$ |
| 1-89 | -CONH- -Hpdc | - | 4 | 1 | 3 | OH | 1 | 3-Thi$^+$ |
| 1-90 | -CONH- -Hpdc | - | 3 | 1 | 5 | -OAc | 1 | 1-Me-2-Quin$^+$ |
| 1-91 | -CONH- -Hpdc | - | 3 | 1 | 6 | OH | 1 | 3-Thi$^+$ |
| 1-92 | -CONH- -Hpdc | - | 3 | 1 | 6 | -OAc | 1 | 3-Thi$^+$ |
| 1-93 | -CONH- -Hpdc | - | 3 | 1 | 5 | -OAc | 1 | 1-Pyr$^+$ |
| 1-94 | -CONH- -Hpdc | - | 3 | 1 | 6 | -SAc | 1 | 1-Quin$^+$ |
| 1-95 | -CONH- -Odc | - | 2 | 1 | 1 | MeO | 1 | $-N^+Me_3$ |
| 1-96 | -CONH- -Odc | - | 4 | 1 | 3 | -OAc | 1 | 2-iQuin$^+$ |
| 1-97 | -CONH- -Odc | - | 4 | 1 | 4 | CarO | 1 | 1-Quin$^+$ |

TABLE 1 (cont.)

| Cpd. No. | $R^1$ | E | l | m | n | $R^4$ | q | Q |
|---|---|---|---|---|---|---|---|---|
| 1-98 | -CONH- -Odc | - | 3 | 1 | 5 | CarO | 1 | 2-Pyr |
| 1-99 | -CONH- -Odc | - | 3 | 1 | 6 | OH | 1 | 3-$Thi^+$ |
| 1-100 | -CONH- -Odc | - | 3 | 1 | 6 | -OAc | 1 | 3-$Thi^+$ |
| 1-101 | -CONH- -Hpdc | - | 3 | 2 | 4 | -OAc | 1 | 3-$Thi^+$ |
| 1-102 | -CONH- -Hpdc | - | 3 | 2 | 5 | OH | 1 | 1-$Quin^+$ |
| 1-103 | -CONH- -Hpdc | - | 3 | 2 | 4 | OH | 1 | 3-$Thi^+$ |
| 1-104 | -CONH- -Hpdc | - | 3 | 2 | 5 | -OAc | 1 | 1-$Quin^+$ |
| 1-105 | -CONH- -Hpdc | - | 3 | 1 | 2 | -OAc | 1 | 1-Me-2-$Pyr^+$ |
| 1-106 | -CONH- -Hpdc | - | 3 | 1 | 2 | -OAc | 1 | 1-Et-2-$Pyr^+$ |
| 1-107 | -CONH- -Hpdc | - | 3 | 1 | 2 | -OAc | 1 | 2-Pyr |
| 1-108 | -CONH- -Hpdc | - | 3 | 1 | 2 | -OAc | 1 | 1-Me-2-$Quin^+$ |
| 1-109 | -CONH- Hpdc | -CONAc- | 3 | 1 | 0 | - | 0 | 1-Me-2-$Pyr^+$ |
| 1-110 | -CONH- Hpdc | -CONAc- | 3 | 1 | 0 | - | 0 | 1-Et-2-$Quin^+$ |
| 1-111 | -CONH- Hpdc | -CONH- | 3 | 1 | 0 | - | 0 | 2-Et-1-$iQuin^+$ |

TABLE 2

| Cpd. No. | $R^1$ | E | l | m | n | $R^4$ | q | Q |
|---|---|---|---|---|---|---|---|---|
| 2-1 | Dc | -CO- | 2 | 3 | 6 | COOH | 1 | 1,1-diEt-2-Pyrd$^+$ |
| 2-2 | Ddc | -CONH- | 3 | 0 | 7 | Mec | 1 | 1-Me-1-Imid$^+$ |
| 2-3 | Tedc | -CONAc- | 4 | 2 | 8 | - | 0 | 1-Me-1-Pyrd$^+$ |
| 2-4 | Hdc | -CO- | 2 | 2 | 9 | Etc | 1 | 1-Me-1-Pip$^+$ |
| 2-5 | Hdc | -CONH- | 3 | 1 | 4 | - | 0 | 3-Thi$^+$ |
| 2-6 | Hdc | -CONAc- | 3 | 2 | 3 | - | 0 | 3-Thi$^+$ |
| 2-7 | Hdc | - | 3 | 3 | 2 | - | 0 | 3-Thi$^+$ |
| 2-8 | Odc | -CONH- | 4 | 0 | 10 | COOH | 1 | 3,4-diMe-5-Thi$^+$ |
| 2-9 | Ei | -CONAc- | 2 | 1 | 0 | Mec | 1 | 1-Imin$^+$ |
| 2-10 | Doc | -CO- | 3 | 3 | 1 | - | 0 | 1-Me-3-Imid$^+$ |
| 2-11 | -CONH-Udc | -CONH- | 4 | 3 | 2 | Etc | 1 | $-N^+Me_3$ |
| 2-12 | -CONH-Tdc | -CONAc- | 2 | 0 | 3 | COOH | 1 | 4-Me-4-Mor$^+$ |
| 2-13 | -CONH-Pdc | -CO- | 3 | 1 | 5 | Mec | 1 | 1-Me-2-Pyr$^+$ |
| 2-14 | -CONH-Hpdc | -CONH- | 3 | 1 | 4 | - | 0 | 3-Thi$^+$ |
| 2-15 | -CONH-Hpdc | -CONAc- | 4 | 2 | 6 | COOH | 1 | 1-Et-2-Pyr$^+$ |
| 2-16 | -CONH-Hpdc | -CO- | 3 | 3 | 7 | Etc | 1 | 1-Me-3-Pyr$^+$ |
| 2-17 | -CONH-Ndc | -CONH- | 2 | 0 | 8 | Mec | 1 | 1,1-diMe-2-Pyrd$^+$ |
| 2-18 | -CONH-Hen | -CONAc- | 3 | 2 | 9 | - | 0 | 1-Me-1-Imid$^+$ |
| 2-19 | Ddc | - | 4 | 2 | 2 | OH | 1 | $-N^+Me_3$ |
| 2-20 | Dco | - | 2 | 1 | 2 | 2-EtOEtO | 1 | 3-Me-4-Thi$^+$ |
| 2-21 | Pdco | - | 4 | 1 | 7 | -OBzc | 1 | 1-Me-1-Pyrd$^+$ |
| 2-22 | Ste | - | 4 | 2 | 10 | -SBoz | 1 | 5-$Tez_2$ |
| 2-23 | Heno | - | 4 | 1 | 2 | -SAc | 1 | 1-Quin$^+$ |
| 2-24 | Tco | - | 3 | 1 | 4 | -OAc | 1 | 3-Thi$^+$ |
| 2-25 | Myr | - | 2 | 1 | 8 | SH | 1 | 5-(2-HOEt)-4-Me-3-Thi$^+$ |
| 2-26 | Pal | - | 3 | 1 | 10 | OH | 1 | 1-Me-2-Quin$^+$ |
| 2-27 | -CONH--Ei | - | 3 | 1 | 4 | MeO | 1 | 3-Thi$^+$ |
| 2-28 | -CONH-Hpdc | - | 3 | 1 | 4 | -OAc | 1 | 3-Thi$^+$ |
| 2-29 | -CONH-Hpdc | -CONH- | 3 | 1 | 0 | - | 0 | 2-Pyr |
| 2-30 | -CONAc-Hpdc | -CONAc- | 3 | 1 | 0 | - | 0 | 2-Pyr |
| 2-31 | -CONAc-Hpdc | -CONAc- | 3 | 1 | 0 | - | 0 | 1-Et-2-Pyr$^+$ |
| 2-32 | -CONAc-Hpdc | -CONH- | 3 | 1 | 0 | - | 0 | 1-Et-2-Pyr$^+$ |

TABLE 3

| Cpd. No. | $R^1$ | E | l | m | n | $R^4$ | q | Q |
|---|---|---|---|---|---|---|---|---|
| 3-1 | Dc | -CO- | 2 | 3 | 6 | COOH | 1 | 1,1-diEt-2-Pyrd$^+$ |
| 3-2 | Ddc | -CONH- | 3 | 0 | 7 | Mec | 1 | 1-Me-1-Imid$^+$ |
| 3-3 | Tedc | -CONAc- | 4 | 2 | 8 | - | 0 | 1-Me-1-Pyrd$^+$ |
| 3-4 | Hdc | -CO- | 2 | 2 | 9 | Etc | 1 | 1-Me-1-Pip$^+$ |
| 3-5 | Hdc | -CONH- | 3 | 1 | 4 | - | 0 | 5-(2-HOEt)-4-Me-3-Thi$^+$ |
| 3-6 | Hdc | -CONAc- | 3 | 2 | 3 | - | 0 | 5-(2-HOEt)-4-Me-3-Thi$^+$ |
| 3-7 | Hdc | - | 3 | 3 | 2 | - | 0 | 5-(2-HOEt)-4-Me-3-Thi$^+$ |
| 3-8 | Odc | -CONH- | 4 | 0 | 10 | COOH | 1 | 3,4-diMe-5-Thi$^+$ |
| 3-9 | Ei | -CONAc- | 2 | 1 | 0 | Mec | 1 | 1-Imin$^+$ |
| 3-10 | Doc | -CO- | 3 | 3 | 1 | - | 0 | 1-Me-3-Imid$^+$ |
| 3-11 | -CONH-Udc | -CONH- | 4 | 3 | 2 | Etc | 1 | $-N^+Me_3$ |
| 3-12 | -CONH-Tdc | -CONAc- | 2 | 0 | 3 | COOH | 1 | 4-Me-4-Mor$^+$ |
| 3-13 | -CONH-Pdc | -CO- | 3 | 1 | 5 | Mec | 1 | 1-Me-2-Pyr$^+$ |
| 3-14 | -CONH-Hpdc | -CONH- | 3 | 1 | 4 | - | 0 | 3-Thi$^+$ |
| 3-15 | -CONH-Hpdc | -CONAc- | 4 | 2 | 6 | COOH | 1 | 1-Et-2-Pyr$^+$ |
| 3-16 | -CONH-Hpdc | -CO- | 3 | 3 | 7 | Etc | 1 | 1-Me-3-Pyr$^+$ |
| 3-17 | -CONH-Ndc | -CONH- | 2 | 0 | 8 | Mec | 1 | 1,1-diMe-2-Pyrd$^+$ |
| 3-18 | -CONH-Hen | -CONAc- | 3 | 2 | 9 | - | 0 | 1-Me-1-Imid$^+$ |
| 3-19 | Udc | - | 3 | 1 | 1 | SH | 1 | 1-Me-3-Imid$^+$ |
| 3-20 | Udco | - | 3 | 1 | 3 | -OBz | 1 | 3-Et-4-Me-5-Thi$^+$ |
| 3-21 | Pal | - | 2 | 2 | 8 | MeO | 1 | 1-Me-1-Imin$^+$ |
| 3-22 | Eio | - | 2 | 2 | 3 | OH | 1 | 1-Et-2-Quin$^+$ |
| 3-23 | -CONH-Hdc | - | 2 | 1 | 4 | SH | 1 | 1-Me-3-Imid$^+$ |

TABLE 4

| Cpd. No. | $R^1$ | E | l | m | n | $R^4$ | q | Q |
|---|---|---|---|---|---|---|---|---|
| 4-1 | Dc | -CO- | 2 | 3 | 6 | COOH | 1 | 1,1-diEt-2-Pyrd$^+$ |
| 4-2 | Ddc | -CONH- | 3 | 0 | 7 | Mec | 1 | 1-Me-1-Imid$^+$ |
| 4-3 | Tedc | -CONAc- | 4 | 2 | 8 | - | 0 | 1-Me-1-Pyrd$^+$ |
| 4-4 | Hdc | -CO- | 2 | 2 | 9 | Etc | 1 | 1-Me-1-Pip$^+$ |
| 4-5 | Hdc | -CONH- | 3 | 1 | 4 | - | 0 | 3-Thi$^+$ |
| 4-6 | Hdc | -CONAc- | 3 | 2 | 3 | - | 0 | 3-Thi$^+$ |
| 4-7 | Hdc | - | 3 | 3 | 2 | - | 0 | 3-Thi$^+$ |
| 4-8 | Odc | -CONH- | 4 | 0 | 10 | COOH | 1 | 3,4-diMe-5-Thi$^+$ |
| 4-9 | Ei | -CONAc- | 2 | 1 | 0 | Mec | 1 | 1-Imin$^+$ |
| 4-10 | Doc | -CO- | 3 | 3 | 1 | - | 0 | 1-Me-3-Imid$^+$ |
| 4-11 | -CONH-Udc | -CONH- | 4 | 3 | 2 | Etc | 1 | $-N^+Me_3$ |
| 4-12 | -CONH-Tdc | -CONAc- | 2 | 0 | 3 | COOH | 1 | 4-Me-4-Mor$^+$ |
| 4-13 | -CONH-Pdc | -CO- | 3 | 1 | 5 | Mec | 1 | 1-Me-2-Pyr$^+$ |
| 4-14 | -CONH-Hpdc | -CONH- | 3 | 1 | 4 | - | 0 | 3-Thi$^+$ |
| 4-15 | -CONH-Hpdc | -CONAc- | 4 | 3 | 6 | - | 0 | 1-Et-2-Pyr$^+$ |
| 4-16 | -CONH-Hpdc | -CO- | 3 | 3 | 7 | Etc | 1 | 1-Me-3-Pyr$^+$ |
| 4-17 | -CONH-Ndc | -CONH- | 2 | 0 | 8 | Mec | 1 | 1,1-diMe-2-Pyrd$^+$ |
| 4-18 | Tdc | - | 2 | 3 | 3 | SH | 1 | $-NMe_2$ |
| 4-19 | Lau | - | 4 | 2 | 4 | -OBoz | 1 | 1-Et-2-Pyrd |
| 4-20 | Myr | - | 3 | 2 | 6 | AllO-COO- | 1 | 1-Me-1-Imid$^+$ |
| 4-21 | -CONH-Hen | - | 2 | 2 | 5 | -OAc | 1 | 1-Pyr$^+$ |

TABLE 5

| Cpd. No. | R2 | E | l | m | n | R4 | q | Q |
|---|---|---|---|---|---|---|---|---|
| 5-1 | Dc | -CO- | 2 | 1 | 0 | - | 0 | 3-Thi+ |
| 5-2 | Udc | -CONH- | 3 | 1 | 1 | Etc | 1 | 1-Me-3-Imid+ |
| 5-3 | Ddc | -CONAc- | 4 | 2 | 2 | COOH | 1 | -N+Me3 |
| 5-4 | Tdc | - | 2 | 3 | 3 | Mec | 1 | -NMe2 |
| 5-5 | Tedc | -CO- | 3 | 1 | 4 | - | 0 | 1-Pip |
| 5-6 | Pdc | -CONH- | 4 | 1 | 5 | Etc | 1 | 1-Me-1-Pip+ |
| 5-7 | Hdc | -CONAc- | 2 | 2 | 6 | COOH | 1 | Mor |
| 5-8 | Hpdc | -COO- | 3 | 3 | 7 | Mec | 1 | 4-Me-4-Mor+ |
| 5-9 | Odc | -CO- | 4 | 1 | 8 | - | 0 | 1-Me-2-Pyr+ |
| 5-10 | Ndc | -CONH- | 2 | 2 | 9 | - | 0 | 1-Et-2-Pyr+ |
| 5-11 | Ei | -CONAc- | 3 | 2 | 10 | Etc | 1 | 3-Pyr |
| 5-12 | Hen | -COO- | 4 | 3 | 0 | COOH | 1 | 1-Me-3-Pyr+ |
| 5-13 | Doc | -CO- | 2 | 0 | 1 | Mec | 1 | 2-Pyr |
| 5-14 | Dco | -CONH- | 3 | 2 | 2 | - | 0 | 4-Thi |
| 5-15 | Udco | -CONAc- | 4 | 2 | 3 | Etc | 1 | 4-Me-5-Thi |
| 5-16 | Lau | - | 2 | 3 | 4 | COOH | 1 | 1-Et-2-Pyrd |
| 5-17 | Tdco | -CO- | 3 | 0 | 5 | Mec | 1 | 1,1-diEt-2-Pyrd+ |
| 5-18 | Myr | -CONH- | 4 | 2 | 6 | - | 0 | 1-Me-1-Imid+ |
| 5-19 | Pdco | -CONAc- | 2 | 2 | 7 | Etc | 1 | 1-Me-1-Pyrd+ |
| 5-20 | Pal | -COO- | 3 | 3 | 8 | COOH | 1 | 1-Me-1-Pip+ |
| 5-21 | Hpdo | -CO- | 4 | 0 | 9 | Mec | 1 | 3,4-diMe-5-Thi$^+$ |
| 5-22 | Ste | -CONH- | 2 | 2 | 10 | - | 0 | 1-Imin$^+$ |
| 5-23 | Ndco | -CONAc- | 3 | 2 | 0 | Etc | 1 | Thz |
| 5-24 | Eio | -COO- | 4 | 3 | 1 | COOH | 1 | 5-(2-HOEt)- -4-Me-3-Thi$^+$ |
| 5-25 | Heno | -CO- | 2 | 0 | 2 | Mec | 1 | 1-Me-3-Imid$^+$ |
| 5-26 | Doco | -CONH- | 3 | 2 | 3 | - | 0 | -N$^+$Me$_3$ |
| 5-27 | -CONH-Dc | -CONAc- | 4 | 2 | 4 | Etc | 1 | -NMe$_2$ |
| 5-28 | -CONH-Udc | - | 2 | 3 | 5 | COOH | 1 | 1-Pip |
| 5-29 | -CONH-Ddc | -CO- | 3 | 0 | 6 | Mec | 1 | 1-Me-1-Pip$^+$ |

TABLE 5 (cont.)

| Cpd. No. | $R^2$ | E | l | m | n | $R^4$ | q | Q |
|---|---|---|---|---|---|---|---|---|
| 5-30 | -CONH-Tdc | -CONH- | 4 | 2 | 7 | - | 0 | Mor |
| 5-31 | -CONH-Tedc | -CONAc- | 2 | 2 | 8 | Etc | 1 | 4-Me-4-$Mor^+$ |
| 5-32 | -CONH-Pdc | -COO- | 3 | 3 | 9 | COOH | 1 | 1-Me-2-$Pyr^+$ |
| 5-33 | -CONH-Hdc | -CO- | 4 | 0 | 10 | Mec | 1 | 1-Et-2-$Pyr^+$ |
| 5-34 | -CONH-Hpdc | -CONH- | 2 | 2 | 0 | - | 0 | 3-Pyr |
| 5-35 | -CONH-Odc | -CONAc- | 3 | 2 | 1 | Etc | 1 | 1-Me-3-$Pyr^+$ |
| 5-36 | -CONH-Ndc | -COO- | 4 | 3 | 2 | COOH | 1 | 2-Pyr |
| 5-37 | -CONH-Ei | -CO- | 2 | 0 | 3 | Mec | 1 | 4-Thi |
| 5-38 | -CONH-Hen | -CONH- | 3 | 2 | 4 | - | 0 | 4-Me-5-Thi |
| 5-39 | -CONH-Doc | -CONAc- | 4 | 2 | 5 | Etc | 1 | 1-Et-2-Pyrd |
| 5-40 | Dc | -CO- | 2 | 3 | 6 | COOH | 1 | 1,1-diEt-2-$Pyrd^+$ |
| 5-41 | Ddc | -CONH- | 3 | 0 | 7 | Mec | 1 | 1-Me-1-$Imid^+$ |
| 5-42 | Tedc | -CONAc- | 4 | 2 | 8 | - | 0 | 1-Me-1-$Pyrd^+$ |
| 5-43 | Hdc | -CO- | 2 | 2 | 9 | Etc | 1 | 1-Me-1-$Pip^+$ |
| 5-44 | Hdc | -CONH- | 3 | 1 | 4 | - | 0 | 3-$Thi^+$ |
| 5-45 | Hdc | -CONAc- | 3 | 2 | 3 | - | 0 | 3-$Thi^+$ |
| 5-46 | Hdc | - | 3 | 3 | 2 | - | 0 | 3-$Thi^+$ |
| 5-47 | Odc | -CONH- | 4 | 0 | 10 | COOH | 1 | 3,4-diMe-5-$Thi^+$ |
| 5-48 | Ei | -CONAc- | 2 | 1 | 0 | Mec | 1 | 1-$Imin^+$ |
| 5-49 | Doc | -CO- | 3 | 3 | 1 | - | 0 | 1-Me-3-$Imid^+$ |
| 5-50 | -CONH-Udc | -CONH- | 4 | 3 | 2 | Etc | 1 | $-N^+Me_3$ |
| 5-51 | -CONH-Tdc | -CONAc- | 2 | 0 | 3 | COOH | 1 | 4-Me-4-$Mor^+$ |
| 5-52 | -CONH-Pdc | -CO- | 3 | 1 | 5 | Mec | 1 | 1-Me-2-$Pyr^+$ |
| 5-53 | -CONH-Hpdc | -CONH- | 3 | 1 | 4 | - | 0 | 3-$Thi^+$ |
| 5-54 | -CONH-Hpdc | -CONAc- | 4 | 3 | 6 | - | 0 | 1-Et-2-$Pyr^+$ |
| 5-55 | -CONH-Hpdc | -CO- | 3 | 3 | 7 | Etc | 1 | 1-Me-3-$Pyr^+$ |
| 5-56 | -CONH-Ndc | -CONH- | 2 | 0 | 8 | Mec | 1 | 1,1-diMe-2-$Pyrd^+$ |
| 5-57 | -CONH-Hen | -CONAc- | 3 | 2 | 9 | - | 0 | 1-Me-1-$Imid^+$ |
| 5-58 | Dc | - | 2 | 1 | 2 | OH | 1 | 3-$Thi^+$ |

TABLE 5 (cont.)

| Cpd. No. | $R^2$ | E | l | m | n | $R^4$ | q | Q |
|---|---|---|---|---|---|---|---|---|
| 5-59 | Tedc | 3,5- -Isoxd | 3 | 0 | 4 | -SAc | 1 | 1-Pip |
| 5-60 | Pdc | 2,4-Thd | 4 | 0 | 5 | OH | 1 | 1-Me-1-Pip$^+$ |
| 5-61 | Hdc | - | 3 | 1 | 3 | -OAc | 1 | 3-Thi$^+$ |
| 5-62 | Hpdc | - | 2 | 2 | 7 | OH | 1 | 4-Me-4-Mor$^+$ |
| 5-63 | Odc | - | 3 | 1 | 8 | SH | 1 | 1-Me-2-Pyr$^+$ |
| 5-64 | Ndc | - | 4 | 2 | 9 | -OAc | 1 | 1-Et-2-Pyr$^+$ |
| 5-65 | Ei | - | 2 | 3 | 10 | 2-ThpO | 1 | 3-Pyr |
| 5-66 | Hen | - | 3 | 1 | 0 | -O-Tms | 1 | 1-Me-3-Pyr$^+$ |
| 5-67 | Doc | - | 4 | 2 | 1 | MeOMeO- | 1 | 2-Pyr |
| 5-68 | Tdco | - | 2 | 1 | 5 | MecO- | 1 | 1,1-diEt-2- -Pyrd$^+$ |
| 5-69 | Hpdo | - | 3 | 1 | 9 | -SAc | 1 | 4-Mor |
| 5-70 | Ndco | - | 2 | 1 | 3 | -SMe | 1 | 1-Pyz$^+$ |
| 5-71 | Eio | - | 3 | 2 | 1 | -SBz | 1 | 1-Pym$^+$ |
| 5-72 | Doco | - | 2 | 2 | 3 | SH | 1 | 2-iQuin$^+$ |
| 5-73 | -CONH-Dc | - | 4 | 2 | 5 | SH | 1 | 1-Pyzn$^+$ |
| 5-74 | -CONH-Udc | - | 2 | 1 | 6 | -OAc | 1 | 3-Et-4-Oxa$^+$ |
| 5-75 | -CONH-Ddc | - | 3 | 2 | 7 | OH | 1 | 3-Me-2-Thi$^+$ |
| 5-76 | -CONH- -Tedc | - | 3 | 2 | 9 | -OCar | 1 | 4-Mor |
| 5-77 | -CONH-Hdc | - | 3 | 1 | 4 | -SAc | 1 | 3-Thi$^+$ |
| 5-78 | -CONH- -Hpdc | - | 2 | 2 | 1 | -OAc | 1 | 3-Thi$^+$ |
| 5-79 | -CONH-Odc | - | 3 | 1 | 2 | -OAc | 1 | 3-Thi$^+$ |
| 5-80 | -CONH-Doc | - | 3 | 2 | 6 | SH | 1 | 3-Thi$^+$ |
| 5-81 | -CONH-Hdc | - | 2 | 1 | 1 | -OBz | 1 | 3-Thi$^+$ |
| 5-82 | -CONH-Hdc | - | 2 | 1 | 2 | OH | 1 | 1-Quin$^+$ |
| 5-83 | -CONH-Hdc | - | 4 | 1 | 3 | -OAc | 1 | 3-Thi$^+$ |
| 5-84 | -CONH-Hdc | - | 3 | 1 | 4 | OH | 1 | 3-Thi$^+$ |

TABLE 5 (cont.)

| Cpd. No. | $R^2$ | E | l | m | n | $R^4$ | q | Q |
|---|---|---|---|---|---|---|---|---|
| 5-85 | -CONH-Hdc | - | 3 | 1 | 5 | OH | 1 | $3\text{-Thi}^+$ |
| 5-86 | -CONH-Hdc | - | 3 | 1 | 6 | OH | 1 | $3\text{-Thi}^+$ |
| 5-87 | -CONH-Hdc | - | 3 | 1 | 6 | -OAc | 1 | $3\text{-Thi}^+$ |
| 5-88 | -CONH--Hpdc | - | 2 | 1 | 2 | -OAc | 1 | $3\text{-Thi}^+$ |
| 5-89 | -CONH--Hpdc | - | 4 | 1 | 3 | OH | 1 | $3\text{-Thi}^+$ |
| 5-90 | -CONH--Hpdc | - | 3 | 1 | 5 | -OAc | 1 | $1\text{-Me-2-Quin}^+$ |
| 5-91 | -CONH--Hpdc | - | 3 | 1 | 6 | OH | 1 | $3\text{-Thi}^+$ |
| 5-92 | -CONH--Hpdc | - | 3 | 1 | 5 | -OAc | 1 | $1\text{-Pyr}^+$ |
| 5-93 | -CONH--Hpdc | - | 3 | 1 | 6 | -OAc | 1 | $1\text{-Quin}^+$ |
| 5-94 | -CONH-Odc | - | 2 | 1 | 1 | MeO | 1 | $-N^+Me_3$ |
| 5-95 | -CONH-Odc | - | 4 | 1 | 3 | -OAc | 1 | $2\text{-}\underline{i}\text{Quin}^+$ |
| 5-96 | -CONH-Odc | - | 4 | 1 | 4 | -OAc | 1 | $3\text{-Thi}^+$ |
| 5-97 | -CONH-Odc | - | 3 | 1 | 5 | -OAc | 1 | 2-Pyr |
| 5-98 | -CONH-Odc | - | 3 | 1 | 6 | OH | 1 | $3\text{-Thi}^+$ |
| 5-99 | -CONH-Odc | - | 3 | 1 | 6 | -OAc | 1 | $3\text{-Thi}^+$ |
| 5-100 | -CONH--Hpdc | - | 3 | 2 | 4 | -OAc | 1 | $3\text{-Thi}^+$ |
| 5-101 | -CONH--Hpdc | - | 3 | 2 | 5 | OH | 1 | $1\text{-Quin}^+$ |
| 5-102 | -CONH--Hpdc | - | 3 | 2 | 4 | OH | 1 | $3\text{-Thi}^+$ |
| 5-103 | -CONH--Hpdc | - | 3 | 2 | 5 | -OAc | 1 | $1\text{-Quin}^+$ |

TABLE 5 (cont.)

| Cpd. No. | $R^2$ | E | l | m | n | $R^4$ | q | Q |
|---|---|---|---|---|---|---|---|---|
| 5-104 | -CONH- -Hpdc | - | 3 | 1 | 2 | -OAc | 1 | $1-Me-2-Pyr^+$ |
| 5-105 | -CONH- -Hpdc | - | 3 | 1 | 2 | -OAc | 1 | $1-Et-2-Pyr^+$ |
| 5-106 | -CONH- -Hpdc | - | 3 | 1 | 2 | -OAc | 1 | 2-Pyr |
| 5-107 | -CONH- -Hpdc | - | 3 | 1 | 2 | -OAc | 1 | $1-Me-2-Quin^+$ |

TABLE 6

| Cpd. No. | $R^2$ | E | l | m | n | $R^4$ | q | Q |
|---|---|---|---|---|---|---|---|---|
| 6-1 | Dc | -CO- | 2 | 3 | 6 | COOH | 1 | $1,1-diEt-2-Pyrd^+$ |
| 6-2 | Ddc | -CONH- | 3 | 0 | 7 | Mec | 1 | $1-Me-1-Imid^+$ |
| 6-3 | Tedc | -CONAc- | 4 | 2 | 8 | - | 0 | $1-Me-1-Pyrd^+$ |
| 6-4 | Hdc | -CO- | 2 | 2 | 9 | Etc | 1 | $1-Me-1-Pip^+$ |
| 6-5 | Hdc | -CONH- | 3 | 1 | 4 | - | 0 | $3-Thi^+$ |
| 6-6 | Hdc | -CONAc- | 3 | 2 | 3 | - | 0 | $3-Thi^+$ |
| 6-7 | Hdc | - | 3 | 3 | 2 | - | 0 | $3-Thi^+$ |
| 6-8 | Odc | -CONH- | 4 | 0 | 10 | COOH | 1 | $3,4-diMe-5-Thi^+$ |
| 6-9 | Ei | -CONAc- | 2 | 1 | 0 | Mec | 1 | $1-Imin^+$ |
| 6-10 | Doc | -CO- | 3 | 3 | 1 | - | 0 | $1-Me-3-Imid^+$ |
| 6-11 | -CONH-Udc | -CONH- | 4 | 3 | 2 | Etc | 1 | $-N^+Me_3$ |
| 6-12 | -CONH-Tdc | -CONAc- | 2 | 0 | 3 | COOH | 1 | $4-Me-4-Mor^+$ |
| 6-13 | -CONH-Pdc | -CO- | 3 | 1 | 5 | Mec | 1 | $1-Me-2-Pyr^+$ |
| 6-14 | -CONH-Hpdc | -CONH- | 3 | 1 | 4 | - | 0 | $3-Thi^+$ |
| 6-15 | -CONH-Hpdc | -CONAc- | 4 | 2 | 6 | COOH | 1 | $1-Et-2-Pyr^+$ |
| 6-16 | -CONH-Hpdc | -CO- | 3 | 3 | 7 | Etc | 1 | $1-Me-3-Pyr^+$ |
| 6-17 | -CONH-Ndc | -CONH- | 2 | 0 | 8 | Mec | 1 | $1,1-diMe-2-Pyrd^+$ |
| 6-18 | -CONH-Hen | -CONAc- | 3 | 2 | 9 | - | 0 | $1-Me-1-Imid^+$ |
| 6-19 | Ddc | - | 4 | 2 | 2 | OH | 1 | $-N^+Me_3$ |
| 6-20 | Dco | - | 2 | 1 | 2 | 2-EtO-EtO- | 1 | $3-Me-4-Thi^+$ |
| 6-21 | Pdco | - | 4 | 1 | 7 | -OBzc | 1 | $1-Me-1-Pyrd^+$ |
| 6-22 | Ste | - | 4 | 2 | 10 | -SBz | 1 | $5-Tez_2$ |
| 6-23 | Heno | - | 4 | 1 | 2 | -SAc | 1 | $1-Quin^+$ |
| 6-24 | Tco | - | 3 | 1 | 4 | MeCar--O- | 1 | $3-Thi^+$ |
| 6-25 | -CONH--Tdc | - | 2 | 1 | 8 | SH | 1 | $5-(2-HOEt)--4-Me-3-Thi^+$ |
| 6-26 | -CONH-Pdc | - | 3 | 1 | 10 | OH | 1 | $1-Me-2-Quin^+$ |
| 6-27 | -CONH-Ei | - | 3 | 1 | 4 | MeO | 1 | $3-Thi^+$ |
| 6-28 | -CONH-Hpdc | - | 3 | 1 | 4 | -OAc | 1 | $3-Thi^+$ |
| 6-29 | -CONH-Hpdc | - | 3 | 1 | 6 | -OAc | 1 | $3-Thi^+$ |

TABLE 7

| Cpd. No. | $R^2$ | E | l | m | n | $R^4$ | q | Q |
|---|---|---|---|---|---|---|---|---|
| 7-1 | Dc | -CO- | 2 | 3 | 6 | COOH | 1 | 1,1-diEt-2-Pyrd$^+$ |
| 7-2 | Ddc | -CONH- | 3 | 0 | 7 | Mec | 1 | 1-Me-1-Imid$^+$ |
| 7-3 | Tedc | -CONAc- | 4 | 2 | 8 | - | 0 | 1-Me-1-Pyrd$^+$ |
| 7-4 | Hdc | -CO- | 2 | 2 | 9 | Etc | 1 | 1-Me-1-Pip$^+$ |
| 7-5 | Hdc | -CONH- | 3 | 1 | 4 | - | 0 | 3-Thi$^+$ |
| 7-6 | Hdc | -CONAc- | 3 | 2 | 3 | - | 0 | 3-Thi$^+$ |
| 7-7 | Hdc | - | 3 | 3 | 2 | - | 0 | 3-Thi$^+$ |
| 7-8 | Odc | -CONH- | 4 | 0 | 10 | COOH | 1 | 3,4-diMe-5-Thi$^+$ |
| 7-9 | Ei | -CONAc- | 2 | 1 | 0 | Mec | 1 | 1-Imin$^+$ |
| 7-10 | Doc | -CO- | 3 | 3 | 1 | - | 0 | 1-Me-3-Imid$^+$ |
| 7-11 | -CONH-Udc | -CONH- | 4 | 3 | 2 | Etc | 1 | $-N^+Me_3$ |
| 7-12 | -CONH-Tdc | -CONAc- | 2 | 0 | 3 | COOH | 1 | 4-Me-4-Mor$^+$ |
| 7-13 | -CONH-Pdc | -CO- | 3 | 1 | 5 | Mec | 1 | 1-Me-2-Pyr$^+$ |
| 7-14 | -CONH-Hpdc | -CONH- | 3 | 1 | 4 | - | 0 | 3-Thi$^+$ |
| 7-15 | -CONH-Hpdc | -CONAc- | 4 | 2 | 6 | COOH | 1 | 1-Et-2-Pyr$^+$ |
| 7-16 | -CONH-Hpdc | -CO- | 3 | 3 | 7 | Etc | 1 | 1-Me-3-Pyr$^+$ |
| 7-17 | -CONH-Ndc | -CONH- | 2 | 0 | 8 | Mec | 1 | 1,1-diMe-2-Pyrd$^+$ |
| 7-18 | -CONH-Hen | -CONAc- | 3 | 2 | 9 | - | 0 | 1-Me-1-Imid$^+$ |
| 7-19 | Udc | - | 3 | 1 | 1 | SH | 1 | 1-Me-3-Imid$^+$ |
| 7-20 | Udco | - | 3 | 1 | 3 | -OBz | 1 | 3-Et-4-Me-5-Thi$^+$ |
| 7-21 | Pal | - | 2 | 2 | 8 | MeO | 1 | 1-Me-1-Imid$^+$ |
| 7-22 | -CONH-Ndc | - | 2 | 2 | 3 | OH | 1 | 1-Et-2-Quin$^+$ |
| 7-23 | -CONH-Odc | - | 2 | 1 | 3 | OH | 1 | 1-Me-3-Imid$^+$ |

TABLE 8

| Cpd. No. | $R^2$ | E | l | m | n | $R^4$ | q | Q |
|---|---|---|---|---|---|---|---|---|
| 8-1 | Dc | -CO- | 2 | 3 | 6 | COOH | 1 | 1,1-diEt-2-Pyrd$^+$ |
| 8-2 | Ddc | -CONH- | 3 | 0 | 7 | Mec | 1 | 1-Me-1-Imid$^+$ |
| 8-3 | Tedc | -CONAc- | 4 | 2 | 8 | - | 0 | 1-Me-1-Pyrd$^+$ |
| 8-4 | Hdc | -CO- | 2 | 2 | 9 | Etc | 1 | 1-Me-1-Pip$^+$ |
| 8-5 | Hdc | -CONH- | 3 | 1 | 4 | - | 0 | 3-Thi$^+$ |
| 8-6 | Hdc | -CONAc- | 3 | 2 | 3 | - | 0 | 3-Thi$^+$ |
| 8-7 | Hdc | - | 3 | 3 | 2 | - | 0 | 3-Thi$^+$ |
| 8-8 | Odc | -CONH- | 4 | 0 | 10 | COOH | 1 | 3,4-diMe-5-Thi$^+$ |
| 8-9 | Ei | -CONAc- | 2 | 1 | 0 | Mec | 1 | 1-Imin$^+$ |
| 8-10 | Doc | -CO- | 3 | 3 | 1 | - | 0 | 1-Me-3-Imid$^+$ |
| 8-11 | -CONH-Udc | -CONH- | 4 | 3 | 2 | Etc | 1 | $-N^+ Me_3$ |
| 8-12 | -CONH-Tdc | -CONAc- | 2 | 0 | 3 | COOH | 1 | 4-Me-4-Mor$^+$ |
| 8-13 | -CONH-Pdc | -CO- | 3 | 1 | 5 | Mec | 1 | 1-Me-2-Pyr$^+$ |
| 8-14 | -CONH-Hpdc | -CONH- | 3 | 1 | 4 | - | 0 | 3-Thi$^+$ |
| 8-15 | -CONH-Hpdc | -CONAc- | 4 | 2 | 6 | COOH | 1 | 1-Et-2-Pyr$^+$ |
| 8-16 | -CONH-Hpdc | -CO- | 3 | 3 | 7 | Etc | 1 | 1-Me-3-Pyr$^+$ |
| 8-17 | -CONH-Ndc | -CONH- | 2 | 0 | 8 | Mec | 1 | 1,1-diMe-2-Pyrd$^+$ |
| 8-18 | Tdc | - | 2 | 3 | 3 | SH | 1 | $-NMe_2$ |
| 8-19 | Lau | - | 4 | 2 | 4 | -OBz | 1 | 1-Et-2-Pyrd |
| 8-20 | Myr | - | 3 | 2 | 6 | AllO-COO- | 1 | 1-Me-1-Imid$^+$ |
| 8-21 | -CONH-Hen | - | 2 | 2 | 5 | -OAc | 1 | 1-Pyr$^+$ |

Table 9

| Cpd. No. | $R^1$ | E | A | B | l | n | Q |
|---|---|---|---|---|---|---|---|
| 9-1 | Dc | 3,5--Isoxd | O | O | 2 | 1 | $3-Thi^+$ |
| 9-2 | Udc | 2,4-Thd | O | S | 3 | 1 | $1-Me-3-Imid^+$ |
| 9-3 | Ddc | 2,4-Oxad | S | O | 4 | 2 | $-N^+Me_3$ |
| 9-4 | Tdc | 2,4-Imd | S | S | 2 | 3 | $-NMe_2$ |
| 9-5 | Tedc | 3,5--Isoxd | O | O | 3 | 4 | 1-Pip |
| 9-6 | Pdc | 2,4-Thd | O | O | 4 | 5 | $1-Me-1-Pip^+$ |
| 9-7 | Hdc | 2,4-Oxad | O | O | 3 | 3 | $3-Thi^+$ |
| 9-8 | Hpdc | 2,4-Imd | O | O | 2 | 7 | $4-Me-4-Mor^+$ |
| 9-9 | Odc | 3,5--Isoxd | O | O | 3 | 8 | $1-Me-2-Pyr^+$ |
| 9-10 | Ndc | 2,4-Thd | O | O | 4 | 9 | $1-Et-2-Pyr^+$ |
| 9-11 | Ei | 2,4-Oxad | O | O | 2 | 10 | 3-Pyr |
| 9-12 | Hen | 2,4-Imd | O | O | 3 | 0 | $1-Me-3-Pyr^+$ |
| 9-13 | Doc | 3,5--Isoxd | O | O | 4 | 1 | 2-Pyr |
| 9-14 | Dco | 2,4-Thd | S | O | 2 | 2 | $3-Me-4-Thi^+$ |
| 9-15 | Udco | 2,4-Oxad | O | S | 3 | 3 | $3-Et-4-Me-5-Thi^+$ |
| 9-16 | Lau | 2,4-Imd | S | S | 4 | 4 | 1-Et-2-Pyrd |
| 9-17 | Tdco | 3,5--Isoxd | O | O | 2 | 5 | $1,1-diEt-2-Pyrd^+$ |
| 9-18 | Myr | 2,4-Thd | S | S | 3 | 6 | $1-Me-1-Imid^+$ |
| 9-19 | Pdco | 2,4-Oxad | S | O | 4 | 7 | $1-Me-1-Pyrd^+$ |
| 9-20 | Pal | 2,4-Imd | O | S | 2 | 8 | $1-Me-1-Imin^+$ |
| 9-21 | Hpdo | 3,5--Isoxd | O | O | 3 | 9 | 4-Thz |
| 9-22 | Ste | 2,4-Thd | S | O | 4 | 10 | $5-Tez_2$ |
| 9-23 | Ndco | 2,4-Oxad | O | O | 2 | 1 | $1-Pyz^+$ |
| 9-24 | Eio | 2,4-Imd | O | O | 3 | 1 | $1-Pym^+$ |

Table 9 (cont)

| Cpd. No. | $R^1$ | E | A | B | l | n | Q |
|---|---|---|---|---|---|---|---|
| 9-25 | Heno | 3,5--Isoxd | S | O | 4 | 2 | 1-Quin$^+$ |
| 9-26 | Doco | 2,4-Thd | O | O | 2 | 3 | 2-iQuin$^+$ |
| 9-27 | Tco | 2,4-Oxad | S | O | 3 | 4 | 3-Thi$^+$ |
| 9-28 | -CONH--Dc | 3,5--Isoxd | O | O | 4 | 5 | 1-Pyzn$^+$ |
| 9-29 | -CONH--Udc | 3,5--Isoxd | O | O | 2 | 6 | 3-Et-4-Oxa$^+$ |
| 9-30 | -CONH--Ddc | 3,5--Isoxd | O | O | 3 | 7 | 3-Me-2-Thi$^+$ |
| 9-31 | -CONH--Tdc | 3,5--Isoxd | S | O | 2 | 8 | 5-(2-HOEt)--4-Me-3-Thi$^+$ |
| 9-32 | -CONH--Tedc | 3,5--Isoxd | O | O | 2 | 9 | 4-Mor |
| 9-33 | -CONH--Pdc | 3,5--Isoxd | S | O | 3 | 10 | 1-Me-2-Quin$^+$ |
| 9-34 | -CONH--Hdc | 3,5--Isoxd | O | O | 3 | 0 | 3-Pyr |
| 9-35 | -CONH--Hpdc | 3,5--Isoxd | O | O | 3 | 3 | 3-Pyr |
| 9-36 | -CONH--Odc | 3,5--Isoxd | O | O | 3 | 2 | 3-Pyr |
| 9-37 | -CONH--Ndc | 3,5--Isoxd | O | S | 2 | 3 | 1-Et-2-Quin$^+$ |
| 9-38 | -CONH--Ei | 3,5--Isoxd | S | O | 3 | 4 | 3-Thi$^+$ |
| 9-39 | -CONH--Hen | 3,5--Isoxd | S | S | 2 | 5 | 1-Pyr$^+$ |
| 9-40 | -CONH--Doc | 3,5--Isoxd | O | O | 3 | 6 | 3-Thi$^+$ |

Table 9 (cont)

| Cpd. No. | $R^1$ | E | A | B | l | n | Q |
|---|---|---|---|---|---|---|---|
| 9-41 | -CONH- -Hdc | 3,5- -Isoxd | O | O | 3 | 0 | 3-Pyr |
| 9-42 | -CONH- -Hdc | 3,5- -Isoxd | O | O | 2 | 2 | 1-Quin$^+$ |
| 9-43 | -CONH- -Hdc | 3,5- -Isoxd | O | O | 4 | 3 | 3-Thi$^+$ |
| 9-44 | -CONH- -Hdc | 3,5- -Isoxd | O | O | 3 | 4 | 3-Thi$^+$ |
| 9-45 | -CONH- -Hdc | 3,5- -Isoxd | O | O | 3 | 4 | 1-Quin$^+$ |
| 9-46 | -CONH- -Hdc | 3,5- -Isoxd | O | O | 3 | 1 | 3-Thi$^+$ |
| 9-47 | -CONH- -Hdc | 3,5- -Isoxd | O | O | 3 | 4 | 2-iQuin$^+$ |
| 9-48 | -CONH- -Hpdc | 3,5- -Isoxd | O | O | 2 | 2 | 3-Thi$^+$ |
| 9-49 | -CONH- -Hpdc | 3,5- -Isoxd | O | O | 4 | 3 | 3-Thi$^+$ |
| 9-50 | -CONH- -Hpdc | 3,4- -Isoxd | O | O | 3 | 1 | 1-Et-2-Pyr$^+$ |
| 9-51 | -CONH- -Hpdc | 3,4- -Isoxd | O | O | 3 | 2 | 1-Et-2-Pyr$^+$ |
| 9-52 | -CONH- -Hpdc | 3,4- -Isoxd | O | O | 3 | 1 | 1-Et-2-Quin$^+$ |
| 9-53 | -CONH- -Hpdc | 3,4- -Isoxd | O | O | 3 | 2 | 1-Et-2-Quin$^+$ |
| 9-54 | -CONH- -Hpdc | 3,4- -Isoxd | O | O | 3 | 1 | 2-Et-3-iQuin$^+$ |
| 9-55 | -CONH- -Hpdc | 3,4- -Isoxd | O | O | 3 | 1 | 2-Et-1-iQuin$^+$ |

Table 9 (cont)

| Cpd. No. | $R^1$ | E | A | B | l | n | Q |
|---|---|---|---|---|---|---|---|
| 9-56 | -CONH- -Hpdc | 3,4- -Isoxd | O | O | 3 | 2 | 3-Thi$^+$ |
| 9-57 | -CONH- -Hpdc | 3,4- -Isoxd | O | O | 3 | 3 | 3-Thi$^+$ |
| 9-58 | -CONH- -Hpdc | 3,4- -Isoxd | O | O | 3 | 4 | 3-Thi$^+$ |
| 9-59 | -CONH- -Odc | 3,4- -Isoxd | O | S | 3 | 2 | 3-Thi$^+$ |
| 9-60 | -CONH- -Hpdc | 3,5- -Isoxd | O | O | 3 | 0 | 1-Me-3-Pyr$^+$ |
| 9-61 | -CONH- -Hpdc | 3,5- -Isoxd | O | O | 3 | 1 | 1-Me-2-Quin$^+$ |
| 9-62 | -CONH- -Hpdc | 3,5- -Isoxd | O | O | 3 | 4 | 3-Thi$^+$ |
| 9-63 | -CONH- -Hpdc | 3,5- -Isoxd | O | O | 3 | 1 | 3-Thi$^+$ |
| 9-64 | -CONH- -Hpdc | 3,5- -Isoxd | O | O | 3 | 4 | 1-Pyr$^+$ |
| 9-65 | -CONH- -Hpdc | 3,5- -Isoxd | O | O | 3 | 4 | 1-Quin$^+$ |
| 9-66 | -CONH- -Odc | 3,5- -Isoxd | O | S | 2 | 2 | 1-Me-3-Imid$^+$ |
| 9-67 | -CONH- -Odc | 3,5- -Isoxd | O | O | 2 | 1 | $-N^+Me_3$ |
| 9-68 | -CONH- -Odc | 3,5- -Isoxd | O | O | 3 | 1 | 2-iQuin$^+$ |
| 9-69 | -CONH- -Odc | 3,5- -Isoxd | O | O | 4 | 4 | 1-Quin$^+$ |
| 9-70 | -CONH- -Odc | 3,5- -Isoxd | O | O | 3 | 1 | 1-Me-2-Pyr$^+$ |

Table 9 (cont)

| Cpd. No. | $R^1$ | E | A | B | l | n | Q |
|---|---|---|---|---|---|---|---|
| 9-71 | -CONH- -Odc | 3,5- -Isoxd | O | O | 3 | 1 | 3-Thi$^+$ |
| 9-72 | -CONH- -Odc | 3,5- -Isoxd | O | O | 3 | 4 | 3-Thi$^+$ |
| 9-73 | -CONH- -Hpdc | 2,4-Thd | O | O | 3 | 4 | 3-Thi$^+$ |
| 9-74 | -CONH- -Hpdc | 2,4-Thd | O | O | 3 | 2 | 1-Quin$^+$ |
| 9-75 | -CONH- -Hpdc | 2,4-Thd | O | O | 3 | 1 | 3-Thi$^+$ |
| 9-76 | -CONH- -Hpdc | 2,4-Thd | O | O | 3 | 2 | 3-Thi$^+$ |
| 9-77 | -CONH- -Hpdc | 2,4-Oxad | O | O | 3 | 3 | 1-Me-2-Pyr$^+$ |
| 9-78 | -CONH- -Hpdc | 2,4-Oxad | O | O | 3 | 3 | 1-Quin$^+$ |
| 9-79 | -CONH- -Hpdc | 2,4-Imd | O | O | 3 | 3 | 3-Thi$^+$ |
| 9-80 | -CONH- -Hpdc | 2,4-Imd | O | O | 3 | 3 | 1-Me-2-Quin$^+$ |
| 9-81 | -CONAc- -Hdc | 3,4-Isoxd | S | O | 3 | 0 | 2-Pyr |
| 9-82 | -CONAc- -Hdc | 3,4-Isoxd | S | O | 3 | 2 | 1-Et-2-Pyr$^+$ |
| 9-83 | -CONAc- -Hpdc | 3,4-Isoxd | S | O | 3 | 1 | 2-Pyr |
| 9-84 | -CONAc- -Hpdc | 3,4-Isoxd | S | O | 3 | 2 | 2-Pyr |
| 9-85 | -CONAc- -Hpdc | 3,4-Isoxd | S | O | 3 | 1 | 1-Et-2-Pyr$^+$ |

Table 9 (cont)

| Cpd. No. | $R^1$ | E | A | B | l | n | Q |
|---|---|---|---|---|---|---|---|
| 9-86 | -CONAc--Hpdc | 3,4-Isoxd | S | O | 3 | 2 | 1-Et-2-$Pyr^+$ |
| 9-87 | -CONAc--Odc | 3,4-Isoxd | S | O | 3 | 1 | 1-Et-2-$Pyr^+$ |
| 9-88 | -CONAc--Odc | 3,4-Isoxd | S | O | 3 | 2 | 2-Pyr |
| 9-89 | -CONAc--Hdc | 3,5-Isoxd | S | O | 3 | 1 | 1-Et-2-$Pyr^+$ |
| 9-90 | -CONAc--Hdc | 3,5-Isoxd | S | O | 3 | 2 | 2-Pyr |
| 9-91 | -CONAc--Hpdc | 3,5-Isoxd | S | O | 3 | 1 | 1-Et-2-$Pyr^+$ |
| 9-92 | -CONAc--Hpdc | 3,5-Isoxd | S | O | 3 | 2 | 1-Et-2-$Pyr^+$ |
| 9-93 | -CONAc--Hpdc | 3,5-Isoxd | S | O | 3 | 1 | 2-Pyr |
| 9-94 | -CONAc--Hpdc | 3,5-Isoxd | S | O | 3 | 2 | 2-Pyr |
| 9-95 | -CONAc--Odc | 3,5-Isoxd | S | O | 3 | 1 | 2-Pyr |
| 9-96 | -CONAc--Odc | 3,5-Isoxd | S | O | 3 | 2 | 1-Et-2-$Pyr^+$ |
| 9-97 | -CONAc--Hdc | 3,5-Isoxd | O | O | 3 | 1 | 2-Pyr |
| 9-98 | -CONAc--Hdc | 3,5-Isoxd | O | O | 3 | 2 | 1-Et-2-$Pyr^+$ |
| 9-99 | -CONAc--Hpdc | 3,5-Isoxd | O | O | 3 | 1 | 2-Pyr |
| 9-100 | -CONAc--Hpdc | 3,5-Isoxd | O | O | 3 | 1 | 1-Et-2-$Pyr^+$ |

Table 9 (cont)

| Cpd. No. | $R^1$ | E | A | B | l | n | Q |
|---|---|---|---|---|---|---|---|
| 9-101 | -CONAc--Hpdc | 3,5-Isoxd | O | O | 3 | 2 | 2-Pyr |
| 9-102 | -CONAc--Hpdc | 3,5-Isoxd | O | O | 3 | 2 | 1-Et-2-$Pyr^+$ |
| 9-103 | -CONAc--Odc | 3,5-Isoxd | O | O | 3 | 1 | 1-Et-2-$Pyr^+$ |
| 9-104 | -CONAc--Odc | 3,5-Isoxd | O | O | 3 | 2 | 2-Pyr |

Table 10

| Cpd. No. | $R^1$ | E | A | B | l | n | Q |
|---|---|---|---|---|---|---|---|
| 10-1 | Dc | 3,5--Isoxd | O | O | 2 | 3 | $3\text{-Thi}^+$ |
| 10-2 | Udc | 2,4-Thd | O | S | 3 | 1 | $1\text{-Me-3-Imid}^+$ |
| 10-3 | Ddc | 2,4-Oxad | S | O | 4 | 2 | $-N^+Me_3$ |
| 10-4 | Tdc | 2,4-Imd | S | S | 2 | 3 | $-NMe_2$ |
| 10-5 | Tedc | 3,5--Isoxd | O | O | 3 | 4 | 1-Pip |
| 10-6 | Pdc | 2,4-Thd | O | O | 4 | 5 | $1\text{-Me-1-Pip}^+$ |
| 10-7 | Hdc | 2,4-Oxad | O | O | 3 | 3 | $3\text{-Thi}^+$ |
| 10-8 | Hpdc | 2,4-Imd | O | O | 2 | 7 | $4\text{-Me-4-Mor}^+$ |
| 10-9 | Odc | 3,5--Isoxd | O | O | 3 | 8 | $1\text{-Me-2-Pyr}^+$ |
| 10-10 | Ndc | 2,4-Thd | O | O | 4 | 9 | $1\text{-Et-2-Pyr}^+$ |
| 10-11 | Ei | 2,4-Oxad | O | O | 2 | 10 | 3-Pyr |
| 10-12 | Hen | 2,4-Imd | O | O | 3 | 0 | $1\text{-Me-3-Pyr}^+$ |
| 10-13 | Doc | 3,5--Isoxd | O | O | 4 | 1 | 2-Pyr |
| 10-14 | Dco | 2,4-Thd | S | O | 2 | 2 | $3\text{-Me-4-Thi}^+$ |
| 10-15 | Udco | 2,4-Oxad | O | S | 3 | 3 | $3\text{-Et-4-Me-5-Thi}^+$ |
| 10-16 | Lau | 2,4-Imd | S | S | 4 | 4 | 1-Et-2-Pyrd |
| 10-17 | Tdco | 3,5--Isoxd | O | O | 2 | 5 | $1,1\text{-diEt-2-Pyrd}^+$ |
| 10-18 | Myr | 2,4-Thd | S | S | 3 | 6 | $1\text{-Me-1-Imid}^+$ |
| 10-19 | Pdco | 2,4-Oxad | S | O | 4 | 7 | $1\text{-Me-1-Pyrd}^+$ |
| 10-20 | Pal | 2,4-Imd | O | S | 2 | 8 | $1\text{-Me-1-Imin}^+$ |
| 10-21 | Hpdo | 3,5--Isoxd | O | O | 3 | 9 | 4-Thz |
| 10-22 | Ste | 2,4-Thd | S | O | 4 | 10 | $5\text{-Tez}_2$ |
| 10-23 | Ndco | 2,4-Oxad | O | O | 2 | 3 | $1\text{-Pyz}^+$ |
| 10-24 | Eio | 2,4-Imd | O | O | 3 | 1 | $1\text{-Pym}^+$ |

Table 10 (cont)

| Cpd. No. | $R^1$ | E | A | B | l | n | Q |
|---|---|---|---|---|---|---|---|
| 10-25 | Heno | 3,5--Isoxd | S | O | 4 | 2 | 1-Quin$^+$ |
| 10-26 | Doco | 2,4-Thd | O | O | 2 | 3 | 2-iQuin$^+$ |
| 10-27 | Tco | 2,4-Oxad | S | O | 3 | 4 | 3-Thi$^+$ |
| 10-28 | -CONH--Dc | 3,5--Isoxd | O | O | 4 | 5 | 1-Pyzn$^+$ |
| 10-29 | -CONH--Udc | 3,5--Isoxd | O | O | 2 | 6 | 2-Pyr |
| 10-30 | -CONH--Ddc | 3,5--Isoxd | O | O | 3 | 7 | 3-Me-2-Thi$^+$ |
| 10-31 | -CONH--Tdc | 3,5--Isoxd | S | O | 2 | 8 | 5-(2-HOEt)--4-Me-3-Thi$^+$ |
| 10-32 | -CONH--Tedc | 3,5--Isoxd | O | O | 2 | 9 | 4-Mor |
| 10-33 | -CONH--Pdc | 3,5--Isoxd | S | O | 3 | 10 | 1-Me-2-Quin$^+$ |
| 10-34 | -CONH--Hdc | 3,5--Isoxd | O | O | 3 | 3 | 3-Thi$^+$ |
| 10-35 | -CONH--Hpdc | 3,5--Isoxd | O | O | 3 | 3 | 3-Thi$^+$ |
| 10-36 | -CONH--Odc | 3,5--Isoxd | O | O | 3 | 2 | 3-Thi$^+$ |
| 10-37 | -CONH--Ndc | 3,5--Isoxd | O | S | 2 | 3 | 1-Et-2-Quin$^+$ |
| 10-38 | -CONH--Ei | 3,5--Isoxd | S | O | 3 | 4 | 3-Thi$^+$ |
| 10-39 | -CONH--Hen | 3,5--Isoxd | S | S | 2 | 5 | 1-Pyr$^+$ |
| 10-40 | -CONH--Doc | 3,5--Isoxd | O | O | 3 | 6 | 3-Thi$^+$ |

Table 10 (cont)

| Cpd. No. | $R^1$ | E | A | B | l | n | Q |
|---|---|---|---|---|---|---|---|
| 10-41 | -CONH- -Hdc | 3,5- -Isoxd | O | O | 3 | 0 | 3-Pyr |
| 10-42 | -CONH- -Hdc | 3,5- -Isoxd | O | O | 2 | 2 | 1-Quin$^+$ |
| 10-43 | -CONH- -Hdc | 3,5- -Isoxd | O | O | 4 | 3 | 3-Thi$^+$ |
| 10-44 | -CONH- -Hdc | 3,5- -Isoxd | O | O | 3 | 4 | 3-Thi$^+$ |
| 10-45 | -CONH- -Hdc | 3,5- -Isoxd | O | O | 3 | 4 | 1-Quin$^+$ |
| 10-46 | -CONH- -Hdc | 3,5- -Isoxd | O | O | 3 | 1 | 3-Thi$^+$ |
| 10-47 | -CONH- -Hdc | 3,5- -Isoxd | O | O | 3 | 4 | 2-iQuin$^+$ |
| 10-48 | -CONH- -Hpdc | 3,5- -Isoxd | O | O | 2 | 2 | 3-Thi$^+$ |
| 10-49 | -CONH- -Hpdc | 3,5- -Isoxd | O | O | 4 | 3 | 3-Thi$^+$ |
| 10-50 | -CONH- -Hpdc | 3,4- -Isoxd | O | O | 3 | 0 | 1-Me-3-Pyr$^+$ |
| 10-51 | -CONH- -Hpdc | 3,4- -Isoxd | O | O | 3 | 1 | 1-Me-3-Quin$^+$ |
| 10-52 | -CONH- -Hpdc | 3,4- -Isoxd | O | O | 3 | 4 | 3-Thi$^+$ |
| 10-53 | -CONH- -Hpdc | 3,4- -Isoxd | O | O | 3 | 1 | 3-Thi$^+$ |
| 10-54 | -CONH- -Hpdc | 3,4- -Isoxd | O | O | 3 | 4 | 1-Pyr$^+$ |
| 10-55 | -CONH- -Hpdc | 3,4- -Isoxd | O | O | 3 | 4 | 1-Quin$^+$ |

Table 10 (cont)

| Cpd. No. | $R^1$ | E | A | B | l | n | Q |
|---|---|---|---|---|---|---|---|
| 10-56 | -CONH- -Odc | 3,4- -Isoxd | O | S | 2 | 1 | 1-Me-3-Imid$^+$ |
| 10-57 | -CONH- -Odc | 3,4- -Isoxd | O | O | 2 | 1 | $-N^+Me_3$ |
| 10-58 | -CONH- -Odc | 3,4- -Isoxd | O | O | 3 | 2 | 2-iQuin$^+$ |
| 10-59 | -CONH- -Odc | 3,4- -Isoxd | O | O | 4 | 4 | $-N^+Me_3$ |
| 10-60 | -CONH- -Odc | 3,5- -Isoxd | O | O | 3 | 1 | 1-Me-2-Pyr$^+$ |
| 10-61 | -CONH- -Odc | 3,5- -Isoxd | O | O | 3 | 1 | 3-Thi$^+$ |
| 10-62 | -CONH- -Hpdc | 3,5- -Isoxd | O | O | 3 | 4 | 3-Thi$^+$ |
| 10-63 | -CONH- -Hpdc | 2,4-Thd | O | O | 3 | 4 | 3-Thi$^+$ |
| 10-64 | -CONH- -Hpdc | 2,4-Thd | O | O | 3 | 5 | 1-Quin$^+$ |
| 10-65 | -CONH- -Hpdc | 2,4-Thd | O | O | 3 | 1 | 3-Thi$^+$ |
| 10-66 | -CONH- -Hpdc | 2,4-Thd | O | O | 3 | 2 | 1-Quin$^+$ |
| 10-67 | -CONH- -Hpdc | 2,4-Oxad | O | O | 3 | 0 | 1-Me-2-Pyr$^+$ |
| 10-68 | -CONH- -Hpdc | 2,4-Oxad | O | O | 3 | 3 | 1-Quin$^+$ |
| 10-69 | -CONH- -Hpdc | 2,4-Imd | O | O | 3 | 3 | 3-Thi$^+$ |
| 10-70 | -CONH- -Hpdc | 2,4-Imd | O | O | 3 | 3 | 1-Me-2-Quin$^+$ |

In the compounds listed above, where the compound is shown as containing a quaternary nitrogen atom, then the compound must also contain an anion to balance the positive charge. Such an anion is not critical and may be chosen from any of the anions exemplified above in relation to $Z^-$ or may, where appropriate be a carboxylate ion to form an inner salt.

Of the compounds listed above, the following are preferred: Compounds No. 1-9, 1-10, 1-32, 1-34, 1-35 1-44, 1-45, 1-46, 1-47, 1-52, 1-53, 1-54, 1-55, 1-56, 1-57, 1-61, 1-63, 1-67, 1-76, 1-77, 1-78, 1-79, 1-80, 1-82, 1-83, 1-84, 1-85, 1-86, 1-87, 1-88, 1-89, 1-90, 1-91, 1-92, 1-93, 1-94, 1-95, 1-96, 1-97, 1-98, 1-99, 1-100, 1-101, 1-102, 1-103, 1-104, 1-105, 1-106, 1-107, 1-108, 1-109, 1-110, 1-111, 2-5, 2-6, 2-7, 2-14, 2-15, 2-28, 2-29, 2-30, 2-31, 2-32, 3-5, 3-7, 3-14, 3-15, 3-23, 4-13, 4-14, 4-15, 4-21, 5-53, 5-54, 5-83, 5-87, 5-88, 7-4, 9-7, 9-8, 9-9, 9-10, 9-21, 9-33, 9-34, 9-35, 9-36, 9-41, 9-42, 9-43, 9-44, 9-45, 9-46, 9-47, 9-48, 9-50, 9-51, 9-52, 9-53, 9-54, 9-55, 9-56, 9-57, 9-58, 9-60, 9-61, 9-62, 9-63, 9-65, 9-70, 9-71, 9-72, 9-73, 9-75, 9-76, 9-77, 9-78, 9-79, 9-80, 9-82, 9-85, 9-86, 9-87, 9-89, 9-91, 9-92, 9-96, 9-98, 9-100, 9-102, 9-103, 10-7, 10-35, 10-36, 10-37, 10-43, 10-50 and 10-51. More preferred compounds are Compounds No. 1-34, 1-44, 1-45, 1-46, 1-52, 1-53, 1-54, 1-55, 1-56, 1-57, 1-82, 1-84, 1-87, 1-90, 1-91, 1-96, 1-100, 1-101, 1-104, 1-106, 1-108, 1-109, 2-14, 2-15, 2-31, 2-32, 3-14, 3-15, 9-35, 9-36, 9-44, 9-45, 9-46, 9-47, 9-50, 9-51, 9-52, 9-56, 9-58, 9-62, 9-65, 9-70, 9-72, 9-73, 9-77, 9-82, 9-86, 9-92, 9-96 and 9-98.

The most preferred compounds are Compounds No:

1-34. 3-{6-Ethoxycarbonyl-6-[(3-heptadecylcarbamoyloxytetrahydropyran-2-yl)methoxycarbonylamino]hexyl} thiazolium salts, especially a dl-3-{6-ethoxycarbonyl-6-[(trans-3-heptadecylcarbamoyloxytetrahydropyran-2-yl)methoxycarbonylamino]hexyl}thiazolium salt, particularly the methanesulphonate

1-53. 3-{5-[(3-Heptadecylcarbamoyloxytetrahydropyran-2-yl)methoxycarbonylamino]pentyl}thiazolium salts, especially a dl-3-{5-[(trans-3-heptadecylcarbamoyloxytetrahydropyran-2-yl)methoxycarbonylamino]-pentyl}thiazolium salt, particularly the bromide

1-57. 1-Ethyl-2-{N-acetyl-N-[3-(N-heptadecylcarbamoyloxy)tetrahydropyran-2-ylmethoxycarbonyl]-aminomethyl}pyridinium salts, especially a dl-1-ethyl-2-{N-acetyl-N-[trans-3-(N-heptadecylcarbamoyloxy)-tetrahydropyran-2-ylmethoxycarbonyl]aminomethyl}pyridinium salt, particularly the chloride

1-92. 3-{7-Acetoxy-8-[(3-heptadecylcarbamoyloxytetrahydropyran-2-yl)methoxy]octyl}thiazolium salts

2-31. 1-Ethyl-2-{N-acetyl-N-[3-(N-acetyl-N-heptadecylcarbamoylthio)tetrahydropyran-2-ylmethoxycarbonyl]-aminomethyl}pyridinium salts, especially a dl-1-ethyl-2-{N-acetyl-N-[cis-3-(N-acetyl-N-heptadecylcarbamoylthio)tetrahydropyran-2-ylmethoxycarbonyl]aminomethyl}pyridinium salt, particularly the chloride

2-32. 1-Ethyl-2-{N-[3-(N-acetyl-(N-heptadecylcarbamoylthio)tetrahydropyran-2-ylmethoxycarbonyl]-aminomethyl}pyridinium salts, especially a dl-1-ethyl-2-{N-[cis-3-(N-acetyl-N-heptadecylcarbamoylthio)-tetrahydropyran-2-ylmethoxycarbonyl]aminomethyl}pyridinium salt, particularly the chloride

9-62. 3-{4-[3-(3-Heptadecylcarbamoyloxytetrahydropyran-2-yl)methoxy-5-isoxazolyl]butyl}thiazolium salts, especially a dl-3-{4-[3-trans-3-heptadecylcarbamoyloxytetrahydropyran-2-yl)methoxy-5-isoxazolyl]-butyl}thiazolium salt, particularly the methanesulphonate.

## PREPARATION OF COMPOUNDS OF THE INVENTION

The compounds of the present invention may be prepared by a variety of processes, for example by any of the following Methods A to K.

### Method A

This method is for preparing a compound of general formula (I) in which $R^2$ represents the group of formula (III), i.e. a compound of formula (IX), as shown in the following reaction scheme:

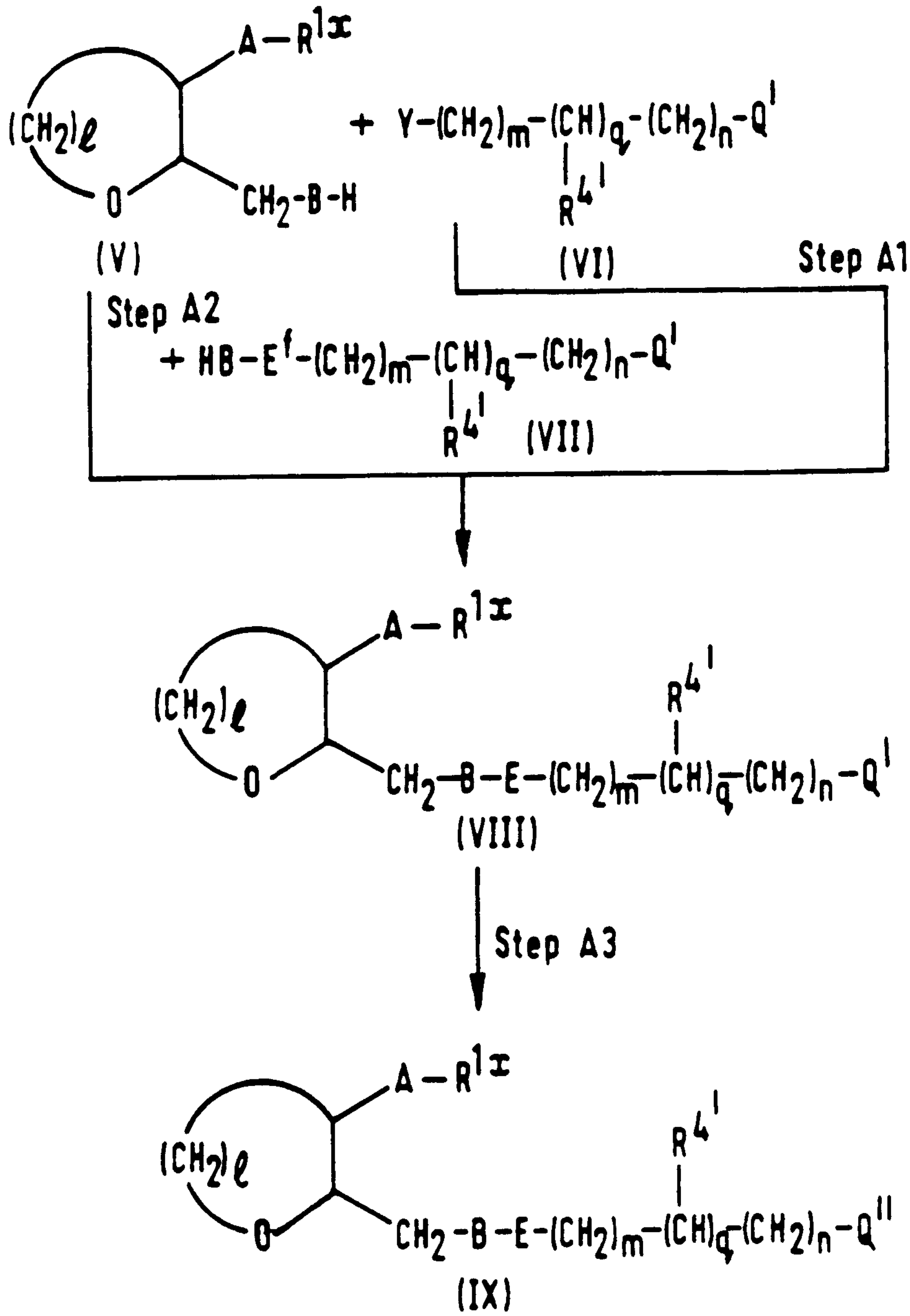
(V)
(VI)
Step A1
Step A2
(VII)
(VIII)
Step A3
(IX)

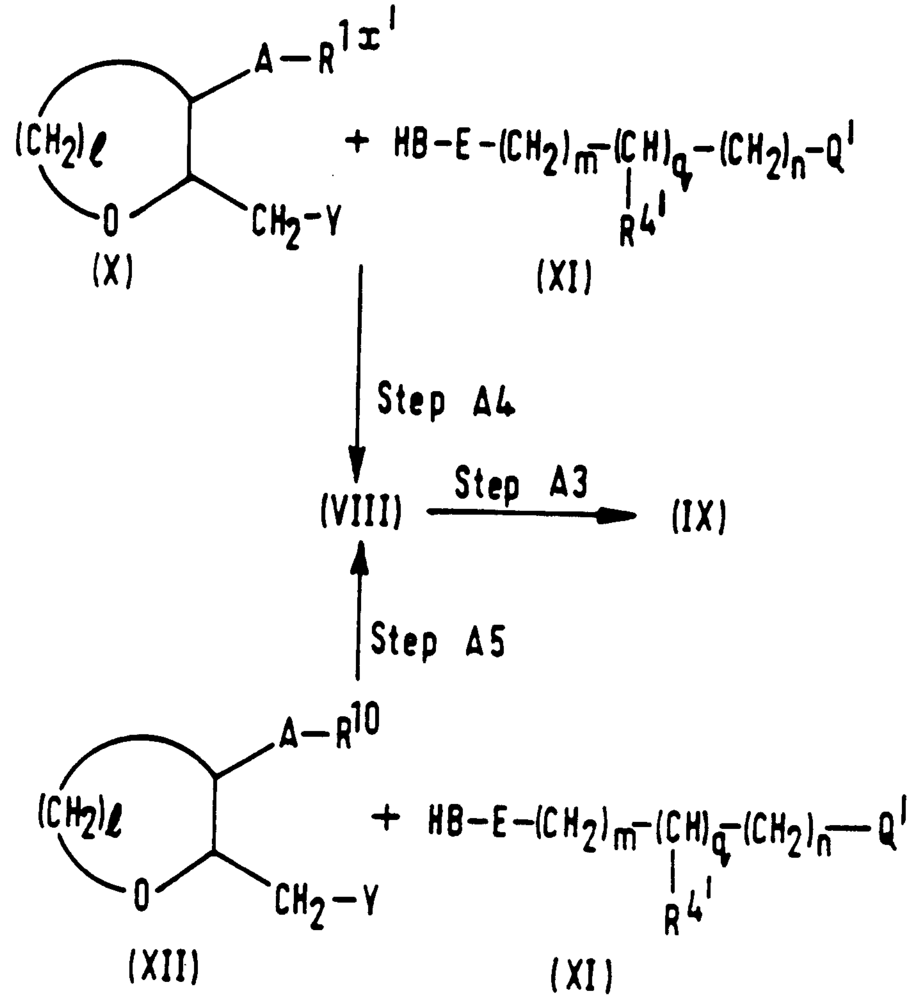


In the above formulae:

A, B, E, l, m, n and q are as defined above.

$R^{1x}$ represents an alkyl group containing from 8 to 22 carbon atoms, an aliphatic carboxylic acyl group containing from 8 to 22 carbon atoms or a group of formula (II):

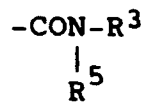

(II)

in which $R^3$ and $R^5$ are as defined above,

i.e. as defined above for $R^1$ or $R^2$.

$R^{1x'}$ represents an alkyl group containing from 8 to 22 carbon atoms.

$R^{4'}$ represents any of the groups defined above for $R^4$, but in which any reactive group is, if necessary, protected. Examples include the $C_1$ - $C_6$ alkanoyloxy groups, such as the formyloxy, acetoxy, propionyloxy, butyryloxy, isobutyryloxy, pentanoyloxy, pivaloyloxy, valeryloxy or isovaleryloxy groups, and halogenated derivatives thereof, such as the chloroacetoxy, dichloroacetoxy, trichloroacetoxy or trifluoroacetoxy groups; lower alkoxyalkanoyloxy groups, such as the methoxyacetoxy group; alkenoyloxy groups, such as the (E)-2-methyl-2-butenoyloxy group; aromatic acyloxy groups, for example, arylcarbonyloxy groups, such as the benzoyloxy, α-naphthoyloxy or β-naphthoyloxy groups; halogenated arylcarbonyloxy groups, such as the o-bromobenzoyloxy or p-chlorobenzoyloxy groups; lower alkylated arylcarbonyloxy groups, such as the 2,4,6-trimethylbenzoyloxy or p-toluoyloxy groups; lower alkoxylated arylcarbonyloxy groups, such as the p-anisoyloxy group; nitrated arylcarbonyloxy groups, such as the p-nitrobenzoyloxy or o-nitrobenzoyloxy

groups; lower alkoxycarbonylated arylcarbonyloxy groups, such as the o-(methoxycarbonyl)benzoyloxy group; arylated arylcarbonyloxy groups, such as the p-phenylbenzoyloxy group; tetrahydropyranyloxy or tetrahydrothiopyranyloxy groups, such as those exemplified below in relation to $R^{11}$; tetrahydrofuranyloxy or tetrahydrothienyloxy groups, such as the tetrahydrofuran-2-yloxy or tetrahydrothien-2-yloxy groups; silyloxy groups, for example, tri(lower alkyl)silyloxy groups, such as the trimethylsilyloxy, triethylsilyloxy, dimethylisopropylsiloxy, t-butyldimethylsilyloxy, diisopropylmethylsilyloxy, di-t-butylmethylsilyloxy or triisopropylsilyloxy groups; tri(lower alkyl)silyloxy groups in which 1 to 2 of the alkyl groups are replaced by aryl groups, such as the diphenylmethylsilyloxy, diphenylbutylsilyloxy, diphenylisopropylsilyloxy or diisopropylphenylsilyloxy groups; alkoxymethoxy groups, for example, lower alkoxymethoxy groups, such as the methoxymethoxy, 1,1-dimethyl-1-methoxymethoxy, ethoxymethoxy, propoxymethoxy, isopropoxymethoxy, butoxymethoxy or t-butoxymethoxy groups; lower alkoxylated (lower alkoxy)methoxy groups, such as the 2-methoxyethoxy-methoxy group; halogenated (lower alkoxy)methoxy groups, such as the 2,2,2-trichloroethoxymethoxy or bis-(2-chloroethoxy)methoxy groups; substituted ethoxy groups, for example, lower alkoxylated ethoxy groups, such as the 1-ethoxyethoxy, 1-methyl-1-methoxyethoxy or 1-(isopropoxy)ethoxy groups; halogenated ethoxy groups, such as the 2,2,2-trichloroethoxy group; arylselenylated lower alkoxy groups substituted with from 1 to 3 aryl groups, such as the phenylselenylmethoxy, 2-phenylselenylethoxy, 3-phenylselenylpropoxy, α-naphthylselenylmethoxy, β-naphthylselenylmethoxy, diphenylselenylmethoxy, triphenylselenylmethoxy, α-naphthyldiphenylselenylmethoxy or 9-anthrylselenylmethoxy groups; lower alkoxy groups substituted with from 1 to 3 aryl groups (which themselves are substituted by substituents such as substituted or unsubstituted lower alkyl, lower alkoxy, nitro, halogen or cyano groups), such as the p-methylbenzyloxy, 2,4,6-trimethylbenzyloxy, 3,4,5-trimethylbenzyloxy, p-methoxybenzyloxy, p-methoxyphenyldiphenylmethoxy, o-nitrobenzyloxy, p-nitrobenzyloxy, p-chlorobenzyloxy, p-bromobenzyloxy, p-cyanobenzyloxy, p-cyanobenzyldiphenylmethoxy, bis(o-nitrophenyl)methoxy or piperonyloxy groups; alkoxycarbonyloxy groups, for example, lower alkoxycarbonyloxy groups, such as the methoxycarbonyloxy, ethoxycarbonyloxy, t-butoxycarbonyloxy or isobutoxycarbonyloxy groups; lower alkoxycarbonyloxy groups (having substituents such as halogen atoms or trialkylsilyl groups), such as the 2,2,2-trichloroethoxycarbonyloxy or 2-trimethylsilylethoxycarbonyloxy groups; alkenyloxycarbonyloxy groups, such as the vinyloxycarbonyloxy or allyloxycarbonyloxy groups; other protected groups of the type commonly used in reactions, for example, aralkyloxycarbonyloxy groups (in which the aryl ring may optionally be substituted with 1 or 2 lower alkoxy or nitro groups), such as the benzyloxycarbonyloxy, p-methoxybenzyloxycarbonyloxy, 3,4-dimethoxybenzyloxycarbonyloxy, o-nitrobenzyloxycarbonyloxy or p-nitrobenzyloxycarbonyloxy groups; and other protected groups, such as the pivaloyloxymethoxycarbonyloxy group. Of these, we prefer: the aliphatic acyl groups; the aromatic acyloxy groups; the ethoxy group and substituted ethoxy groups, such as the 2-(phenylselenyl)ethoxy group; aralkyloxy groups; tetrahydropyranyloxy groups; aralkyloxy groups; alkoxycarbonyloxy groups; alkenyloxycarbonyloxy groups; and aralkyloxycarbonyloxy groups. The above are examples of protected hydroxy groups which may be represented by $R^{4'}$. Examples of protected thio groups include the thio groups corresponding to the protected hydroxy groups exemplified above. Examples of protected carboxy groups include the ester groups exemplified above in relation to $R^4$.

$R^{10}$ represents a hydroxy-protecting or mercapto-protecting group, e.g. as exemplified in relation to the hydroxy-protecting groups forming part of the groups which may be represented by $R^{4'}$.

$E^f$ represents a heterocyclic group containing from 5 to 14, preferably from 5 to 10 and more preferably from 5 to 7, ring atoms, as defined above for E.

Q' represents a group having the formula -O-$R^{11}$ [in which $R^{11}$ represents a hydroxy-protecting group, for example: a tetrahydropyranyl or tetrahydrothiopyranyl group which may be substituted or unsubstituted, such as the tetrahydropyran-2-yl, 3-bromotetrahydropyran-2-yl 4-methoxytetrahydropyran-4-yl, tetrahydrothiopyran-2-yl or 4-methoxytetrahydrothiopyran-4-yl groups; a lower alkoxymethyl group such as the methoxymethyl, t-butoxymethyl, 2-methoxyethoxymethyl, 2,2,2-trichloroethoxymethyl or bis(2-chloroethoxy)methyl groups; or an aralkyl group such as the benzyl, p-methoxybenzyl, o-nitrobenzyl, p-nitrobenzyl, p-halobenzyl (e.g. p-chlorobenzyl or p-bromobenzyl group), p-cyanobenzyl, diphenylmethyl, triphenylmethyl, α-naphthyldiphenylmethyl, or p-methoxyphenyldiphenylmethyl groups, of which we prefer the tetrahydropyranyl, lower alkoxymethyl and aralkyl groups] or any one of the heterocyclic groups defined above for Q in which, if necessary, any reactive group is protected.

Q" represents a group of formula Y, defined below, or any one of the heterocyclic groups represented by Q, in which Q is as defined above.

Y represents a halogen atom (for example a chlorine, bromine or iodine atom), a lower alkylsulphonyloxy group (for example a methanesulphonyloxy, ethanesulphonyloxy or trifluoromethanesulphonyloxy group), a trihalomethoxy group (for example a trichloromethoxy or tribromomethoxy group) or an arylsulphonyloxy

group (for example a benzenesulphonyloxy or p-toluenesulphonyloxy group).

Step A1

In this Step a compound of formula (V) having a terminal hydroxy or mercapto group (-B-H) on the methyl group at the position α to the ethereal oxygen atom is reacted with a compound of formula (VI), to give the compound of formula (VIII). This is a simple alkylation reaction and may be carried out by means well known for this type of reaction. For example, the reaction may be carried out by reacting the compound of formula (V) with the compound of formula (VI) in the presence of a base.

The reaction is preferably effected in the presence of a solvent, the nature of which is not critical, provided that it does not interfere with the reaction. Examples of suitable solvents include: ether, such as diethyl ether, tetrahydrofuran, or dioxane; aromatic hydrocarbons, such as benzene or toluene; amides, such as dimethylformamide or dimethylacetamide; dimethyl sulphoxide; or hexamethylphosphoric triamide; preferably benzene, dimethylformamide or hexamethylphosphoric triamide.

There is also no particular restriction on the nature of the base to be employed, provided that it does not affect other parts of the compounds involved in the reaction. The base functions as an acid-binding agent and any base capable of fulfilling this function may be employed in the present invention, for example: organic bases, such as triethylamine, 1,5-diazabicyclo[5.4.0]-undec-5-ene, pyridine, 2,6-lutidine, dimethylaniline or 4-(N,N-dimethylamino)pyridine; alkali metal hydroxides, such as sodium hydroxide or potassium hydroxide; and alkali metal hydrides, such as sodium hydride or potassium hydride; of these, the alkali metal hydroxides are preferred.

The reaction will take place over a wide range of temperatures and the precise temperature chosen is not particularly critical. We generally find it convenient to conduct the reaction at a temperature from 0°C to 150°C, more preferably at from 60°C to 90°C. The time required for the reaction may vary widely, depending on many factors, notable the reaction temperature and the nature of the starting materials, but a period of from 1 hour to 3 days, more preferably from 4 to 16 hours, will normally suffice.

After completion of the reaction, the compound of formula (VIII) can be collected from the reaction mixture by conventional means. For instance, one suitable recovery technique comprises: adding an organic solvent immiscible with water to the reaction mixture; washing with water; and evaporating off the solvent. The desired compound thus obtained can be further purified, if necessary, by such conventional techniques as recrystallization, reprecipitation and the various chromatography techniques, notably column chromatography.

Step A2

In this Step, a compound of formula (V) is reacted with a compound of formula (VII) under the conditions of the Mitsunobu reaction.

Such a reaction may be carried out in the presence of a solvent using a lower dialkyl azodicarboxylate, such as dimethyl azodicarboxylate or diethyl azodicarboxylate, and triphenylphosphine.

There is no particular limitation on the nature of the solvent, provided that it does not interfere with the reaction. Examples of suitable solvents include: ethers, such as diethyl ether or tetrahydrofuran; and aromatic hydrocarbons, such as benzene or toluene.

The reaction will take place over a wide range of temperatures and the precise temperature chosen is not particularly critical. We generally find it convenient to conduct the reaction at a temperature from 0°C to 100°C. The time required for the reaction may vary widely, depending on many factors, notably the reaction temperature and the nature of the starting materials, but a period of from 15 minutes to 2 hours will normally suffice.

After completion of the reaction, the compound of formula (VIII) can be collected from the reaction mixture by conventional means. The desired compound thus obtained can be isolated by various chromatography techniques, notably column chromatography.

Step A3

In Step A3, the desired compound of formula (IX) is prepared by converting the group of formula -O-$R^{11}$, when the compound of formula (VIII) contains this group as Q', into a group of formula Y.

First, the hydroxy-protecting group, $R^{11}$, is removed. The nature of the reaction employed to remove this group will, of course, depend on the nature of the group to be removed. When the hydroxy-protecting group is a tetrahydropyranyl group, a tetrahydrofuranyl group, a substituted ethyl group or a lower

alkoxymethyl group, it can be removed by treatment with an acid in a solvent. Examples of suitable acids include acetic acid, p-toluenesulphonic acid, hydrochloric acid or a mixture of acetic acid and sulphuric acid. The reaction is preferably effected in the presence of a solvent, the nature of which is not critical, provided that it does not interfere with the reaction. Examples of suitable solvents include: alcohols, such as methanol or ethanol; ethers, such as tetrahydrofuran or dioxane; and mixtures of one or more of these organic solvents with water.

The reaction will take place over a wide range of temperatures and the precise temperature chosen is not particularly critical. We generally find it convenient to conduct the reaction at a temperature from 0°C to 100°C, more preferably at from 20°C to 60°C. The time required for the reaction may vary widely, depending on many factors, notably the reaction temperature and the nature of the starting materials, but a period of from 10 minutes, more commonly from 1 hour, to 24 hours will normally suffice.

When the hydroxy-protecting group is an aralkyl group, it can be removed by contact with a reducing agent. For example, the reduction can be carried out by catalytic reduction at room temperature by using a catalyst, such as palladium on activated carbon, platinum or Raney nickel, in the presence of hydrogen gas. This reaction is preferably effected in the presence of a solvent, the nature of which is not critical, provided that it does not interfere with the reaction. Examples of suitable solvents include: alcohols, such as methanol or ethanol; ethers, such as tetrahydrofuran or dioxane; fatty acids, such as acetic acid; and mixtures of one or more of these organic solvents with water.

The reaction will take place over a wide range of temperatures and the precise temperature chosen is not particularly critical. We generally find it convenient to conduct the reaction at a temperature from 0°C to about room temperature. The time required for the reaction may vary widely, depending on many factors, notable the reaction temperature and the nature of the starting materials, especially the reducing agent, but a period of from 5 minutes to 12 hours will normally suffice.

Alternatively, the deprotection reaction can be conducted by reacting the protected compound with a metal, such as metallic lithium or sodium, with liquid ammonia or with an alcohol, such as methanol or ethanol, at a relatively low temperature, e.g. from -78°C to -20°C.

Where the protecting group is an aralkyl group, it can also be removed by using a mixture of aluminium chloride and sodium iodide or an alkylsilyl halide, such as trimethylsilyl iodide. The reaction is preferably carried out in the presence of a solvent, the nature of which is not critical, provided that it does not interfere with the reaction. Examples of preferred solvents include: nitriles such as acetonitrile; halogenated hydrocarbons, particularly halogenated aliphatic hydrocarbons, such as methylene chloride or chloroform; and mixtures of any two or more of the above solvents. The reaction temperature may vary widely, depending upon many factors, notably the nature of the starting materials, but we generally find it convenient to carry out the reaction at a temperature of from 0°C to 50°C.

When the compound of formula (VIII) contains a mercapto sulphur atom and the hydroxy-protecting group is a benzyl group, this can often best be removed by treatment with aluminium chloride and sodium iodide. When the protecting group is a di- or tri- arylmethyl group, this is preferably removed by treatment with an acid, e.g. trifluoroacetic acid, hydrochloric acid or acetic acid.

Where the hydroxy-protecting group is a silyl group, it can be removed by treatment with a compound producing a fluoride anion, such as tetrabutylammonium fluoride. The reaction is preferably effected in the presence of a solvent, the nature of which is not critical, provided that it does not interfere with the reaction. Examples of suitable solvents include: ethers, such as tetrahydrofuran, or dioxane.

The reaction will take place over a wide range of temperatures and the precise temperature chosen is not particularly critical. We generally find it convenient to conduct the reaction at about room temperature. The time required for the reaction may vary widely, depending on many factors, notably the reaction temperature and the nature of the starting materials, but a period of from 10 to 18 hours will normally suffice.

Where the hydroxy-protecting group is an aliphatic acyl group, an aromatic acyl group or an alkoxycarbonyl group, the protecting group can be removed by treatment with a base. There is no particular restriction on the nature of the base to be employed in this reaction, provided that other parts of the molecule are not affected. Examples of preferred bases include: metal alcoholates, particularly alkali metal alcoholates, such as sodium methoxide; ammonium hydroxide; alkali metal carbonates, such as sodium carbonate or potassium carbonate; alkali metal hydroxides, such as sodium hydroxide or potassium hydroxide; and mixtures of concentrated ammonia and methanol. The reaction is preferably effected in the presence of a solvent, the nature of which is not critical, provided that it has no adverse effect on the reaction, and any solvent conventionally used in hydrolysis reactions may equally be employed here. Suitable examples include such organic solvents as alcohols (such as tetrahydrofuran or dioxane), water or a mixture of one or more of the above organic solvents and water. The reaction temperature and the time

required for the reaction may vary widely, depending upon the nature of the starting materials and the bases employed and there is no particular restriction. However, in order to avoid adverse reactions, we normally prefer to carry out the reaction at a temperature of from 0°C to 150°C and for a period of from 1 hour to 10 hours.

Where the hydroxy-protecting group is an alkenyloxycarbonyl group, it can be removed by treatment with a base in a similar manner to that described above for deprotection when the hydroxy-protecting group is an aliphatic acyl group, an aromatic acyl group or an alkoxycarbonyl group. Where the protecting group is an allyloxycarbonyl group, it can also be removed simply by using palladium and triphenylphosphine or nickel tetracarbonyl, and this reaction has the advantage that there is little if any side reaction.

After completion of the reaction, the desired compound can be isolated from the reaction mixture by conventional means. The product may then, if desired, be further purified by such conventional techniques as recrystallization, preparative thin layer chromatography or column chromatography.

Next, the deprotected hydroxy group is converted to an ester by acylation, for example, by methanesulphonylation, toluenesulphonylation, trifluoromethanesulphonylation or trifluoroacetylation, or it is halogenated.

The ester synthesis is preferably effected in the presence of a solvent, the nature of which is not critical, provided that it does not interfere with the reaction. Examples of suitable solvents include: halogenated hydrocarbons, particularly halogenated aliphatic hydrocarbons, such as chloroform, methylene chloride or dichloroethane; ethers, such as diethyl ether, tetrahydrofuran or dioxane; and aromatic hydrocarbons, such as benzene or toluene. Of these, methylene chloride or benzene are preferred.

The reaction is preferably effected in the presence of a base, the nature of which is not critical, provided that it does not affect other parts of the compounds. The base functions as an acid-binding agent and any base capable of fulfilling this function may be employed in the present invention, for example: organic bases, such as triethylamine, pyridine, 2,6-lutidine or N,N-dimethylaniline.

The reaction will take place over a wide range of temperatures and the precise temperature chosen is not particularly critical. We generally find it convenient to conduct the reaction at a temperature from 0°C to 25°C. The time required for the reaction may vary widely, depending on many factors, notably the reaction temperature and the nature of the starting materials, but a period of from 30 minutes to 24 hours will normally suffice.

The nature of the halogenation reaction is not critical, provided that it can replace a hydroxy group by a halogen atom. In general, it is preferably carried out using a carbon tetrahalide and triphenylphosphine, or using a phosphorus trihalide.

The reaction is preferably effected in the presence of a solvent, the nature of which is not critical provided that it does not interfere with the reaction. Examples of suitable solvents include: halogenated hydrocarbons, particularly halogenated aliphatic hydrocarbons, such as chloroform, methylene chloride or dichloroethane; and nitriles, such as acetonitrile.

The reaction will take place over a wide range of temperatures and the precise temperature chosen is not particularly critical. We generally find it convenient to conduct the reaction at a temperature from -25°C to room temperature. The time required for the reaction may vary widely, depending on many factors, notably the reaction temperature and the nature of the starting materials, but a period of from 1 to 60 minutes hours will normally suffice.

The halogen-substituted compound can also be synthesized by reaction of the ester synthesized as described above with an alkali metal halide, such as sodium iodide, sodium bromide or potassium chloride. The reaction is preferably effected in the presence of a solvent, the nature of which is not critical, provided that it does not interfere with the reaction. It is preferably a polar solvent capable of dissolving an alkali metal halide. Examples include: ketones, such as acetone, sulphoxides, such as dimethyl sulphoxide; fatty acid amides, such as dimethylformamide; and phosphorus triamides, such as hexamethylphosphoric triamide. Of these, we prefer dimethylformamide.

The reaction will take place over a wide range of temperatures and the precise temperature chosen is not particularly critical. We generally find it convenient to conduct the reaction at a temperature from 20°C to 80°C. The time required for the reaction may vary widely, depending on many factors, notably the reaction temperature and the nature of the starting materials, but a period of from 1 to 24 hours will normally suffice.

After completion of the reaction, the desired compound can be isolated from the reaction mixture by conventional means. The product may then, if desired, be further purified by such conventional techniques as recrystallization, preparative thin layer chromatography and column chromatography.

Step A4

This is essentially the same as Step A1, except that, whereas, in Step A1, the group -B-H is on the compound of formula (V) (the cyclic ether) and the group Y is on the compound of formula (VI), in Step A4, the group Y is on the compound of formula (X) (the cyclic ether) and the group -B-H is on the compound of formula (XI). The reaction may be carried out employing the same reagents and reaction conditions as hereinbefore described with reference to Step A1. The resulting compound of formula (VIII) may then be subjected to Step A3, as described above.

Step A5

In this Step, a compound of formula (VIII) is prepared by reacting a compound of formula (XI) having a terminal hydroxy or mercapto group in its molecule with a compound of formula (XII), which functions as an alkylating agent, in a similar manner to that described in Step A1 or A4, to give an ether or thioether compound, followed by removing the hydroxy- or mercapto-protecting group represented by $R^{10}$; the hydroxy or mercapto group of the resulting compound may then be alkylated with a compound of formula $R^{1y}$-Y (in which $R^{1y}$ represents an alkyl group containing from 8 to 22 carbon atoms, and Y is as defined above). Alternatively, it may be acylated by using a reactive derivative of a carboxylic acid having the formula $R^{1z}$-Y (in which Y is as defined above and $R^{1z}$ represents a straight or branched chain aliphatic acyl group containing from 8 to 22 carbon atoms). As a still further alternative, it may be carbamated using a compound of formula $R^3$-N=C=O (in which $R^3$ is as defined above).

The first step of this reaction will take place as described in Step A1 and may be carried out using similar reagents and reaction conditions to those employed in that Step.

The nature of the reaction employed to remove the hydroxy- or mercapto- protecting group $R^{10}$ will, of course, depend on the nature of the group to be removed, but these groups can be removed as described above in relation to the removal of the hydroxy-protecting group $R^{11}$ in Step A3.

Where the resulting deprotected compound is to be alkylated, the reaction may be carried out as described above in relation to the alkylation reaction of Step A1, employing the same reagents and reaction conditions.

Where Y represents a halogen atom, acylation is preferably carried out in a solvent in the presence of a base. Where a solvent is employed, its nature is not critical, provided that it does not interfere with the reaction and that the starting material can dissolve in it, at least to some extent. Preferred examples of such solvents include: halogenated hydrocarbons, particularly halogenated aliphatic hydrocarbons, such as methylene chloride, chloroform or 1,2-dichloroethane; ethers, such as diethyl ether, tetrahydrofuran or dioxane; and aromatic hydrocarbons such as benzene or toluene.

There is likewise no particular limitation on the nature of the base to be employed in the reaction, and any base commonly used for this type of reaction may equally be used here, provided that it has no adverse effect on the reaction, and in particular, that it has no effect on other parts of the molecule. In general, we prefer to use an amine, preferably triethylamine, diethylamine or pyridine.

The reaction will take place over a wide range of temperatures, and the precise temperature chosen is not critical to the invention. However, we generally find it convenient to carry out the reaction at a temperature of from 0°C to 120°C. The time required for the reaction may vary widely, depending upon many factors, notably the nature of the starting materials, the solvents of the bases and the reaction temperature. However, when the reaction is carried out at a temperature in the range suggested above, a period of from 2 to 24 hours will normally suffice.

Where Y represents a trihalomethoxy group, a lower alkanesulphonyloxy group, a halogenated lower alkanesulphonyloxy group, an arylsulphonyloxy group, an aliphatic acyloxy group or an aromatic acyloxy group, a base is not always required for the reaction, as the reaction occurs spontaneously. Nonetheless, the reaction rate is accelerated in the presence of the base, and, if a base is employed, it is preferably selected from those described above for the case where Y represents a halogen atom.

In this case, too, the reaction is preferably effected in the presence of a solvent, the nature of which is not critical, provided that it has no adverse effect on the reaction. Examples of suitable solvents include: halogenated hydrocarbons, particularly halogenated aliphatic hydrocarbons, such as chloroform, methylene chloride or 1,2-dichloroethane; and ethers, such as diethyl ether, tetrahydrofuran or dioxane. Of these, we prefer methylene chloride or tetrahydrofuran.

The reaction will take place over a wide range of temperatures, and the precise temperature chosen is not critical to the invention. However, we generally find it convenient to carry out the reaction at a temperature of from 0°C to 50°C, preferably from 0°C to 20°C. The time required for the reaction may

vary widely, depending upon many factors, notable the nature of the starting materials, the solvents or the bases (if employed) and the reaction temperature. However, when the reaction is carried out at a temperature in the range suggested above, a period of from 30 minutes to 24 hours will normally suffice.

After completion of the reaction, the desired compound can be recovered from the reaction mixture by conventional means. If required, the compound can be further purified by such conventional purification techniques as recrystallization, preparative thin layer chromatography or column chromatography.

The formation of a carbamate is preferably effected as a two stage reaction, in which first a compound having the formula $R^3$-COOH (in which $R^3$ is as defined above) is reacted with diphenylphosphoryl azide (DPPA) dissolved in an inert solvent, such as chloroform, toluene, benzene, methylene chloride or tetrahydrofuran, to form a compound of formula $R^3$-N=C=O (in which $R^3$ is as defined above). This reaction is preferably carried out in toluene or benzene in the presence of an organic base, such as triethylamine or tributylamine. The cyclic ether compound is then added to the solution of this compound of formula $R^3$-N=C=O, and then the mixture is heated at 60°C to 150°C for 2 to 24 hours to afford the desired compound of formula (VIII). We prefer that, immediately after the isocyanate compound is synthesized, the reaction mixture should be washed with a saturated aqueous solution of sodium bicarbonate and water to remove phosphorus compounds followed by distilling off the solvent, drying and dissolving the residue in a solvent (preferably toluene) selected from the above illustrated solvents. The cyclic ether compound should then be added; or a commercial isocyanate is reacted in the same solvent as above.

After completion of the reaction, the desired compound can be recovered from the reaction mixture by conventional means. If required, the compound can be further purified by such conventional purification techniques as recrystallization, preparative thin layer chromatography or column chromatography.

The resulting compound of formula (VIII) may then be subjected to Step A3, as described above.

Method B

In this Method there are prepared compounds of formula (I) in which $R^1$ represents the group of formula (III), i.e. a compound of formula (XV), as shown in the following reaction scheme:

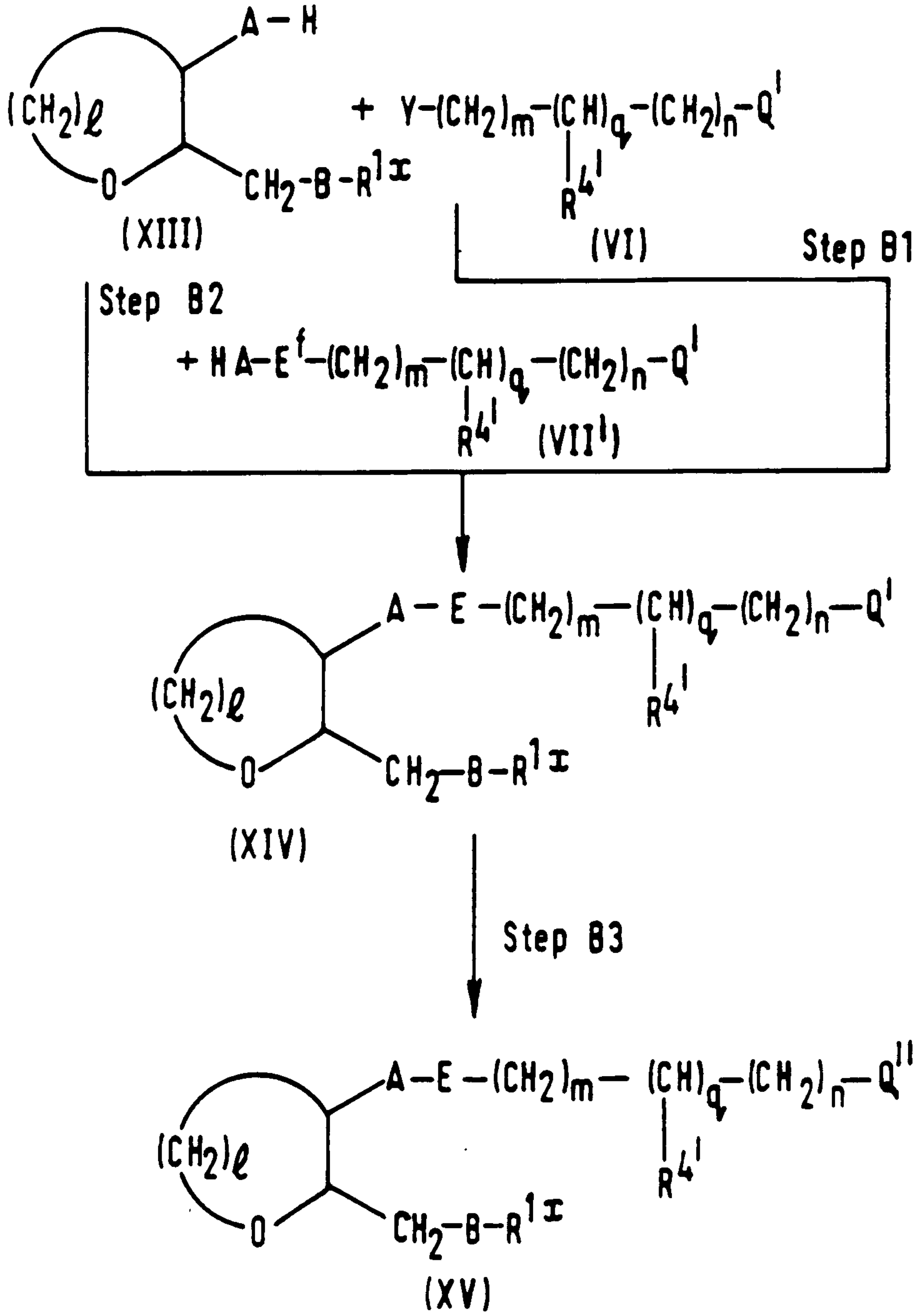


(XIII) (VI) (VII') (XIV) (XV)

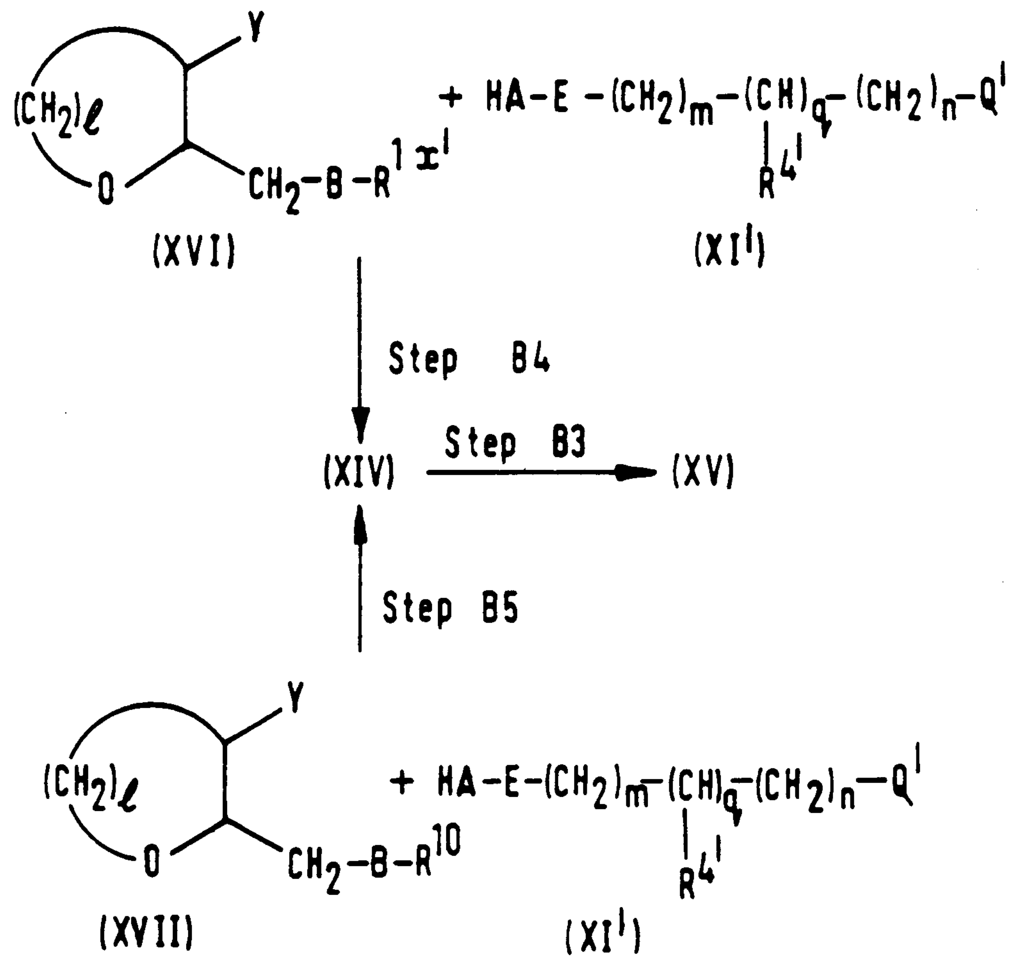


In the above formulae:

A, B, E, l, m, n, q, $R^{1x}$, $R^{1x'}$, $R^{4'}$, $R^{10}$, $E^f$, Q', Q" and Y are as defined above.

These reactions are the same as the reactions involved in the corresponding Steps of Method A, except that, in this case, the group of formula (III) is at the $\beta$ position to the oxygen atom of the cyclic ether. Each of these Steps may, of course, be carried out in precisely the same way as the corresponding Step of Method A, employing the same reagents and reaction conditions. In Steps B4 and B5, an inversion of the stereochemistry at the $\beta$-position takes place.

Method C

This reaction produces compounds of the invention in which the group of formula (III) is on the methyl group at the position $\alpha$ to the oxygen atom of the cyclic ether and E represents a group of formula -C(=O)- or -CO-$NR^6$-, i.e. a compound of formula (XVIII) or (XXI), as shown in the following reaction scheme:

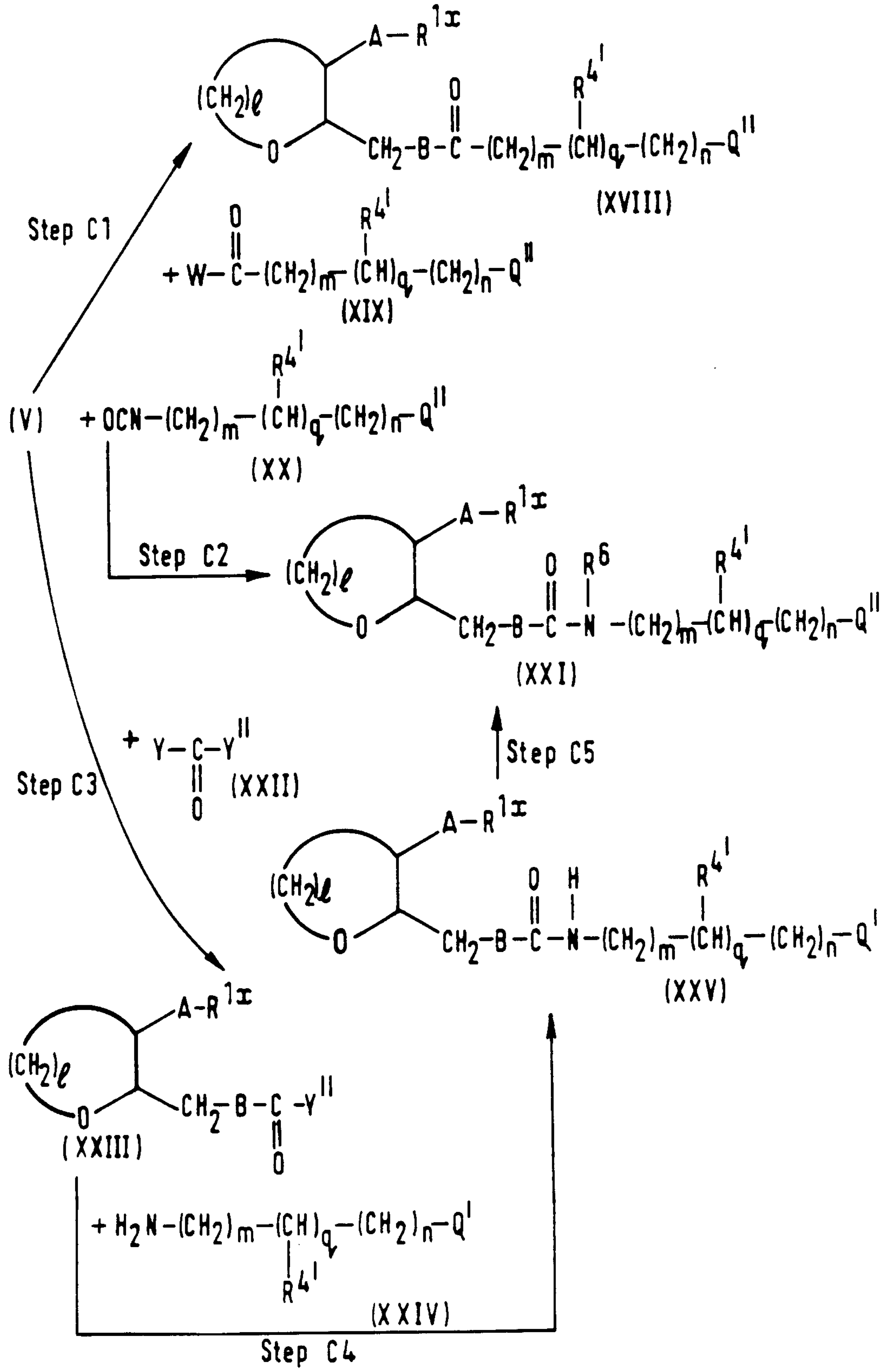


(XVIII) (XIX) (V) (XX) (XXI) (XXII) (XXV) (XXIII) (XXIV)

In the above formulae:

A, B, l, m, n, q, $R^{1x}$, $R^{4'}$, $R^6$, Q', Q" and Y are as defined above:

W represents a residue of a reactive carboxylic acid, preferably the same group as those defined as Y, a lower aliphatic acyloxy group or an aromatic acyloxy group (which may be any of those acyloxy groups defined above as protected hydroxy groups to be represented by $R^{4'}$);and

Y" represents a leaving group, such as a halogen atom, an aralkyloxy group (e.g. a benzyloxy group) or a trihalomethoxy group (e.g. a trichloromethoxy group).

Step C1

In this Step, a compound of formula (XVIII) is prepared by reacting a compound of formula (V) (see Method A) having a terminal hydroxy or mercapto group with a reactive derivative of an acid (XIX), in the presence or absence of a base.

The reaction is the same as the acylation reaction described in Step A5 of Method A, and may be carried out using the same reagents and reaction conditions.

Step C2

In Step C2, a carbamate or thiocarbamate is prepared by reaction of a compound of formula (V), which has a terminal hydroxy or mercapto group, with an isocyanate compound of formula (XX), followed, if desired, by substitution of the imino group to prepare a compound of formula (XXI) from the resulting carbamate or thiocarbamate.

The isocyanate compound of formula (XX) can be synthesized without difficulty, for example, by allowing a compound having the formula:

$$HOOC-(CH_2)_m-\underset{\substack{|\\ R^{4'}}}{(CH)}_q-(CH_2)_n-Q"$$

(in which m, q, n, $R^{4'}$ and Q" are as defined above) to react with diphenylphosphoryl azide in an inert solvent, such as chloroform, toluene, benzene, methylene chloride or tetrahydrofuran, preferably toluene or benzene, and in the presence of an organic base, such as triethylamine or tributylamine, preferably at a temperature of from 0°C to 150°C. The desired compound of formula (XXI) can be prepared directly by adding the compound of formula (V) to a solution of the compound of formula (XX) obtained as described above and then heating for 2 to 24 hours at 60°C to 150°C to react further. Preferably, the compound of formula (XX) at the time of its synthesis is washed with a saturated aqueous solution of sodium bicarbonate and with water in order to remove the phosphorus compound, and then, after removal of the solvent, dried, dissolved in any desired one of the solvents mentioned above (preferably toluene) and mixed with the compound of formula (V) to react.

The imino-substitution reaction can be achieved by reaction with, for example, an alkyl halide, a carboxylic acid halide or a carboxylic acid anhydride in the presence of a base.

The reaction is preferably effected in the presence of a solvent, the nature of which is not critical, provided that it does not interfere with the reaction. Examples of suitable solvents include: aromatic hydrocarbons, such as benzene or toluene; and organic amines, such as pyridine.

There is also no particular restriction on the nature of the base to be employed, provided that it does not interfere with other parts of the molecule. It is preferably: an organic base, e.g. an amine, for example, triethylamine, diisopropylethylamine, 4-(N,N-dimethylaminopyridine or pyridine; or an inorganic base, e.g. an alkali metal hydride, for example sodium hydride or potassium hydride.

The reaction will take place over a wide range of temperatures and the precise temperature chosen is not particularly critical. We generally find it convenient to conduct the reaction at a temperature from 20°C to 120°C. The time required for the reaction may vary widely, depending on many factors, notably the reaction temperature and the nature of the starting materials and base employed, but a period of from 1 to 24 hours will normally suffice.

After completion of the reaction, the desired compound can be isolated from the reaction mixture by conventional means. If required, the compound can be further purified by such conventional purification methods as recrystallization, preparative thin layer chromatography and column chromatography.

Step C3

In this Step, a compound of formula (XXIII) is prepared by reacting a compound of formula (V), which has a terminal hydroxy or mercapto group, with a compound of formula (XXII) in the presence of an organic base.

There is no particular restriction on the nature of the solvent to be employed, provided that it does not interfere with the reaction and that it can dissolve the starting materials at least to some extent. Examples of preferred solvents include: halogenated hydrocarbons, such as methylene chloride or chloroform; aromatic hydrocarbons, such as benzene, toluene or xylene; and ethers, such as tetrahydrofuran or dioxane.

There is also no particular restriction on the nature of the base to be employed, provided that it does not interfere with other parts of the molecule. It is preferably an organic base, e.g. an amine, for example, triethylamine, 1,5-diazabicyclo[5.4.0]undec-5-ene, pyridine, 2,6-lutidine, dimethylaniline or 4-(*N*,*N*-dimethylamino)pyridine.

The reaction will take place over a wide range of temperatures and the precise temperature chosen is not particularly critical. We generally find it convenient to conduct the reaction at a temperature from 0°C to 100°C, preferably from 0°C to 50°C. The time required for the reaction may vary widely, depending on many factors, notably the reaction temperature and the nature of the starting materials and base employed, but a period of from 30 minutes to 24 hours will normally suffice.

After completion of the reaction, the desired compound can be isolated from the reaction mixture by conventional means. If required, the compound can be further purified by such conventional purification methods as recrystallization, preparative thin layer chromatography and column chromatography.

Alternatively, the compound of formula (XXIII) may be employed as such in the next Step, without isolation.

## Step C4

In Step C4, the compound of formula (XXIII) prepared as described in Step C3 is reacted with an amine compound of formula (XXIV) to give a compound of formula (XXV). This reaction is preferably carried out in the presence of a solvent and of a base, and is preferably effected under the conditions described above in Step C3.

## Step C5

In this Step, a compound of formula (XXI) is prepared by converting a group of formula $-OR^{11}$ represented by Q' to a group Y and then, if desired, substituting the imino group with a group $R^6$. The first of these reactions may be carried out in a similar manner to that described for Step A3, and the second of these reactions may be carried out in a similar manner to that described for the substituting reaction in Step C2.

## Method D

This reaction produces compounds of the invention in which the group of formula (III) is at the position $\beta$ to the oxygen atom of the cyclic ether and E represents a group of formula $-CO-NR^6-$, i.e. a compound of formula (XXVI) or (XXVII), as shown in the following reaction scheme:

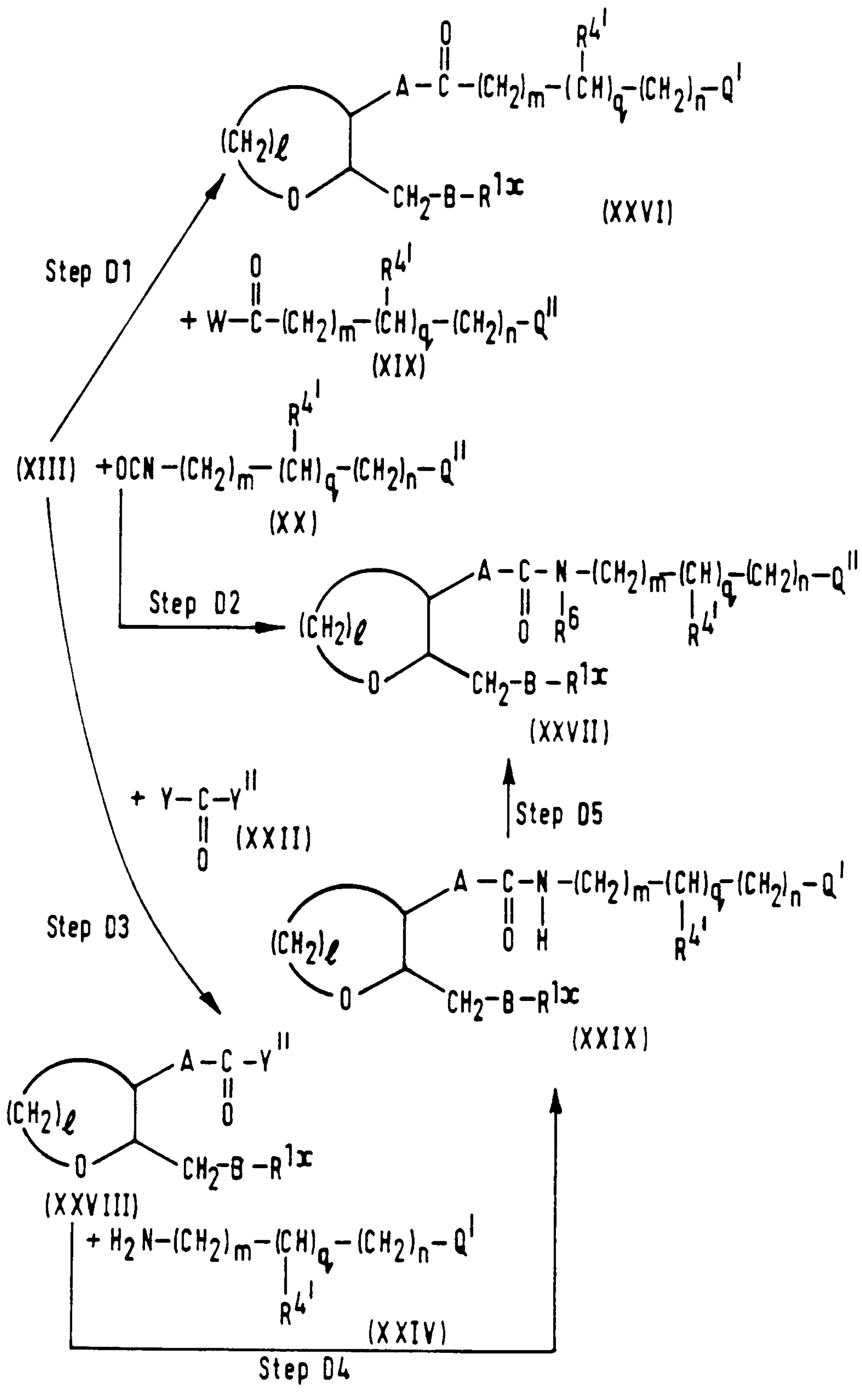


In the above formulae:

A, B, l, m, n, q, $R^{1x}$, $R^{4'}$, $R^6$, Q', Q", W, Y and Y" are as defined above.

These reactions are the same as the reactions involved in the corresponding Steps of Method C, except that, in this case, the group of formula (III) is at the $\beta$ position to the oxygen atom of the cyclic ether. Each of these Steps may, of course, be carried out in precisely the same way as the corresponding Step of

Method C, employing the same reagents and reaction conditions.

Method E

This reaction produces compounds of the invention in which the group of formula (III) is on the methyl group at the position α to the oxygen atom of the cyclic ether and E represents a group of formulas -CO-O-, i.e. a compound of formula (XXXII), as shown in the following reaction scheme:

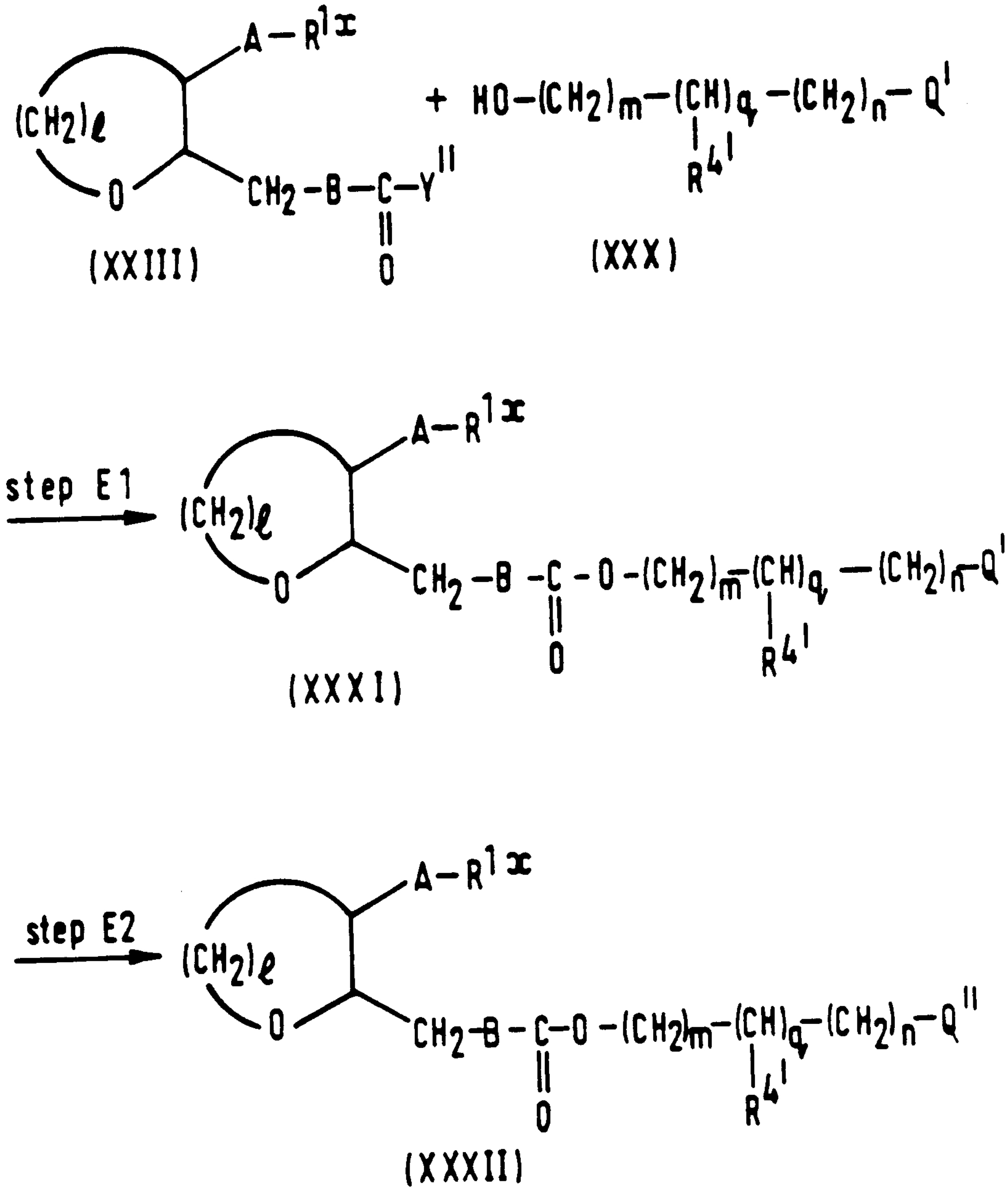


(XXIII) (XXX) (XXXI) (XXXII)

In the above formulae:
A, B, l, m, n, q, $R^{1x}$, $R^{4'}$, Q', Q" and Y" are as defined above.

Step E1

In this Step, a compound of formula (XXIII) is reacted with a hydroxy compound of formula (XXX) to give a compound of formula (XXXI). The reaction is essentially the same as that described in Step C4 of Method C and may be carried out using similar reagents and reaction conditions.

Step E2

In this Step, a compound of formula (XXXII) is prepared by converting a group of formula $-OR^{11}$ represented by Q' to a group Y. This reaction may be carried out in a similar manner to that described for the protecting reaction in Step A3.

Method F

This reaction produces compounds of the invention in which the group of formula (III) is at the position $\beta$ to the oxygen atom of the cyclic ether and E represents a group of formula -CO-O-, i.e. a compound of formula (XXXIV), as shown in the following reaction scheme:

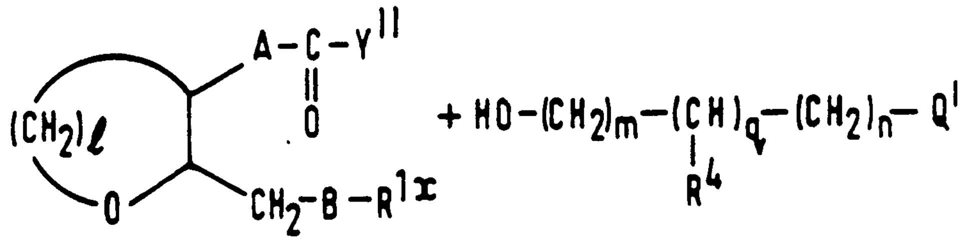

(XXVIII) (XXX)

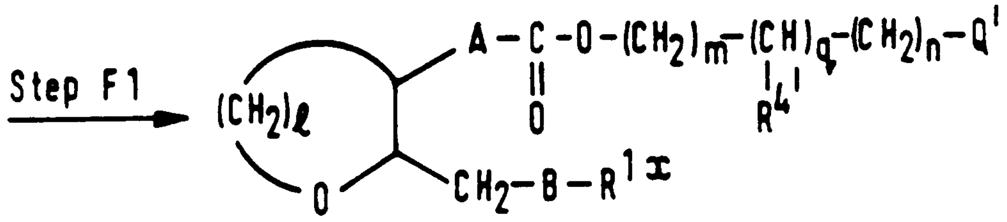


(XXXIII)

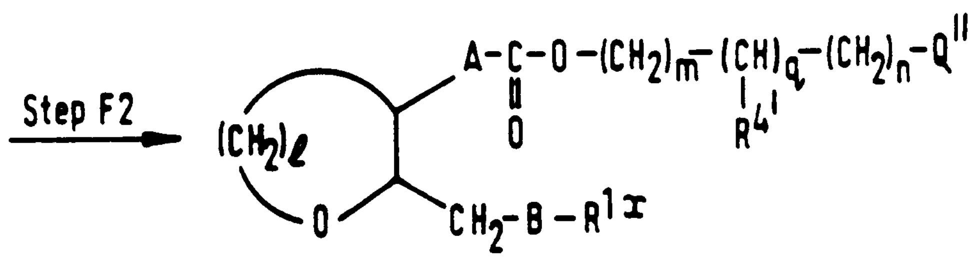


(XXXIV)

In the above formulae:

A, B, l, m, n, q, $R^{1x}$, $R^{4'}$, Q', Q" and Y" are as defined above.

These reactions are the same as the reactions involved in the corresponding Steps of Method E, except that, in this case, the group of formula (III) is at the $\beta$ position to the oxygen atom of the cyclic ether. Each of these Steps may, of course, be carried out in precisely the same way as the corresponding Step of Method E, employing the same reagents and reaction conditions.

Method G

This Method provides an alternative way of preparing compounds of formula (IX), as defined in Method A, and is illustrated by the following reaction scheme:

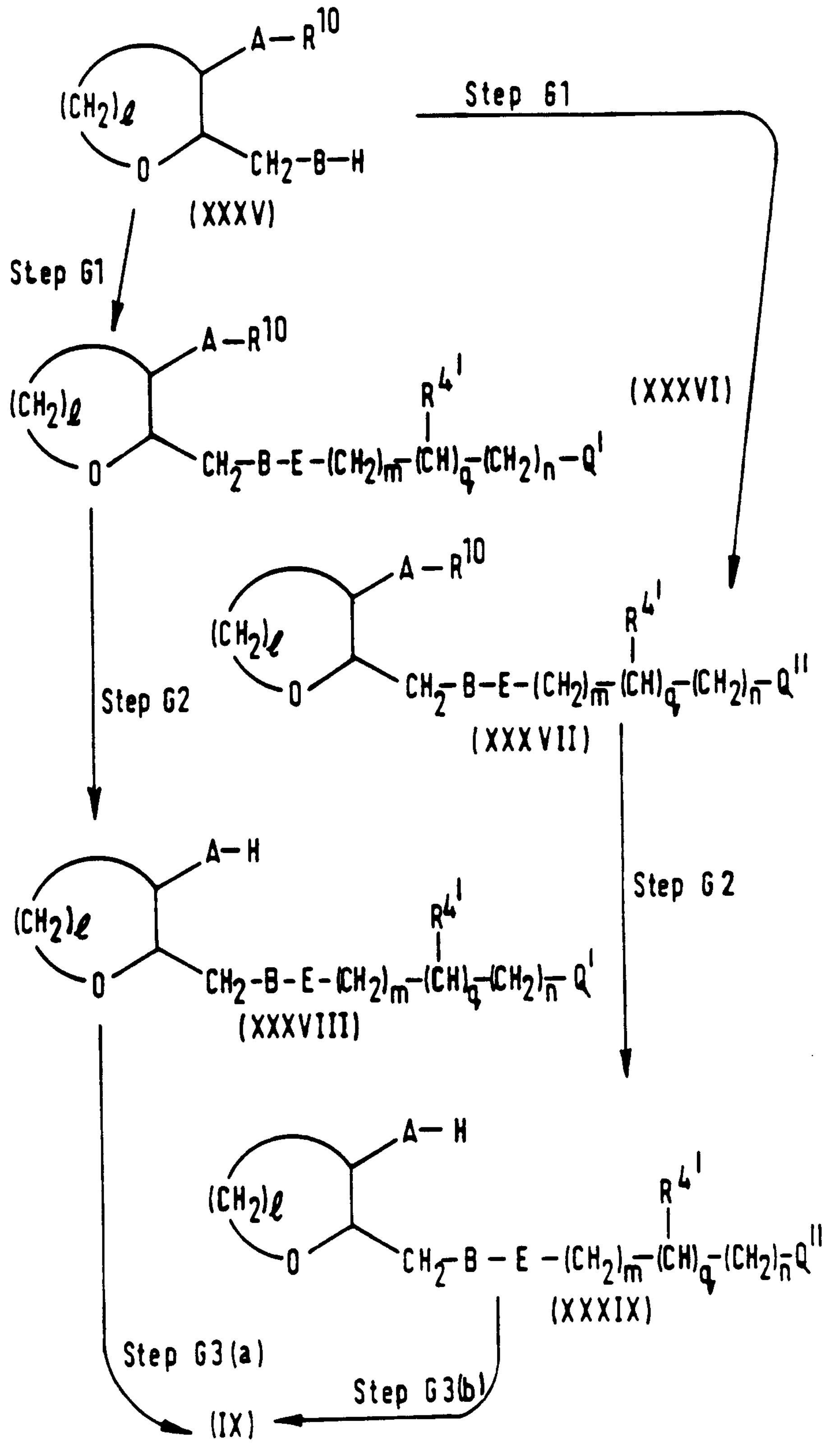

In the above formulae:
A, B, E, l, m, n, q, $R^{10}$, $R^{4'}$, Q' and Q" are as defined above.

Step G1

In this Step, a compound of formula (XXXV) having a protected hydroxy or mercapto group at position 3 and a free hydroxy or mercapto group on the methyl group at position 2 is converted to a compound of formula (XXXVI) and/or a compound of formula (XXXVII) by a sequence of reactions similar to those described in Steps A1 - A5, C1 - C5, E1 and E2, or any appropriate combination thereof.

Step G2

In this Step, the hydroxy- or mercapto- protecting group $R^{10}$ is removed by a reaction similar to those described in Step A3 of Method A, and which may be carried out using the same reagents and reaction conditions.

Step G3

In this Step, a compound of formula (IX) is prepared by either:
(a) in the case of the compound of formula (XXXVIII), converting a group of formula $-OR^{11}$ represented by Q', to a group Y, by the method described in Step A3, and, before or after this reaction, subjecting the compound to an alkylation, acylation or carbamoylation reaction similar to that described in the second part of Step A5, using the same reagents and reaction conditions, or
(b) in the case of the compound of formula (XXXIX), only subjecting the compound to an alkylation, acylation carbamoylation reaction similar to that described in the second part of Step A5, using the same reagents and reaction conditions,

Method H

This Method provides an alternative way of preparing compounds of formula (XV), as defined in Method B, and is illustrated by the following scheme:

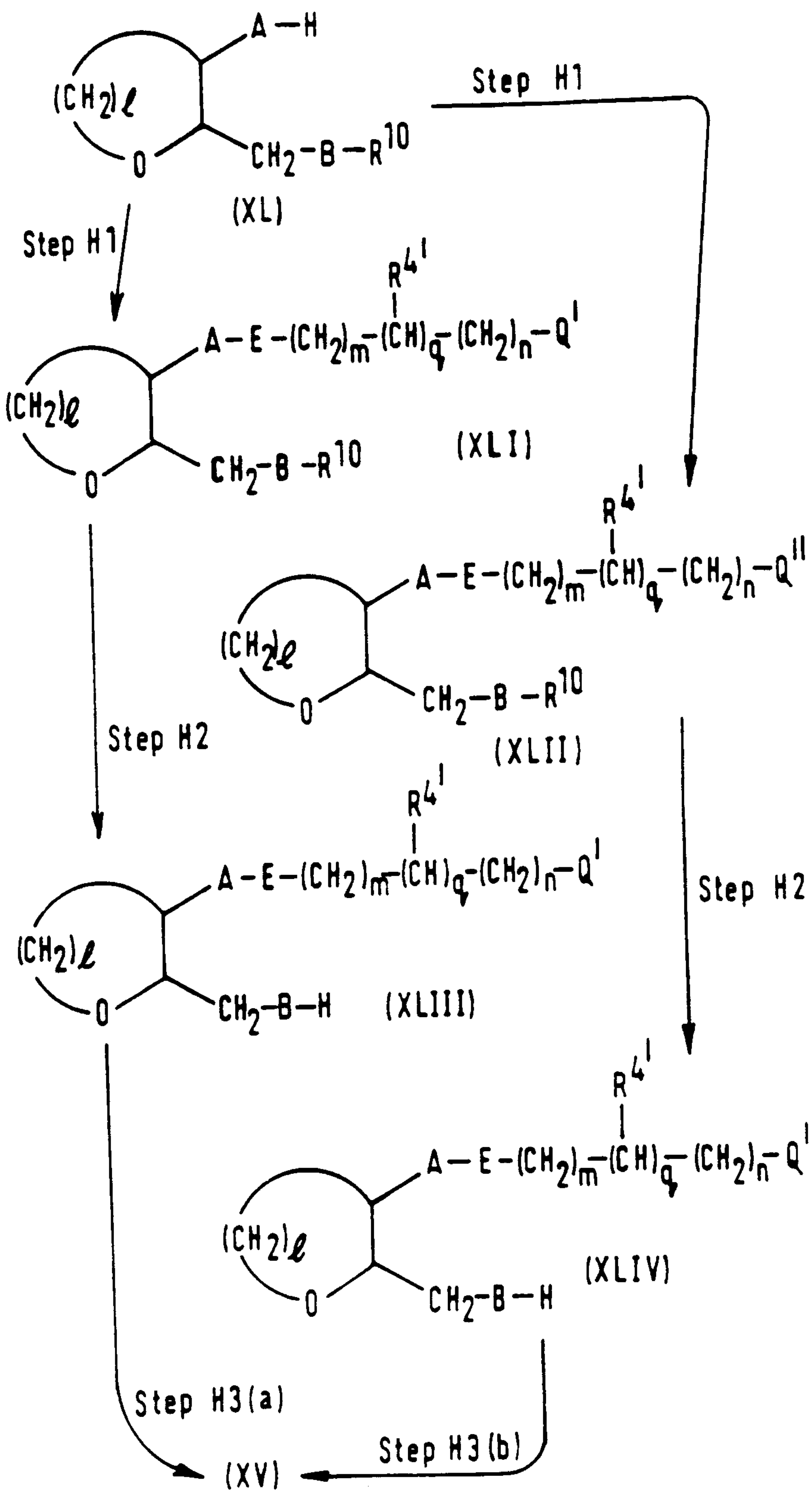


In the above formulae:

A, B, E, l, m, n, q, $R^{10}$, $R^{4'}$, Q' and Q" are as defined above.

These reactions are the same as the reactions involved in the corresponding Steps of Method G, except that, in this case, the group of formula (III) is at the β position to the oxygen atom of the cyclic ether. Each of these Steps may, of course, be carried out in precisely the same way as the corresponding Step of

Method G, employing the same reagents and reaction conditions.

Method I

This Method provides an alternative method of preparing compounds of the invention having a group of formula (III) on the methyl group at the 2-position, as illustrated in the following reaction scheme:

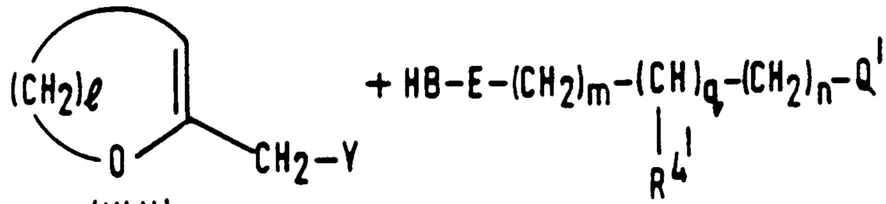

(XLV)

(XLVI)

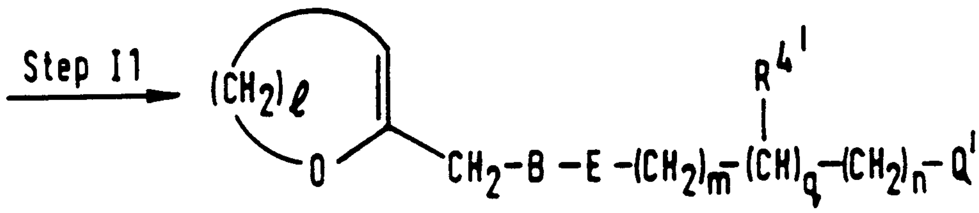


(XLVII)

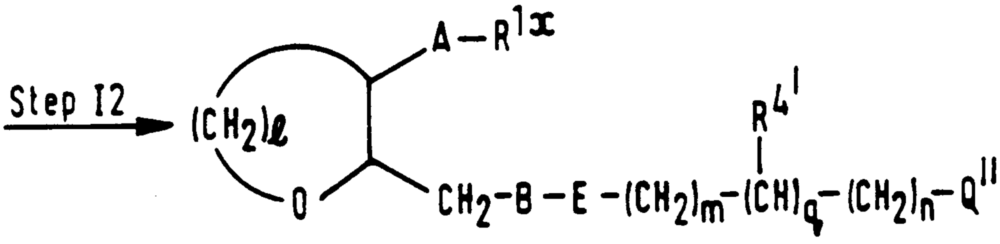


(XLVIII)

In the above formulae:
A, B, E, l, m, n, q, $R^{1x}$, $R^{4'}$, Y, Q' and Q" are as defined above.

Step I1

In this Step, a compound of formula (XLV) is reacted with a hydroxy or mercapto compound of formula (XLVI) to give a compound of formula (XLVII). This is an alkylation reaction similar to that described in Step A1 and may be carried out using the same reagents and reaction conditions.

Step I2

In this Step, a compound of formula (XLVIII) is prepared from the compound of formula (XLVII) by following essentially the same procedure as described in Japanese Patent Application Kokai No. 267592/86, and then the second half of Step A5.

Method J

This is a process for preparing a compound of the invention where a group of formulas (III) is on the methyl group at the 2-position and E represents a direct bond, as illustrated by the following reaction scheme:

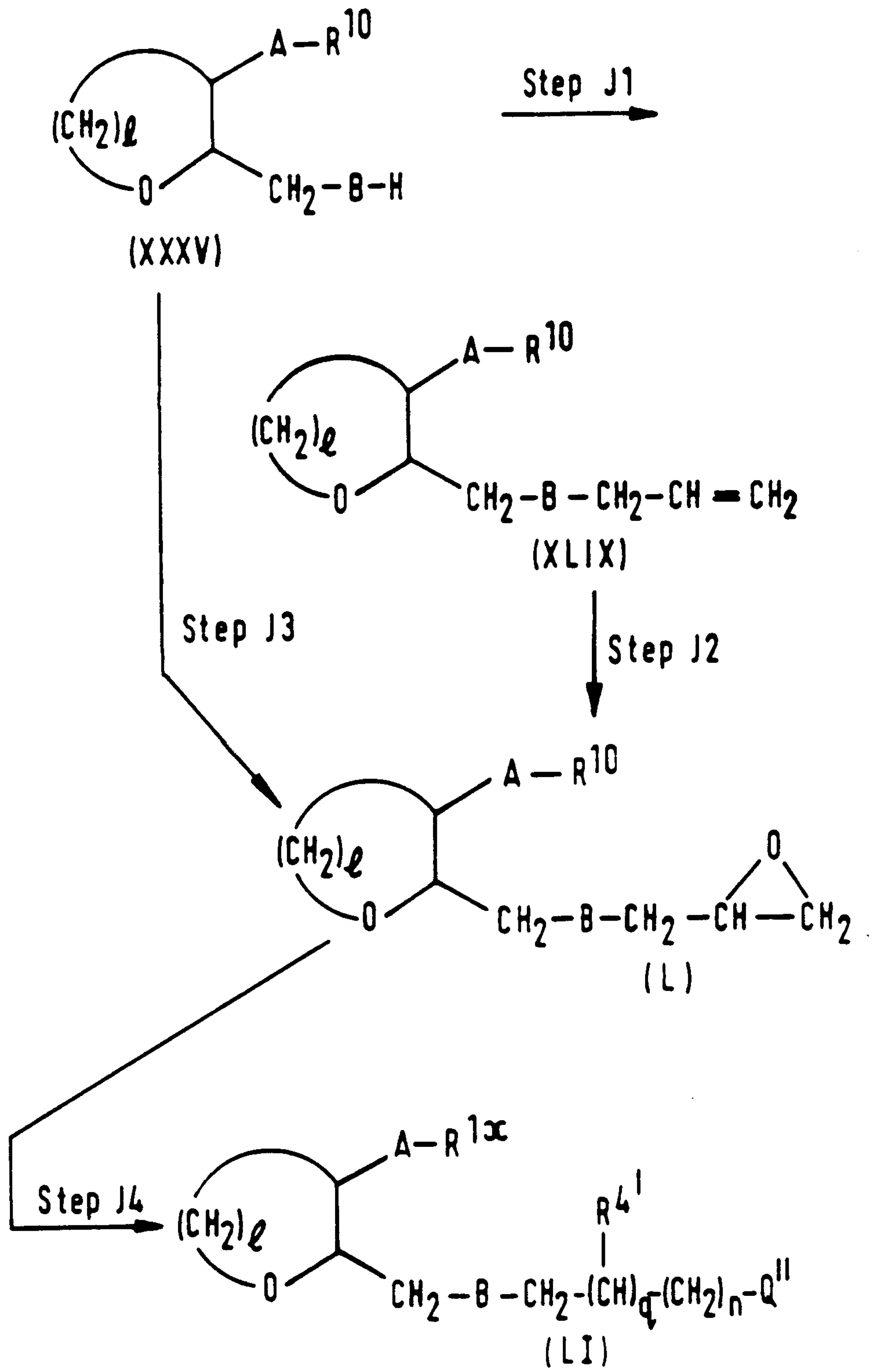


In the above formulae:
A, B, l, n, q, $R^{1x}$, $R^{4'}$, $R^{10}$ and Q" are as defined above.

Step J1

In this Step, a compound of formula (XLIX) is prepared by reacting a compound of formula (XXXV) with an active allyl compound, preferably an allyl halide (such as allyl chloride, allyl bromide or allyl iodide) in a solvent and in the presence of a base.

The nature of the solvent employed is not critical, provided that it does not interfere with the reaction. Examples of suitable solvents include: ethers, such as diethyl ether, tetrahydrofuran, or dioxane; aromatic hydrocarbons, such as benzene or toluene; and amides, such as dimethylformamide or dimethylacetamide; of these the amides are preferred.

There is also no particular restriction on the nature of the base to be employed, provided that it does not affect other parts of the compounds involved in the reaction. The base functions as an acid-binding agent and any base capable of fulfilling this function may be employed in the present invention, for example: alkali metal hydrides, such as sodium hydride or potassium hydride; alkali metal hydroxides, such as sodium hydroxide or potassium hydroxide; and organic bases, particularly amines, such as triethylamine, 1,5-diazabicyclo[5.4.0]undec-5-ene, pyridine, 2,6-lutidine, dimethylaniline or 4-(N,N-dimethylamino)pyridine; of these, the alkali metal hydrides are preferred.

The reaction will take place over a wide range of temperatures and the precise temperature chosen is not particularly critical. We generally find it convenient to conduct the reaction at a temperature from 0°C to 100°C. The time required for the reaction may vary widely, depending on many factors, notably the reaction temperature and the nature of the starting materials, but a period of from 1 hour to 3 days, more preferably from 1 to 24 hours, will normally suffice.

Step J2

In this Step, an epoxide compound of formula (L) is prepared by oxidation of the double bond in the compound of formula (XLIX) to convert it to an epoxide group.

The reaction is preferably effected in the presence of a solvent, the nature of which is not critical, provided that it does not interfere with the reaction. Examples of suitable solvents include: halogenated hydrocarbons, particularly halogenated aliphatic hydrocarbons, such as chloroform or methylene chloride; and ethers, such as diethyl ether or tetrahydrofuran.

There is no particular limitation on the nature of the oxidising agent used and any such agent conventionally used for this type of reaction may equally be used here. Preferred oxidising agents include: organic peroxides, such as peracetic acid, perbenzoic acid, benzoyl peroxide and m-chloroperbenzoic acid.

The reaction will take place over a wide range of temperatures and the precise temperature chosen is not particularly critical. We generally find it convenient to conduct the reaction at a temperature from -20°C to +80°C. The time required for the reaction may vary widely, depending on many factors, notably the reaction temperature and the nature of the starting materials, but a period of from 1 hour to 24 hours will normally suffice.

Step J3

In this Step, a compound of formula (L) is prepared directly from a compound of formula (XXXV) by reaction with an epihalohydrin (e.g. an epichlorohydrin, epibromohydrin or epiiodohydrin). The reaction will take place under the conditions described in Step J1.

Step J4

In this Step, the epoxide compound of formula (L) is reacted with a compound of formula $M-(CH_2)_{(n-1)}-Q'$ (in which: Q' and n are as defined above; and M represents a metal atom or a multivalent metal atom in association with a suitable anion, for example, an alkali metal atom, such as lithium, sodium or potassium, or a halogenated metal atom, such as halogenated magnesium atom or a halogenated zinc atom), which can be prepared by conventional means, after which it may be subjected to the reactions described in the latter half of Step A5 and Step A3.

The reaction with the compound of formula $M-(CH_2)_{(n-1)}-Q'$ is preferably effected in the presence of a solvent, the nature of which is not critical. Examples of suitable solvents include: aromatic hydrocarbons, such a toluene or benzene; and ethers, such as diethyl ether or tetrahydrofuran; of these, the ethers are preferred.

The reaction will take place over a wide range of temperatures and the precise temperature chosen is not particularly critical. We generally find it convenient to conduct the reaction at a temperature from -78 °C to +65 °C. The time required for the reaction may vary widely, depending on many factors, notably the reaction temperature and the nature of the starting materials, but a period of from 15 minutes to 24 hours will normally suffice.

Method K

In this Method, a compound of formula (I) of this invention is prepared by reacting a compound of formula (IX), (XV), (XVIII), (XXI), (XXVI), (XXVII), (XXXII), (XXXIV), (XLVIII) or (LI), which may have been prepared as described above, when Q" represents a group having the formula Y (in which Y is as defined above), with an amine compound of formula (LII) or $Q^1$, and then, if desired, deprotecting the protecting group of $R^5$ and/or $R^6$ and/or deprotecting the protecting group in the group $R^{4'}$, for example as illustrated by the following reaction:

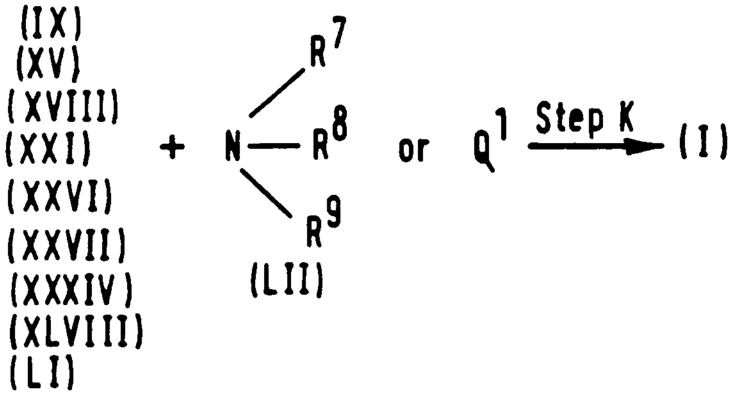

(IX)
(XV)
(XVIII)
(XXI)
(XXVI)
(XXVII)
(XXXIV)
(XLVIII)
(LI)
\+
(LII)
or
(I)

In the above formula, $R^7$, $R^8$ and $R^9$ are as defined and $Q^1$ represents a heterocyclic compound corresponding to the definition of Q.

The reaction is preferably effected in the presence of a solvent. There is no particular restriction on the nature of the solvent to be employed, provided that it does not interfere with the reaction and that it can dissolve the starting materials at least to some extent. Examples of preferred solvents include: aromatic hydrocarbons, such as benzene, toluene or xylene; halogenated hydrocarbons, particularly halogenated aliphatic hydrocarbons, such a methylene chloride or chloroform; lower alcohols, such as methanol, ethanol or isopropanol; amides, such as dimethylformamide; ethers, such as diethyl ether, tetrahydrofuran or dioxane; acetonitrile; water; or a mixture of any 2 or more, e.g. 2 to 3, of these solvents, such as a mixture of chloroform, dimethylformamide and isopropanol, e.g. in a volume ratio or about 3 : 5 : 5. Of these, a mixture of chloroform, dimethylformamide and isopropanol or an aromatic hydrocarbon are preferred.

The reaction will take place over a wide range of temperatures and the precise temperature chosen is not particularly critical. We generally find it convenient to conduct the reaction at a temperature from 20 °C to 80 °C. The time required for the reaction may vary widely depending on many factors, notably the reaction temperature and the nature of the starting materials and base employed, but a period of from 1 to 48 hours will normally suffice. When gaseous amines are employed, the reaction is preferably carried out in a nitrogen atmosphere and in a sealed reactor (e.g. a sealed tube).

The protecting group or groups may then be removed. Although the nature of the deprotecting reaction will vary depending on the nature of the protected group, it may be performed by known methods as shown below.

When the carboxy protecting group is a lower alkyl group, the protecting group can be removed by treatment with a base. The conditions employed in this reaction are similar to those described when the hydroxy-protecting group is a lower aliphatic acyl group or an aromatic acyl group.

When the carboxy protecting group is an aralkyl group or a halogenated lower alkyl group, the protecting group can be removed by contact with a reducing agent. Preferred examples of the reducing agent include: zinc and acetic acid when the carboxy-protecting group is a halogenated lower alkyl; catalytic reduction using palladium on activated carbon or platinum when the carboxy-protecting group is an aralkyl group; or an alkali metal sulphide such as potassium sulphide or sodium sulphide. The reaction may be carried out in a solvent and there is no particular limitation on the nature of the solvent, provided that it does

not participate in the reaction. Preferred examples of such solvents include: alcohols such as methanol or ethanol; ethers such as tetrahydrofuran or dioxane; fatty acids such as acetic acid; and mixtures of one or more of the above organic solvent and water. The reaction is usually carried out at a temperature between 0°C to room temperature. The time required for the reaction may vary depending on the nature of the starting materials and the reducing agents, but the reaction is usually carried out for a period of from 5 minutes to 12 hours.

When the carboxy-protecting group is an alkoxymethyl group, the protecting group can be removed by treatment with an acid. Preferred acids include hydrochloric acid or acetic acid-sulphuric acid. There is no particular limitation on the nature of the solvent, provided that it does not interfere with the reaction. Preferred example of such solvents include: alcohols such as methanol or ethanol; ethers such as tetrahydrofuran or dioxane; and mixtures of any one or more of the above organic solvents and water. The reaction is usually carried out at a temperature in the range of from 0°C to 50°C. The time required for the reaction may vary depending on the nature of the starting materials and the acid, but the reaction is usually carried out for a period of from 10 minutes to 18 hours.

After completion of the reaction, the desired compound may be recovered from the reaction mixture by conventional means. For example, after insoluble materials have been filtered off, the solvent is distilled off and the residue is purified by such conventional purification methods as recrystallization, preparative thin layer chromatography or column chromatography to give a purified product.

When the imino-protecting group is a lower aliphatic or aromatic acyl group or an alkoxycarbonyl group, the protecting group can be removed by treatment with a base. The reaction conditions are similar to those employed when the hydroxy-protecting group is a lower aliphatic acyl group or an aromatic acyl group.

When the imino-protecting group is an alkenyloxycarbonyl group, the protecting group can be removed by treatment with a base in a similar manner to that described when the hydroxy-protecting group is a lower aliphatic acyl group or an aromatic acyl group.

When the imino protecting group is an aryloxycarbonyl group, a deprotecting reaction can be simply carried out by using palladium and triphenyl phosphine or nickel tetracarbonyl and few side reactions occur.

The deprotection of the imino-protecting group described above may simultaneously remove a carboxy-protecting group.

After completion of the reaction, the desired compound can be recovered from the reaction mixture by using conventional methods. For example, a purified product can be obtained by such conventional purification methods as recrystallization, preparative thin layer chromatography or column chromatography and the like.

The deprotection of the carboxy-protecting group and the imino-protecting group can be carried out in any desired order.

If desired, the compounds at any appropriate stage may be protected, for example by esterification, in particular by a protecting group capable of being hydrolysed in vivo. This reaction can be performed by using well known methods in this art.

For example, ester compounds protected with a carboxy-protecting group which can be hydrolyzed in vivo can be prepared by reacting the carboxy group with: an aliphatic acyloxymethyl halide, such as acetoxymethyl chloride, propionyloxymethyl bromide or pivaloyloxymethyl chloride; a lower alkoxy carbonyloxyethyl halide, such as 1-methoxycarbonyloxyethyl chloride or 1-ethoxycarbonyloxyethyl iodide; a phthalidyl halide or a (2-oxo-5-methyl-1,3-dioxolen-4-yl)methyl halide, e.g. at from 0°C to 50°C. There is no particular limitation on the nature of the solvent employed, provided that is does not interfere with the reaction and a preferred solvent is a polar solvent such as dimethylformamide. The reaction temperature and time vary depending upon the nature of the starting material, the solvents and the reagents. The reaction is preferably carried out within a temperature range of from 0°C to 100°C over a period of from 30 minutes to 10 hours.

After completion of the reaction, the desired compound may be recovered from the reaction mixture by conventional means. For example, one suitable recovery technique comprises: filtering off any insoluble material precipitated from the reaction mixture; and removing the solvent, e.g. by distillation, if necessary under reduced pressure, to give the desired compound. If necessary, this compound may be further purified by such conventional techniques as recrystallisation or the various chromatography techniques, e.g. preparative thin layer chromatography or column chromatography.

When Q' or Q" represents any one of the heterocyclic groups defined for Q, each of the compounds of formula (VIII), (IX), (XIV), (XV), (XVIII), (XXI), (XXV), (XXVI), (XXVII), (XXIX), (XXXI), (XXXII), (XXXIII), (XXXIV), (XLVIII) and (LI) is a compound of the present invention. However, if desired, the protecting groups can be removed and, further if desired, the deprotected groups may be protected again by protecting groups capable of being hydrolysed in vivo in a similar manner to that described in Step K, to prepare the

corresponding compounds of formula (I).

PREPARATION OF STARTING MATERIALS

Certain of the starting materials used in the preparation of the compounds of the invention are novel. These may be prepared by methods well known *per se*, for example as follows:

Method L

In this method are prepared compounds of formulae (V), (X), (XVI), (XVII), (XXXV) and (XL) from the corresponding known compound of formula (LIII), i.e. 3,4-dihydro-2*H*-pyran, dihydrofuran or 6,7-dihydrooxepine, as shown in the following reaction schemes:

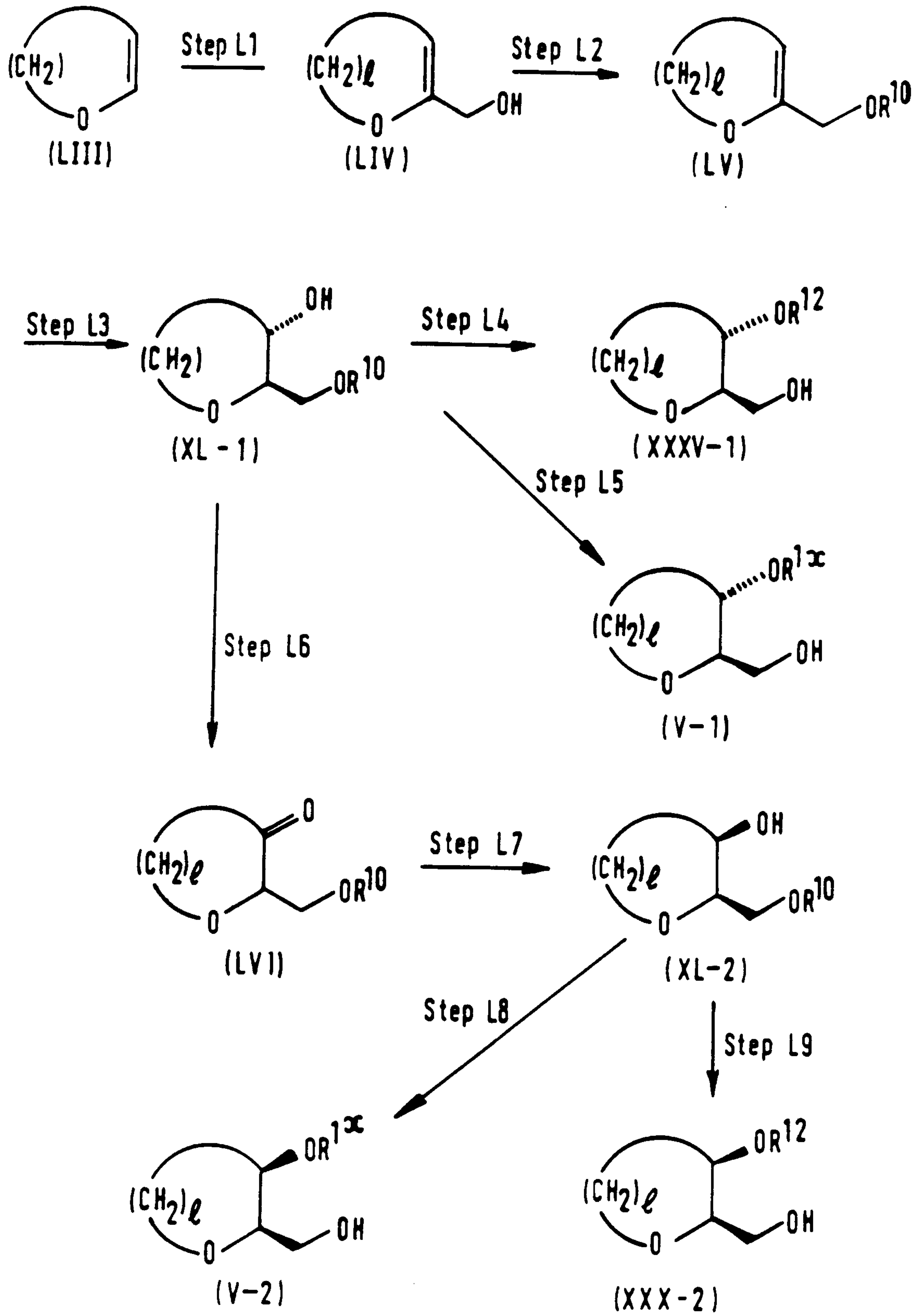


(LIII) (LIV) (LV)

(XL-1) (XXXV-1)

(V-1)

(LVI) (XL-2)

(V-2) (XXX-2)

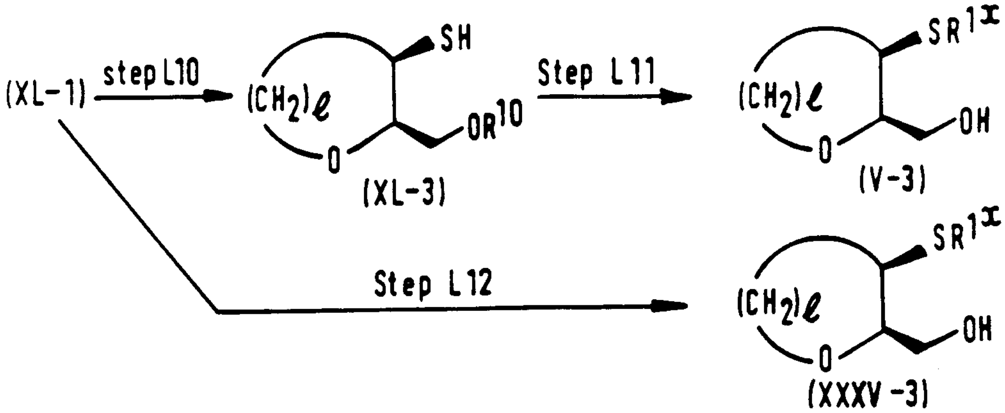
(XL-1)
step L10
(XL-3)
Step L11
(V-3)
Step L12
(XXXV-3)

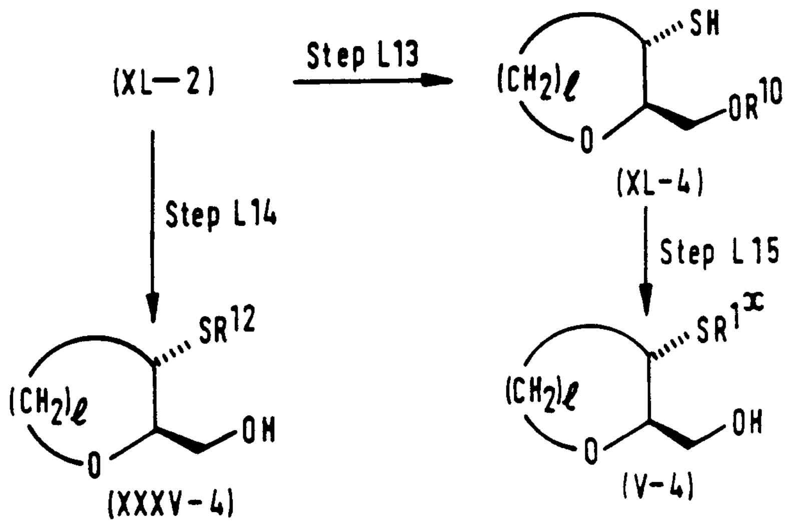
(XL—2)
Step L13
(XL-4)
Step L14
Step L15
(XXXV-4)
(V-4)

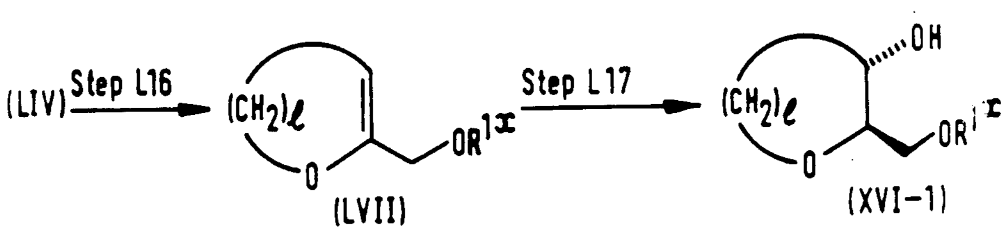
(LIV)
Step L16
(LVII)
Step L17
(XVI-1)

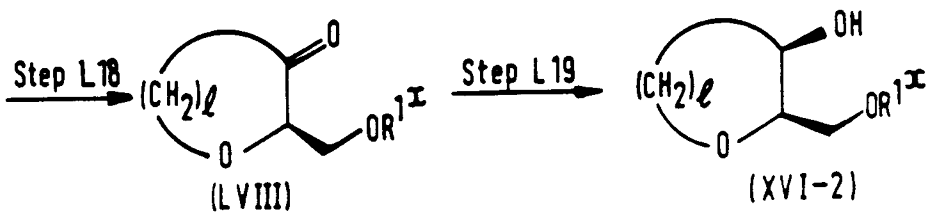
Step L18
(LVIII)
Step L19
(XVI-2)

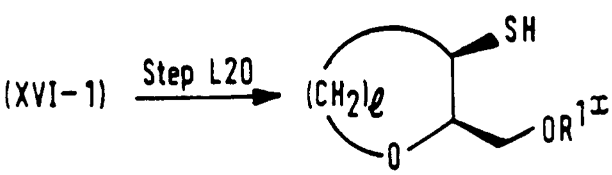
(XVI-1)
Step L20

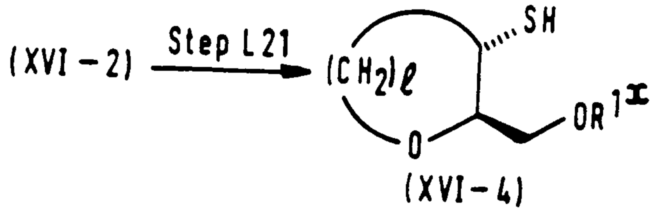


(XVI-2) (XVI-4)

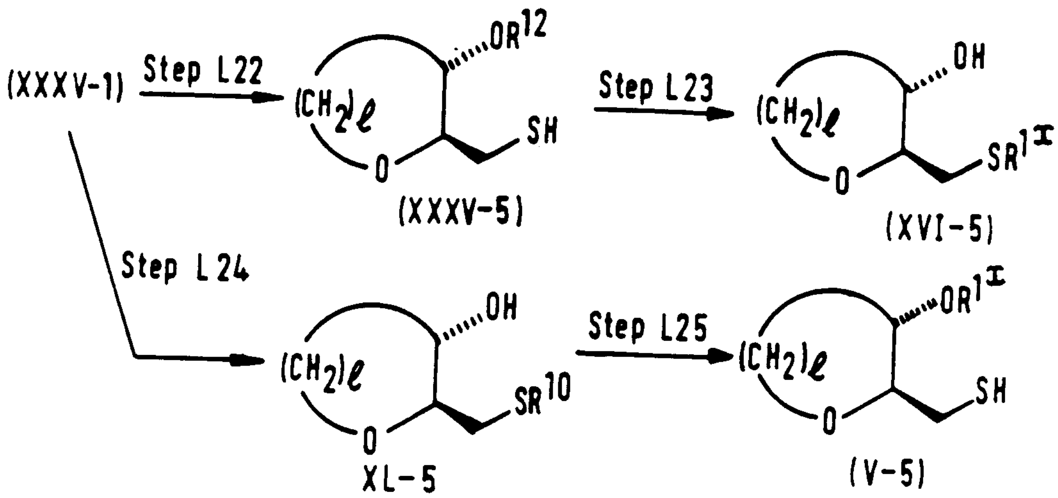


(XXXV-1) (XXXV-5) (XVI-5) XL-5 (V-5)

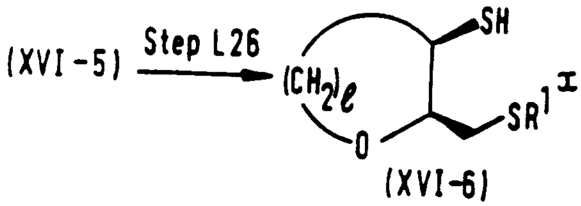


(XVI-5) (XVI-6)

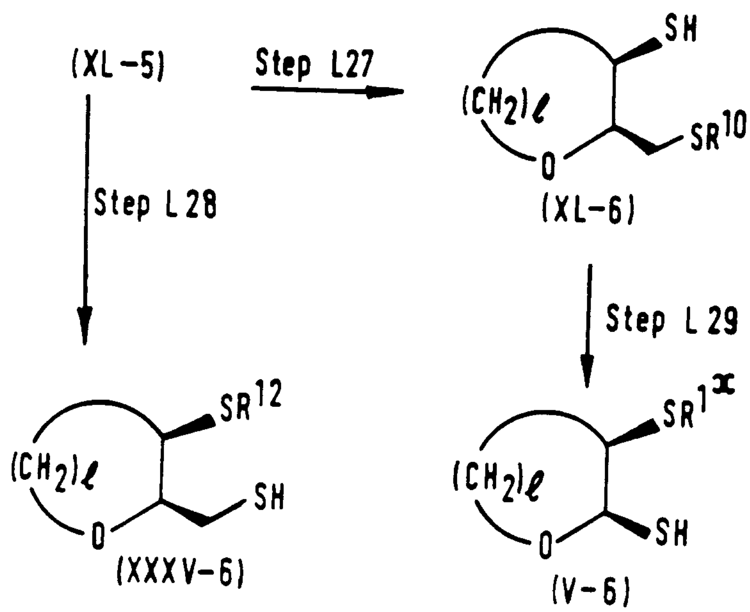


(XL-5) (XL-6) (XXXV-6) (V-6)

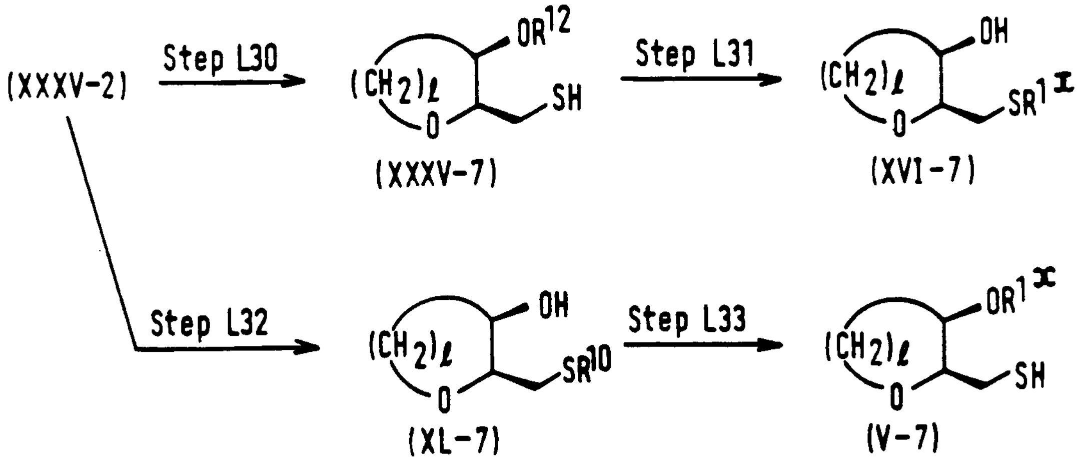


(XXXV-2) (XXXV-7) (XVI-7) (XL-7) (V-7)

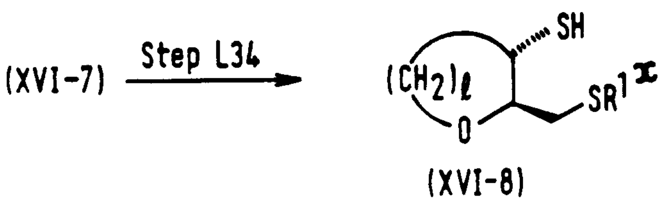


(XVI-7) (XVI-8)

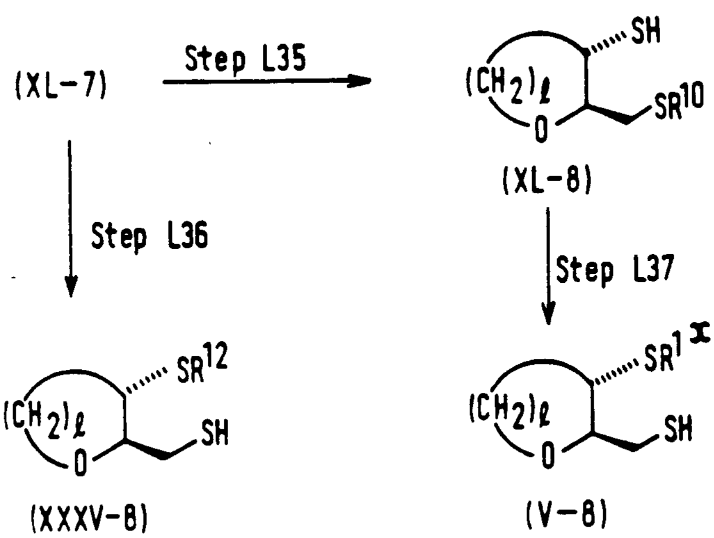


(XL-7) (XL-8) (XXXV-8) (V-8)

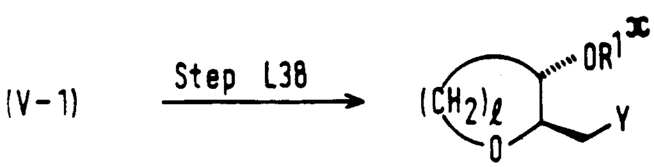


(V-1)

(V-2)

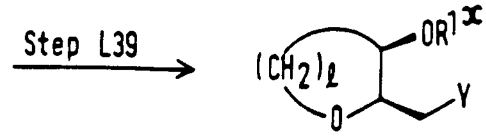


(X-2)

(V-3)

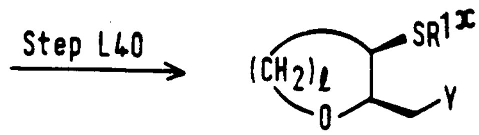


(X-3)

(V-4)

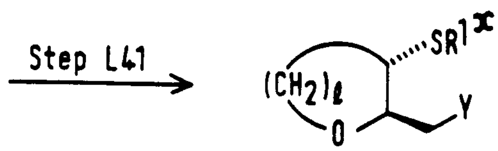


(X-4)

(XVI-1)

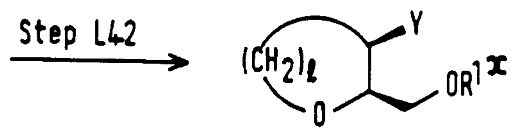


(XVII-1)

(XVI-2)

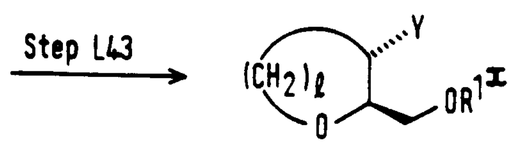


(XVII-2)

(XVI-5)

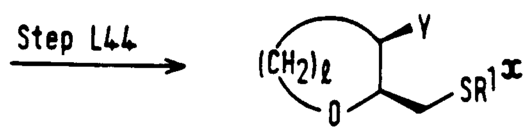


(XVII-3)

(XVI-7)

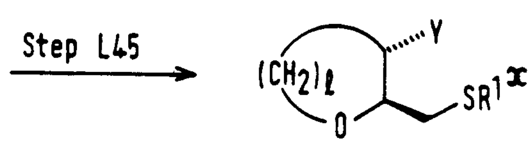


(XVII-4)

In the above formulae:

l, $R^{1x}$, $R^{10}$ and Y are as defined above; and

$R^{12}$ represents a hydroxy- or mercapto- protecting group, which may be selected from those groups defined above in relation to $R^{10}$, but (since it appears in the same compounds as $R^{10}$) should be removable independently of $R^{10}$.

Since many of the reactions involved in the above reaction schemes are repeated several times, the reactions may be summarised as follows:

Reaction 1:

In this reaction a compound of formula (LIV) is prepared by hydroxymethylation of a compound of formula (LIII). This reaction may be conducted according to the method described by A. Lebouc et al. [Synthesis, 610 (1979)].

Reaction 2:

In this reaction a compound of formula (LV) is prepared by protecting the hydroxy group of the compound of formula (LIV) with a protecting group $R^{10}$, which may be as described above. This reaction may be conducted by conventional methods for protecting a hydroxy group. When the substrate contains a mercapto group instead of a hydroxy group, for example, the mercapto group of a compound of formula (XL-3), this may be protected with a mercapto-protecting group as described above.

Reaction 3:

In this reaction a racemic compound of formula (XL-1) having trans hydroxy groups is prepared by hydroboration of the double bond of a compound of formula (LV), preferably using a borane as the hydroborating agent. The reaction can be carried out asymmetrically and an optically active compound is obtained by using for example, monoisopinocamphenylborane according to Brown's method [J. Org. Chem., 47, 5074 (1982)].

Reaction 4:

This reaction consists of alkylation, acylation or carbamation following the procedure described in the second part of Step A5. A mercapto group of, for example, a compound of formula (XL-3) can be similarly converted instead of the hydroxy group.

Reaction 5:

This reaction consists of deprotecting a protected hydroxy or mercapto group and may be carried out in a similar manner to that described in Step A3.

Reaction 6:

In this reaction a compound of formula (LVI) is prepared by oxidation of a compound of formula (XL-1) with Jones' reagent using chromic acid or pyridinium chlorochromate to convert a hydroxy group to a carbonyl group.

Reaction 7:

This reaction consists in the formation of a pair of hydroxy groups in cis relationship by stereo-selective reduction of a carbonyl group of a compound of formula (LVI) with L-selectride to give a compound of formula (XL-2).

Reaction 8:

This reaction is for preparing an ester of a compound of formula (XL-3) by acylation of a hydroxy group of a compound of formula (XL-1) following a procedure simto that described in Step A3, for example, by methanesulphonylation, toluenesulphonylation, trifluoromethanesulphonylation or trifluoroacetylation followed by converting the resulting acyloxy group to a protected thiol group having a sterically inverted configuration using for, example, thioacetic acid.

Reaction 9:

This reaction consists in the preparation of a compound of formula (XXXV-1) by protecting the free hydroxy group of a compound of formula (XL-1) with a protecting group, preferably a tetrahydropyranyl group, differing from the one described in Reaction 1.

Reaction 10:

This reaction consists in the preparation of a compound of formula (X-1) by converting the hydroxy group of a compound of formula (V-1) to a group Y following a procedure similar to that described in Step A3.

The Steps of the above reaction schemes L employing the reactions defined above are tabulated in the following Table 11.

Table 11

| Step | Reaction | Step | Reaction | Step | Reaction |
|---|---|---|---|---|---|
| L1 | 1 | L16 | 4 | L31 | 4, 5 |
| L2 | 2 | L17 | 3 | L32 | 8, 5 |
| L3 | 3 | L18 | 6 | L33 | 4, 5 |
| L4 | 9, 5 | L19 | 7 | L34 | 8, 5 |
| L5 | 4, 5 | L20 | 8, 5 | L35 | 8, 5 |
| L6 | 6 | L21 | 8, 5 | L36 | 8, 5 |
| L7 | 7 | L22 | 8, 5 | L37 | 4, 5 |
| L8 | 4, 5 | L23 | 4, 5 | L38 | 10 |
| L9 | 9, 5 | L24 | 8, 5 | L39 | 10 |
| L10 | 8, 5 | L25 | 4, 5 | L40 | 10 |
| L11 | 4, 5 | L26 | 8, 5 | L41 | 10 |
| L12 | 8, 5 | L27 | 8, 5 | L42 | 10 |
| L13 | 8, 5 | L28 | 8, 5 | L43 | 10 |
| L14 | 8, 5 | L29 | 4, 5 | L44 | 10 |
| L15 | 4, 5 | L30 | 8, 5 | L45 | 10 |

Method M

Optically active starting materials can be stereoselectively prepared from an optically active tartaric acid as shown in the following reaction schemes.

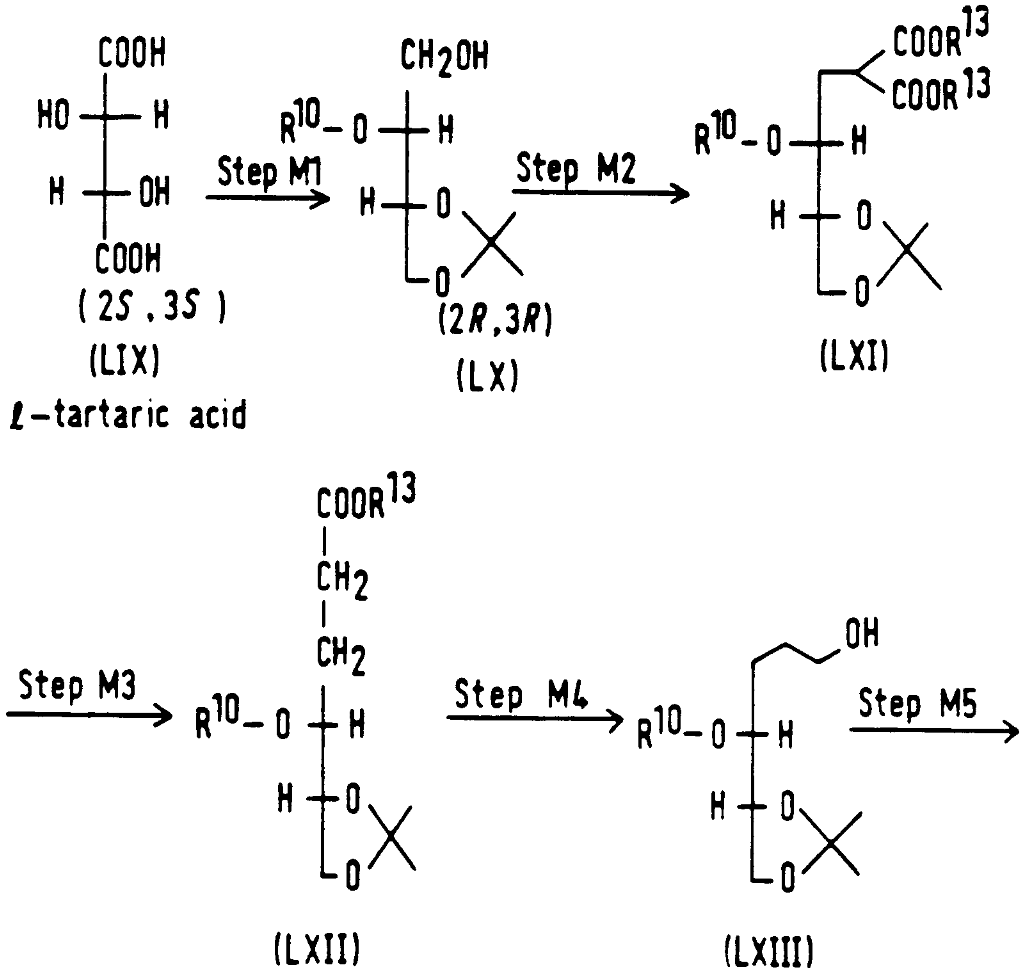


(2*S*,3*S*) (LIX) *l*-tartaric acid

(2*R*,3*R*) (LX)

(LXI)

(LXII)

(LXIII)

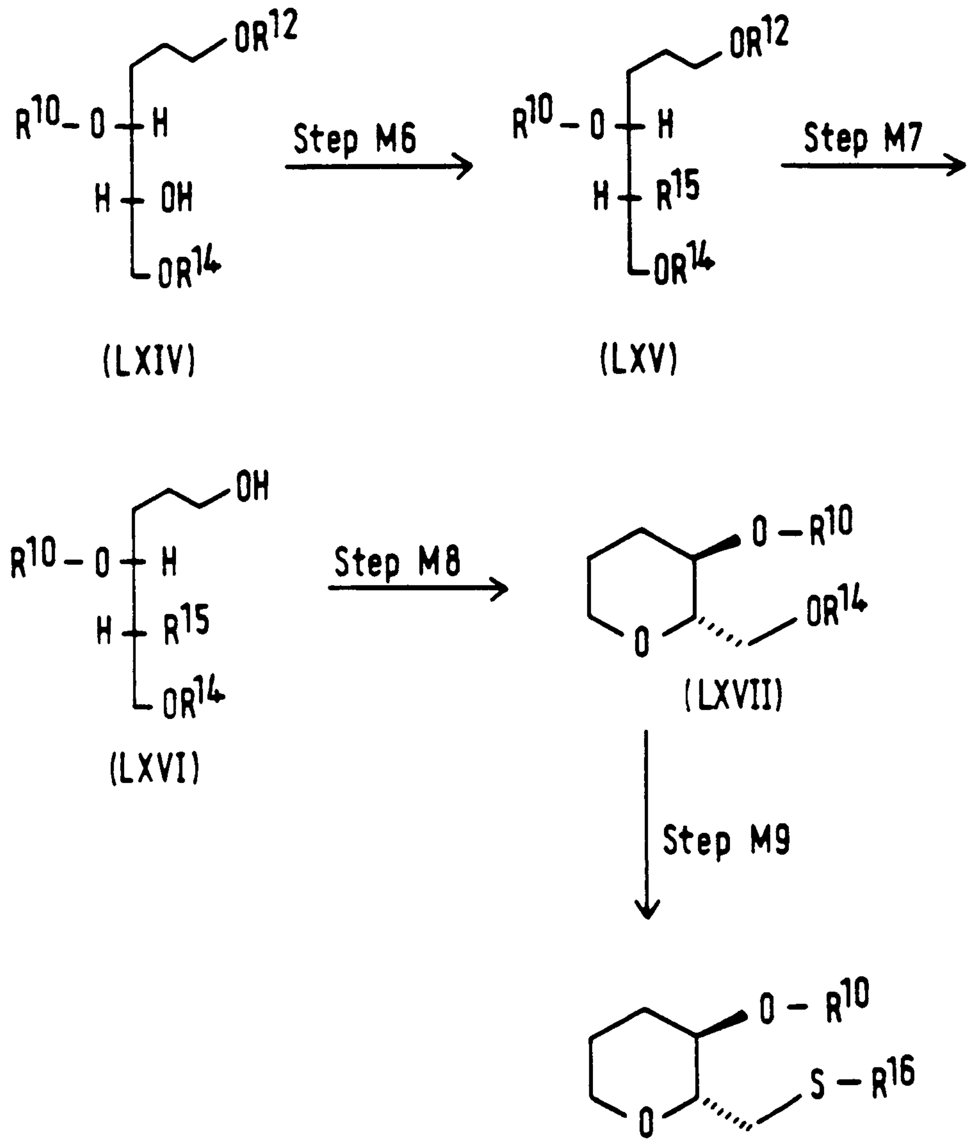


(LXIV) (LXV)

(LXVI) (LXVII)

(LXIX)

(LXIII)

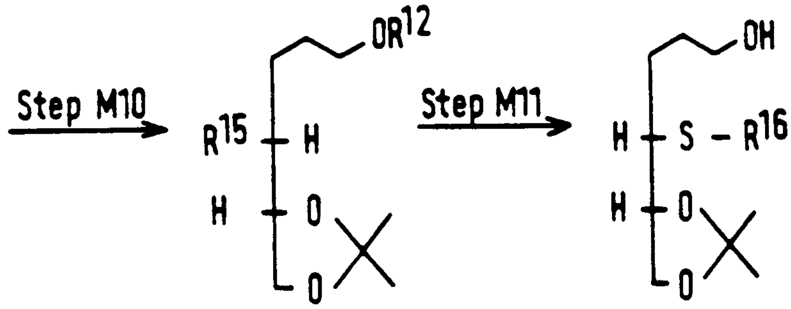


(LXX) (LXXI)

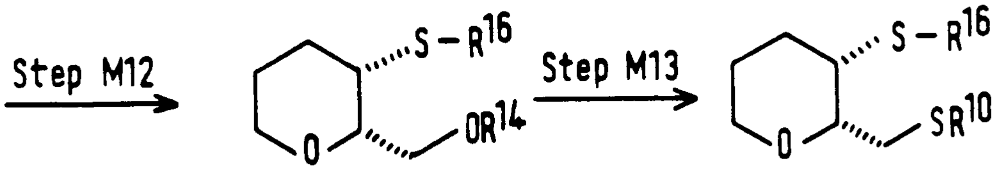


(LXXII) (LXXIII)

(LXIV)

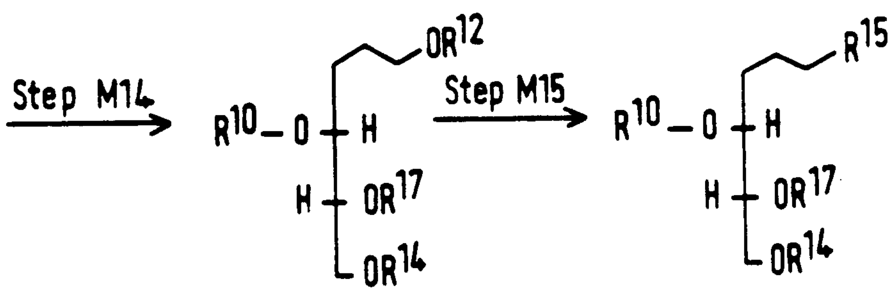


(LXXIV) (LXXV)

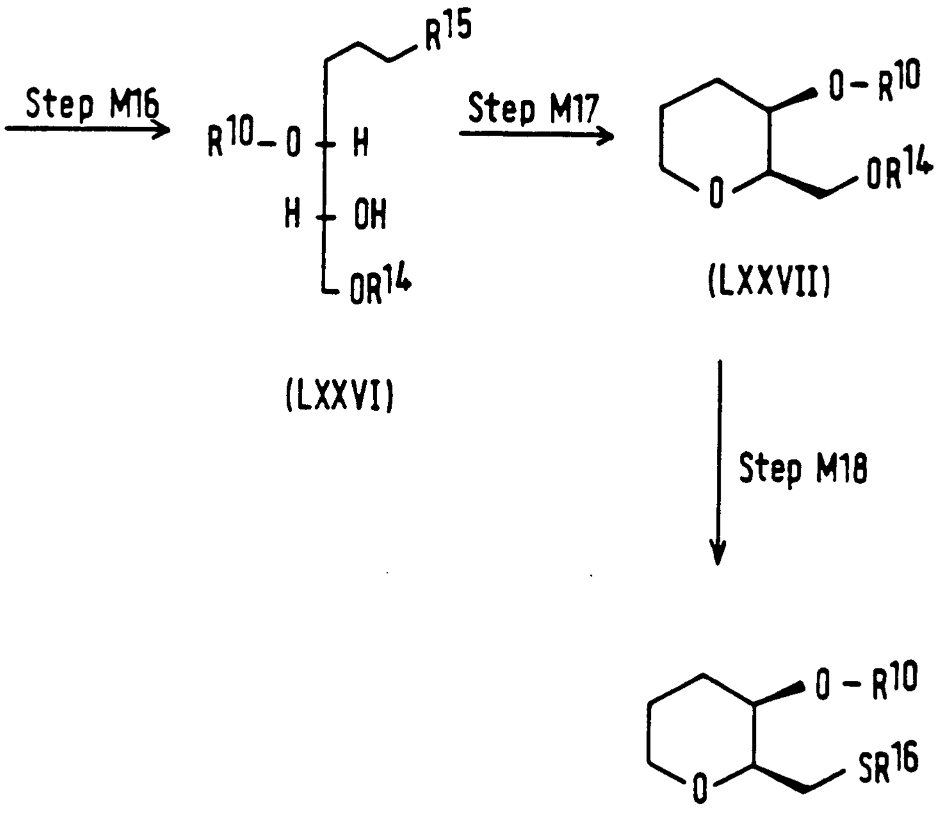


(LXXVI)

(LXXVII)

(LXXVIII)

(LXXI)

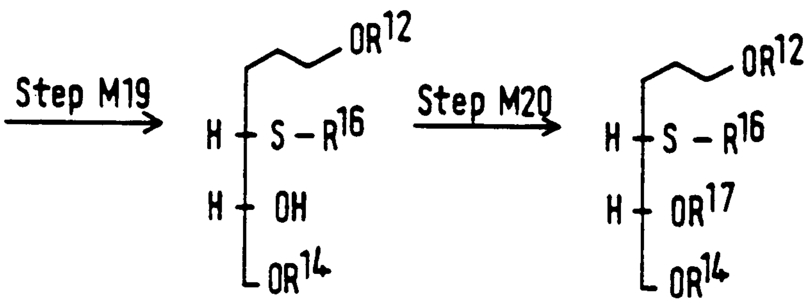


(LXXIX)

(LXXX)

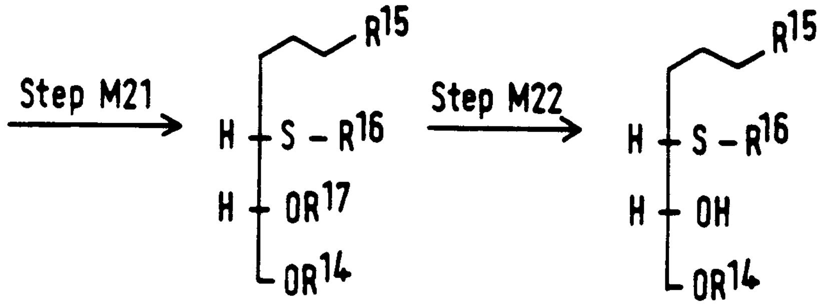


(LXXXI) (LXXXII)

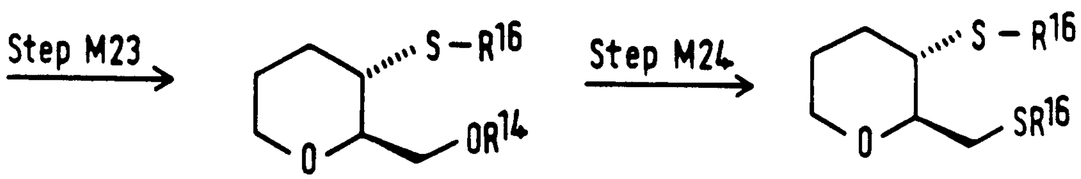


(LXXXIII) (LXXXIV)

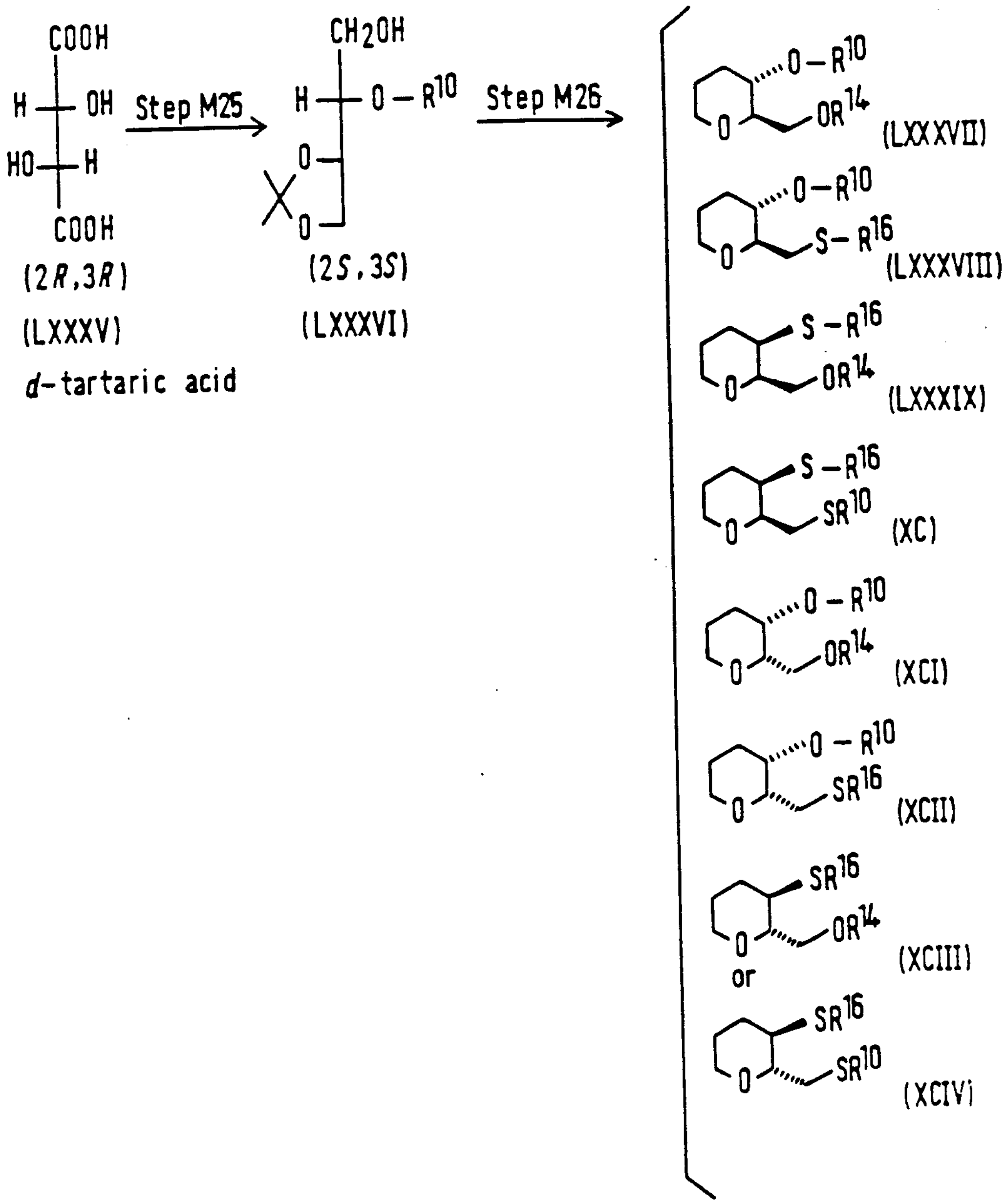


(2R,3R)
(LXXXV)
d-tartaric acid

(2S,3S)
(LXXXVI)

(LXXXVII)
(LXXXVIII)
(LXXXIX)
(XC)
(XCI)
(XCII)
(XCIII)
or
(XCIV)

(LX)

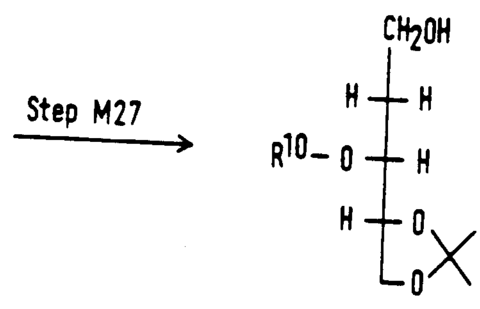


(XCV)

In the above formulae:

$R^{10}$ and $R^{12}$ are as defined above;

$R^{13}$ represents a lower (e.g. $C_1$ - $C_4$, more preferably $C_1$ or $C_2$) alkyl group;

$R^{14}$, $R^{16}$ and $R^{17}$ represent hydroxy- or mercapto-protecting groups, like those represented by $R^{10}$ and $R^{12}$, and may be selected from the protecting groups exemplified above for $R^{10}$ and $R^{12}$; preferably, $R^{10}$, $R^{12}$, $R^{14}$, $R^{16}$ $R^{17}$ are so selected that they can be selectively de-protected, when two or more among them are

used in a same molecule; and
$R^{15}$ represents a lower alkylsulphonyloxy group or an arylsulphonyloxy group similar to those defined above for Y.

Preferably, $R^{10}$ represents a benzyl group; $R^{12}$ represents a silyl group; $R^{14}$ represents a di- or triarylmethyl group; $R^{16}$ represents a lower aliphatic acyl group; and $R^{17}$ represents a tetrahydropyranyl group.

The starting material is a compound of formula (LIX), which is (2S, 3S), i.e. l-tartaric acid, which is reacted as described by Ohno et al. [Ohno et al., Tetrahedron Lett., 23, 3507 (1982)] in Step M1, to give a (2R, 3R) compound of formula (LX).

In Step M2, a compound of formula (LXI) is prepared by the acylation of the primary hydroxy group of the compound of formula (LX), following a procedure similar to that described in Step A3, followed by substituting the acyloxy group with iodine and reacting it with a di(lower alkyl) malonate.

A compound of formula (LXII) having two more carbon atoms than does the compound of formula (LX) can obtained by decarboxylation of the compound of formula (LXI) for example, by heating it in an aqueous dimethyl sulphoxide solution of sodium chloride (Step M3).

The alcoholic compound (LXIII) can be prepared by reducing the compound of formula (LXII) using a reducing agent such as lithium aluminium hydride in a suitable solvent, e.g. an ether such as those exemplified elsewhere herein (Step M4). After protection of the hydroxy group of the compound with a group of formula $R^{12}$ (preferably a diphenyl-t-butylsilyl group) (Step M5) followed by deprotection of the isopropylidene group, protection of the resulting primary hydroxy group with a group of formula $R^{14}$ - (preferably a triphenylmethyl group) and acylation of the resulting secondary hydroxy group in a similar manner to that described in Step A3 give rise to a compound of formula (LXV) (Step M6). An optically active compound (LXVII) can be prepared by deprotecting the hydroxy-protecting group, $R^{12}$, of the compound of formula (LXV) (Step M7) (using a fluoride anion when $R^{12}$ represents a silyl group), followed by cyclization accompanied by inversion of the steric configuration at the 2-position by treatment with a base (e.g. potassium t-butoxide in t-butanol) (Step M8).

In a similar manner to Reaction 8, the compound of formula (LXVII) can be converted to the corresponding mercapto compound (LXIX) with retention of its steric configuration (Step M9).

Alternatively, the compound of formula (LXIII) can be converted to a compound having a protected thiol group with inversion of steric configuration (Steps M10 and M11).

A compound of formula (LXXII) having optical activity can be prepared from a compound of formula (LXXI) following a similar procedure to that described in Steps M5 - M8 (Step M12).

A compound of formula (LXXIII) having optical activity can then be prepared from a compound of formula (LXXII) by following a similar procedure to that described in Step M9 (Step M13).

After protection of the secondary hydroxy group of the compound of formula (LXIV) with a group of formula $R^{17}$ (Step M14) and selective deprotection of the protecting group $R^{12}$, a compound of formula (LXXV) can be prepared by acylation of the resulting hydroxy group by a similar method to that described in Step A3 (Step M15).

An optically active compound of formula (LXXVII) can be prepared with retention of steric configuration by deprotecting a hydroxy-protecting group, $R^{17}$, of a secondary hydroxy group of a compound of formula (LXXV) (Step M16) followed by carrying out a similar method to that described in Step M8 (Step M17). Then by following a similar procedure to that described in Step M9, a mercapto compound (LXXVIII) can be prepared (Step M18).

A compound of formula (LXXIX) can be prepared from a compound of formula (LXXI) in a similar manner to that described in Step M5 (Step M19).

A compound of formula (LXXX) can then be prepared from the compound of formula (LXXIX) in a similar manner to that described in Step M14 (Step M20).

A compound of formula (LXXXI) can be prepared from the compound of formula (LXXX) in a similar manner to that described in Step M15 (Step M21).

A compound of formula (LXXXII) can be prepared from the compound of formula (LXXXI) in a similar manner to that described in Step M16 (Step M22).

An optically active compound of formula (LXXXIII) can be prepared from the compound of formula (LXXXII) in a similar manner to that described in Step M17 (Step M23).

An optically active compound of formula (LXXXIV) can be prepared from the compound of formula (LXXXIII) in a similar manner to that described in Step M9 (Step M24).

A (2S, 3S) compound of formula (LXXXVI) can be prepared from d-tartaric acid, which has the formula (LXXXV) and has the (2R, 3R) configuration as a starting material (Step M25), in a manner similar to that described in Step M1.

Optically active compounds of formula (LXXXVII -XCIV) can be prepared from the compound (LXXXVI) by the similar methods to those described in Steps M2 - M24 (Step M26).

On the other hand, starting materials having a 5-membered ring (l = 2) for preparing compounds of formula (I) in which l = 2 can be prepared by synthesizing a cyano compound using a metal cyanide in place of the malonate in Step M2, followed by alcoholysis by conventional means to give an ester. An alcohol compound of formula (XCV), which has one more carbon atom than the compound of formula (LX), prepared from this ester by reduction can then be converted to the corresponding optically active compounds using the methods described in Steps M5 - M24.

A starting material having a 7-membered ring (l = 4) for preparing a compound of formula (I) in which l = 4 can be converted to the corresponding optically active compounds and may be prepared from a compound of formula (XCV) as a starting material by using the methods described in Steps M2 - M24.

A starting material for use in the invention can be prepared by deprotection of either protecting group of the optically active compound prepared above, followed by alkylation, acylation or carbamation by Reaction 4, as described in Step A5.

The compounds of the present invention have shown excellent PAF antagonistic activity and anti-inflammatory activity, in terms of the duration of the effect and/or bioavailability. They are, accordingly, useful as a new type of anti-shock agent, anti-thrombotic agent, anti-asthmatic agent, anti-allergic agent and anti-inflammatory agent.

The compounds of the invention may be administered orally or parenterally as required and may, if desired, be formulated into appropriate pharmaceutical formulations, depending upon the desired route of administration. For example, for oral administration, the compounds may be formulated as tablets, capsules, granules, powders or syrups. For parenteral administration, they may be formulated as injectible solutions or suspensions or as suppositories. Although the preferred dose will vary, depending upon the nature of the disorder, the symptoms, age, condition and body weight of the patient and the route of administration, a preferred dose for an adult human patient would normally be expected to be from 0.1 to 200 mg/kg body weight per day, and this could be administered in a single dose or in divided doses.

The invention is further illustrated by the following non-limiting Examples. Preparation of certain of the starting materials employed in these Examples is illustrated by the subsequent Preparations. The biological activities of certain of the compounds of the invention are then illustrated in the subsequent Experiments. In the Examples and Preparations, values of optical rotation were measured using the sodium D-line, i.e. all are $[\alpha]_D$.

## EXAMPLE 1

### dl-3-[7-(trans-3-Hexadecyloxytetrahydropyran-2-ylmethoxy)heptyl]thiazolium methanesulphonate

(a) A solution of 0.51 ml of methanesulphonyl chloride dissolved in 5 ml of benzene was added dropwise to a solution of 2.067 g of dl-7-(trans-3-hexadecyloxytetrahydropyran-2-ylmethoxy)-1-heptanol (prepared as described in Preparation 55) and 1.83 ml of triethylamine dissolved in 15 ml of benzene, whilst ice-cooling. The reaction mixture was stirred at room temperature for 15 minutes, after which it was washed with water, dried over anhydrous magnesium sulphate and concentrated by evaporation under reduced pressure, to give 2.406 g of crude dl-7-(trans-3-hexadecyloxytetrahydropyran-2-ylmethoxy)heptyl methanesulphonate as a viscous oil.

(b) 1.20 g of the methanesulphonate [prepared as described in step (a) above] and 1.56 ml of thiazole were dissolved in 3 ml of toluene, and the solution was stirred on an oil bath kept at 70 °C for 5 days. At the end of this time, the mixture was allowed to cool, the solvent was distilled off under reduced pressure, and the residue was subjected to column chromatography through 40 g of silica gel. 0.741 g of the title compound was obtained as a viscous oil from the fractions eluted with a 60 : 35 : 5 by volume mixture of methylene chloride, methanol and water.

Nuclear Magnetic Resonance Spectrum (90 MHz, $CDCl_3$) $\delta$ ppm:

0.7 - 2.45 (45H, multiplet);
2.77 (3H, singlet);
2.95 - 4.05 (10H, multiplet);
4.75 (2H, triplet, J = 7.5 Hz);
8.4 - 8.6 (2H, muliplet);
10.91 (1H, multiplet).

EXAMPLE 2

dl-3-[7-(cis-3-Hexadecyloxytetrahydropyran-2-ylmethoxy)heptyl]thiazolium methanesulphonate

In a similar manner to that described in Example 1(a), 1.42 g of crude dl-7-(cis-3-hexadecyloxytetrahydropyran-2-ylmethoxy)heptyl methanesulphonate was obtained as a viscous oil from 1.215 g of dl-7-(cis-3-hexadecyloxytetrahydropyran-2-ylmethoxy)-1-heptanol (prepared as described in Preparation 58). 0.71 g of the resulting oily product was treated in a similar manner to that described in Example 1(b), to give 0.322 g of the title compound as viscous oil.
Nuclear Magnetic Resonance Spectrum (90 MHz, $CDCl_3$) δ ppm;
0.75 - 2.30 (45H, multiplet);
2.77 (3H, singlet);
3.1 - 3.75 (9H, multiplet);
3.85 - 4.20 (1H, multiplet);
4.77 (2H, triplet, J = 7.5 Hz);
8.4 - 8.7 (2H, multiplet);
10.97 (1H, multiplet).

| Elemental analysis: | | | | |
|---|---|---|---|---|
| Calculated for $C_{33}H_{63}NO_6S_2 . H_2O$: | C, 60.79%; | H, 10.05%; | N, 2.15%; | S, 9.83%. |
| Found : | C, 60.39%; | H, 9.94%; | N, 2.16%; | S, 9.52%. |

EXAMPLE 3

dl-3-[5-(trans-3-Hexadecyloxytetrahydropyran-2-ylmethoxycarbonylamino)pentyl]thiazolium bromide

1.84 ml of thiazole was added to a solution of 711 mg of dl-trans-3-hexadecyloxytetrahydropyran-2-ylmethyl N-(5-bromopentyl)carbamate (prepared as described in Preparation 47) dissolved in 2 ml of toluene, and the mixture was heated at 80 °C for 86 hours. At the end of this time, the reaction mixture was evaporated to dryness under reduced pressure, and the residue was subjected to column chromatography through 17 g of silica gel. Those fractions eluted with mixtures of methylene chloride and methanol ranging from 19 : 1 to 17 : 3 by volume were collected and then subjected to medium pressure liquid chromatography through a Lobar B column. 480 mg of the title compound were obtained as a powder from the fractions eluted with the same solvent mixtures.
Nuclear Magnetic Resonance Spectrum (90 MHz, $CD_3OD$) δ ppm:
0.7 - 2.4 (41H, multiplet);
2.9 - 4.5 (10H, multiplet);
4.62 (2H, triplet, J = 7 Hz);
8.31 (1H, doublet, J = 4 Hz);
8.52 (1H, doublet, J = 4 Hz).
Infrared Absorption Spectrum ($CHCL_3$) $\nu_{max}$ $cm^{-1}$:
3470 (-NH) and 1710 (-O-CO-).

EXAMPLE 4

dl-3-[5-(trans-3-Heptadecylcarbamoyloxytetrahydropyran-2-ylmethoxycarbonylamino)pentyl]thiazolium bromide

400 mg of dl-trans-2-[N-(5-bromopentyl)carbamoyloxymethyl]tetrahydropyran-3-yl N-heptadecylcarbamate (prepared as described in Preparation 6) were dissolved in 1 ml of toluene, and then 0.47 ml of thiazole was added to the resulting mixture, after which the mixture was heated at 80 °C for 66 hours. The reaction mixture was then evaporated to dryness under reduced pressure, and the residue was subjected to column chromatography through 10 g of silica gel. 390 mg of the title compound, melting at 54 - 56 °C, were obtained as a powder from those fractions eluted with mixtures of methylene chloride and methanol ranging from 19 : 1 to 4 : 1 by volume.
Nuclear Magnetic Resonance Spectrum

(270 MHz, $CD_3OD$) δ ppm:

0.90 (3H, triplet, J = 7 Hz);
1.20 - 1.80 (37H, multiplet);
2.02 (2H, quintet, J = 7 Hz);
2.10 - 2.30 (1H, multiplet);
3.00 - 3.20 (4H, multiplet);
3.30 - 3.50 (2H, multiplet);
3.85 - 3.95 (1H, multiplet);
4.05 (1H, doublet of doublets, J = 11 & 6 Hz);
4.17 (1H, doublet of doublets, J = 11 & 1 Hz);
4.49 (1H, ddd, J = 10, 10 & 5 Hz);
4.61 (2H, triplet, J = 7 Hz);
8.30 (1H, doublet, J = 4 Hz);
8.50 (1H, doublet, J = 4 Hz).

| Elemental analysis: | | | | |
|---|---|---|---|---|
| Calculated for $C_{33}H_{60}BrN_3O_5S$ . 1.5 $H_2O$:<br>Found : | C, 55.22%;<br>C, 55.22%; | H, 8.85%;<br>H, 8.56%; | N, 5.85%;<br>N, 5.73%; | S, 4.47%.<br>S, 4.22%. |

EXAMPLE 5

3-{5-[2S, 3R)-3-Heptadecylcarbamoyloxytetrahydropyran-2-yl)methoxycarbonylamino]pentyl}thiazolium bromide

Following a procedure similar to that described in Example 4, 553.0 mg of the title compound, melting at 97.0-99.0 °C, were obtained as a white powder starting from 564.0 mg of (2S, 3R)-2-[N-(5-bromopentyl)-carbamoyloxymethyl]tetrahydropyran-3-yl N-heptadecylcarbamate (prepared as described in Preparation 31) and 0.66 ml of thiazole.
$[\alpha]^{25}$ -27.3 ° (c = 1.05, methanol).
FAB Mas Spectrum (m/e): 610 ($M^+$ - $Br^-$)
[FAB is Fast Atom Bombardment].

| Elemental analysis: | | | | |
|---|---|---|---|---|
| Calculated for $C_{33}H_{60}BrN_3O_5S$ . 1.2 $H_2O$):<br>Found : | C, 55.63%;<br>C, 55.58%; | H, 8.83%;<br>H, 8.62%; | N, 5.90%;<br>N, 5.78%; | S, 4.50%.<br>S, 4.36%. |

EXAMPLE 6

3-{5-(2R, 3S)-3-Heptadecylcarbamoyloxytetrahydropyran-2-yl)methoxycarbonylamino]pentyl}thiazolium bromide

Following a procedure similar to that described in Example 4, 523.8 mg of the title compound, melting at 97.0 - 99.0 °C, were obtained as a white powder starting from 540.0 mg of (2R, 3S)-2-[N-(5-bromopentyl)-carbamoyloxymethyl]tetrahydropyran-3-yl N-heptadecylcarbamate (prepared as described in Preparation 32) and 0.63 ml of thiazole.
$[\alpha]^{25}$ +27.2 ° (c = 1.05, methanol).
FAB Mass Spectrum (m/e): 610 ($M^+$ - $Br^-$).

| Elemental analysis: | | | | |
|---|---|---|---|---|
| Calculated for $C_{33}H_{60}BrN_3O_5S$ . 1.5 $H_2O$:<br>Found: | C, 55.22%;<br>C, 55.18%; | H, 8.85%;<br>H, 8.40%; | N, 5.85%;<br>N, 5.86%; | S, 4.47%.<br>S, 4.32%. |

EXAMPLE 7

dl-3-{5-[(cis-3-Heptadecylcarbamoylthiotetrahydropyran-2-yl)methoxycarbonylamino]pentyl}thiazolium bromide

600 mg of dl-[cis-3-(N-heptadecylcarbamoylthio)-tetrahydropyran-2-ylmethyl] N-(5-bromopentyl)-carbamate (prepared as described in Preparation 42) were dissolved in 1 ml of toluene, and then 0.68 ml of thiazole were added to the resulting mixture. The mixture was then heated on an oil bath kept at 80 °C for 64 hours. At the end of this time, the reaction mixture was evaporated to dryness under reduced pressure, and the residue was subjected to column chromatography through 15 g of silica gel. 595 mg of the title compound were obtained as a white powder, melting at 122 - 125 °C, from the fractions eluted with mixtures of methylene chloride and methanol ranging from 9 : 1 to 4 : 1 by volume.

Nuclear Magnetic Resonance Spectrum (90 MHz, $CD_3OD$) $\delta$ ppm:

0.7 - 2.3 (43H, multiplet);
2.9 - 4.2 (10H, multiplet);
4.67 (2H, triplet, J = 7 Hz);
8.34 (1H, doublet, J = 4 Hz);
8.56 (1H, doublet, J = 4 Hz).

Infrared Absorption Spectrum (KBr) $\nu_{max}$ $cm^{-1}$:

3320 (-NH), 1700 (-O-CO-) and 1640 (-S-CO-).

| Elemental analysis: | | | | |
|---|---|---|---|---|
| Calculated for $C_{33}H_{60}BrN_3O_4S_2$ . 1.5 $H_2O$: | C, 54.01%; | H, 8.65%; | N, 5.73%; | S, 8.74%. |
| Found : | C, 54.29%; | H, 8.21%; | N, 5.74%; | S, 8.36%. |

EXAMPLE 8

dl-3-{5-[(cis-3-Heptadecylcarbamoyloxytetrahydropyran-2-yl)methoxycarbonylamino]pentyl}thiazolium bromide

Following a procedure similar to that described in Example 4 but using 550.2 mg of dl-cis-2-[N-(5-bromopentyl)carbamoyloxy]methyltetrahydropyran-3-yl N-heptadecylcarbamate (prepared as described in Preparation 67) and 0.65 ml of thiazole, 526.9 mg of the title compound was obtained as a white powder, melting at 115 - 120 °C.

Nuclear Magnetic Resonance Spectrum (90 MHz, $CD_3OD$ + $CDCl_3$ = 1 : 1 by volume) $\delta$ ppm:

0.7 - 2.3 (43H, multiplet);
3.0 - 3.2 (4H, multiplet);
3.4 - 3.8 (2H, multiplet);
3.9 - 4.2 (3H, multiplet);
4.66 (2H, triplet, J = 7 Hz);
4.7 - 4.9 (1H, multiplet);
8.28 (1H, doublet, J = 4 Hz);
8.52 (1H, doublet, J = 4 Hz).

Infrared Absorption Spectrum ($CHCl_3$) $\nu_{max}$ $cm^{-1}$:

3460 (-NH-), 1705, 1695 (-O-CO-).

| Elemental Analysis: | | | | | |
|---|---|---|---|---|---|
| Calculated for $C_{33}H_{60}BrN_3O_5S.3H_2O$: | C, 53.21%; | H, 8.12%; | Br, 13.48%; | N, 5.64%; | S, 3.30%. |
| Found: | C, 53.31%; | H, 7.99%; | Br, 13.78%; | N, 5.51%; | S, 3.59%. |

EXAMPLE 9

dl-3-{6-Ethoxycarbonyl-6-[(trans-3-heptadecylcarbamoyloxytetrahydropyran-2-yl)methoxycarbonylamino]-hexyl}thiazolium methanesulphonate

0.099 g of methanesulphonyl chloride was added, whilst ice-cooling, to a solution of 0.363 g of ethyl 2-[-(dl-trans-3-heptadecylcarbamoyloxytetrahydropyran-2-yl)methoxycarbonylamino]-5-hydroxyheptanoate (prepared as described in Preparation 64) and 0.16 ml of triethylamine in 5 ml of benzene. The mixture was then stirred at room temperature for 15 minutes. At the end of this time, the reaction mixture was washed with water, dried over anhydrous magnesium sulphate and concentrated by evaporation under reduced pressure. The resulting oily residue (0.357 g) and 0.41 ml of thiazole were dissolved in 2.0 ml of toluene, and the mixture was heated for 4 days on an oil bath kept at 90°, whilst stirring. The solvent was then distilled off under reduced pressure, and the residue was subjected to column chromatography through 30 g of silica gel, eluted with a gradient system of methylene chloride and methanol ranging from 3 : 1 to 1 : 1 by volume, to give 0.205 g of the title compound as a white powder, melting at 52 - 60°C.

Nuclear Magnetic Resonance Spectrum
(270 MHz, $CD_3OD$) $\delta$ ppm:

0.90 (3H, triplet, J=7.0 Hz);
1.25 (3H, triplet, J=7.0 Hz);
1.3 - 1.9 (39H, multiplet);
2.01 (2H, multiplet);
2.20 (1H, multiplet);
2.70 (3H, singlet);
3.06 (2H, triplet, J=6.9 Hz);
3.44 (2H, multiplet);
3.89 (1H, multiplet);
4.0 - 4.3 (2H, multiplet);
4.16 (2H, quartet, J=7.0 Hz);
4.49 (1H, multiplet);
4.60 (2H, triplet, J=7.5 Hz);
8.29 (1H, doublet, J=3.7 Hz);
8.50 (1H, doublet, J=3.7 Hz).

| Elemental Analysis: | | | |
|---|---|---|---|
| Calculated for $C_{33}H_{69}N_3O_{10}S_2$: | C, 57.61%; | H, 8.78%; | N, 5.30%. |
| Found: | C, 57.57%; | H, 8.94%; | N, 5.17%. |

EXAMPLE 10

S-{dl-cis-2-[N-(2-Pyridylmethyl)carbamoyloxy]methyltetrahydropyran-3-yl} N-(heptadecyl)thiocarbamate

A solution of 0.778 g of phenyl chlorocarbonate in 8 ml of methylene chloride was added to a solution of 1.424 g of S-[dl-(cis-2-hydroxymethyltetrahydropyran-3-yl)]N-(heptadecyl)thiocarbamate (prepared as described in Preparation 41) and 0.54 ml of pyridine in 20 ml of methylene chloride. The mixture was then stirred at room temperature for 1 hour. At the end of this time, the reaction mixture was poured into water and extracted three times with methylene chloride. The combined extracts were washed, in turn, with 10% w/v aqueous hydrochloric acid, a saturated aqueous solution of sodium bicarbonate and a saturated aquous solution of sodium chloride, and they were then dried over anhydrous magnesium sulphate. The solvent was then removed from the reaction mixture by distillation under reduced pressure, to leave 2.01 g of the crude carbonate, which was dissolved in 28 ml of chloroform. 0.68 ml of 2-(aminomethyl)pyridine was added to the resulting solution, and the mixture was heated under reflux for 44 hours. At the end of this time, the reaction mixture was evaporated to dryness under reduced pressure, and the residue was subjected to column chromatography through 40 g of silica gel. Those fractions eluted with a gradient system of hexane and ethyl acetate ranging from 2 : 1 to 0 : 1 by volume were collected and then reprecipitated from a mixture of hexane and methylene chloride to give 1.675 g of the title compound as a white solid, melting at 88 - 90°C.

Nuclear Magnetic Resonance Spectrum
(90 MHz, $CDCl_3$) δ ppm:
0.7 - 2.2 (37H, multiplet);
3.1 - 4.3 (8H, multiplet);
3.26 (2H, quartet);
4.51 (2H, doublet, J = 6 Hz);
5.38 (1H, multiplet);
5.82 (1H, multiplet);
7.23, 7.67 and 8.56 (4H, multiplet).
Infrared Absorption Spectrum ($CHCl_3$) $\nu_{max}$ $cm^{-1}$:
3440 (-NH-), 1720 (-CO-), 1670 (-S-CO-).

| Elemental Analysis: | | | | |
|---|---|---|---|---|
| Calculated for $C_{31}H_{53}N_3O_4S$: | C, 66.04%; | H, 9.47%; | N, 7.45%; | S, 5.69%. |
| Found; | C, 66.12%; | H, 9.38%; | N, 7.53%; | S, 5.63%. |

EXAMPLE 11

S-{dl-cis-2-[N-Acetyl-N-(2-pyridylmethyl)carbamoyloxy]methyltetrahydropyran-3-yl} N-acetyl-N-(heptadecyl)thiocarbamate

A solution of 1.632 g of S-{dl-cis-2-[N-(2-pyridylmethyl)carbamoyloxy]methyltetrahydropyran-3-yl}N-(heptadecyl)thiocarbamate (prepared as described in Example 10), 3.54 g of 4-dimethylaminopyridine and 2.73 ml of acetic anhydride in 32 ml of toluene was heated at 80 °C for 65 hours, whilst stirring. At the end of this time, the reaction mixture was evaporated to dryness under reduced pressure, and the residue was subjected to column chromatography through 40 g of silica gel and then to medium pressure liquid chromatography through a Lobar B column. Those fractions eluted with a 15 : 5 : 4 by volume mixture of hexane, methylene chloride and ethyl acetate were collected to give 0.493 g of the title compound as an oil.
Nuclear Magnetic Resonance Spectrum (60 MHz, $CDCl_3$) δ ppm:
0.7 - 2.2 (37H, multiplet);
2.39 (3H, singlet);
3.58 (3H, singlet);
3.0 - 4.4 (8H, multiplet);
5.08 (2H, singlet);
7.13, 7.63 and 8.53 (4H, multiplet).

EXAMPLE 12

S-{dl-cis-2-[N-(2-Pyridylmethyl)carbamoyloxymethyl]tetrahydropyran-3-yl} N-acetyl-N-(heptadecyl)-thiocarbamate

After the elution of the fraction containing the compound of Example 11, a further elution was carried out using a 1 : 1 : 1 by volume mixture of hexane, methylene chloride and ethylacetate to give an oil. This oil was further purified by column chromatography through a Lobar B column using the same solvent system as above to give 0.379 g of the title compound
Nuclear Magnetic Resonance Spectrum
(60 MHz, $CDCl_3$) δ ppm:
0.7 - 2.2 (37H, multiplet);
2.41 (3H, singlet);
3.2 - 4.6 (10H, multiplet);
5.90 (1H, multiplet);
7.25 (2H, multiplet);
7.67 (1H, multiplet);
8.56 (1H, multiplet).

EXAMPLE 13

dl-1-Ethyl-2-{N-acetyl-N-[cis-3-(N-acetyl-N-heptadecylcarbamoylthio)tetrahydropyran-2-yl]-methoxycarbonyl}aminomethylpyridinium chloride

A mixture of 0.493 g of S-{dl-cis-2-[N-acetyl-N-(2-pyridylmethyl)carbamoyloxy]methyltetrahydropyran-3-yl}N-acetyl-N-(heptadecyl)thiocarbamate (prepared as described in Example 11) and 10 ml of ethyl iodide was heated under reflux for 91 hours. At the end of this time, the reaction mixture was evaporated to dryness under reduced pressure, and the residue was dissolved in 70% v/v aqueous methanol. The resulting solution was passed through a column packed with 44 ml of an ion exchange resin (IRA-410, $Cl^-$ form, Rohm & Haas), and the column was washed with 70% v/v aqueous methanol. The eluate and the washings were combined and then concentrated by evaporation under reduced pressure, to give a crude chloride. This crude chloride was subjected to column chromatography through 10 g of silica gel and then to medium pressure liquid chromatography through a Lobar B Column.

The fraction eluted with a 9 : 1 by volume mixture of methylene chloride and methanol was collected to give 0.368 g of the title compound.

Nuclear Magnetic Resonance Spectrum
(60 MHz, $CD_3OD$) $\delta$ ppm:

0.7 - 2.2 (40H, multiplet);
1.66 (3H, triplet, J = 7 Hz);
2.37 (3H, singlet);
2.61 (3H, singlet);
3.1 - 4.4 (8H, multiplet);
4.77 (2H, quartet J = 7 Hz);
5.40 (2H, singlet);
7.98 (2H, multiplet);
8.53 (1H, multiplet);
9.03 (1H, multiplet).

EXAMPLE 14

dl-trans-2-[N-(2-Pyridylmethyl)carbamoyloxymethyl]-tetrahydropyran-3-yl N-heptadecylcarbamate

A solution of 0.795 g of phenyl chlorocarbonate in 8 ml of methylene chloride was added to a solution of 1.400 g of dl-(trans-2-hydroxymethyltetrahydropyran-3-yl) N-heptadecylcarbamate (prepared as described in Preparation 4) and 0.55 ml of pyridine in 20 ml of methylene chloride, and then the mixture was stirred at room temperature for 1 hour. At the end of this time, the reaction mixture was poured into water and extracted three times with methylene chloride. The combined extracts were washed, in turn, with 10% w/v aqueous hydrochloric acid, a saturated aqueous solution of sodium bicarbonate and a saturated aqueous solution of sodium chloride and were then dried over anhydrous magnesium sulphate.

The solvent was then distilled off under reduced pressure, to leave 2.04 g of a crude carbonate. The whole of this crude carbonate was dissolved in 28 ml of chloroform, and then 0.70 ml of 2-(aminomethyl)-pyridine was added to the solution. The resulting mixture was heated under reflux for 49 hours. At the end of this time, the reaction mixture was evaporated to dryness under reduced pressure, and the residue was subjected to column chromatography through 40 g of silica gel. Those fractions eluted with a gradient system of hexane and ethyl acetate ranging from 1 : 1 to 0 : 1 by volume were collected and reprecipitated from a mixture of hexane and methylene chloride, to give 1.663 g of the title compound as a white solid, melting at 78 - 80 °C.

Nuclear Magnetic Resonance Spectrum
(90 MHz, $CDCl_3$) $\delta$ ppm:

0.7 - 2.4 (37H, multiplet);
2.9 - 3.7 (4H, multiplet);
3.7 - 4.9 (7H, multiplet);
5.87 (1H, multiplet);
7.22 (2H, multiplet);
7.66 (1H, multiplet);
8.54 (1H, multiplet).

Mass Spectrum (m/e): 547 ($M^+$).

| Elemental Analysis: | | | |
|---|---|---|---|
| Calculated for $C_{31}H_{53}N_3O_5$: | C, 67.97%; | H, 9.75%; | N, 7.67%. |
| Found: | C, 67.94%; | H, 9.65%; | N, 7.69%. |

EXAMPLE 15

dl-trans-2-[N-Acetyl-N-(2-pyridylmethyl)carbamoyl-oxymethyl]tetrahydropyran-3-yl N-heptadecylcarbamate

A solution of 1.613 g of dl-trans-2-[N-(2-pyridylmethyl)carbamoyloxymethyl]tetrahydropyran-3-yl N-heptadecylcarbamate (prepared as described in Example 14), 3.60 g of 4-dimethylaminopyridine and 2.78 ml of acetic anhydride in 32 ml of toluene was heated at 80°C for 86 hours, whilst stirring. The reaction mixture was then evaporated to dryness under reduced pressure, and the residue was subjected to column chromatography through 40 g of silica gel, and then to medium pressure liquid chromatography through a Lobar B column. The fraction eluted with a 3 : 2 by volume mixture of hexane and ethyl acetate was collected and then reprecipitated from hexane to give 0.408 g of the title compound as a solid, melting at 79 - 81°C.

Nuclear Magnetic Resonance Spectrum
(60 MHz, $CDCl_3$) δ ppm:
0.7 - 2.4 (37H, multiplet);
2.62 (3H, singlet);
2.8 - 3.5 (4H, multiplet);
3.7 - 5.2 (7H, multiplet);
5.11 (2H, singlet);
7.15 (2H, multiplet);
7.65 (1H, multiplet);
8.55 (1H, multiplet).

| Elemental Analysis: | | | |
|---|---|---|---|
| Calculated for $C_{33}H_{55}N_3O_6$: | C, 67.20%; | H, 9.40%; | N, 7.12%. |
| Found: | C, 66.95%; | H, 9.67%; | N, 7.08%. |

EXAMPLE 16

dl-1-Ethyl-2-{N-acetyl-N-[trans-3-(N-heptadecylcarbamoyloxy)tetrahydropyran-2-yl]methoxycarbonyl}-aminomethylpyridinium chloride

A mixture of 0.365 g of dl-trans-2-[N-acetyl-N-(2-pyridylmethyl)carbamoyloxymethyl]tetrahydropyran-3-yl N-heptadecylcarbamate (prepared as described in Example 15) and 8 ml of ethyl iodide was heated under reflux for 40 hours. At the end of this time, the reaction mixture was evaporated to dryness under reduced pressure, and the residue was dissolved in 70% v/v aqueous methanol. The solution was passed through a column packed with 35 ml of an ion exchange resin (IRA-410, $Cl^-$ form. Rohm & Haas), and the column was washed with 70% v/v aqueous methanol. The eluate and the washings were combined and then concentrated by evaporation under reduced pressure, to give a crude chloride. This crude chloride was subjected to column chromatography through 10 g of silica gel, and then to medium pressure liquid chromatography through a Lobar B Column. The fraction eluted with a 9 : 1 by volume mixture of methylene chloride and methanol was collected to give 0.345 g of the title compound.

Nuclear Magnetic Resonance Spectrum
(60 MHz, $CD_3OD$) δ ppm:
0.7 - 2.3 (40H, multiplet);
1.67 (3H, triplet J=7 Hz);
2.64 (3H, singlet);
2.8 - 5.1 (11H, multiplet);
4.81 (2H, quartet J=7 Hz);

5.45 (2H, singlet);
8.06 (2H, multiplet);
8.60 (1H, multiplet);
9.10 (1H, multiplet).

EXAMPLE 17

dl-1-Ethyl-2-{N-[cis-3-(N-acetyl-N-heptadecylcarbamoylthio)tetrahydropyran-2-yl]-methoxycarbonylaminomethyl}pyridinium chloride

A mixture of 0.379 g of S-{dl-cis-2-[N-(2-pyridylmethyl)carbamoyloxymethyl]tetrahydropyran-3-yl}N-acetyl-N-(heptadecyl)thiocarbamate (prepared as described in Example 12) and 8 ml of ethyl iodide was heated under reflux for 91 hours. At the end of this time, the reaction mixture was dissolved in 70% v/v aqueous methanol and passed through a column packed with 35 ml of an ion-exchange resin (IRA-410, $Cl^-$ form). The column was washed with 70% v/v aqueous methanol. The eluate and the washings were combined and then concentrated by evaporation under reduced pressure, to give a crude chloride. This crude chloride was subjected to column chromatoography through 10 g of silica gel, and then to medium pressure liquid chromatography through a Lobar B column. The fraction eluted with a 9 : 1 by volume mixture of methylene chloride and methanol was collected to give 0.302 g of the title compound.
Nuclear Magnetic Resonance Spectrum
(60 MHz, $CD_3OD$) $\delta$ ppm:
0.7 - 2.2 (40H, multiplet);
1.63 (3H, triplet J = 7 Hz);
2.38 (3H, singlet);
3.2 - 5.0 (13H, multiplet);
4.73 (2H, quartet, J = 7 Hz.);
8.13 (2H, multiplet);
8.55 (1H, multiplet);
9.05 (1H, multiplet).

EXAMPLE 18

dl-3-{3-(trans-3-Heptadecylcarbamoyloxytetrahydropyran-2-ylmethoxy)-5-isoxazolyl]butyl}thiazolium methanesulphonate

0.10 ml of methanesulphonyl chloride was added, whilst ice-cooling, to a solution of 0.470 g of dl-trans-2-[5-(4-hydroxybutyl)-3-isoxazolyloxymethyl]-tetrahydropyran-3-yl N-heptadecylcarbamate (prepared as described in Preparation 69) and 0.24 ml of triethylamine dissolved in 10 ml of benzene. The mixture was then stirred at room temperature for 15 minutes, after which it was washed with water, dried over anhydrous magnesium sulphate and concentrated by evaporation under reduced pressure. The residue was dissolved in 3 ml of toluene, and 0.60 ml of thiazole was added to the solution. The mixture was then heated on an oil bath kept at 85 °C for 4 days. At the end of this time, the solvent was removed by distillation under reduced pressure, after which the residue was subjected to column chromatography through 30 g of silica gel. 0.349 g of the title compound was obtained, as a white powder, melting at 82 - 86 °C, from those fractions eluted with mixtures of methylene chloride and methanol ranging from 2 : 1 to 1 : 1 by volume.
Nuclear Magnetic Resonance Spectrum
(270 MHz, $CD_3OD$) $\delta$ ppm:
0.90 (3H, triplet, J = 7.0 Hz);
1.2 - 2.3 (38H, multiplet);
2.69 (3H, singlet);
2.76 (2H, triplet, J = 7.3 Hz);
3.04 (2H, triplet, J = 7.0 Hz);
3.43 (1H, multiplet);
3.58 (1H, multiplet);
3.93 (1H, doublet, J = 11.5 Hz);
4.25 (1H, multiplet, J = 11.5 Hz);
4.56 (1H, multiplet);
4.62 (2H, triplet, J = 7.3 Hz);

5.85 (1H, singlet);
8.29 (1H, doublet, J = 4.0 Hz);
8.49 (1H, doublet, J = 4.0 Hz).

| Elemental Analysis: | | | |
|---|---|---|---|
| Calculated for $C_{35}H_{61}N_3O_8S$: | C, 58.71%; | H, 8.59%; | N, 5.87%. |
| Found: | C, 58.35%; | H, 8.81%; | N, 5.71%. |

EXAMPLE 19

dl-3-{7-Hydroxy-8-[(trans-3-heptadecylcarbamoyloxytetrahydropyran-2-yl)methoxy]octyl}thiazolium p-toluenesulphonate

150 mg of dl-trans-2-[2-hydroxy-8-(p-toluenesulphonyloxy)octyloxymethyl]tetrahydropyran-3-yl N-heptadecylcarbamate (prepared as described in Preparation 76) and 0.15 ml of thiazole were dissolved in 1 ml of toluene, and the solution was heated on an oil bath kept at 80 °C for 42 hours. At the end of this time, the solvent was removed by distillation under reduced pressure, after which the residue was subjected to column chromatography through 4 g of silica gel. 56 mg of the title compound were obtained, as a powder melting at 81 - 84 °C, from the fractions eluted with a 9 : 1 by volume mixture of methylene chloride and methanol.
Nuclear Magnetic Resonance Spectrum: (60 MHz, $CD_3OD$) δ ppm:
0.7 - 2.5 (45H, multiplet);
2.35 (3H, singlet);
3.04 (2H, triplet, J = 6.5 Hz);
3.3 - 4.1 (8H, multiplet);
4.58 (2H, doublet, J = 7 Hz);
4.3 - 4.9 (1H, multiplet);
7.22 (2H, doublet, J = 8 Hz);
7.73 (2H, doublet, J = 8 Hz);
8.27 (1H, doublet, J = 4 Hz);
8.60 (1H, doublet, J = 4 Hz).

EXAMPLE 20

dl-3-{7-Acetoxy-8-[(trans-3-heptadecylcarbamoyloxy-tetrahydropyran-2-yl)methoxy]octyl}thiazolium p-toluenesulphonate

163 mg of isomer I of dl-trans-2-[2-acetoxy-8-p-toluenesulphonyloxyoctyloxymethyl]tetrahydropyran-3-yl N-heptadecylcarbamate (prepared as described in Preparation 77) and 0.15 ml of thiazole were dissolved in 1 ml of toluene, and the mixture was heated on an oil bath kept at 80 °C for 110 hours. At the end of this time, the solvent was removed by distillation under reduced pressure, after which the residue was subjected to column chromatography through 4 g of silica gel. 87 mg of the title compound were obtained, as a resin, from the fractions eluted with mixtures of methylene chloride and methanol ranging from 93 : 7 to 9 : 1 by volume.
Nuclear Magnetic Resonance Spectrum: (60 MHz, $CD_3OD$) δ ppm:
0.7 - 2.3 (47H, multiplet);
2.00 (3H, singlet);
2.33 (3H, singlet);
2.80 - 4.20 (9H, multiplet);
4.2 - 4.7 (1H, multiplet);
4.55 (2H, triplet, J = 7 Hz);
4.75 - 5.2 (1H, multiplet);
7.20 (2H, doublet, J = 8 Hz);
7.72 (2H, doublet, J = 8 Hz);
8.28 (1H, doublet, J = 4 Hz);
8.50 (1H, doublet, J = 4 Hz).

| Elemental Analysis: | | | | |
|---|---|---|---|---|
| Calculated for $C_{44}H_{74}N_2O_9S_2 . 0.5H_2O$:<br>Found: | C, 62.31%;<br>C, 62.29%; | H, 8.91%;<br>H, 9.08%; | N, 3.30%;<br>N, 2.92%; | S, 7.56%.<br>S, 7.46%. |

PREPARATION 1

6-Benzyloxymethyl-3,4-dihydro-2H-pyran

A solution of 5.71 g of 6-hydroxymethyl-3,4-dihydro-2H-pyran dissolved in 100 ml of dimethylformamide was added dropwise to a mixture of 2.18 g of sodium hydride (as a 55% w/w dispersion in mineral oil) and dimethylformamide, whilst ice-cooling. The mixture was then stirred at room temperature for 1 hour, after which 6.33 g of benzyl chloride were added to it. This resulting mixture was then stirred for 16 hours, after which it was poured into 1 litre of water. The resulting mixture was then extracted twice with ethyl acetate. The combined extracts were washed with water, dried over anhydrous magnesium sulphate and concentrated by evaporation under reduced pressure. The oily residue (13 g) was subjected to column chromatography through 200 g of silica gel. 9.40 g of the title compound were obtained as a colourless oil from those fractions eluted with mixtures of diethyl ether and hexane ranging from 4 : 100 to 5 : 100 by volume. It boiled at 125 - 130 ° C (bath temperature)/1 mmHg (133 Pa).
Nuclear Magnetic Resonance Spectrum
(90 MHz, $CDCl_3$) δ ppm:
1.65 - 2.2 (4H, multiplet);
3.87 (2H, singlet);
4.03 (2H, multiplet);
4.57 (2H, singlet);
4.80 (1H, triplet, J = 3.5 Hz);
7.2 - 7.6 (5H, multiplet).

| Elemental analysis: | | |
|---|---|---|
| Calculated for $C_{13}H_{16}O_2$:<br>Found : | C, 76.44%;<br>C, 76.36%; | H, 7.90%.<br>H, 7.90%. |

PREPARATION 2

dl-trans-2-Benzyloxymethyltetrahydropyran-3-ol

A 1 M solution of borane in 29.3 ml of tetrahydrofuran was added dropwise to a solution of 9.00 g of 6-benzyloxymethyl-3,4-dihydro-2H-pyran (prepared as described in Preparation 1) dissolved in 30 ml of tetrahydrofuran, whilst maintaining the temperature in the range from -5 ° C to 0 ° C. The reaction mixture was then stirred at room temperature for 3 hours, after which a 10% w/v aqueous solution of sodium hydroxide was added dropwise. 10.8 ml of 30% v/v aqueous hydrogen peroxide were than added, whilst keeping the temperature in the range from 32 to 40 ° C. The mixture was then stirred for a further 1 hour at room temperature, after which the organic layer was separated, washed with water, dried over anhydrous magnesium sulphate and concentrated by evaporation under reduced pressure. The oily residue (10.5 g) was purified by column chromatography through 250 g of silica gel. 8.82 g of the title compound were obtained from those fractions eluted with a 1 : 20 by volume mixture of ethyl acetate and methylene chloride. It boiled at 130 - 135 ° C (bath temperature)/1 mmHg (133 Pa).
Nuclear Magnetic Resonance Spectrum
(90 MHz, $CDCl_3$) δ ppm:
1.15 - 2.25 (4H, multiplet);
2.83 (1H, doublet, J = 3 Hz);
3.1 - 3.6 (3H, multiplet);
3.68 (2H, doublet, J = 5 Hz);
3.75 - 4.05 (1H, multiplet);

4.58 (2H, singlet);
7.2 - 7.5 (5H, multiplet).
Mass Spectrum (m/e): 222 ($M^+$).

| Elemental analysis: | | |
|---|---|---|
| Calculated for $C_{13}H_{18}O_2$: | C, 70.24%; | H, 8.16%. |
| Found : | C, 70.07%; | H, 8.04%. |

PREPARATION 3

dl-trans-2-Benzyloxymethyltetrahydropyran-3-yl N-heptadecylcarbamate

A solution of 6.082 g of stearic acid, 3.84 ml of diphenylphosphoryl azide and 2.48 ml of triethylamine dissolved in 200 ml of benzene was heated under reflux for 3 hours. The reaction mixture was then cooled, after which it was washed first with an aqueous solution of sodium bicarbonate and then with a saturated aqueous solution of sodium chloride. It was then dried over anhydrous magnesium sulphate and the solvent was distilled off. The residue was dissolved in 160 ml of benzene, and 1.980 g of dl-trans-2-benzyloxymethyltetrahydropyran-3-ol (prepared as described in Preparation 2) was added to the solution. The mixture was heated under reflux for 38 hours under an atmosphere of nitrogen. At the end of this time, the reaction mixture was washed, in turn, with an aqueous solution of sodium bicarbonate and with water, dried over anhydrous magnesium sulphate and evaporated to dryness under reduced pressure. The residue was subjected to column chromatography through 60 g of silica gel. 3.904 g of the title compound were obtained as a white solid, melting at 61 - 63 °C, from those fractions eluted with a 6 : 3 : 1 by volume mixture of hexane, methylene chloride and diethyl ether.
Infrared Absorption Spectrum ($CHCl_3$) $\nu_{max}$ $cm^{-1}$:
3460 (-NH-) and 1720 (-O-CO-).
Mass Spectrum (m/e): 503 ($M^+$) and 412 ($M^+$ - $C_7H_7$).

| Elemental analysis: | | | |
|---|---|---|---|
| Calculated for $C_{31}H_{53}NO_4$: | C, 73.91%; | H, 10.60%; | N, 2.78%. |
| Found : | C, 74.27%; | H, 10.70%; | N, 2.71%. |

PREPARATION 4

dl-(trans-2-Hydroxymethyltetrahydropyran-3-yl) N-heptadecylcarbamate

3.800 g of dl-trans-2-benzyloxymethyltetrahydropyran-3-yl N-heptadecylcarbamate (prepared as described in Preparation 3) were dissolved in 120 ml of methanol and allowed to react with hydrogen at room temperature for 8 hours in the presence of 10% w/w palladium on activated carbon in a Paal's apparatus at 4 atmospheres (about 4 bar). The catalyst was then filtered off, and the solvent was removed from the filtrate by distillation under reduced pressure, to give 2.729 g of the title compound as a white solid, melting at 84 - 86 °C (after recrystallization from diethyl ether).
Infrared Absorption Spectrum ($CHCl_3$ $\nu_{max}$ $cm^{-1}$:
3570 (-OH), 3450 (-NH) and 1710 (-O-CO-).
Mass Spectrum (m/e): 413 ($M^+$) and 382 ($M^+$ - $CH_2OH$).

| Elemental analysis: | | | |
|---|---|---|---|
| Calculated for $C_{24}H_{47}NO_4$: | C, 69.69%; | H, 11.45%; | N, 3.39%. |
| Found : | C, 69.38%; | H, 11.35%; | N, 3.52%. |

PREPARATION 5

dl-(trans-2-Hydroxymethyltetrahydropyran-3-yl) N-octadecylcarbamate

Following a procedure similar to that described in Preparation 3, 5.405 g of nonadecanoic acid were reacted with 1.118 g of dl-trans-2-benzyloxymethyltetrahydropyran-3-ol (prepared as described in Preparation 2). The resulting product was purified by column chromatography through silica gel eluted with a 6 : 3 : 1 by volume mixture of hexane, methylene chloride and diethyl ether, to give 1.417 g of dl-(trans-2-benzyloxymethyltetrahydropyran-3-yl) N-octadecylcarbamate as a white solid.

1.395 g of this product was dissolved in 30 ml of tetrahydrofuran, without further purification, and was then hydrogenated at room temperature for 7 hours in the presence of 10% w/w palladium on activated carbon in a Paal's apparatus at 4 atmospheres (about 4 bar). At the end of this time, the catalyst was filtered off and the solvent was stripped from the filtrate by evaporation under reduced pressure, to give 1.128 g of the title compound as a white solid, melting at 84 - 86 °C (after recrystallization from diethyl ether).

Nuclear Magnetic Resonance Spectrum (90 MHz, $CDCl_3$) $\delta$ ppm:
0.7 - 2.4 (39H, multiplet);
2.80 (1H, multiplet);
3.0 - 4.2 (7H, multiplet);
4.5 - 4.9 (2H, multiplet).
Infrared Absorption Spectrum ($CHCl_3$) $\nu_{max}$ $cm^{-1}$:
3600 (-OH), 3460 (-NH) and 1710 (-O-CO-).
Mass Spectrum (m/e): 427 ($M^+$) and 396 ($M^+$ - $CH_2OH$).

| Elemental analysis: | | | |
|---|---|---|---|
| Calculated for $C_{25}H_{49}NO_4$: | C, 70.21%; | H, 11.55%; | N, 3.28%. |
| Found : | C, 69.91%; | H, 11.55%; | N, 3.19%. |

PREPARATION 6

dl-trans-2-[N-(5-Bromopentyl)carbamoyloxymethyl]-tetrahydropyran-3-yl N-heptadecylcarbamate

1.56 ml of diphenylphosphoryl azide and 1.68 ml of triethylamine were added to a solution of 1.41 g of 6-bromohexanoic acid dissolved in 40 ml of benzene. The mixture was then heated under reflux for 3 hours. At the end of this time, the reaction mixture was washed with a saturated aqueous solution of sodium bicarbonate and then with a saturated aqueous solution of sodium chloride. It was then dried over anhydrous magnesium sulphate and concentrated by evaporation under reduced pressure. The residue was dissolved in 20 ml of toluene, and then 1.000 g of dl-(trans-2-hydroxymethyltetrahydropyran-3-yl) N-octadecylcarbamate (prepared as described in Preparation 5) and 1.68 ml of triethylamine were added to the resulting solution. The mixture was then heated on an oil bath kept at 85 °C for 67 hours. At the end of this time, the reaction mixture was concentrated by evaporation under reduced pressure, and the residue was subjected to column chromatography through 30 g of silica gel. Those fractions eluted with mixtures of hexane and ethyl acetate ranging from 4 : 1 to 3 : 1 by volume were collected and then subjected to medium pressure liquid chromatography using a Lobar B column. 0.815 g of the title compound was obtained as a white waxy material, melting at 71 - 75 °C, from those fractions eluted with the same solvent mixtures.

Nuclear Magnetic Resonance Spectrum (90 MHz, $CDCl_3$) $\delta$ ppm:
0.7 - 2.4 (43H, multiplet);
2.6 - 3.8 (6H, multiplet);
3.38 (2H, triplet, J = 7 Hz);
3.8 - 4.9 (6H, multiplet).
Infrared Absorption Spectrum ($CHCl_3$) $\nu_{max}$ $cm^{-1}$:
3450 (-NH) and 1720 (-OCONH-).
Mass Spectrum (m/e): 606, 604 ($M^+$), 525 ($M^+$ - Br) and 524 ($M^+$ - HBr).

| Elemental analysis: | | | |
|---|---|---|---|
| Calculated for $C_{30}H_{57}BrN_2O_5$: | C, 59.49%; | H, 9.49%; | N, 4.62%. |
| Found : | C, 59.92%; | H, 9.44%; | N, 4.81%. |

PREPARATION 7

(2R, 3S)-3-O-Benzyl-4-iodo-1,2-O-O-isopropylidenebutane-1,2,3-triol

(a) A solution of 21.00 ml of methanesulphonyl chloride in 100 ml of benzene was added dropwise to a solution of 57.00 g of (2R, 3R)-3-O-benzyl-1,2,-O,O-isopropylidenethreitol [prepared by the method described by Ohno et al., Chem. Pharm. Bull., 33, 572 (1985)] and 44.10 ml of triethylamine in 1 litre of benzene, whilst ice-cooling. The mixture was stirred at room temperature for 1 hour, after which it was washed with water, dried over anhydrous magnesium sulphate and concentrated by evaporation under reduced pressure, to give 74.80 g of (2R, 3R)-2-benzyloxy-3,4-isopropylidenedioxybutyl methanesulphonate as a colourless oily material.

Nuclear Magnetic Resonance Spectrum

(90 MHz, $CDCl_3$) $\delta$ ppm:

1.32 (3H, singlet);
1.40 (3H, triplet)
2.92 (3H, singlet);
3.4 - 4.5 (6H, multiplet)
4.65 (2H, singlet);
7.25 (5H, multiplet).

(b) A mixture of 74.80 g of the methanesulphonate prepared as described in step (a) above, 113.92 g of sodium bicarbonate and 169.39 g of sodium iodide in 1.1 litre of acetone was heated under reflux for 12 hours. At the end of this time, the reaction mixture was cooled, after which it was filtered with the aid of a Celite (trade mark) filter aid to remove insoluble materials. The solvent was stripped from the filtrate, and the residue was diluted with water and then extracted three times with ethyl acetate. The combined extracts were washed with water, dried over anhydrous magnesium sulphate and concentrated by evaporation under reduced pressure. The oily residue was subjected to column chromatography through 600 g of silica gel. 75.64 g of the title compound were obtained, as a colourless oil, from those fractions eluted with a 95 : 5 by volume mixture of hexane and ethyl acetate. It boiled at 130 - 150 °C/l mmHg (133 Pa).

$[\alpha]^{26}$ +8.40° (c = 1.25, $CHCl_3$).

Nuclear Magnetic Resonance Spectrum

(90 MHz, $CDCl_3$ $\delta$ ppm:

1.36 (3H, singlet);
1.44 (3H, singlet);
3.16 (1H, doublet of doublets, J = 10.5 & 7 Hz);
3.34 (1H, doublet of doublets, J = 10.5 & 7 Hz);
3.57 (1H, ddd, J = 7, 5 & 5 Hz);
3.78 (1H, doublet of doublets, J = 8 & 6.5 Hz);
4.00 (1H, doublet of doublets, J = 8 & 6.5 Hz);
4.33 (1H, doublet of triplets, J = 6.5 & 5 Hz);
4.74 (2H, AB-quartet, J = 12 Hz);
7.40 (5H, multiplet).

Infrared Absorption Spectrum ($CHCl_3$) $\nu_{max}$ $cm^{-1}$:

518 (C-I).

Mass Spectrum (m/e): 362 ($M^+$) and 347 ($M^+$ - $CH_3$).

| Elemental analysis: | | | |
|---|---|---|---|
| Calculated for $C_{14}H_{19}O_3I$: | C, 46.22%; | H, 5.29%; | I, 35.04%. |
| Found : | C, 46.47%; | H, 5.18%; | I, 35.11%. |

PREPARATION 8

(2S, 3R)-3-O-Benzyl-4-iodo-1,2,-O,O-isopropylidenebutane-1,2,3-triol

Following a procedure similar to that described in Preparation 1, 63.45 g of (2S, 3S)-2-benzyloxy-3,4-isopropylidenedioxybutyl methanesulphonate were prepared from 48.45 g of (2S, 3S)-3-O-benzyl-1,2,-O,O-isopropylidenethreitol [itself prepared by the method of Ohno et al., Chem. Pharm. Bull., 33, 572 (1985)], 37.50 ml of triethylamine and 17.80 ml of methanesulphonyl chloride. Then, following a procedure similar to that described in Preparation 1, 66.87 g of the title compound were obtained by treating this methanesulphonate with 96.80 g of sodium bicarbonate and 143.90 g of sodium iodide.
$[\alpha]^{26}$ -8.40° (c=1.00, $CHCl_3$).

PREPARATION 9

Ethyl (4R, 5R)-4-benzyloxy-2-ethoxycarbonyl-5,6-isopropylidenedioxyhexanoate

A solution of 40.00 g of diethyl malonate dissolved in 200 ml of dimethylformamide was added dropwise to a mixture of 12.00 g of sodium hydride (as a 55% w/w dispersion in mineral oil) and 600 ml of dimethylformamide, whilst maintaining the temperature within the range between 5 and 8°C. When the dropwise addition was complete, the mixture was stirred at room temperature for 1 hour, and then a solution of 75.38 g of (2R, 3S)-3-O-benzyl-4-iodo-1,2-O,O-isopropylidenebutane-1,2,3-triol (prepared as described in Preparation 7) dissolved in 300 ml of dimethylformamide was added dropwise whilst maintaining the temperature at 5 to 8°C. The mixture was then stirred at 100°C for 2 hours, after which the mixture was cooled, poured into 2 litres of water and then extracted three times with ethyl acetate. The combined extracts were washed with water, dried over anhydrous magnesium sulphate and concentrated by evaporation under reduced pressure. The oily residue was subjected to column chromatography through 1 kg of silica gel. 68.92 g of the title compound were obtained, as a colourless oil boiling at 170 - 180°C/l mmHg (133 Pa), from those fractions eluted with a 9 : 1 by volume mixture of hexane and ethyl acetate.
$[\alpha]^{25}$ +39.1° (c=1.00, $CHCl_3$).
Nuclear Magnetic Resonance Spectrum
(90 MHz, $CDCl_3$) $\delta$ ppm:
1.22 (3H, triplet, J=7.5 Hz);
1.24 (3H, triplet, J=7.5 Hz);
1.38 (3H, singlet);
1.46 (3H, singlet);
2.01 (2H, triplet, J=6.5 Hz);
3.4 - 4.4 (5H, multiplet);
4.15 (4H, multiplet);
4.68 (2H, AB-quartet, J=12 Hz);
7.38 (5H, multiplet).
Infrared Absorption Spectrum ($CHCl_3$) $\nu_{max}$ $cm^{-1}$:
1730 (-O-CO-).
Mass Spectrum (m/e): 379 ($M^+$ - $CH_3$).

| Elemental analysis: | | |
|---|---|---|
| Calculated for $C_{21}H_{30}O_7$: | C, 63.94%; | H, 7.67%. |
| Found : | C, 63.66%; | H, 7.49%. |

PREPARATION 10

Ethyl (4S, 5S)-4-benzyloxy-2-ethoxycarbonyl-5,6-isopropylidenedioxyhexanoate

Following a procedure similar to that described in Preparation 9, 58.40 g of the title compound were obtained from 35.48 g of diethyl malonate, 10.63 g of sodium hydride (as a 55% w/w dispersion in mineral oil) and 66.87 g of (2S, 3R)-3-O-benzyl-4-iodo-1,2,O,O-isopropylidenebutane-1,2,3-triol (prepared as described in Preparation 8).

$[\alpha]^{26}$ -39.5° (c = 1.00, $CHCl_3$).

PREPARATION 11

Ethyl (4R, 5R)-4-benzyloxy-5,6-isopropylidenedioxyhexanoate

68.70 g of ethyl (4R, 5R)-4-benzyloxy-2-ethoxy-carbonyl-5,6-isopropylidenedioxyhexanoate (prepared as described in Preparation 9), 12.20 g of sodium chloride and 6.51 ml of water were mixed with 1.1 litre of dimethyl sulphoxide. The mixture was then heated under reflux on an oil bath kept at 210°C for 2 hours. At the end of this time, the reaction mixture was cooled and then poured into 2.5 litres of water and extracted three times with ethyl acetate. The combined extracts were washed with water, dried over anhydrous magnesium sulphate and concentrated by evaporation under reduced pressure. The resulting oily residue was subjected to column chromatography through 1 kg of silica gel. 41.79 g of the title compound were obtained from those fractions eluted with a 95 : 5 by volume mixture of hexane and ethyl acetate. It was in the form of a colourless oil boiling at 150 - 160°C/l mmHg (133 Pa).
$[\alpha]^{26}$ +47.6° (c = 1.32, methanol).
Nuclear Magnetic Resonance Spectrum
(90 MHz, $CDCl_3$) δ ppm:
1.23 (3H, triplet, J = 7.5 Hz);
1.38 (3H, singlet);
1.45 (3H, singlet);
1.6 - 2.0 (2H, multiplet);
2.43 (2H, triplet, J = 7.5 Hz);
3.3 - 3.6 (1H, multiplet);
3.6 - 4.4 (3H, multiplet);
4.10 (2H, quartet, J = 7.5 Hz);
4.69 (2H, AB-quartet, J = 12 Hz);
7.38 (5H, singlet).
Infrared Absorption Spectrum ($CHCl_3$) $\nu_{max}$ $cm^{-1}$:
1730 (-O-CO-).
Mass Spectrum (m/e): 322 ($M^+$) and 307 ($M^+$ - $CH_3$).

| Elemental analysis: | | |
|---|---|---|
| Calculated for $C_{18}H_{26}O_5$: | C, 67.06%; | H, 8.13%. |
| Found : | C, 67.06%; | H, 8.13%. |

PREPARATION 12

Ethyl (4S, 5S)-4-benzyloxy-5,6-isopropylidenedioxyhexanoate

Following a procedure similar to that described in Preparation 11, 41.79 g of the title compound were obtained from 58.68 g of ethyl (4S, 5S)-4-benzyloxy-2-ethoxycarboyl-5,6-isopropylidenedioxyhexanoate (prepared as described in Preparation 10), 10.43 g of sodium chloride and 5.56 ml of water.
$[\alpha]^{26}$ -47.4° (c = 1.30, $CHCl_3$).

PREPARATION 13

(4R, 5R)-4-Benzyloxy-5,6-isopropylidenedioxyhexan-1-ol

A solution of 47.65 g of ethyl (4R, 5R)-4-benzyloxy-5,6-isopropylidenedioxyhexanoate (prepared as described in Preparation 11) dissolved in 250 ml of tetrahydrofuran was added dropwise to a suspension of 6.75 g of lithium aluminium hydride in 750 ml of tetrahydrofuran at a temperature maintained within the range between 5 and 8°C. The reaction mixture was then stirred at room temperature for 2 hours, after which 27.00 ml of a 4% w/v aqueous solution of sodium hydroxide was added dropwise to it at a temperature maintained within the range between 4 and 7°C. The suspension was then filtered with the aid of a Celite filter aid, and the filtrate was evaporated to dryness under reduced pressure. The residue was

subjected to column chromatography through 800 g of silica gel. 37.34 g of the title compound were obtained, as a colourless oil boiling at 150 - 160 °C/l mmHg (133 Pa), from those fractions eluted with a 2 : 1 by volume mixture of hexane and ethyl acetate.
$[\alpha]^{26}$ +41.8° (c=1.06, $CHCl_3$).
Nuclear Magnetic Resonance Spectrum
(90 MHz, $CDCl_3$) δ ppm:
1.36 (3H, triplet);
1.44 (3H, singlet);
1.5 - 1.8 (4H, multiplet);
1.71 (1H. singlet);
3.4 - 3.8 (4H, multiplet);
4.02 (1H, doublet of triplets, J=7.5, 6 Hz);
4,25 (1H, doublet of triplets, J=7.5, 6 Hz);
4,71 (2H, AB-quartet, J=12 Hz);
7.38 (5H, multiplet).
Infrared Absorption Spectrum ($CHCl_3$) $\nu_{max}$ $cm^{-1}$:
3450 (-OH).
Mass Spectrum (m/e): 280 ($M^+$) and 265 ($M^+$ - $CH_3$).

| Elemental analysis: | | |
|---|---|---|
| Calculated for $C_{16}H_{24}O_4$:<br>Found : | C, 68.55%;<br>C, 68.23%; | H, 8.63%.<br>H, 8.58%. |

PREPARATION 14

(4S, 5S)-4-Benzyloxy-5,6-isopropylidenedioxyhexan-1-ol

Following a procedure similar to that described in Preparation 13, 32.15 g of the title compound were obtained, as a colourless oil, from 41.00 g of ethyl (4S, 5S)-4-benzyloxy-5,6-isopropylidenedioxyhexanoate (prepared as described in Preparation 12) and 5.78 g of lithium aluminium hydride.
$[\alpha]^{26}$ -42,5° (c=1.10, $CHCl_3$).

PREPARATION 15

(2R, 3R)-3-Benzyloxy-6-(t-butyldiphenylsilyloxy)-1,2-isopropylidenedioxyhexane

A solution of 20.77 g of t-butyldiphenylsilyl chloride dissolved in 90 ml of dimethylformamide was added dropwise to a solution of 19.27 g of (4R, 5R)-4-benzylkoxy-5,6-isopropylidenedioxyhexan-1-ol (prepared as described in Preparation 13) and 10.29 g of imidazole in 300 ml of dimethylformamide, whilst maintaining the temperature in the range from 5 to 7°C. The reaction mixture was then stirred at room temperature for 3 hours, after which it was poured into 2 litres of water and then extracted three times with ethyl acetate. The combined extracts were washed with water, dried over anhydrous magnesium sulphate and then concentrated by evaporation under reduced pressure. The oily residue was subjected to column chromatography through 700 g of silica gel. 32.80 g of the title compound were obtained, as a colourless oil, from those fractions eluted with mixtures of hexane and ethyl acetate ranging from 98 : 2 to 95 : 5 by volume.
$[\alpha]^{26}$ +21.4°C (c=1.11, $CHCl_3$).
Nuclear Magnetic Resonance Spectrum
(90 MHz, $CDCl_3$) δ ppm:
1.04 (9H, singlet);
1.37 (3H, singlet);
1.43 (3H, singlet);
1.4 - 1.8 (4H, multiplet);
3.3 - 3.8 (4H, multiplet);
4.00 (1H, doublet of triplets, J=7.5 & 6 Hz);
4.20 (1H, doublet of triplets, J=7.5 & 6 Hz);

4.66 (2H, AB-quartet, J = 12 Hz);
7.2 - 7.8 (15H, multiplet).
Infrared Absorption Spectrum ($CHCl_3$) $\nu_{max}$ $cm^{-1}$:
1100 (Si-O).
Mass Spectrum (m/e): 503 ($M^+$ - $CH_3$).

| Elemental analysis; | | |
|---|---|---|
| Calculated for $C_{32}H_{42}O_4Si$: | C 74.09%; | H, 8.16%. |
| Found : | C, 74.20%; | H, 8.18%. |

PREPARATION 16

(2S, 3S)-3-Benzyloxy-6-(t-butyldiphenylsilyloxy)-1,2-isopropylidenedioxyhexane

Following a procedure similar to that described in Preparation 15, 30.97 g of the title compound were obtained, as a colourless oil, from 18.00 g of (4S, 5S)-4-benzyloxy-5,6-isopropylidenedioxyhexan-1-ol (prepared as described in Preparation 14), 9.62 g of imidazole and 19.41 g of t-butyldiphenylsilyl chloride.
$[\alpha]^{26}$ -20.8° (c = 1.25, $CHCl_3$).

PREPARATION 17

(2R, 3R)-3-Benzyloxy-6-(t-butyldiphenylsilyloxy)hexane-1,2-diol

32.49 g of (2R, 3R)-3-benzyloxy-6-(t-butyldiphenyl-silyloxy)-1,2-isopropylidenedioxyhexane (prepared as described in Preparation 15) were dissolved in a mixture of 300 ml of acetic acid and 30 ml of water. The resulting solution was stirred at room temperature for 17 hours and then at 50 °C for 2 hours. At the end of this time, the solution was cooled, and the solvent was removed by distillation under reduced pressure. The residue was subjected to column chromatography through 500 g of silica gel. 27.40 g of the title compound were obtained, as a colourless oil, from the fraction eluted with a 1 : 2 by volume mixture of ethyl acetate and hexane.
$[\alpha]^{26}$ -20.4° (c = 1.12, $CHCl_3$).
Nuclear Magnetic Resonance Spectrum
(90 MHz, $CDCl_3$) δ ppm:
1.05 (9H, singlet);
1.5 - 1.9 (4H, multiplet);
1.9 - 2.3 (1H, multiplet);
2.3 - 2.65 (1H, multiplet);
3.4 - 3.9 (6H, multiplet);
4.53 (2H, AB-quartet, J = 12 Hz);
7.2 - 7.8 (15H, multiplet).
Infrared Absorption Spectrum ($CHCl_3$) $\nu_{max}$ $cm^{-1}$:
3590, 3460 (-OH) and 1100 (Si-O).
Mass Spectrum (m/e): 479 ($M^+$ + 1)

PREPARATION 18

(2S, 3S)-3-Benzyloxy-6-(t-butyldiphenylsilyloxy)-hexane-1,2-diol

Following a procedure similar that described in Preparation 17, 26.15 g of the title compound were obtained, as a colourless oil, from 30.48 g of (2S, 3S)-3-benzyloxy-6-(t-butyldiphenylsilyoxy)-1,2-isopropylidenedioxyhexane (prepared as described in Preparation 16), 300 ml of acetic acid and 30 ml of water.
$[\alpha]^{26}$ +20.6° (C = 1.15, $CHCl_3$).

PREPARATION 19

(2R, 3R)-3-Benzyloxy-6-(t-butyldiphenylsilyloxy)-1-triphenylmethoxyhexan-2-ol

18.33 g of triphenylmethyl chloride were added to a solution of 26.22 g of (2R, 3R)-3-benzyloxy-6-(t-butyldiphenylsilyloxy)hexane-1,2-diol (prepared as described in Preparation 17) and 18.40 ml of triethylamine in 500 ml of toluene. The resulting mixture was heated under reflux for 3 hours. At the end of this time, it was cooled and then diluted with water. It was then extracted three times with ethyl acetate. The combined extracts were washed, in turn, with water, with a saturated aqueous solution of sodium bicarbonate and with a saturated aqueous solution of sodium chloride, after which they were dried over anhydrous magnesium sulphate, and the solvent was stripped off by evaporation under reduced pressure. The resulting oily residue was dissolved in 270 ml of tetrahydrofuran, and then 90 ml of a saturated aqueous solution of sodium bicarbonate were added, and the resulting mixture was stirred at room temperature of 1 hour. After this work-up, 40 g of the oily product were subjected to column chromatography through 500 g of silica gel. 37.01 g of the title compound were obtained, as a colourless oil, from those fractions eluted with mixtures of hexane and ethyl acetate ranging from 95 : 5 to 9 : 1 by volume.
$[\alpha]^{25}$ -3.55° (c = 1.03, $CHCl_3$).
Nuclear Magnetic Resonance Spectrum
(90 MHz, $CDCl_3$) $\delta$ ppm:
1.05 (9H, singlet);
1.4 - 1.8 (4H, multiplet);
2.30 (1H, doublet, J = 6 Hz);
3.22 (2H, doublet, J = 6 Hz);
3.5 - 3.9 (4H, multiplet);
4.45 (2H, AB-quartet, J = 12 Hz);
7.1 - 7.8 (30H, multiplet).
Infrared Absorption Spectrum ($CHCl_3$) $\nu_{max}$ $cm^{-1}$;
3580 (-OH) and 1100 (O-Si).
Mass Spectrum (m/e) : 477 [$M^+$ - HC)$CH_3)_3$].

| Elemental analysis: | | |
|---|---|---|
| Calculated for $C_{48}H_{52}O_4Si$: | C, 79.96%; | H, 7.27%. |
| Found : | C, 79.71%; | H, 7.11%. |

PREPARATION 20

(2S, 3S)-3-Benzyloxy-6-(t-butyldiphenylsilyloxy)-1-triphenylmethoxyhexan-2-ol

Following a procedure similar to that described in Preparation 19, 36,50 g of the title compound were obtained, as a colourless oil, from 25.96 g of (2S, 3S)-3-benzyloxy-6-(t-butyldiphenylsilyloxy)hexane-1,2-diol (prepared as described in Preparation 18), 18.20 ml of triethylamine and 18.11 g of triphenylmethyl chloride.
$[\alpha]^{25}$ + 3.56 (C = 1.01, $CHCl_3$).

PREPARATION 21

(2R, 3R)-3-Benzyloxy-6-hydroxy-1-triphenylmethoxy-2-hexyl methanesulphonate

(a) 4.75 ml of methanesulphonyl chloride were added dropwise, whilst ice-cooling at 5°C, to a solution of 36.89 g of (2R, 3R)-3-benzyloxy-6-(t-butyldiphenylsilyloxy)-1-triphenylmethoxyhexan-2-ol (prepared as described in Preparation 19) and 8.56 ml of triethylamine dissolved in 500 ml of methylene chloride. The mixture was stirred at room temperature for 1 hour, after which it was poured into water. The organic layer was separated, washed with a saturated aqueous solution of sodium chloride, dried over anhydrous magnesium sulphate and concentrated by evaporation under reduced pressure, to give 40.91 g of (2R,3R)-3-benzyloxy-6-(t-butyldiphenylsilyloxy)-1-triphenylmethoxy-2-hexyl methanesulphonate as a colourless oil.
Nuclear Magnetic Resonance Spectrum

(90 MHz, $CDCl_3$) δ ppm:
1.01 (9H, singlet);
1.3 - 1.8 (4H, multiplet);
2.96 (3H, singlet);
3.0 - 3.9 (5H, multiplet);
4.50 (2H, singlet);
4.6 - 4.9 (1H, multiplet);
7.1 - 7.8 (30H, multiplet).

(b) 61.4 ml of 1N solution of tetrabutylammonium fluoride in tetrahydrofuran were added dropwise, whilst ice-cooling at 5°C, to a solution of 40.91 g of the methanesulphonate [prepared as described in step (a) above] in 500 ml of tetrahydrofuran, and the mixture was stirred at room temperature for 14 hours. It was ten diluted with water, and extracted three times with ethyl acetate. The combined extracts were washed with water, dried over anhydrous magnesium sulphate and concentrated by evaporation under reduced pressure, and the residue was subjected to column chromatography through 700 g of silica gel. 26.45 g of the title compound were obtained, as a colourless oil, from those fractions eluted with mixtures of hexane and ethyl acetate ranging from 4 : 1 - 2 : 1 by volume.
$[\alpha]^{25}$ +21.7° (c=1.22, $CHCl_3$).
Nuclear Magnetic Resonance Spectrum
(90 MHz, $CDCl_3$) δ ppm:
1.37 (1H, singlet);
1.4 - 1.8 (4H, multiplet);
3.00 (3H, singlet);
3.30 (1H, doublet of doublets, J=11 & 6 Hz);
3.4 - 3.6 (2H, multiplet);
3.60 (1H, doublet of doublets, J=11 & 3 Hz);
3.6 - 3.9 (1H, multiplet);
4.56 (2H, singlet);
4.82 (1H, ddd, J=6, 6 & 3 Hz);
7.2 - 7.6 (20H, multiplet).
Infrared Absorption Spectrum ($CDCl_3$) $\nu_{max}$ $cm^{-1}$:
3500 (-OH), 1360 and 1170 ($-SO_2-$).
Mass Spectrum (m/e: 483 ($M^+$ - $C_6H_5$) and 468
($M^+$ - $C_6H_5CH_3$).

PREPARATION 22

(2S, 3S)-3-Benzyloxy-6-hydroxy-1-triphenylmethoxy-2-hexyl methanesulphonate

Following a procedure similar to that described in Preparation 21, 25.18 g of the title compound were obtained, as a colourless oil, from 35.60 g of (2S, 3S)-3-benzyloxy-6-(t-butyldiphenylsilyloxy)-1-triphenylmethoxyhexan-2-ol (prepared as described in Preparation 20).
$[\alpha]^{25}$ -21.7° (c=1.23, $CHCl_3$).

PREPARATION 23

(2S, 3R)-3-Benzyloxy-2-triphenylmethoxymethyltetra-hydropyran

A solution of 26.08 g of (2R, 3R)-3-benzyloxy-6-hydroxy-1-triphenylmethoxy-2-hexyl methanesulphonate (prepared as described in Preparation 21) dissolved in 290 ml of t-butanol was added dropwise to a solution of 7.01 g of potassium t-butoxide in 250 ml of t-butanol, whilst maintaining the temperature at 25°C. The mixture was then stirred at 40°C for 4 hours. At the end of this time, 0.56 ml of acetic acid were added to the mixture, and the solvent was removed by distillation under reduced pressure. The residue was diluted with water, and then extracted three times with ethyl acetate. The combined extracts were washed with a saturated aqueous solution of sodium bicarbonate and with a saturated aqueous solution of sodium chloride, dried over anhydrous magnesium sulphate and concentrated by evaporation under reduced pressure. The residue was then subjected to column chromatography through 430 g of silica gel. 21.04 g of the title compound were obtained from those fractions eluted with a 95 : 5 by volume mixture of hexane and ethyl acetate, as crystals melting at 86.0 - 88.0°C (after recrystallization from methanol).

$[\alpha]^{25}$ -32.8° (c=1.01, $CHCl_3$).
Nuclear Magnetic Resonance Spectrum
(270 MHz, $CDCl_3$) δ ppm:
1.41 (1H, dddd, J=12.3, 10.9, 9.3 & 6.7 Hz);
1.70 (2H, multiplet);
2.26 (1H, ddddd, J=12.3, 4.2, 3.9, 3.9 & 1 Hz);
3.20 (1H, doublet of doublets, J=9.8 & 5.0 Hz);
3.37 (1H, ddd, J=9.3, 5.0 & 2.0 Hz);
3.39 (1H, ddd, J=11.4, 9.3 & 5.3 Hz);
3.48 (1H, doublet of doublets, J=9.8 & 2.0 Hz);
3.49 (1H, ddd, J=10.9, 9.3 & 4.2 Hz);
4.00 (1H, dddd, J=11.4, 2.9, 2.9 & 1 Hz);
4.38 (2H, AB-quartet, J=11.5 Hz);
7.0 - 7.5 (20H, multiplet).
Infrared Absorption Spectrum ($CHCl_3$) $\nu_{max}$ $cm^{-1}$:
1595, 1490, 1450 ($C_6H_5$-), 1090 and 1070 (C-O-C).
Mass Spectrum (m/e): 387 ($M^+$ - $C_6H_5$) and 373 ($M^+$ - $C_7H_7$).

| Elemental analysis: | | |
|---|---|---|
| Calculated for $C_{32}H_{32}O_3$: | C, 82.73%; | H, 6.94%. |
| Found : | C, 82.56%; | H, 6.83%. |

## PREPARATION 24

### (2R, 3S)-3-Benzyloxy-2-triphenylmethoxymethyltetrahydropyran

Following a procedure similar to that described in Preparation 21, 20.12 g of the title compound were obtained, as crystals, melting at 86.5 - 88.5°C, from 24.98 g of (2S, 3S)-3-benzyloxy-6-hydroxy-1-triphenylmethoxy-2-hexyl methanesulphonate (prepared as described in Preparation 22) and 6.06 g of potassium t-butoxide.
$[\alpha]^{25}$ +33.0 (c=1.00, $CHCl_3$).

## PREPARATION 25

### (2S, 3R)-3-Hydroxy-2-triphenylmethoxymethyltetrahydropyran

3.513 g of (2S, 3R)-3-benzyloxy-2-triphenylmethoxymethyltetrahydropyran (prepared as described in Preparation 23) were dissolved in 120 ml of ethanol and hydrogenated at room temperature for 30 hours in the presence of 1.852 g of 10% w/w palladium on activated carbon in a Paal's apparatus at a hydrogen pressure of 4 atmospheres (about 4 bars). At the end of this time, the catalyst was filtered off and the filtrate was freed from the solvent by evaporation under reduced pressure. The residue was subjected to column chromatography through 90 g of silica gel. 2.557 g of the title compound were obtained, as a colorless oil, from those fractions eluted with a 7 : 1 by volume mixture of hexane and ethyl acetate.
$[\alpha]^{25}$ +38.2° (c=1.07, $CHCl_3$).
Nuclear Magnetic Resonance Spectrum
(90 MHz, $CDCl_3$) δ ppm:
1.2 - 1.8 (3H, multiplet);
1.9 - 2.3 (1H, multiplet);
3.00 (1H, singlet);
3.1 - 3.7 (5H, multiplet);
3.75 - 4.05 (1H, multiplet);
7.2 - 7.7 (15H, multiplet).
Infrared Absorption Spectrum ($CHCl_3$) $\nu_{max}$ $cm^{-1}$:
3500 (OH), 1600, 1490, 1450 (-Ph) and 1090 (C-O-C).
Mass Spectrum (m/e): 374 ($M^+$) and 297

($M^+$ - $C_6H_5$).

PREPARATION 26

(2R, 3S)-3-Hydroxy-2-(triphenylmethoxymethyl)tetrahydropyran

Following a procedure similar to that described in Preparation 25, 2.075 g of the title compound were obtained, as a colourless oil, from 2.835 g of (2R, 3S)-3-benzyloxy-2-triphenylmethoxymethyl pyran (prepared as described in Preparation 24) in the presence of 1.499 g of 10% w/w palladium on activated carbon.
$[\alpha]^{25}$ -38.0° (c = 1.12, $CHCl_3$).

PREPARATION 27

(2S, 3R)2-2(Triphenylmethoxymethyl)tetrahydropyran-3-yl N-heptadecylcarbamate

A solution of 4.782 g of stearic acid, 3.62 ml of diphenylphosphoryl azide and 2.34 ml of triethylamine dissolved in 100 ml of benzene was heated under reflux for 3 hours. At the end of this time, the reaction mixture was cooled and then diluted with ethyl acetate. It was then washed with a saturated aqueous solution of sodium bicarbonate and dried over anhydrous magnesium sulphate. The solvent was then removed by distillation under reduced pressure, and the residue was dissolved in 25 ml of toluene. A solution of 2.34 ml of triethylamine and 2.518 g of (2S, 3R)-3-hydroxy-2-triphenylmethoxymethyl-tetrahydropyran (prepared as described in Preparation 25) dissolved in 25 ml of toluene was then added to the resulting solution. The mixture was then heated at 100°C for 90 hours, after which it was allowed to cool and then poured into a saturated aqueous solution of sodium bicarbonate. This was then extracted three times with ethyl acetate. The combined extracts were dried over anhydrous magnesium sulphate and concentrated by evaporation under reduced pressure, to give an oily residue. This was subjected to column chromatography through 120 g of silica gel. 3.131 g of the title compound were obtained, as a colourless oil, from those fractions eluted with a 7 : 1 by volume mixture of hexane and ethyl acetate.
$[\alpha]^{25}$ -28.5° (c = 1.04 $CHCl_3$).
Nuclear Magnetic Resonance Spectrum
(90 MHz, $CDCl_3$) $\delta$ ppm:
0.7 - 2.4 (37H, multiplet);
2.8 - 3.6 (6H, multiplet);
3.8 - 4.1 (1H, multiplet);
4.2 - 4.8 (2H, multiplet);
7.1 - 7.6 (15H, multiplet).
Infrared Absorption Spectrum ($CHCl_3$) $\nu_{max}$ $cm^{-1}$:
3460 (NH), 1720 and 1510 (-NH-CO).
Mass Spectrum (m/e): 412 ($M^+$ - $C_{19}H_{15}$), 396
($M^+$ - $C_{19}H_{15}O$) and 382 ($M^+$ - $C_{21}H_{17}O$).

PREPARATION 28

(2R, 3S)-2-(Triphenylmethoxymethyl)tetrahydropyran-3-yl N-heptadecylcarbamate

Following a procedure similar to that described in Preparation 11, 2.491 g of the title compound was obtained, as a colourless oil, from 3.553 g of stearic acid, 2.69 ml of diphenylphosphoryl azide and 1.871 g of (2R, 3S)-3-hydroxy-2-(triphenylmethoxymethyl)tetrahydropyran (prepared as described in Preparation 26).
$[\alpha]^{25}$ +28.8° (c = 1.13, $CHCl_3$).

PREPARATION 29

(2S, 3R)-2-Hydroxymethyltetrahydropyran-3-yl N-heptadecylcarbamate

240.0 mg of p-toluenesulphonic acid were added to a solution of 2.753 g of (2S, 3R)-2-(triphenylmethoxymethyl)tetrahydropyran-3-yl N-heptadecylcarbamate (prepared as described in Preparation 27) dissolved in 55 ml of methanol, and the mixture was heated under reflux for 1 hour. At the end of this time, the reaction mixture was cooled, and 352.6 mg of sodium bicarbonate were added. The methanol was then removed by distillation under reduced pressure, and ethyl acetate was added. Insoluble materials were filtered off, and the filtrate was concentrated by evaporation under reduced pressure. The residue was subjected to column chromatography through 55 g of silica gel. 1.476 g of the title compound was obtained from those fractions eluted with mixtures of hexane and ethyl acetate ranging from 2 : 1 to 1 : 1 by volume in the form of crystals melting at 92.0 - 93.5 °C (after recrystallisation from diethyl ether).
$[\alpha]^{25}$ -7.20° (c=1.00, $CHCl_3$).
Nuclear Magnetic Resonance Spectrum
(90 MHz, $CDCl_3$) $\delta$ ppm:
0.7 - 2.3 (37H, multiplet);
2.6 - 2.85 (1H, multiplet);
2.9 - 3.8 (6H, multiplet);
3.8 - 4.1 (1H, multiplet);
4.4 - 4.9 (2H, multiplet).
Infrared Absorption Spectrum ($CHCl_3$) $\nu_{max}$ $cm^{-1}$:
3450 (-NH, -OH), 1710 and 1510 (-NH-CO).
Mass Spectrum (m/e): 414 ($M^+$ + 1) and 382 ($M^+$ - $CH_2OH$).

| Elemental analysis: | | | |
|---|---|---|---|
| Calculated for $C_{24}H_{47}NO_4$: | C, 69.69%; | H, 11.45%; | N, 3.39%. |
| Found: | C, 69.33%; | H, 11.40%; | N, 3.53%. |

## PREPARATION 30

### (2R, 3S)-2-Hydroxymethyltetrahydropyran-2-yl N-heptadecylcarbamate

Following a procedure similar to that described in Preparation 29), 1.305 g of the title compound was obtained, as crystals melting at 92.5 - 93.5 °C, from 2.400 of (2R, 3S)-2-(triphenylmethoxymethyl)-tetrahydropyran-3-yl N-heptadecylcarbamate (prepared as described in Preparation 28).
$[\alpha]^{25}$ +7.25° (c=1.02, $CHCl_3$).

## PREPARATION 31

### (2S, 3R)-2-[N-(5-Bromopentyl)carbamoyloxymethyl]-tetrahydropyran-3-yl N-heptadecylcarbamate

Following a procedure similar to that described in Preparation 6, 1.270 g of the title compound was obtained, as a waxy solid melting at 71.0 - 72.0 °C, from 1.706 g of 5-bromohexanoic acid, 1.88 ml of diphenylphosphoryl azide and 1.206 g of (2S, 3R)-2-hydroxymethyltetrahydropyran-3-yl N-heptadecylcarbamate (prepared as described in Preparation 29).
$[\alpha]^{25}$ -26.4° (c=1.18, $CHCl_3$).

## PREPARATION 32

### (2R, 3S)-2-[N-(5-Bromopentyl)carbamoyloxymethyl]-tetrahydropyran-3-yl N-heptadecylcarbamate

Following a procedure similar to that described in Preparation 6), 1.283 g of the title compound was obtained, as a waxy solid melting at 71.5 - 72.0 °C, from 1.698 g of 5-bromohexanoic acid, 1.88 ml of diphenylphosphoryl azide and 1.200 g of (2R, 3S)-2-hydroxymethyltetrahydropyran-2-yl N-heptadecylcarbamate (prepared as described in Preparation 30).
$[\alpha]^{25}$ +26.5° (c=1.00, $CHCl_3$).

PREPARATION 33

dl-cis-2-Benzyloxymethyltetrahydropyran-3-ol

(a) 2.22 g of dl-trans-2-benzyloxymethyltetrahydropyran-3-ol (prepared as described in Preparation 2) were dissolved in 20 ml of acetone, and were then oxidized with 6 ml of Jones' reagent (containing 1.60 g of chromic anhydride), whilst ice-cooling. The reaction mixture was then stirred at room temperature for 1 hour, after which it was poured into water and extracted twice with ethyl acetate. The combined extracts were washed with water, dried and concentrated by evaporation under reduced pressure, to give 2.08 of a crude oily ketonic compound.

(b) The whole of the ketonic compound prepared as described in step (a) above was dissolved, without further purification, in 10 ml of tetrahydrofuran and was then reduced at a temperature between 0 to 5°C with 12 ml of a 1M tetrahydrofuran solution of L-selectride. The solution was then stirred for 30 minutes whilst ice-cooling and for 2 hours at room temperature. 6 ml of a 10% w/v aqueous solution of sodium hydroxide were then added dropwise at 5 - 15°C, and then 6 ml of 35% v/v aqueous hydrogen peroxide were added dropwise to the mixture at 15 - 30°C. The organic layer was then separated, dried over anhydrous magnesium sulphate and concentrated by evaporation under reduced pressure, to give an oily residue. This was purified by column chromatography through 60 g of silica gel. Those fractions eluted with mixtures of hexane and ethyl acetate ranging from 100 : 15 to 2 : 1 by volume were worked up , to give 1.135 g of the title compound as a colourless liquid boiling at a bath temperature of 130 - 140°C/l mmHg 133 Pa).

Nuclear Magnetic Resonance Spectrum
($90$ MHz, $CDCl_3$) $\delta$ ppm:
1.25 - 2.30 (4H, multiplet);
2.68 (1H, doublet, J=6 Hz);
3.3 - 3.7 (4H, multiplet);
3.80 (1H, multiplet);
4.03 (1H, multiplet);
4.59 (2H, singlet);
7.2 - 7.5 (5H, multiplet).
Mass Spectrum (m/e): 222 ($M^+$).

PREPARATION 34

dl-cis-Hydroxymethyltetrahydropyran-3-yl N-octadecylcarbamate

2.627 of nonadecanoic acid were reacted, in a similar manner to that described in Preparation 3, with 0.815 g of dl-cis-2-benzyloxymethyltetrahydropyran-3-ol (prepared as described in Preparation 33). The resulting crude product was purified by column chromatography through 70 g of silica gel. Those fractions eluted with a 1 : 5 by volume mixture of ethyl acetate and hexane afforded 1.05 g of dl-cis-2-benzyloxymethyltetrahydropyran-3-yl N-octadecylcarbamate.

The whole of this product was dissolved in 30 ml of a 2 : 1 by volume mixture of tetrahydrofuran and methanol, and was then hydrogenated at room temperature for 8 hours in the presence of 0.50 g of a 10% w/w palladium on activated carbon catalyst and hydrogen at an initial pressure of 4 atmospheres (about 4 bars) in a Paar's apparatus. The catalyst was removed by filtration, and then the solvent was distilled off under reduced pressure. The residue was subjected to column chromatography through 20 g of silica gel. Those fractions eluted with mixtures of ethyl acetate and hexane ranging from 1 : 5 to 1 : 2 by volume were worked up and then recrystallized from a mixture of diethyl ether and hexane, to give 0.732 g of the title compound as crystals melting at 85 - 86°C.

Nuclear Magnetic Resonance Spectrum
(90 MHz, $CDCl_3$) $\delta$ ppm:
0.7 - 2.2 (39H, multiplet);
2.9 - 3.8 (7H, multiplet);
3.9 - 4.2 (1H, multiplet);
4.65 - 5.1 (2H, multiplet);
Infrared Absorption Spectrum ($CHCl_3$) $\nu_{max}$ $cm^{-1}$:
3600(-OH), 3450 (-NH-), 1700 (-O-CO-).
Mass Spectrum (m/e): 427 ($M^+$), 396 ($M^+$ - $CH_2OH$).

| Elemental Analysis: | | | |
|---|---|---|---|
| Calculated for $C_{25}H_{49}NO_4$: | C, 70.21%; | H, 11.55%; | N, 3.28%. |
| Found: | C, 70.27%; | H, 11.73%; | N, 3.28%. |

PREPARATION 35

dl-trans-2-Benzyloxymethyl-3-(tetrahydropyran-2-yloxy)tetrahydropyran

2.22 g of dl-trans-2-benzyloxymethyltetrahydropyran-3-ol (prepared as described in Preparation 2), 2.65 ml of dihydropyran and 0.05 g of pyridinium p-toluenesulphonate were dissolved in 40 ml of methylene chloride, and the mixture was stirred at room temperature for 4 hours. The solvent was then distilled off to give a residue, which was subjected to column chromatography through 80 g of silica gel. Those fractions eluted with mixtures of hexane and diethyl ether ranging from 6 : 1 to 5 : 1 by volume were worked up, to afford 2.93 g of the title compound as a colourless oil.

Nuclear Magnetic Resonance Spectrum
($90$ MHz, $CDCl_3$) $\delta$ ppm:
1.1-2.4 (10H, multiplet);
3.15-4.15 (8H, multiplet);
4.57 (2H, AB-quartet, J = 12 Hz);
4.78 (1H, multiplet);
7.35 (5H, multiplet).
Mass Spectrum (m/e): 306 ($M^+$).

| Elemental Analysis: | | |
|---|---|---|
| Calculated for $C_{18}H_{26}O_4$: | C, 70.56%; | H, 8.55%. |
| Found: | C, 70.65%; | H, 8.45%. |

PREPARATION 36

dl-trans-3-(Tetrahydropyran-2-yloxy)tetrahydropyran-2-ylmethanol

2.93 g of dl-trans-2-benzyloxymethyl-3-(tetrahydropyran-2-yloxy)tetrahydropyran (prepared as described in Preparation 35) were dissolved in 130 ml of tetrahydrofuran and were then hydrogenated at room temperature for 8 hours in the presence of 1.30 g of a 10% w/w palladium on activated carbon catalyst and of hydrogen at an initial pressure of 4 atmospheres (about 4 bars). The catalyst was removed by filtration, and then the solvent was distilled off. The resulting residue was purified by column chromatography through 50 g of silica gel. Those fractions eluted with mixtures of hexane and diethyl ether ranging from 2 : 1 to 1 : 1 by volume were worked up to give 1.87 g of the title compound as a colourless oil.

Nuclear Magnetic Resonance Spectrum
(90 MHz, $CDCl_3$) $\delta$ ppm:
2.2 - 2.4 (10H, multiplet);
2.77 (1H, triplet, J = 7 Hz);
3.1 - 4.1 (8H, multiplet);
4.70 (1H, multiplet).
Infrared Absorption Spectrum ($CDCl_3$) $\nu_{max}$ $cm^{-1}$:
3600, 3480 (-OH).

| Elemental Analysis: | | |
|---|---|---|
| Calculated for $C_{11}H_{20}O_4$: | C, 61.12%; | H, 9.33%. |
| Found: | C, 60.75%; | H, 9.29%. |

PREPARATION 37

dl-(trans-3-Hydroxytetrahydropyran-2-yl)methyl N-octadecylcarbamate

6.247 g of nonadecanoic acid were reacted with 1.809 g of dl-trans-3-(tetrahydropyran-2-yloxy)-tetrahydropyran-2-ylmethanol (prepared as described in Preparation 36) on an oil bath at 85°C for 24 hours, in a manner similar to that described in preparation 3. The reaction mixture was then cooled to room temperature, after which solids were filtered off and the solvent was removed by evaporation under reduced pressure. The residue was purified by column chromatography through 100 g of silica gel. Those fractions eluted with a 1 : 5 : 5 by volume mixture of diethyl ether, hexane and methylene chloride were worked up to give 4.20 g of dl-[trans-3-(tetrahydropyran-2-yloxy)tetrahydropyran-2-yl]methyl N-octadecylcarbamate as a solid.

The whole of this compound was dissolved in 30 ml of a 1 : 2 by volume mixture of tetrahydrofuran and methanol, and then 0.20 g of camphorsulphonic acid was added to the solution. The reaction mixture was then stirred at room temperature for 45 minutes, after which 10 ml of a saturated aqueous solution of sodium bicarbonate was added. The solvent was removed by evaporation under reduced pressure, and diethyl ether was added to the residue. The ethereal layer was separated, washed with water and dried over anhydrous magnesium sulphate. The diethyl ether was then evaporated off under reduced pressure. The residue (3.57 g) was subjected to column chromatography through 70 g of silica gel. Those fractions eluted with a 1 : 5 : 5 by volume mixture of diethyl ether, hexane and methylene chloride were worked up and then recrystallized from a mixture of diethyl ether and hexane, to give 3.289 g of the title compound as white crystals melting at 55 - 56°C.

Nuclear Magnetic Resonance Spectrum
(90 MHz, $CDCl_3$) $\delta$ ppm:
0.75 - 2.30 (39H, multiplet);
3.00 - 3,60 (5H, multiplet);
3.69 (1H, doublet, J = 4 Hz);
3.85 - 4.20 (2H, multiplet);
4.76 (1H, doublet of doublets, $J_1$ = 13 Hz, $J_2$ = 3 Hz);
4.95 (1H, multiplet).

Infrared Absorption Spectrum ($CHCl_3$) $\nu_{max}$ $cm^{-1}$:
3450(-NH-, -OH), 1700 (-O-CO-).

Mass Spectrum (m/e): 427 ($M^+$), 409 ($M^+$ - $H_2O$).

| Elemental Analysis: | | | |
|---|---|---|---|
| Calculated for $C_{25}H_{49}NO_4$: | C, 70.21%; | H, 11.55%; | N, 3.28%. |
| Found: | C, 70.31%; | H, 11.42%; | N, 3.27%. |

PREPARATION 38

dl-S-(cis-2-Benzyloxymethyltetrahydropyran-3-yl) thioacetate

1.04 ml of methanesulphonyl chloride were added dropwise, whilst ice-cooling, to a solution of 2.00 g of dl-trans-2-benzyloxymethyltetrahydropyran-3-ol (prepared as described in Preparation 2) and 2.51 ml of triethylamine in 40 ml of benzene. The reaction mixture was then stirred at room temperature for 1 hour, after which it was washed with water and dried over anhydrous magnesium sulphate. The solvent was then evaporated off under reduced pressure, to give a crude methanesulphonate, as an oil. This was dissolved, without further purification, in 10 ml of dimethylformamide.

Meanwhile, a solution of 0.77 ml of thioacetic acid in 5 ml of dimethylformamide was added dropwise to a suspension of 0.47 g of sodium hydride (as a 55% w/w dispersion in mineral oil) in 5 ml of dimethylformamide, whilst cooling; the mixture was then stirred at room temperature for 1 hour. At the end of this time, the solution of the crude methanesulphonate prepared as described above was added to this mixture, and the mixture was heated for 16 hours at 80°C and then for 10 hours at 100°C, whilst stirring. The mixture was then cooled to room temperature, poured into water and then extracted with ethyl acetate. The extract was washed with water and dried over anhydrous magnesium sulphate, and then the solvent was evaporated off under reduced pressure, to give an oily residue. This residue was purified by column

chromatography through 50 g of silica gel. Fractions eluted with mixtures of diethyl ether and hexane ranging from 3 : 97 to 10 : 90 by volume were worked up, to give 1.448 g of the title compound as an oil.
Nuclear Magnetic Resonance Spectrum
(90 MHz, $CDCl_3$) $\delta$ ppm:
1.10 - 2.20 (4H, multiplet);
2.30 (3H, singlet);
3.20 - 4.20 (6H, multiplet);
4.52 (2H, AB-quartet, J = 12 Hz);
7.20 - 7.50 (5H, multiplet).
Infrared Absorption Spectrum ($CHCl_3$) $\nu_{max}$ $cm^{-1}$:
1685 (-S-CO-).
Mass Spectrum (m/e): 280 ($M^+$).

| Elemental Analysis: | | | |
|---|---|---|---|
| Calculated for $C_{15}H_{20}O_3S$: | C, 64.25%; | H, 7.19%; | S, 11.44%. |
| Found: | C, 64.19%; | H, 6.96%; | S, 11.67%. |

PREPARATION 39

dl-cis-Benzyloxymethyltetrahydropyran-3-thiol

1.04 ml of an approximately 28% w/v methanolic solution of sodium methoxide was added dropwise at -10 °C to 1.422 g of dl-S-(cis-2-benzyloxymethyltetrahydropyran-3-yl) thioacetate (prepared as described in Preparation 38) dissolved in 30 ml of methanol. The reaction mixture was stirred at -10 to 0 °C for 2 hours, and then 0.33 ml of methanesulphonic acid was added. The reaction mixture was then poured into water and extracted with ethyl acetate. The extracts were washed with water, dried over anhydrous magnesium sulphate and concentrated by evaporation under reduced pressure to give an oily residue. The residue was subjected to column chromatography through 30 g of silica gel. Those fractions eluted with mixtures of diethyl ether and hexane ranging from 3 : 97 to 5 : 95 by volume were worked up, to give 1.146 g of the title compound as an oil.
Nuclear Magnetic Resonance Spectrum
(90 MHz, $CDCl_3$) $\delta$ ppm:
1.10 - 2.40 (4H, multiplet);
1.66 (1H, doublet, J = 10 Hz);
2.95 - 3.25 (1H, multiplet);
3.25 - 3.85 (4H, multiplet);
3.85 - 4.20 (1H, multiplet);
4.55 (2H, AB-quartet, J = 12 Hz);
7.10 - 7.50 (5H, multiplet).
Infrared Absorption Spectrum ($CHCl_3$) $\nu_{max}$ $cm^{-1}$:
2580 (-SH).
Mass Spectrum (m/e): 238 ($M^+$).

| Elemental Analysis: | | | |
|---|---|---|---|
| Calculated for $C_{13}H_{18}O_2S$: | C, 65.51%; | H, 7.61%; | S, 13.45%. |
| Found: | C, 65.62%; | H, 7.83%; | S, 13.19%. |

PREPARATION 40

S-[dl-(cis-2-Benzyloxymethyltetrahydropyran-3-yl)] N-(heptadecyl)thiocarbamate

A similar reaction and treatment procedure to that described in Preparation 3 was repeated, but using 7.75 g of stearic acid in place of the nonadecanoic acid and 2.60 g of dl-cis-2-benzyloxymethyltetrahydropyran-3-thiol (prepared as described in Preparation 39) in place of the dl-trans-2-benzyloxymethyltetrahydropyran-3-ol, to give 5.29 g of the title compound as white crystals melting at 80 - 81 °C, after recrystallization from a mixture of diethyl ether and hexane.

Nuclear Magnetic Resonance Spectrum
(90 MHz, $CDCl_3$) δ ppm:
0.8 - 2.2 (37H, multiplet);
3.1 - 4.2 (8H, multiplet);
4.57 (2H, AB-quartet, $J_{AB}$ = 12 Hz);
5.31 (1H, multiplet);
7.35 (5H,, multiplet).

Infrared Absorption Spectrum ($CHCl_3$) $\nu_{max}$ $cm^{-1}$:
3430 (-NH-), 1670 (-SCO-).

| Elemental Analysis: | | | | |
|---|---|---|---|---|
| Calculated for $C_{31}H_{53}NO_3S$: | C, 71.63%; | H, 10.28%; | N, 2.69%; | S, 6.17%. |
| Found: | C, 71.73%; | H, 10.19%; | N, 2.64%; | S, 6.43%. |

PREPARATION 41

S-[dl-(cis-2-Hydroxymethyltetrahydropyran-3-yl)] N-(heptadecyl)thiocarbamate

A mixture of 200 ml of acetonitrile and 100 ml of methylene chloride was cooled with ice-water. 6.67 g of aluminium chloride and 7.50 g of sodium iodide were added, in turn, to the resulting mixture, followed by a solution of 5.20 g of S-[dl-(cis-2-benzyloxymethyltetrahydropyran-3-yl)] N-(heptadecyl)thiocarbamate (prepared as described in Preparation 40) dissolved in 100 ml of methylene chloride. The mixture was stirred at room temperature for 4 hours. It was then mixed with water and passed through a layer of Celite filter aid to remove insoluble materials. Methylene chloride was added, and the organic layer was separated. The aqueous layer was extracted with methylene chloride. The combined extracts and organic layer were washed, in turn, with an aqueous solution of sodium thiosulphate and with water, dried over anhydrous magnesium sulphate and concentrated by evaporation under reduced pressure. The residue was subjected to column chromatography through 90 g of silica gel. 4.04 g of the title compound were obtained, as white crystals melting at 90 - 91 °C (after recrystallisation from hexane), from those fractions eluted with mixtures of hexane, methylene chloride and ethyl acetate ranging from 10 : 10 : 1 to 2 : 2 : 1 by volume.

Nuclear Magnetic Resonance Spectrum
(90 MHz, $CDCl_3$) δ ppm:
0.7 - 2.3 (38H, multiplet);
3.15 - 4.15 (8H, multiplet);
5.53 (1H, multiplet).

Infrared Absorption Spectrum ($CHCl_3$) $\nu_{max}$ $cm^{-1}$:
3430 (-NH-, -OH), 1650 (-SCO-).

| Elemental Analysis: | | | | |
|---|---|---|---|---|
| Calculated for $C_{24}H_{47}NO_3S$: | C, 67.08%; | H, 11.02%; | N, 3.26%; | S, 7.46%. |
| Found: | C, 67.04%; | H, 10.98%; | N, 3.31%; | S, 7.63%. |

PREPARATION 42

dl-[cis-3-(N-Heptadecylcarbamoylthio)tetrahydropyran-2-yl]methyl N-(5-bromopentyl)carbamate

1.50 ml of diphenylphosphoryl azide and 1.62 ml of triethylamine were added to a solution of 1.36 g of 6-bromohexanoic acid dissolved in 40 ml of benzene. The mixture was then heated under reflux for 3 hours. At the end of this time, it was washed with a saturated aqueous solution of sodium bicarbonate and with a saturated aqueous solution of sodium chloride. It was then dried over anhydrous magnesium sulphate and

concentrated by evaporation under reduced pressure. The residue was dissolved in 20 ml of toluene, and 1.000 g of S-[dl-(cis-2-hydroxymethyltetrahydropyran-3-yl)] N-(heptadecyl)thiocarbamate (prepared as described in Preparation 41) was added to the solution so obtained. The resulting mixture was then heated on an oil bath kept at 85 °C for 64 hours. At the end of this time, the reaction mixture was evaporated to dryness under reduced pressure, and the residue was purified by column chromatography through 30 g of silica gel and by medium pressure liquid chromatography using a Lobar B column. 1.279 g of the title compound was obtained, as a waxy solid melting at 73 - 75 °C, from those fractions eluted with a 4 : 1 by volume mixture of hexane and ethyl acetate.

Nuclear Magnetic Resonance Spectrum
(90 MHz, $CDCl_3$) $\delta$ ppm:
0.7 - 2.2 (43H, multiplet);
2.9 - 4.3 (10H, multiplet);
3.38 (2H, triplet, J = 7 Hz);
4.77 (1H, multiplet);
5.33 (1H, multiplet).

Infrared Absorption Spectrum ($CHCl_3$) $\nu_{max}$ $cm^{-1}$:
3450 (-NH-), 1720 (-O-CO-) and 1675 (-S-CO-).

Mass Spectrum (m/e): 623, 621 ($M^+$ + 1) and 541 ($M^+$ - Br).

| Elemental analysis: | | | | | |
|---|---|---|---|---|---|
| Calculated for $C_{30}H_{57}BrN_2O_4S$: | C, 57.95%; | H, 9.24%; | Br, 12.85%; | N, 4.51%; | S, 5.61%. |
| Found : | C, 57.85%; | H, 9.34%; | Br, 12.85%; | N, 4.52%; | S, 5.28%. |

PREPARATION 43

dl-trans-2-Benzyloxymethyl-3-hexadecyloxytetrahydropyran

A solution of 2.22 g of dl-trans-2-benzyloxymethyltetrahydropyran-3-ol (prepared as described in Preparation 2) in 10 ml of dimethylformamide was added dropwise, with ice-cooling, to 10 ml of dimethylformamide containing 0.480 g of a 55% w/w suspension of sodium hydride in mineral oil. The reaction mixture was stirred at room temperature for 60 minutes, after which 5.49 g of hexadecyl bromide were added, and the resulting mixture was stirred for a further 4 hours. Finally, the mixture was stirred at 60 °C for 60 minutes and then cooled. It was then poured into 100 ml of water, and extracted twice with ethyl acetate. The combined extracts were washed with water, dried over anhydrous magnesium sulphate and condensed by evaporation under reduced pressure. The resulting oily residue was subjected to column chromatography through 100 g of silica gel. Those fractions eluted with mixtures of diethyl ether and hexane ranging from 1 : 20 to 1 : 10 by volume gave 3.82 g of the title compound as a solid having a low melting point, i.e. 28.5 - 29.5 °C (after recrystallization from cold methanol).

Nuclear Magnetic Resonance Spectrum
(90 MHz, $CDCl_3$) $\delta$ ppm:
0.7 - 2.4 (35H, multiplet);
3.0 - 4.2 (8H, multiplet);
4.60 (2H, AB-quartet, J = 13 Hz);
7.2 - 7.45 (5H, multiplet).

Mass Spectrum (m/e): 446 ($M^+$).

| Elemental Analysis: | | |
|---|---|---|
| Calculated for $C_{29}H_{50}O_3$: | C, 77.97%; | H, 11.28%. |
| Found: | C, 78.06%; | H, 11.31%. |

PREPARATION 44

dl-trans-3-Hexadecyloxy-2-hydroxymethyltetrahydropyran

1.5 g of 10% w/w palladium on activated carbon was added to a solution of 3.757 g of dl-trans-2-benzyloxymethyl-3-hexadecyloxytetrahydropyran (prepared as described in Preparation 43) in 150 ml of methanol, and the whole was mixed with hydrogen by shaking in a Paar's apparatus at room temperature under a pressure of 4 atmospheres (about 4 bars). After 20 hours, the catalyst was removed by filtration, and the solvent was then removed by distillation, to give 2.749 g of the title compound as a solid, melting at 41 - 42°C (after recrystallization from cold hexane).

Infrared Absorption Spectrum ($CHCl_3$) $\nu_{max}$ $cm^{-1}$:

3600, 3470 (-OH).

Mass Spectrum (m/e): 357 ($M^+$ + 1), 356 ($M^+$).

| Elemental Analysis: | | |
|---|---|---|
| Calculated for $C_{22}H_{44}O_3$: | C, 74.10%; | H, 12.43%. |
| Found: | C, 74.12%; | H, 12.11%. |

PREPARATION 45

dl-cis-2-Benzyloxymethyl-3-hexadecyloxytetrahydropyran

A mixture of 1.037 g of dl-cis-2-benzyloxymethyltetrahydropyran-3-ol (prepared as described in Preparation 33), 1.709 g of hexadecyl bromide, 0.77 g of potassium hydroxide and 15 ml of toluene was heated, with stirring, at 120°C for 10 hours. At the end of this time, the reaction mixture was cooled and then poured into water. The aqueous layer was extracted twice with diethyl ether. The organic layer and the extracts were combined, washed with water, dried over anhydrous magnesium sulphate and condensed by evaporation under reduced pressure. The resulting oily residue (3.3 g) was subjected to column chromatography through 50 g of silica gel. The fraction eluted with a 1 : 10 by volume mixture of diethyl ether and hexane gave 1.455 g of the title compound as a colourless oily substance.

Nuclear Magnetic Resonance Spectrum

(90 MHz, $CDCl_3$) $\delta$ ppm:

0.7 - 2.3 (35H, multiplet);
3.1 - 3.8 (5H, multiplet);
3.61 (2H, singlet);
3.85 - 4.15 (1H, multiplet);
4.55 (2H, AB-quartet, J = 13 Hz);
7.2 - 7.5 (5H, multiplet).

Mass Spectrum (m/e): 447 ($M^+$ + 1).

| Elemental Analysis: | | |
|---|---|---|
| Calculated for $C_{29}H_{50}O_3$: | C, 77.97%; | H, 11.28%. |
| Found: | C, 77.68%; | H, 11.16%. |

PREPARATION 46

dl-cis-3-Hexadecyloxy-2-hydroxymethyltetrahydropyran

1.409 g of dl-cis-2-benzyloxymethyl-3-hexadecyloxytetrahydropyran (prepared as described in Preparation 45) was dissolved in 100 ml of a 1 : 1 by volume mixture of methanol and ethanol. 0.70 g of a 10% w/w palladium on activated carbon catalyst was then added to the resulting solution. Catalytic reduction using the same procedure as described in Preparation 44 yielded 1.116 g of a crude substance, which was then subjected to column chromatography through 30 g of silica gel. Those fractions eluted with mixtures of

diethyl ether and hexane ranging from 1 : 20 to 1 : 5 by volume gave 1.031 of the title compound, melting at 42 - 43 °C (after recrystallisation from cold hexane).
Infrared Absorption Spectrum ($CHCl_3$) $\nu_{max}$ $cm^{-1}$:
3600, 3460(-OH).
Mass Spectrum (m/e): 357 ($M^+$ + 1), 356 ($M^+$).

| Elemental Analysis: | | |
|---|---|---|
| Calculated for $C_{22}H_{44}O_3$: | C, 74.10%; | H, 12.43%. |
| Found: | C, 73.85%; | H, 12.13%. |

PREPARATION 47

dl-[trans-3-Hexadecyloxytetrahydropyran-2-yl]methyl N-(5-bromopentyl)carbamate

1.09 ml of diphenylphosphoryl azide and 1.17 ml of triethylamine were added to a solution of 985 mg of 6-bromohexanoic acid dissolved in 30 ml of benzene. The mixture was then heated under reflux for 3 hours. At the end of this time, the reaction mixture was washed with a saturated aqueous solution of sodium bicarbonate and with a saturated aqueous solution of sodium chloride, dried over anhydrous magnesium sulphate and concentrated by evaporation under reduced pressure. The residue was dissolved in 12 ml of toluene. 600 mg of dl-(trans-3-hexadecyloxytetrahydropyran-2-yl)methanol (prepared as described in Preparation 44) and 1.17 ml of triethylamine were added to the solution, and the mixture was heated at 85 °C for 15 hours, after which it was evaporated to dryness under reduced pressure. The residue was subjected to column chromatography through 20 g of silica gel. Those fractions eluted with mixtures of hexane and ethyl acetate ranging from 17 : 3 to 4 : 1 by volume were collected and then purified by medium pressure liquid chromatography through a Lobar B column. 744 mg of the title compound were isolated, as an oil, from those fractions eluted with mixtures of the above solvent system.
Nuclear Magnetic Resonance Spectrum
(90 MHz, $CDCl_3$) $\delta$ ppm:
0.7 - 2.4 (41H, multiplet);
2.9 - 3.7 (7H, multiplet);
3.38 (2H, triplet, J = 7 Hz);
3.96 (1H, multiplet);
4.16 (1H, doublet of doublets, J = 12, 5 Hz);
4.40 (1H, doublet of doublets, J = 12, 3 Hz);
4.75 (1H, multiplet).
Mass Spectrum (m/e): 550, 548 ($M^+$ + 1) and 468 ($M^+$ - Br).

| Elemental analysis: | | | | |
|---|---|---|---|---|
| Calculated for $C_{28}H_{54}BrNO_4$: | C, 61,30%; | H, 9.92%; | Br, 14.56%; | N, 2.55%. |
| Found : | C, 61.19%; | H, 9.79%; | Br, 14.24%; | N, 2.67%. |

PREPARATION 48

4,5-Dihydrofurfuryl alcohol

358 g of a 15.08% w/w solution of butyllithium in hexane were added dropwise to 58.7 g of dihydrofuran in 350 ml of anhydrous tetrahydrofuran over a period of 30 minutes at 5 - 10 °C, whilst ice-cooling. The reaction mixture was then heated at 50 °C for 2 hours, whilst stirring. At the end of this time, the mixture was cooled to 0 °C in an ice-bath. 25.0 g of paraformaldehyde were added all at once to the reaction mixture, which was then heated for 2 hours at 50 °C. The reaction mixture was cooled to room temperature and then washed with 500 ml of ice-water; the aqueous layer was then extracted five times with methylene chloride. The organic layer and the extracts were combined, dried over anhydrous magnesium sulphate and concentrated by evaporation under reduced pressure to give 14 g of a residue. The residue was then

distilled under reduced pressure, to give 8.97 g of the title compound as a colourless oil boiling at 66 - 67 °C/7mmHg (about 933Pa). The title compound is preferably used as a reagent in the next step (e.g. Preparation 49) immediately after distillation because it dimerizes easily.
Nuclear Magnetic Resonance Spectrum
(90 MHz, $C_6D_6$) δ ppm:
2.21 (2H, broad triplet, J = 9 Hz);
2.98 (1H, broad triplet, J = 6 Hz);
3.98 (2H, doublet, J = 6 Hz);
4.00 (2H, triplet, J = 9 Hz);
4.68 (1H, multiplet).
Mass Spectrum (m/e): 200 ($M^+$ x 2), 101 ($M^+$ + 1), 100 ($M^+$).

PREPARATION 49

2-Benzyloxymethyl-4,5-dihydrofuran

7.69 g of sodium hydride (as a 55% w/w suspension in mineral oil) were suspended in 150 ml of dimethylformamide, and 17.64 g of 4,5-dihydrofurfuryl alcohol (prepared as described in Preparation 48) in 30 ml dimethylformamide were added dropwise at 5 - 10 °C over 30 minutes, whilst ice-cooling. The mixture was stirred at room temperature for 1 hour, and then 20.93 ml of benzyl bromide were added dropwise thereto at 10 - 15 °C over a further 30 minutes, whilst ice-cooling. The reaction mixture was stirred at room temperature for 1 hour, poured into 2 litres of water and extracted twice with ethyl acetate. The extracts were combined, washed with water, dried over anhydrous magnesium sulphate and evaporated to dryness under reduced pressure, to give 17.8 g of an oily residue. This residue was purified by column chromatography through 400 g of silica gel. Those fractions eluted with a 7 : 100 by volume mixture of hexane and diethyl ether afforded 3.71 g of the title compound as a colourless oil.
Nuclear Magnetic Resonance Spectrum
(90 MHz, $CDCl_3$) δ ppm:
2.65 (2H, triplet of multiplets, J = 10 Hz);
3.58 (2H, singlet);
4.03 (2H, multiplet);
4.39 (2H, triplet, J = 10 Hz);
4.93 (1H, multiplet);
7.35 (5H, multiplet).
Mass Spectrum (m/e): 190 ($M^+$).

PREPARATION 50

dl-trans-2-Benzyloxymethyltetrahydrofuran-3-ol

3.389 of 2-benzyloxymethyl-4,5-dihydrofuran (prepared as described in Preparation 49) were hydroborated in a similar manner to that described in Preparation 2. 3.93 g of the resulting crude product were purified by column chromatography through 120 g of silica gel. Those fractions eluted with a 1 : 2 by volume mixture of hexane and ethyl acetate were condensed by evaporation under reduced pressure, to afford 2.012 g of the title compound as a colourless oil.
Nuclear Magnetic Resonance Spectrum
(90 MHz, $CDCl_3$) δ ppm:
1.6 - 2.4 (2H, multiplet);
2.28 (1H, multiplet);
3.43 (1H, doublet of doublets, $J_1$ = 10 Hz & $J_2$ = 6 Hz);
3.60 (1H, doublet of doublets, $J_1$ = 10 Hz & $J_2$ = 4.5 Hz).
3.75 - 4.10 (3H, multiplet);
4.27 (1H, multiplet);
4.58 (2H, singlet);
7.30 (5H, singlet).
Mass Spectrum (m/e): 208 ($M^+$).

PREPARATION 51

dl-trans-2-Benzyloxymethyltetrahydrofuran-3-yl N-heptadecylcarbamate

1.967 of dl-trans-2-benzyloxymethyltetrahydrofuran-3-ol (prepared as described in Preparation 50) were treated in a manner similar to that described in Preparation 3 to give 3.711 g of the title compound as white crystals melting at 54 - 56 °C, after recrystallization from hexane.
Mass Spectrum (m/e): 489 ($M^+$), 398 ($M^+$ - $C_7H_7$).
Nuclear Magnetic Resonance Spectrum
(270 MHz, $CDCl_3$) $\delta$ ppm:
0.8 - 1.7 (33H, multiplet);
1.90 - 2.30 (2H, multiplet);
3.15 (2H, doublet of triplets, $J_1 = J_2 = 6.6$ Hz);
3.59 (2H, doublet, $J = 4.4$ Hz);
3.88 (1H, multiplet);
4.05 (2H, multiplet);
4.56 (2H, singlet);
4.67 (1H, multiplet);
5.10 (1H, multiplet);
7.32 (5H, multiplet).
Infrared Absorption Spectrum ($CHCl_3$) $\nu_{max}$ $cm^{-1}$:
3450 (-NH-), 1720 (-OCONH).

| Elemental Analysis: | | | |
|---|---|---|---|
| Calculated for $C_{30}H_{51}NO_4$:<br>Found: | C, 73.57%;<br>C, 73.09%; | H, 10.50%;<br>H, 10.33%; | N, 2.86%.<br>N, 2.87%. |

PREPARATION 52

dl-trans-2-Hydroxymethyltetrahydrofuran-3-yl N-heptadecylcarbamate

1.142 g of dl-trans-2-benzyloxymethyltetrahydrofuran-3-yl N-heptadecylcarbamate (prepared as described in Preparation 51) were subjected to debenzylation in a similar manner to that described in Preparation 4, to give 0.841 g of the title compound as white crystals melting at 77 - 78 °C, after recrystallization from a mixture of diethyl ether and hexane.
Nuclear Magnetic Resonance Spectrum
(270MHz, $CDCl_3$) $\delta$ ppm:
0.8 - 1.7 (33H, multiplet);
1.95 - 2.25 (2H, multiplet);
2.41 (1H, triplet, $J = 6.2$ Hz);
3.16 (2H, doublet of triplets, $J_1 = J_2 = 6.6$ Hz);
3.70 (2H, multiplet);
3.80 - 4.10 (3H, multiplet);
4.72 (1H, multiplet);
5.01 (1H, multiplet).
Infrared Absorption Spectrum ($CHCl_3$) $\nu_{max}$ $cm^{-1}$:
3600 (-OH), 3450 (-NH-), 1710 (-OCONH-).

| Elemental Analysis: | | | |
|---|---|---|---|
| Calculated for $C_{23}H_{45}NO_4$:<br>Found: | C, 69.13%;<br>C, 68.98%; | H, 11.35%;<br>H, 11.22%; | N, 3.50%.<br>N, 3.70%. |

Mass Spectrum (m/e): 400 ($M^+$ + 1), 399 ($M^+$), 368 ($M^+$ - $OCH_3$).

PREPARATION 53

6-[7-(Tetrahydropyran-2-yloxy)heptyloxymethyl]-3,4-dihydro-2H-pyran

(a) A solution of 2.32 ml of methanesulphonyl chloride dissolved in 10 ml of benzene was added dropwise, whilst ice-cooling, to a solution of 4.32 g of 7-(tetrahydropyran-2-yloxy)-1-heptanol and 5.56 ml of triethylamine dissolved in 80 ml of benzene. The mixture was stirred at room temperature for 1 hour, after which it was washed with water, dried over anhydrous magnesium sulphate and concentrated by evaporation under reduced pressure, to give 5.86 g of 7-(tetrahydropyran-2-yloxy)-heptyl methanesulphonate, as an oil.

(b) A solution of 2.28 g of 6-hydroxymethyl-3,4-dihydro-2H-pyran in 5 ml of dimethylformamide was added dropwise to a suspension of 0.87 g of sodium hydride (as a 55% w/w dispersion in mineral oil) in 20 ml of dimethylformamide. The mixture was stirred at room temperature for 1 hour, after which a solution of the whole of the methanesulphonate prepared as described in step (a) above dissolved in 5 ml of dimethylformamide was added dropwise to it. The mixture was then stirred on an oil bath kept at 70°C for 1 hour, after which it was poured into 200 ml of water and then extracted twice with diethyl ether. The combined extracts were washed with water, dried over anhydrous magnesium sulphate and concentrated by evaporation under reduced pressure. The oily residue (6.47 g) was subjected to column chromatography through 180 g of silica gel. 5.26 g of the title compound were obtained, as a colourless oil, from those fractions eluted with mixtures of hexane and diethyl ether ranging from 1 : 10 to 1 : 5 by volume.

Nuclear Magnetic Resonance Spectrum
($90$ MHz, $CDCl_3$) $\delta$ ppm:
1.1 - 2.2 (20H, multiplet);
3.25 - 4.15 (10H, multiplet);
4.60 (1H, multiplet);
4.80 (1H, multiplet).
Mass Spectrum (m/e): 312 ($M^+$) and 225 ($M^+ - C_5H_9O$).

| Elemental analysis | | |
|---|---|---|
| Calculated for $C_{18}H_{32}O_4$: | C, 69.19%; | H, 10.33%. |
| Found : | C, 69.16%; | H, 10.22%. |

PREPARATION 54

dl-trans-2-[7-(Tetrahydropyran-2-yloxy)heptyloxymethyl]tetrahydropyran-3-ol

5.26 g of 6-[7-(tetrahydropyran-2-yloxy)heptyloxymethyl]-3,4-dihydro-2H-pyran (prepared as described in Preparation 53) were hydroborated by following a procedure similar to that described in Preparation 2. The crude product (5.64 g) was subjected to column chromatography through 100 g of silica gel. 4.17 g of the title compound were obtained, as a colourless oil, from those fractions eluted with a 2 : 1 by volume mixture of hexane and ethyl acetate.

Nuclear Magnetic Resonance Spectrum
(90 MHz, $CDCl_3$) $\delta$ ppm:
1.1 - 2.3 (20H, multiplet);
3.10 (1H, doublet, J = 2.5 Hz);
3.15 - 4.10 (12H, multiplet);
4.60 (1H, multiplet).
Mass Spectrum (m/e); 329 ($M^+$ - 1).

| Elemental analysis: | | |
|---|---|---|
| Calculated for $C_{18}H_{34}O_5$: | C, 65.42%; | H, 10.37%. |
| Found : | C, 65.05% | H, 10.07%. |

PREPARATION 55

dl-7-(trans-3-Hexadecyloxytetrahydropyran-2-yl)-methoxy-1-heptanol

A solution of 1.652 g of dl-trans-2-[7-tetrahydropyran-2-yloxy)heptyloxymethyl]tetrahydropyran-3-ol (prepared as described in Preparation 54) in 5 ml of dimethylformamide was added dropwise at room temperature to a suspension of 0.24 g of sodium hydride (as a 55% w/w dispersion in mineral oil) in 5 ml of dimethylformamide. The reaction mixture was stirred at room temperature for 30 minutes, after which it was heated on an oil bath kept at 60°C for a further 30 minutes. At the end of this time, 1.83 g of hexadecyl bromide were added at room temperature, after which the mixture was heated on an oil bath kept at 60°C for 30 minutes, whilst stirring. The mixture was then cooled, and 0.24 g of sodium hydride (as a 55% w/w dispersion in mineral oil) was added to it at room temperature. The mixture was then heated on an oil bath kept at 60°C for 30 minutes. The reaction mixture was allowed to cool to room temperature and a further 1.83 g of hexadecyl bromide was added to it. The reaction mixture was then heated on an oil bath kept at 60°C for 30 minutes, whilst stirring, after which it was cooled and poured into water and then extracted three times with methylene chloride. The combined extracts were dried over anhydrous magnesium sulphate and concentrated by evaporation under reduced pressure. 40 ml of methanol and 0.5 ml of concentrated hydrochloric acid were added to a solution of the oily residue (5.74 g) dissolved in 15 ml of tetrahydrofuran, and the mixture was stirred at room temperature for 30 minutes. The solvent was then removed by distillation under reduced pressure, and the residue was diluted with water and then extracted twice with diethyl ether. The combined extracts were washed with a saturated aqueous solution of sodium bicarbonate and with a saturated aqueous solution of sodium chloride, dried over anhydrous magnesium sulphate and concentrated by evaporation under reduced pressure. The oily residue (5.14 g) was subjected to column chromatography through 90 g of silica gel. 2.123 g of the title compound were obtained, as a viscous oil, from those fractions eluted with a 2 : 1 by volume mixture of hexane and ethyl acetate.

Nuclear Magnetic Resonance Spectrum
(90 MHz, $CDCl_3$) δ ppm:
0.75 - 2.05 (44H, multiplet);
2.1 - 2.4 (1H, multiplet);
3.00 - 3.80 (12H, multiplet);
3.80 - 4.10 (1H, multiplet).

Mass Spectrum (m/e): 471 ($M^+$ + 1).

| Elemental analysis: | | |
|---|---|---|
| Calculated for $C_{29}H_{58}O_4$: | C, 73.99%; | H, 12.42%. |
| Found: | C, 73.69%; | H, 12.30%. |

PREPARATION 56

dl-2-[7-(Tetrahydropyran-2-yloxy)heptyloxymethyl]-tetrahydropyran-3-one

A solution of 2.387 g of dl-trans-2-[7-(tetrahydropyran-2-yloxy)heptyloxymethyl]tetrahydropyran-3-ol (prepared as described in Preparation 54) in 10 ml of methylene chloride was added all at once to a mixture of 2.33 g of pyridine chlorochromate, 1.78 g of sodium acetate and 10 ml of methylene chloride. The mixture was then stirred at room temperature for 3 hours, after which it was mixed with 30 ml of diethyl ether, and the solution was passed through a chromatography column containing 50 g of silica gel. The column was then washed with diethyl ether and the eluate was combined with the washings and then concentrated by evaporation under reduced pressure. The oily residue (2.24 g) was subjected to column chromatography through 60 g of silica gel. 1.786 g of the title compound was obtained, as an oil, from those fractions eluted with mixtures of hexane and ethyl acetate ranging from 3 : 1 to 2 : 1 by volume.

Infrared Absorption Spectrum ($CHCl_3$) $\nu_{max}$ $cm^{-1}$:
1730.

Mass Spectrum (m/e): 244 ($M^+$ - $C_5H_8O$) and 227 ($M^+$ - $C_5H_9O_2$).

PREPARATION 57

dl-cis-2-[7-(Tetrahydropyran-2-yloxy)heptyloxy-methyl]tetrahydropyran-3-ol

Following a procedure similar to that described in Preparation 33(b), 1.718 g of dl-2-[7-(tetrahydropyran-2-yloxy)heptyloxymethyl]tetrahydropyran-3-one (prepared as described in Preparation 56) was reduced to give 1.320 g of the title compound as a colourless oil.
Nuclear Magnetic Resonance Spectrum
(90 MHz, $CDCl_3$) δ ppm:
1.15 - 2.40 (20H, multiplet);
2.95 (1H, doublet, J = 4 Hz);
3.25 - 4.25 (10H, multiplet);
4.60 (1H, multiplet).
Mass Spectrum 330 ($M^+$) and 329 ($M^+$ - 1).

| Elemental analysis: | | |
|---|---|---|
| Calculated for $C_{18}H_{34}O_5$: | C, 65.42%; | H, 10.37%. |
| Found : | C, 65.22%; | H, 10.44%. |

PREPARATION 58

dl-7-(cis-3-Hexadecyloxytetrahydropyran-2-ylmethoxy)-1-heptanol

1.150 g of dl-cis-2-[7-(tetrahydropyran-2-yloxy)heptyloxymethyl]tetrahydropyran-3-ol (prepared as described in Preparation 57) were treated in a similar manner to that described in Preparation 55 to afford 1.282 g of the title compound as a viscous oil.
Nuclear Magnetic Resonance Spectrum
(90 MHz, $CDCl_3$) δ ppm:
0.7 - 2.35 (45H, multiplet);
2.16 (1H, multiplet);
3.10 - 3.85 (11H, multiplet);
3.90 - 4.20 (1H, multiplet).
Mass Spectrum (m/e): 470 ($M^+$) and 411 ($M^+$ - $C_3H_7O$).

| Elemental analysis: | | |
|---|---|---|
| Calculated for $C_{29}H_{58}O_4$: | C, 73.99%; | H, 12.42%. |
| Found : | C, 73.72%; | H, 12.31%. |

PREPARATION 59

5-(2-Methoxyethoxy)methoxy-1-pentanol

A solution of 50,00 g of 1,5-pentanediol dissolved in 100 ml of dimethylformamide was added dropwise to a mixture of 23.00 g of sodium hydride (as a 55% w/w dispersion in mineral oil) in 300 ml of dimethylformamide, while ice-cooling at 5 to 7°C. The mixture was stirred at room temperature for 1 hour, after which 65.79 g of 2-methoxyethoxymethyl chloride dissolved in 100 ml of dimethylformamide were added dropwise, whilst ice-cooling at 5 to 7°C. The mixture was then stirred at room temperature for 3 hours, after which it was poured into 2.5 litres of water and then extracted five times with methylene chloride. The combined extracts were washed with water, dried over anhydrous magnesium sulphate and concentrated by evaporation under reduced pressure. The residue was subjected to column chromatography through 850 g of silica gel, and 48.40 g of the title compound were obtained, as a colourless oil, from those fractions eluted with mixtures of methylene chloride and methanol ranging from 98 : 2 to 95 : 5 by

volume.
Nuclear Magnetic Resonance Spectrum
(90 MHz, $CDCl_3$) δ ppm:
1.3 - 1.8 (7H, multiplet);
3.39 (3H, singlet);
3.4 - 3.8 (8H, multiplet);
4.72 (2H, singlet).
Infrared Absorption Spectrum ($CHCl_3$) $\nu_{max}$ $cm^{-1}$:
3480(-OH) and 1050 (C-O-C).
Mass Spectrum (m/e): ($M^+$ + 1) and 117 [$M^+$ - $C_3H_7O_2$].

| Elemental analysis: | | |
|---|---|---|
| Calculated for $C_9H_{20}O_4$: | C, 56.23%; | H, 10.49%. |
| Found: | C, 55.95%; | H, 10.28%. |

PREPARATION 60

1-Bromo-5-(2-methoxyethoxy)methoxypentane

79.32 g of triphenylphospine were added, whilst ice-cooling (at 5 to 8 °C), to a solution of 48.40 g of 5-(2-methoxyethoxy)methoxy-1-pentanol (prepared as described in Preparation 59) and 100.19 g of carbon tetrabromide dissolved in 500 ml of methylene chloride. The mixture was stirred at room temperature for 1 hour, after which the solvent was removed by evaporation under reduced pressure. Diethyl ether was then added to the resulting residue. The insoluble materials were filtered off, and the filtrate was concentrated by evaporation under reduced pressure. The residue was subjected to column chromatography through 850 g of silica gel, and 57.87 g of the title compound were obtained, as a colourless oil, from those fractions eluted with a 1 : 9 by volume mixture of ethyl acetate and hexane.
Nuclear Magnetic Resonance Spectrum
(90 MHz, $CDCl_3$) δ ppm:
1.4 - 1.8 (4H, multiplet);
1.90 (2H, quintet, J = 6.5 Hz);
3.40 (3H, singlet);
3.4 - 3.8 (8H, multiplet);
4.72 (2H, singlet).
Infrared Absorption Spectrum ($CHCl_3$) $\nu_{max}$ $cm^{-1}$:
1045 (C-O-C) and 565 (-Br).
Mass Spectrum (m/e): 225, 223 ($M^+$ - $OCH_3$), 181 and 179 ($M^+$ - $OCH_2CH_2OCH_3$).

| Elemental analysis: | | | |
|---|---|---|---|
| Calculated for $C_{19}H_{19}BrO_3$: | C, 42.37%; | H, 7.51%; | Br, 31.32%. |
| Found: | C, 42.39%; | H, 7.31%; | Br, 31.13%. |

PREPARATION 61

Diethyl 2-[5-(2-methoxyethoxy)methoxypentyl]malonate

A solution of 37.80 of diethyl malonate dissolved in 20 ml of absolute ethanol was added dropwise to a solution of sodium ethoxide prepared by gradually adding 3.00 g of metallic sodium to 30 ml of absolute ethanol. To the mixture was added a solution of 30.01 g of 1-bromo-5-(2-methoxyethoxy)methoxypentane (prepared as described in Preparation 60) dissolved in 10 ml of absolute ethanol. The mixture was heated under reflux for 21 hours, after which it was allowed to cool, and then the solvent was distilled off. The residue was mixed with water, and the mixture was extracted three times with ethyl acetate. The combined extracts were washed with water, dried over anhydrous magnesium sulphate and concentrated by evaporation under reduced pressure. The residue was subjected to column chromatography through 850 g of silica gel, and 32.78 g of the title compound were obtained, as a colourless oil, from those fractions eluted with a 4 : 1 by volume mixture of hexane and ethyl acetate.

Nuclear Magnetic Resonance Spectrum
(90 MHz, $CDCl_3$) $\delta$ ppm:
1.26 (6H, triplet, J = 7.0 Hz);
1.3 - 2.1 (8H, multiplet);
3.2 - 3.8 (7H, multiplet);
3.40 (3H, singlet);
4.20 (4H, quartet, J = 7.0 Hz);
4.71 (2H, singlet).

Infrared Absorption Spectrum ($CHCl_3$) $\nu_{max}$ $cm^{-1}$:
1725 (-CO-) and 1040 (-C-O-C).

Mass Spectrum (m/e): ($M^+$ - $C_3H_7O$) and 259 ($M^+$ - $C_3H_7O_2$).

| Elemental analysis: | | |
|---|---|---|
| Calculated for $C_{16}H_{30}O_7$: | C, 57.47%; | H, 9.04%. |
| Found: | C, 57.56%; | H, 8.98%. |

## PREPARATION 62

### Ethyl hydrogen 2-[5-(2-methoxyethoxy)methoxypentyl]malonate

1.5 ml of an aqueous solution containing 0.203 g of potassium hydroxide was added, whilst ice-cooling, to a solution of 1.029 g of diethyl 2-[5-(2-methoxyethoxy)methoxypentyl]malonate (prepared as described in Preparation 61) dissolved in 1.5 ml of ethanol. The reaction mixture was stirred at room temperature for 5 hours, after which it was washed with diethyl ether. The aqueous layer was adjusted to a pH value of 2 by adding 10% w/v aqueous hydrochloric acid, and it was then extracted four times with methylene chloride. The combined extracts were dried over anhydrous magnesium sulphate and concentrated by evaporation under reduced pressure. The oily residue (0.94 g) was subjected to column chromatography through 20 g of silica gel, and 0.764 g of the title compound was obtained, as a colourless oil, from those fractions eluted with mixtures of hexane and ethyl acetate ranging from 2 : 1 to 1 : 1 by volume.

Nuclear Magnetic Resonance Spectrum
(90 MHz, $CDCl_3$) $\delta$ ppm:
1.28 (3H, triplet, J = 7 Hz);
1.15 - 2.10 (8H, multiplet);
3.25 - 3.80 (7H, multiplet);
3.42 (3H, singlet);
4.24 (2H, quartet, J = 7 Hz);
4.73 (2H, singlet).

| Elemental analysis: | | |
|---|---|---|
| Calculated for $C_{14}H_{26}O_7$: | C, 54.88%; | H, 8.55%. |
| Found: | C, 54.70%; | H, 8.45%. |

## PREPARATION 63

### Ethyl 2-[(dl-trans-3-heptadecylcarbamoyloxytetrahydropyran-2-yl)methoxycarbonylamino]-5-[(2-methoxyethoxy)methoxy]heptanoate

A mixture of 0.731 g of ethyl hydrogen 2-[5-(2-methoxyethoxy)methoxypentyl]malonate (prepared as described in Preparation 62), 0.51 ml of diphenylphosphoryl azide and 0.50 ml of triethylamine dissolved in 15 ml of benzene was heated under reflux for 4 hours. At the end of this time, the reaction mixture was

cooled; it was then washed, in turn, with a saturated aqueous solution of sodium bicarbonate and with water, dried over anhydrous magnesium sulphate and concentrated by evaporation under reduced pressure. The oily residue (0.748 g) was dissolved in 15 ml of toluene, and 0.329 of dl-(trans-2-hydroxymethyltetrahydropyran-3-yl) N-heptadecylcarbamate (prepared as described in Preparation 4) and 0.50 ml of triethylamine were added to the solution, which was then heated on an oil bath at 90 °C for 24 hours, whilst stirring. The solvent was then removed by distillation under reduced pressure, and the residue was subjected to column chromatography through 30 g of silica gel. 0.596 g of the title compound was obtained, as a waxy material, from those fractions eluted with mixtures of hexane and ethyl acetate ranging from 2 : 1 to 1 : 1 by volume.

Nuclear Magnetic Resonance Spectrum
($90$ MHz, $CDCl_3$) $\delta$ ppm:
- 0.8 - 2.5 (48H, multiplet);
- 3.17 (2H, doublet of triplets, $J_1 = J_2 = 7$ Hz);
- 3.43 (3H, singlet);
- 3.35 - 4.95 (14H, multiplet);
- 4.23 (2H, quartet, $J = 7$ Hz);
- 4.73 (2H, singlet);
- 5.37 (1H, multiplet).

Mass Spectrum (m/e): 641 ($M^+$ - $C_3H_7O_2$) and 627 ($M^+$ - $C_4H_9O_2$).

## PREPARATION 64

### Ethyl 2-[(dl-trans-3-heptadecylcarbamoyloxytetrahydropyran-2-yl)methoxycarbonylamino]-5-hydroxyheptanoate

0.2 ml of acetyl chloride was added, whilst ice-cooling, to a solution of 0.584 g of ethyl 2-[(dl-trans-3-heptadecylcarbamoyloxytetrahydropyran-2-yl)methoxycarbonylamino]-5-[(2-methoxyethoxy)methoxy]-heptanoate (prepared as described in Preparation 63) in 10 ml of ethanol. The mixture was then stirred at room temperature for 4.5 hours, after which it was diluted with 50 ml of ethyl acetate and then washed with a saturated aqueous solution of sodium bicarbonate and with water. The reaction mixture was then dried, and the solvent was removed by distillation under reduced pressure. The waxy residue (0.479 g) was subjected to column chromatography through 20 g of silica gel. 0.405 g of the title compound was obtained, as a waxy solid melting at 43 - 46 °C, from those fractions eluted with mixtures of hexane and ethyl acetate ranging from 1 : 1 to 2 : 1 by volume.

Nuclear Magnetic Resonance Spectrum
(90 MHz, $CD_3OD$) $\delta$ ppm:
- 0.8 - 2.40 (48H, multiplet);
- 3.08 (2H, triplet, $J = 7$ Hz);
- 3.2 - 4.7 (7H, multiplet);
- 3.55 (2H, triplet, $J = 6$ Hz);
- 4.17 (2H, quartet, $J = 7$ Hz).

Infrared Absorption Spectrum ($CHCl_3$) $\nu_{max}$ $cm^{-1}$:
3500 (-OH), 3450 (-NH) and 1720 (-O-CO-).

## PREPARATION 65

### dl-cis-Benzyloxymethyltetrahydropyran-3-yl N-heptadecylcarbamate

Following a procedure similar to that described in Preparation 3, but using 2.758 g of stearic acid, 2.09 ml of diphenylphosphoryl azide and 0.862 g of dl-cis-2-benzyloxymethyltetrahydropyran-3-ol (prepared as described in Preparation 33), 1.407 g of the title compound was obtained as crystals. These crystals melted at 63.0 - 65.0 °C after recrystallisation from a mixture of diethyl ether and hexane.

Nuclear Magnetic Resonance Spectrum
(90 MHz, $CDCl_3$) $\delta$ ppm:
- 0.7 - 2.4 (37H, multiplet);
- 3.0 - 3.8 (6H, multiplet);
- 3.9 4.2 (1H, multiplet);
- 4.55 (2H, AB-quartet, $J = 12$ Hz);

4.5 - 5.0 (2H, multiplet);
7.2 - 7.4 (5H, multiplet).
Infrared Absorption Spectrum ($CHCl_3$) $\nu_{max}$ $cm^{-1}$:
3460 (-NH-), 1715 (-O-CO-).
Mass Spectrum (m/e): 504 ($M^+$ + 1), 396 ($M^+$ - $OC_7H_7$), 382 ($M^+$ - $CH_2OC_7H_7$).

| Elemental analysis: | | | |
|---|---|---|---|
| Calculated for $C_{31}H_{53}NO_4$: | C, 73.91%; | H, 10.60%; | N, 2.78%. |
| Found: | C, 73.76%; | H, 10.72%; | N, 2.79%. |

PREPARATION 66

dl-cis-2-Hydroxymethyltetrahydropyran-3-yl N-heptadecylcarbamate

Following a procedure similar to that described in Preparation 4, but using a solution of 1.300 g of dl-cis-2-benzoyloxymethyltetrahydropyran-3-yl N-heptadecylcarbamate (prepared as described in Preparation 65) in 30 ml of ethanol and 0.819 of 10% w/w palladium on activated carbon, 0.977 g of the title compound was obtained as crystals. These crystals melted at 81.0 - 82.0°C after recrystallisation from diethyl ether.
Nuclear Magnetic Resonance Spectrum
(90 MHz, $CDCl_3$) $\delta$ ppm:
0.7 - 2.3 (37H, multiplet);
2.9 - 3.1 (1H, multiplet);
3.0 - 3.7 (6H, multiplet);
3.9 - 4.2 (1H, multiplet);
4.7 - 5.1 (2H, multiplet).
Infrared Absorption Spectrum ($CHCl_3$) $\nu_{max}$ $cm^{-1}$:
3600 (-OH), 3460 (-NH-), 1700 (-O-CO-).
Mass Spectrum (m/e): 413 ($M^+$), 382 ($M^+$ - $CH_2OH$).

PREPARATION 67

dl-cis-2-[N-(5-Bromopentyl)carbamoylmethyl]tetrahydropyran-3-yl-heptadecylcarbamate

Following a procedure similar to that described in Preparation 6, but using 1.245 g of 5-bromohexanoic acid, 1.37 ml of diphenylphosphoryl azide and 0.880 g of dl-cis-2-hydroxymethyltetrahydropyran-3-yl N-heptadecylcarbamate (prepared as described in Preparation 66), 0.933 g of the title compound was obtained as a waxy solid.

The compound melted at 95.0 - 96.0°C after recrystallisation from diethyl ether.
Nuclear Magnetic Resonance Spectrum
(90 MHz, $CDCl_3$) $\delta$ ppm:
0.7 - 2.3 (43H, multiplet ;
3.0 - 3.8 (6H, multiplet);
3.38 (2H, triplet, J=7 Hz);
4.00 (1H, multiplet);
4.13 (2H, doublet, J=7 Hz);
4.6 - 5.1 (3H, multiplet).
Infrared Absorption Spectrum ($CHCl_3$) $\nu_{max}$ $cm^{-1}$:
3460 (-NH-), 1720 (-O-CO-).
Mass Spectrum (m/e): 606, 604 ($M^+$), 524 ($M^+$ - HBr).

| Elemental analysis: | | | | |
|---|---|---|---|---|
| Calculated for $C_{30}H_{57}BrN_2O_5$: | C, 59.49%; | H, 9.49%; | Br, 13.19%; | N, 4.62%. |
| Found : | C, 59.61%; | H, 9.58%; | Br, 13.14%; | N, 4.74%. |

PREPARATION 68

3-Hydroxy-5-[4-(2-tetrahydropyranyl)oxybutyl]isoxazole

13.95 ml of a 15% by weight solution of butyllithium in hexane were added dropwise, at -25° to -15°C, to a solution of 3.08 ml of diisopropylamine in 100 ml of tetrahydrofuran. The mixture was stirred at -20°C for 15 minutes, after which a solution of 0.990 g of 3-hydroxy-5-methylisoxazole in 10 ml of tetrahydrofuran was added to the mixture at -20° to -10°C over a period of 10 minutes. The mixture was then stirred at -10°C for 30 minutes, after which it was cooled to -50°C, and then 3.35 g of 1-bromo-3-(2-tetrahydropyranyl)oxypropane were added to it all at once. The mixture was then stirred at 15 - 20°C for 2 hours, after which it was mixed with 1.20 ml of acetic acid. It was then poured into water. The organic layer was separated and the aqueous layer was adjusted to a pH value of 3 by adding 10% w/v aqueous hydrochloric acid followed by extraction twice with diethyl ether. The combined organic layer and extracts were washed with water, dried over anhydrous magnesium sulphate and concentrated by evaporation under reduced pressure, to give a residue, which was subjected to column chromatography through 60 g of silica gel. 1.720 g of the title compound was obtained, as a colourless oil, from the fractions eluted with mixtures of ethyl acetate and hexane ranging from 1 : 2 to 1 : 1 by volume.
Nuclear Magnetic Resonance Spectrum: (90 MHz, $CDCl_3$) δ ppm:
9.91 (1H, singlet);
5.68 (1H, singlet);
4.58 (1H, singlet);
3.2 - 4.1 (4H, multiplet);
1.3 - 2.1 (10H, multiplet).
Mass Spectrum (m/e): 241 ($M^+$), 186, 157 and 140.

PREPARATION 69

dl-trans-2-[5-(4-Hydroxybutyl)-3-isoxazolyloxymethyl]tetrahydropyran-3-yl N-heptadecylcarbamate

A solution of 0.321 g of dimethyl azodicarboxylate in 2 ml of tetrahydrofuran was added to a solution of 0.827 g of dl-(trans-2-hydroxymethyltetrahydropyran-3-yl) N-heptadecylcarbamate (prepared as described in Preparation 4), 0.482 g of 3-hydroxy-5-[4-(2-described in Preparation 68) and 0.577 g of triphenylphosphine in 14 ml of tetrahydrofuran, and the mixture was stirred at room temperature for 4.5 hours. At the end of this time, the solvent was removed by distillation under reduced pressure, after which the residue was subjected to column chromatography through 30 g of silica gel. The fractions eluted with a 1 : 1 by volume mixture of diethyl ether and hexane were collected to give 1.3 g of an oily material, which was dissolved in 10 ml of methanol. 0.05 g of camphorsulphonic acid was added to the resulting solution, and the mixture was stirred at room temperature for 16 hours. At the end of this time, the solvent was removed by distillation under reduced pressure, after which water was added to the residue, and the resulting mixture was extracted with methylene chloride. The extract was dried over anhydrous magnesium sulphate and concentrated by evaporation under reduced pressure, to give 1.19 g of a solid residue, which was subjected to column chromatography through 40 g of silica gel. 0.555 g of the title compound was obtained from the fractions eluted with a 1 : 1 by volume mixture of hexane and ethyl acetate. It melted at 86 - 87°C (after recrystallisation from a mixture of methylene chloride and diethyl ether).
Nuclear Magnetic Resonance Spectrum
(90 MHz, $CDCl_3$) δ ppm:
0.75 - 2.50 (42H, multiplet);
2.65 (2H, triplet, J = 6.5 Hz);
3.12 (2H, triplet of doublets, $J_1$ = 6.5 Hz, $J_2$ = 6 Hz);
3.3 - 4.9 (9H, multiplet);
5.67 (1H, singlet).
Infrared Absorption Spectrum ($CHCl_3$) $\nu_{max}$ $cm^{-1}$:

3600 (-OH), 3450 (-NH-) and 1720 (-OCONH-).
Mass Spectrum (m/e): 552 ($M^+$).

| Elemental Analysis: | | | |
|---|---|---|---|
| Calculated for $C_{31}H_{56}N_2O_6$: | C, 67.35%; | H, 10.21%; | N, 5.06%. |
| Found: | C, 67.57%; | H, 10.42%; | N, 4.83%. |

PREPARATION 70

2-Hydroxy-8-(tetrahydropyran-2-yloxy)octanenitrile

(a) A solution of 20.37 g of 7-(tetrahydropyran-2-yloxy)-1-heptanol in 200 ml of methylene chloride was added, whilst ice-cooling, to a mixture of 40.60 g of pyridinium chlorochromate, 3.86 g of sodium acetate and 200 ml of methylene chloride. The resulting mixture was stirred at 0 °C for 1 hour and then at room temperature for 3 hours, after which it was diluted with 1.5 litre of diethyl ether and passed through a column containing 250 g of silica gel. The eluate was concentrated by evaporation under reduced pressure, and the residue was subjected to column chromatography through 400 g of silica gel. 14.11 g of 7-(tetrahydropyran-2-yloxy)heptanal were obtained, as a colourless oil, from those fractions eluted with mixtures of hexane and ethyl acetate ranging from 40 : 1 to 9 : 1 by volume.
Nuclear Magnetic Resonance Spectrum
(60 MHz, $CDCl_3$) $\delta$ ppm:
1.1 - 2.1 (14H, multiplet);
2.42 (2H, multiplet);
3.1 - 4.1 (4H, multiplet);
4.56 (1H, multiplet);
9.83 (1H, triplet, J = 2 Hz).
(b) 14.11 g of the aldehyde prepared as described in step (a) above and 12.86 g of potassium cyanide were dissolved in 300 ml of a 1 : 1 by volume mixture of dioxane and water, and then 32.9 ml of 6N aqueous hydrochloric acid were added dropwise over a period of 10 minutes, whilst ice-cooling. When the dropwise addition was complete, the reaction mixture was stirred for 1 hour at 0 °C, after which it was poured into 600 ml of water and then extracted twice with ethyl acetate. The combined extracts were washed with water, dried over anhydrous magnesium sulphate and concentrated by evaporation under reduced pressure. The residue was subjected to column chromatography through 300 g of silica gel. 11.90 g of the title compound were obtained, as a colourless oil, from those fractions eluted with mixtures of hexane and ethyl acetate ranging from 1 : 9 to 1 : 4 by volume.
Nuclear Magnetic Resonance Spectrum
(60 MHz, $CDCl_3$) $\delta$ ppm:
1.2 - 2.2 (16H, multiplet);
3.1 - 4.2 (5H, multiplet);
4.40 (1H, multiplet);
4.58 (1H, multiplet).
Infrared Absorption Spectrum ($CHCl_3$) $\nu_{max}$ $cm^{-1}$:
3600, 3360 and 2250.

PREPARATION 71

Methyl 2,8-dihydroxyoctanoate

7.73 g of 2-hydroxy-8-(tetrahydropyran-2-yloxy)-octanenitrile (prepared as described in Preparation 70) were dissolved in a mixture of 80 ml of diethyl ether and 80 ml of methanol, and the solution was saturated with hydrogen chloride, whilst ice-cooling. The reaction mixture was stirred at room temperature for 3 hours, and then 160 ml of water was added to the mixture over a period of 10 minutes, whilst ice-cooling. It was then stirred at room temperature for 1 hour, after which it was diluted with water and then extracted six times with ethyl acetate. The combined extracts were washed with water, dried over anhydrous magnesium sulphate and concentrated by evaporation under reduced pressure. The residue was subjected to column chromatography through 120 g of silica gel. 3.75 g of the title compound were obtained, as a colourless oil,

from those fractions eluted with mixtures of hexane and ethyl acetate ranging from 4 : 1 to 2 : 1 by volume.
Nuclear Magnetic Resonance Spectrum
(90 MHz, $CDCl_3$) $\delta$ ppm:
1.2 - 2.0 (10H, multiplet);
1.67 (1H, multiplet);
2.85 (1H, multiplet);
3.63 (2H, triplet, J = 7 Hz);
4.70 (1H, multiplet).
Infrared Absorption Spectrum ($CHCl_3$) $\nu_{max}$ $cm^{-1}$:
3550 and 1735
Mass Spectrum (m/e): 191 ($M^+$ + 1), 131 and 113.

| Elemental Analysis: | | |
|---|---|---|
| Calculated for $C_9H_{18}O_4$: | C, 56.82%; | H, 9.54%. |
| Found: | C, 56.46%; | H, 9.45%. |

PREPARATION 72

2,8-Bis(tetrahydropyran-2-yloxy)-1-octanol

2.71 g of methyl 2,8-dihydroxyoctanoate (prepared as described in Preparation 71), 3.90 ml of 3,4-dihydro-2H-pyran and 0.072 of pyridine p-toluenesulphonate were dissolved in 50 ml of methylene chloride, and the resulting solution was stirred at room temperature for 15 hours. At the end of this time, the solvent was removed by evaporation under reduced pressure, and the residue was dissolved in 30 ml of tetrahydrofuran. The solution was added dropwise to a mixture of 1.081 g of lithium aluminium hydride and 30 ml of tetrahydrofuran over a period of 10 minutes, whilst ice-cooling. When the dropwise addition was complete, the mixture was stirred at room temperature for 1 hour, and then 4.3 ml of a 4% w/v aqueous solution of sodium hydroxide was added dropwise to it, whilst ice-cooling. The reaction mixture was filtered by passing it through a layer of a Celite filter aid, and insoluble materials were washed with 100 ml of tetrahydrofuran. The filtrate and the washings were combined and concentrated by evaporation under reduced pressure, and the residue was subjected to column chromatography through 100 g of silica gel. 4.51 g of the title compound were obtained, as a colourless oil, from those fractions eluted with mixtures of hexane and ethyl acetate ranging from 1 : 4 to 2 : 3 by volume.
Nuclear Magnetic Resonance Spectrum
(90 MHz, $CDCl_3$):
1.1 - 2.1 (22H, multiplet);
2.18 (1H, triplet, J = 6 Hz);
3.15 - 4.30 (9H, multiplet);
4.35 - 4.85 (2H, multiplet).
Mass Spectrum (m/e): 331, 330 ($M^+$ + 1) and 329.

| Elemental Analysis: | | |
|---|---|---|
| Calculated for $C_{18}H_{34}O_5$: | C, 65.42%; | H, 10.37%. |
| Found: | C, 65.04%; | H, 10.08 %. |

PREPARATION 73

6-[2,8-Bis(tetrahydropyran-2-yloxy)octyloxymethyl]-3,4-dihydro-2H-pyran

(a) A solution of 2.174 g of 6-hydroxymethyl-3,4-dihydro-2H-pyran and 5.31 ml of triethylamine in 45 ml of benzene was cooled in an ice bath, and then 2.21 ml of methanesulphonyl chloride was added to the solution. The reaction mixture was stirred at room temperature for 1 hour, after which it was washed with water, dried over anhydrous magnesium sulphate and concentrated by evaporation under reduced

pressure, to give 3.22 g of an oily residue.

(b) A solution of 1.049 g of 2,8-bis(tetrahydropyran2-yloxy)-1-octanol (prepared as described in Preparation 72) in 10 ml of dimethylformamide was added, whilst ice-cooling, to a mixture of 0.152 g of sodium hydride (as a 55% dispersion in mineral oil) and 10 ml of dimethylformamide. The mixture was then stirred at room temperature for 1 hour. At the end of this time, a solution of the residue prepared as described in step (a) above dissolved in 15 ml of dimethylformamide was added to the mixture, which was then stirred at room temperature for 19 hours. It was then poured into 350 ml of water and extracted twice with ethyl acetate. The combined extracts were washed with water, dried over anhydrous magnesium sulfate and concentrated by evaporation under reduced pressure. The residue was subjected to column chromatography through 60 g of silica gel. 0.934 g of the title compound was obtained, as a colourless oil, from the fractions eluted with a 9 : 1 by volume mixture of hexane and ethyl acetate.

Nuclear Magnetic Resonance Spectrum

(60 MHz, $CDCl_3$) δ ppm:

1.0 - 2.3 (26H, multiplet);

3.1 - 4.2 (11H, multiplet);

4.35 - 4.95 (3H, multiplet).

0.295 g of the starting material, 2,8-bis(tetrahydropyran-2-yloxy)-1-octanol (the compound of Preparation 72), was recovered from the fractions eluted with mixtures of hexane and ethyl acetate ranging from 1 : 4 to 1 : 1 by volume.

## PREPARATION 74

### dl-trans-2-[2,8-Bis(tetrahydropyran-2-yloxy)octyloxymethyl]tetrahydropyran-3-ol

1.46 ml of a 1M borane-tetrahydrofuran complex was added dropwise, whilst ice-cooling, to a solution of 0.934 g of 6-[2,8-bis(tetrahydropyran-2-yloxy)octyloxymethyl]-3,4-dihydro-2H-pyran (prepared as described in Preparation 73) in 3 ml of tetrahydrofuran. The mixture was then stirred at room temperature for 4.5 hours. At the end of this time, 0.80 ml of a 10% w/v aqueous solution of sodium hydroxide and 0.60 ml of a 30% v/v aqueous solution of hydrogen peroxide were added, in turn, to the mixture at 30 to 40 °C, and then the mixture was stirred at room temperature for a further 1 hour. The reaction mixture was then diluted with 50 ml of water and extracted twice with ethyl acetate. The combined extracts were washed with water, dried over anhydrous magnesium sulphate and concentrated by evaporation under reduced pressure, to give a residue, which was purified by column chromatography through 20 g of silica gel and by medium pressure liquid chromatography using a Lobar B column. 0.615 g of the title compound was obtained, as a colourless oil, from the fractions eluted with a 7 : 3 by volume mixture of hexane and ethyl acetate.

Nuclear Magnetic Resonance Spectrum

(90 MHz, $CDCl_3$) δ ppm:

1.1 - 2.5 (27H, multiplet);

2.8 - 4.2 (10H, multiplet);

4.4 - 5.0 (2H, multiplet).

Mass Spectrum (m/e): 445, 444 ($M^+$) and 443.

## PREPARATION 75

### dl-trans-2-(2,8-Dihydroxyoctoyloxymethyl)tetrahydropyran-3-yl N-heptadecylcarbamate

A solution of 0.94 g of stearic acid, 0.71 ml of diphenylphosphoryl azide and 0.46 ml of triethylamine in 20 ml of benzene was heated under reflux for 3 hours. At the end of this time, the mixture was cooled and washed with 20 ml of saturated aqueous solution of sodium bicarbonate and with water. The solvent was removed by distillation under reduced pressure, and 0.90 g of the residue and 0.586 g of dl-trans-2-[2,8-bis-(tetrahydropyran-2-yloxy)octyloxymethyl]tetrahydropyran-3-ol (prepared as described in Preparation 74) were dissolved in 15 ml of toluene. 0.46 ml of triethylamine were added to the resulting mixture, which was then heated under reflux on an oil bath kept at 100 °C for 88 hours. At the end of this time, the solvent was removed by distillation under reduced pressure, and the residue thus obtained was subjected to column chromatography through 20 g of silica gel. 0.695 g of an oily product was obtained by collecting the fractions eluted with mixtures of hexane and ethyl acetate ranging from 3 : 17 to 1 : 4 by volume and concentrating it by evaporation under reduced pressure. This oily product was dissolved in 15 ml of methanol, and 55 mg of p-toluenesulphonic acid were added to the resulting solution. The mixture was then

heated under reflux for 1 hour and cooled. 121 mg of sodium bicarbonate were added, and then the solvent was removed by distillation under reduced pressure. The residue was mixed with ethyl acetate and insoluble materials were filtered off. The filtrate was concentrated by evaporation under reduced pressure, to give a residue. This residue was subjected to column chromatography through 12 g of silica gel. 0.481 g of the title compound was obtained from the fractions eluted with mixtures of hexane and ethyl acetate ranging from 2 : 3 to 1 : 4 by volume. It melted at 67 - 68 °C (after recrystallisation from a mixture of diethyl ether and hexane).

Nuclear Magnetic Resonance Spectrum
($90$ MHz, $CDCl_3$) $\delta$ ppm:
0.7 - 2.3 (47H, multiplet);
2.13 (10H, multiplet);
2.9 - 4.2 (12H, multiplet);
4.4 - 5.0 (2H, multiplet).
Infrared Absorption Spectrum ($CHCl_3$) $\nu_{max}$ $cm^{-1}$:
3450 and 1710.
Mass Spectrum (m/e): 557 ($M^+$) and 456.

| Elemental Analysis: | | | |
|---|---|---|---|
| Calculated for $C_{32}H_{63}NO_6$:<br>Found: | C, 68.90%;<br>C, 68.45%; | H, 11.38%;<br>H, 11.75%; | N, 2.51%.<br>N, 2.69%. |

PREPARATION 76

dl-trans-2-[2-Hydroxy-8-(p-toluenesulphonyloxy)octyloxymethyl]tetrahydropyran-3-yl N-heptadecylcarbamate

0.458 g of dl-trans-2-[(2,8-dihydroxyoctyloxy)methyl]tetrahydropyran-3-yl N-heptadecylcarbamate (prepared as described in Preparation 75), 0.27 ml of triethylamine and 5 mg of 4-dimethylaminopyridine were dissolved in 10 ml of methylene chloride. 0.188 g of p-toluenesulphonyl chloride were added to the mixture, whilst ice-cooling, after which the mixture was stirred at room temperature for 14 hours. At the end of this time, the reaction mixture was washed with water, dried over anhydrous magnesium sulphate and concentrated by evaporation by column chromatography through 12 g of silica gel and by medium pressure liquid chromatography through a Lobar B column. 0.492 g of the title compound was obtained, as a white wax, from the fractions eluted with mixtures of hexane and ethyl acetate ranging from 3 : 2 by 1 : 1 by volume.

Nuclear Magnetic Resonance Spectrum
($90$ MHz, $CDCl_3$) $\delta$ ppm:
0.8 - 2.3 47H, multiplet);
2.45 (3H, multiplet);
2.50 (1H, multiplet);
3.0 - 4.9 (8H, multiplet);
4.03 (2H, doublet, J = 7 Hz);
4.4 - 5.0 (2H, multiplet);
7.37 (2H, doublet, J = 9 Hz);
7.81 (2H, doublet, J = 9 Hz).
Mass Spectrum (m/e): 712 ($M^+$ + 1) and 540.

| Elemental Analysis: | | | | |
|---|---|---|---|---|
| Calculated for $C_{39}H_{69}NO_8S$:<br>Found: | C, 65.79%;<br>C, 65.76%; | H, 9.77%;<br>H, 9.87%; | N, 1.97%;<br>N, 1.97%; | S, 4.50%.<br>S, 4.50%. |

PREPARATION 77

dl-trans-2-[2-Acetoxy-8-p-toluenesulphonyloxyoctyloxymethyl]tetrahydropyran-3-yl N-heptadecylcarbamate

0.04 ml of acetyl chloride was added dropwise, whilst ice-cooling, to a solution of 0.311 g of dl-trans-2-[2-hydroxy-8-(p-toluenesulphonyloxy)octyloxymethyl]tetrahydropyran-3-yl N-heptadecylcarbamate (prepared as described in Preparation 76) and 0.09 ml of triethylamine in 6 ml of benzene. The mixture was then stirred at room temperature for 3 hours, after which it was washed with water, dried over anhydrous magnesium sulphate and concentrated by evaporation under reduced pressure. The residue was subjected to column chromatography through 10 g of silica gel followed by medium pressure liquid chromatography through a Lobar B column. 163 mg of one isomer of the title compound (designated as Isomer I) were obtained, as a waxy material, from those fractions eluted with a 3 : 1 by volume mixture of hexane and ethyl acetate. It had an Rf value of 0.59 on thin layer chromatography on silica gel using a 1 : 1 by volume mixture of hexane and ethyl acetate as the developing solvent.

Nuclear Magnetic Resonance Spectrum
(60 MHz, $CDCl_3$) $\delta$ ppm:
0.7 - 2.6 (47H, multiplet);
2.05 (3H, singlet);
2.46 (3H, singlet);
2.8 - 4.0 (9H, multiplet);
4.00 (2H, triplet, J = 6 Hz);
4.2 - 5.2 (3H, multiplet);
7.36 (2H, doublet, J = 9 Hz);
7.82 (2H, doublet, J = 9 Hz).

Infrared Absorption Spectrum ($CHCl_3$) $\nu_{max}$ $cm^{-1}$:
3460 and 1720

72 mg of another isomer designated as Isomer II) were isolated, as an oil, from those fractions eluted with a 2 : 1 by volume mixture of hexane and ethyl acetate. It had an Rf value of 0.45 on thin layer chromatography under the same conditions as described above.

Nuclear Magnetic Resonance Spectrum
(60 MHz, $CDCl_3$) $\delta$ ppm:
0.7 - 2.6 (47H, multiplet);
2.27 (3H, singlet);
2.45 (3H, singlet);
2.8 - 4.0 (9H, multiplet);
4.00 (2H, triplet, J = 6 Hz);
4.2 - 5.3 (3H, multiplet);
7.37 (2H, doublet, J = 8 Hz);
7.82 (2H, doublet, J = 8 Hz).

Infrared Absorption Spectrum ($CHCl_3$) $\nu_{max}$ $cm^{-1}$:
3460, 1720 and 1710.

PREPARATION 78

S-[dl-(cis-2-Benzyloxymethyltetrahydropyran-3-yl)] N-(octadecyl)thiocarbamate

3.38 g of nonadecanoic acid were allowed to react with 1.124 g of dl-cis-2-benzyloxymethyltetrahydropyran-3-thiol (prepared as described in Preparation 39) in a similar manner to that described in Preparation 3 to give a crude product. This product was purified by column chromatography through 80 g of silica gel. Those fractions eluted with mixture of diethyl ether and hexane ranging from 1 : 5 to 1 : 2 by volume were worked up, to give 2.283 g of the title compound as white crystals melting at 85 - 86 °C (after recrystallisation from a mixture of hexane and diethyl ether).

Nuclear Magnetic Resonance Spectrum
(90 MHz, $CDCl_3$) $\delta$ ppm:
0.8 - 2.2 (39H, multiplet);
3.1 - 4.2 (8H, multiplet);
4.57 (2H, AB-quartet, $J_{AB}$ = 12Hz);
5.33 (1H, multiplet);

7.35 (5H, multiplet).
Infrared Absorption Spectrum ($CHCl_3$) $\nu_{max}$ $cm^{-1}$:
3450 and 1675 (-S-CO-).

| Elemental Analysis: | | | | |
|---|---|---|---|---|
| Calculated for $C_{32}H_{55}NO_3S$: | C, 71.99%; | H, 10.38%; | N, 2.62%; | S, 6.01%. |
| Found: | C, 71.87%; | H, 10.30%; | N, 2.67%; | S, 6.17%. |

PREPARATION 79

S-(dl-(cis-2-Hydroxymethyltetrahydropyran-3-yl)] N-(octadecyl)thiocarbamate

2.32 g of aluminium chloride and 2.61 g of sodium iodide were added succesively to a mixture of 70 ml of acetonitrile and 35 ml of methylene chloride, whilst ice-cooling. A methylene chloride solution containing 1.859 g of S-[dl-(cis-2-benzyloxymethyltetrahydropyran3-yl)] N-(octadecyl)thiocarbamate (prepared as described in Preparation 78) was then added, and the reaction mixture was stirred at room temperature for 7 hours. It was then diluted with water and purified by filtration with a Celite filter aid. The filtrate was mixed with methylene chloride. The methylene chloride layer was separated and the aqueous layer was extracted with methylene chloride. The combined extract and methylene chloride layer were washed successively with an aqueous solution of sodium thiosulphate and then with water, dried over anhydrous magnesium sulphate and concentrated by evaporation under reduced pressure. The residue was purified by column chromatography through 45 g of silica gel. Those fractions eluted with mixtures of ethyl acetate and hexane ranging from 1 : 5 to 1 : 2 by volume were worked up, to give 1.200 g of the title compound as white crystals, melting at 93 - 94 °C (after recrystallisation from diethyl ether).
Nuclear Magnetic Resonance Spectrum
(90 MHz, $CDCl_3$) $\delta$ ppm:
0.7 - 2.3 (40H, multiplet);
3.15 - 4.15 (8H, multiplet);
5.53 (1H, multiplet).
Infrared Absorption Spectrum ($CHCl_3$) $\nu_{max}$ $cm^{-1}$:
3450 (-NH-, -OH), 1650 (-S-CO-).

| Elemental Analysis: | | | | |
|---|---|---|---|---|
| Calculated for $C_{25}H_{49}NO_3S$: | C, 67.67%; | H, 11.13%; | N, 3.16%; | S, 7.23%. |
| Found: | C, 67.65%; | H, 11.24%; | N, 2.96%; | S, 7.51%. |

PREPARATION 80

2-Hydroxymethyl-7-(2-methoxyethoxy)methoxy-1-heptanol

A solution of 10.29 g of diethyl 2-[5-(2-methoxyethoxy)methoxypentyl]malonate (prepared as described in Preparation 61) dissolved in 100 ml of tetrahydrofuran was added dropwise to 2.40 g of lithium aluminium hydride dispersed in 100 ml of tetrahydrofuran, whilst ice-cooling (at 5 to 7 °C). The mixture was then stirred at room temperature for 3 hours, and then 9.60 ml of a 4% w/v aqueous solution of sodium hydroxide were added dropwise whilst maintaining the temperature at from 5 to 9 °C. The mixture was stirred for 30 minutes at room temperature, after which it was filtered with a Celite filter aid, and the filtrate was concentrated by evaporation under reduced pressure. The residue was subjected to column chromatography through 105 g of silica gel, and 6.94 g of the title compound were obtained, as a colourless oil, from those fractions eluted with mixtures of methylene chloride and methanol ranging from 98 : 2 to 95 : 5 by volume.
Nuclear Magnetic Resonance Spectrum
(90 MHz, $CDCl_3$) $\delta$ ppm:
1.2 - 2.0 (9H, multiplet);

2.52 (2H, multiplet);
3.40 (3H, singlet);
3.5 - 4.0 (10H, multiplet);
4.71 (2H, multiplet).

Infrared Absorption Spectrum ($CHCl_3$) $\nu_{max}$ $cm^{-1}$:
3420 (OH) and 1040 (C-O-C-).

Mass Spectrum (m/e): 251 ($M^+$ + 1).

| Elemental Analysis: | | |
|---|---|---|
| Calculated for $C_{12}H_{26}O_5$: | C, 57.57%; | H, 10.47%. |
| Found: | C, 57.46%; | H, 10.22%. |

PREPARATION 81

(2RS)-2-Benzyloxymethyl-7-(2-methoxyethoxy)methoxy-1-heptanol

A solution of 3.06 g of 2-hydroxymethyl-7-(2-methoxyethoxy)methoxy-1-heptanol (prepared as described in Preparation 80) dissolved in 20 ml of dimethylformamide was added dropwise to 587 mg of sodium hydride (as a 55% w/w dispersion in mineral oil) dispersed in 40 ml of dimethylformamide, whilst ice-cooling (at 5 to 7°C). The mixture was then stirred at room temperature for 1 hour, after which 1.60 ml of benzyl bromide were added dropwise, whilst ice-cooling (at 5 to 7°C). The reaction mixture was stirred at room temperature for 2 hours, after which it was poured into 300 ml of water and then extracts were washed with water, dried over anhydrous magnesium sulphate and concentrated by evaporation under reduced pressure. The resulting oily residue was subjected to column chromatography through 60 g of silica gel, and 2.37 g of the title compound were obtained, as a colourless oil, from the fractions eluted with mixtures of hexane and ethyl acetate ranging from 2 : 1 to 1 : 1 by volume.

Nuclear Magnetic Resonance Spectrum
(90 MHz, $CDCl_3$ $\delta$ ppm:
1.2 - 2.0 (9H, multiplet);
2.47 (1H, triplet J=6 Hz);
3.39 (3H, singlet);
3.4 - 3.8 (10H, multiplet);
4.52 (2H, singlet);
4.71 (2H, multiplet);
7.26 (5H, singlet).

Infrared Absorption Spectrum ($CHCl_3$) $\nu_{max}$ $cm^{-1}$:
3500 (-OH) and 1040 (C-O-C-).

Mass Spectrum (m/e): 341 ($M^+$ + 1).

| Elemental Analysis: | | |
|---|---|---|
| Calculated for $C_{19}H_{32}O_5$: | C, 67.03%; | H, 9.47%. |
| Found: | C, 66.88%; | H, 9.39%. |

EXPERIMENT 1

Inhibition of PAF-induced hypotension

The test animals employed were rats of the Wistar-Imamichi strain, each weighing between 350 and 450 g.

Under Inactin anesthesia (90 mg/kg administered intraperitoneally) the left femoral artery and vein of each test animal were cannulated to enable the arterial blood pressure to be monitored continuously and for drug administration, respectively. At intervals of about 5 minutes, each animal was given by intravenous injection 10 ng/kg of synthetic l-$C_{16:0}$ PAF until a steady hypotensive response was achieved. At this time,

the drug to be tested was administered by intravenous injection in doses increasing cumulatively by a factor of 4. Within 1 minute of this injection, a further 10 ng/kg of the $C_{16:0}$ PAF was administered. The hypotensive response to PAF was inhibited by the test drug in a dose-related manner.

The PAF was administered in the form of a solution in physiological saline containing 0.25% w/v bovine serum albumin. The test drugs were dissolved in physiological saline containing 20% v/v ethanol.

The 50% inhibitory dose ($ID_{50}$) was calculated from the dose-response curve constructed for the inhibition of PAF-induced hypotension.

This test was carried out using compounds of the invention, as well as the prior art compound CV-3988, disclosed in US Patent No. 4,408,052 and represented by the foregoing formula (B). The results are shown in the following Table 12.

EXPERIMENT 2

Inhibition of PAF-induced platelet aggregation in vitro

Blood was drawn from a rabbit and immediately mixed with one nineth of its volume of a 3.8% v/w aqueous solution of sodium citrate. A platelet-rich plasma (PRP) was obtained as a supernatant by centrifugation of the blood at 150 x g for 15 minutes at room temperature. The precipitated fraction was centrifuged for a further 15 minutes at 100 x g to obtain a platelet-poor plasma (PPP) as a supernatant. Appropriate proportions of this PRP and PPP were mixed to obtain a plasma having a platelet count of 6 x $10^5/\mu l$.

Platelet aggregation was determined by the method of Born et al. [G.V.R. Born et al., J. Physiol., 62, 67-68 (1962)] where an increase in light transmission is measured by an aggregometer.

25 $\mu l$ of a saline solution containing the compound to be tested at an appropriate concentration was added to 250 $\mu l$ of the above plasma. One minute thereafter, 25 $\mu l$ of a saline solution of synthetic $C_{16:0}$ PAF (at a concentration sufficient to give a final concentration of 1 x $10^{-8}$ - 3 x $10^{-8}$ M) was added and aggregation was observed for 5 minutes. The aggregation resulting from the addition of PAF alone, without the prior addition of the test compound, was taken at 100%.

The $IC_{50}$ values (i.e. concentrations to inhibit aggregation by 50%) were calculated from dose-response curves and are shown in Table 12.

Table 12

| Test compound | Inhibition of Hypotension ID$_{50}$ (mg/kg) | Inhibition of Platelet aggregation IC$_{50}$ (M) |
| --- | --- | --- |
| Cpd. of Ex. 1 | 0.17 | $1.1 \times 10^{-6}$ |
| Cpd. of Ex. 2 | 0.13 | $2.4 \times 10^{-6}$ |
| Cpd. of Ex. 3 | 0.11 | $9.7 \times 10^{-7}$ |
| Cpd. of Ex. 4 | 0.027 | $2.0 \times 10^{-7}$ |
| Cpd. of Ex. 7 | 0.17 | $4.2 \times 10^{-6}$ |
| Cpd. of Ex. 9 | 0.018 | $4.1 \times 10^{-7}$ |
| Cpd. of Ex. 13 | 0.030 | $3.9 \times 10^{-7}$ |
| Cpd. of Ex. 16 | 0.0074 | $1.5 \times 10^{-7}$ |
| Cpd. of Ex. 17 | 0.21 | $2.9 \times 10^{-6}$ |
| Cpd. of Ex. 18 | 0.08 | $4.4 \times 10^{-6}$ |
| Cpd. of Ex. 19 | 0.21 | $9.0 \times 10^{-7}$ |
| Cpd. of Ex. 20 | 0.06 | $2.7 \times 10^{-7}$ |
| CV - 3988 | 0.42 | $8.7 \times 10^{-6}$ |

**Claims**

**Claims for the following Contracting States : BE, CH, DE, FR, GB, IT, LI, LU, NL, SE**

**1.** Compounds of formula (I):

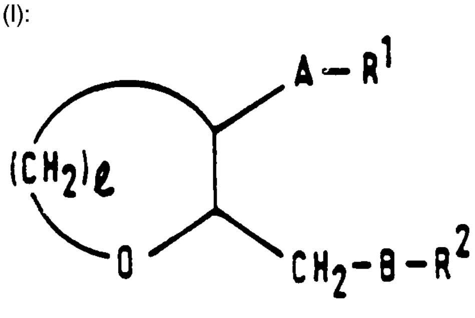

(I)

in which:
l is an integer of from 2 to 4;
A and B are the same or different and each represents an oxygen atom or a sulphur atom;
one of $R^1$ and $R^2$ represents an alkyl group containing from 8 to 22 carbon atoms, an aliphatic carboxylic acyl group containing from 8 to 22 carbon atoms or a group of formula (II):

$$\begin{array}{l} -CON-R^{3} \\ \quad\;\; | \\ \quad\;\; R^{5} \end{array} \qquad (II)$$

in which $R^3$ represents an alkyl group containing from 8 to 22 carbon atoms, and
$R^5$ represents a hydrogen atom, a $C_1$ - $C_4$ alkyl group, a $C_1$ - $C_4$ alkanoyl group or an aralkyl group in which the alkyl part is $C_1$ - $C_4$,
and the other of $R^1$ and $R^2$ represents a group of formula (III):

$$\begin{array}{l} -E-(CH_2)_m-(CH)_q-(CH_2)_n-Q \\ \qquad\qquad\qquad\;\; | \\ \qquad\qquad\qquad\;\; R^{4} \end{array} \qquad (III)$$

in which E represents a single bond, a bivalent heterocyclic group or a group of formula

$$\begin{array}{c} -C-, \\ \| \\ O \end{array} \qquad \begin{array}{c} -C-O- \\ \| \\ O \end{array} \quad \text{or} \quad \begin{array}{l} -C-N- \\ \;\;\|\;\;\; | \\ \;\;O\;\; R^{6} \end{array}$$

in which $R^6$ represents a hydrogen atom or an imino-protecting group;
m is the cypher 0 or an integer from 1 to 3;
n is the cypher 0 or an integer from 1 to 10;
q is the cypher 0 or the integer 1;
$R^4$ represents a hydroxy group, a $C_1$ - $C_4$ alkanoyloxy group, a $C_1$ - $C_4$ alkoxy group, a $C_7$ - $C_9$ aralkyloxy group, a carbamoyloxy group, an alkylcarbamoyloxy group in which the alkyl part is $C_1$ - $C_4$, a dialkylcarbamoyloxy group in which each alkyl part is $C_1$ - $C_4$, a mercapto group, a $C_1$ - $C_4$ alkylthio group, a $C_7$ - $C_9$ aralkylthio group, a $C_1$ - $C_4$ alkanoylthio group, a carbamoylthio group, an alkylcarbamoylthio group in which the alkyl part is $C_1$ - $C_4$, a dialkylcarbamoylthio group in which each alkyl part is $C_1$ - $C_4$ or a carboxy group;
Q represents a group of formula (IV):

$$\begin{array}{l} \quad\; R^{7} \\ \quad / \\ -N \\ \quad \backslash \\ \quad\; R^{8} \end{array} \qquad (IV)$$

in which $R^7$ and $R^8$ are the same or different and each represents a hydrogen atom or a $C_1$ - $C_6$ alkyl group,
or a monovalent heterocyclic group;
said heterocyclic groups having from 5 to 14 ring atoms, of which from 1 to 4 are nitrogen and/or oxygen and/or sulphur hetero-atoms, at least one of these being a nitrogen atom, and said heterocyclic group being unsubstituted or having at least one of substituents (a) and/or substituents (b), defined below;
substituents (a):
oxygen atoms and $C_6$ - $C_{14}$ aryl groups;
substituents (b):
halogen atoms, hydroxy groups, $C_1$ - $C_4$ alkyl groups, $C_1$ - $C_4$ haloalkyl groups, $C_1$ - $C_4$ hydroxyalkyl groups, $C_1$ - $C_4$ alkoxy groups, $C_1$ - $C_6$ alkanoyl groups, $C_3$ - $C_6$ alkenoyl groups, $C_7$ - $C_{15}$ aromatic carboxylic acyl groups, carbamoyl groups, $C_7$ - $C_{15}$ aralkyl groups, $C_2$ - $C_5$ alkoxycarbonyl groups, cyano groups, amino groups, alkylamino groups in which the alkyl part is $C_1$ - $C_4$, dialkylcarbamoyloxy groups in which each alkyl part is $C_1$ - $C_4$ and nitro groups;
said aryl groups and the aryl parts of aralkyl groups and aromatic acyl groups having from 6 to 14 ring atoms and being unsubstituted or having at least one of substituents (b), defined above;
and pharmaceutically acceptable salts and esters thereof.

**2.** Compounds according to Claim 1, having the formula (I) as shown in Claim 1 in which one of $R^1$ and $R^2$ represents a group of formula (III'):

$$-E-(CH_2)_m-\underset{COO^-}{\underset{|}{CH}}-(CH_2)_n-Q^+ \qquad (III')$$

in which E, m and n are as defined in Claim 1 and $Q^+$ represents a quaternised heterocyclic group or a group of formula (IV'):

$$-\overset{R^7}{\overset{/}{N^+}}\underset{\setminus R^8}{}-R^9 \qquad (IV')$$

in which $R^7$, $R^8$ and $R^9$ are the same or different and each represents a hydrogen atom or a $C_1$ - $C_6$ alkyl group.

**3.** Compounds according to Claim 1 or Claim 2, in which said salt is in the form of a quaternary ammonium salt or an acid addition salt, in which Q represents a group of formula (IV"):

$$-\overset{R^7}{\overset{/}{N^+}}\underset{\setminus R^8}{}-R^9 \qquad Z^- \qquad (IV")$$

in which $R^7$, $R^8$ are as defined in Claim 1, $R^9$ is as defined in claim 2 and $Z^-$ represents a complementary pharmaceutically acceptable anion,
or a quaternised heterocyclic group together with a complementary pharmaceutically acceptable anion.

**4.** Compounds according to Claim 3, in which said complementary pharmaceutically acceptable anion is a halogen atom, an anionic residue of another mineral acid, a $C_1$ - $C_6$ alkylsulphonyloxy group, an arylsulphonyloxy group or an anion derived from an organic carboxylic acid or an amino acid.

**5.** Compounds according to any one of the preceding Claims, which are represented by the formula (Ia):

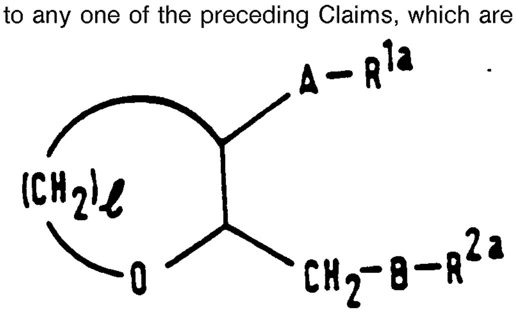

(Ia)

in which: l, A and B are as defined in Claim 1, and
one of $R^{1a}$ and $R^{2a}$ represents an alkyl group containing from 8 to 22 carbon atoms, an aliphatic carboxylic acyl group containing from 8 to 22 carbon atoms or a group of formula (II), as defined in Claim 1, and the other of $R^{1a}$ and $R^{2a}$ represents a group of formula (IIIa):

$$-E-(CH_2)_m-\underset{R^{4a}}{\underset{|}{(CH)}}_q-(CH_2)_n-Q \qquad (IIIa)$$

in which m, n, q and Q are as defined in Claim 1, and
E represents a single bond, a bivalent heterocyclic group or a group of formula

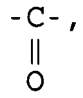, 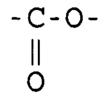 or 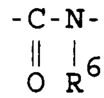

in which $R^6$ is as defined in Claim 1;
$R^{4a}$ represents a hydroxy group, a $C_1$ - $C_4$ alkanoyloxy group, a $C_1$ - $C_4$ alkoxy group, a $C_7$ - $C_9$ aralkyloxy group, a carbamoyloxy group, an alkylcarbamoyloxy group in which the alkyl part is $C_1$ - $C_4$, a dialkylcarbamoyloxy group in which each alkyl part is $C_1$ - $C_4$, a mercapto group, a $C_1$ - $C_4$ alkylthio group, a $C_7$ - $C_9$ aralkylthio group, a $C_1$ - $C_4$ alkanoylthio group, a carbamoylthio group, an alkylcarbamoylthio group in which the alkyl part is $C_1$ - $C_4$, a dialkylcarbamoylthio group in which each alkyl part is $C_1$ - $C_4$, a carboxy group or an alkoxycarbonyl group in which the alkoxy part is $C_1$ - $C_4$;
said heterocyclic groups having from 5 to 10 ring atoms, of which from 1 to 4 are nitrogen and/or oxygen and/or sulphur hetero-atoms, the heterocyclic groups represented by E being unsubstituted and the heterocyclic groups represented by Q being unsubstituted or having at least one $C_1$ - $C_4$ alkyl substituent.

**6.** Compounds according to any one of the preceding Claims, in which the cyclic ether portion of the compound has the 3S configuration.

**7.** Compounds according to Claim 6, in which the cyclic ether portion of the compound has the (3S, 2R) configuration.

**8.** Compounds according to Claim 6, in which the cyclic ether portion of the compound has the (3S, 2S) configuration.

**9.** Compounds according to any one of Claims 1 to 4 and 6 to 8, in which:
l is an integer of from 2 to 4;
A and B are the same or different and each represents an oxygen atom or a sulphur atom;
one of $R^1$ or $R^2$ represents an alkyl group containing from 10 to 22 carbon atoms, an aliphatic carboxylic acyl group containing from 10 to 22 carbon atoms or a group of formula (IIa):

$-CONH-R^3$ (IIa)

in which $R^3$ represents an alkyl group containing from 10 to 22 carbon atoms,
and the other represents a group of formula (IIIb):

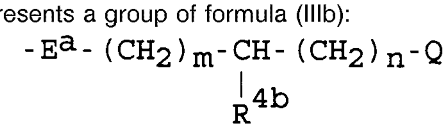

(IIIb)

in which $E^a$ represents a single bond or a group of formula

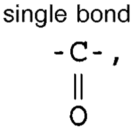, 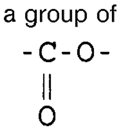 or 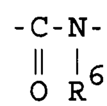

in which $R^6$ represents a hydrogen atom or an imino-protecting group;
m is the cypher 0 or an integer from 1 to 3;
n is the cypher 0 or an integer from 1 to 10;
$R^{4b}$ represents a hydrogen atom, a carboxy group or an alkoxycarbonyl group in which the alkoxy part is $C_1$ - $C_4$;
Q represents a group of formula (IV):

$$-N\genfrac{}{}{0pt}{}{/R^{7}}{\backslash R^{8}} \qquad (IV)$$

in which $R^7$ and $R^8$ are the same or different and each represents a hydrogen atom or a $C_1$ - $C_6$ alkyl group,
or a monovalent heterocyclic group having from 5 to 7 ring atoms, of which from 1 to 4 atoms are nitrogen and/or oxygen and/or sulphur hetero-atoms, at least one of these being a nitrogen atom, and the heterocyclic groups represented by Q being unsubstituted or having at least one substituent selected from $C_1$ - $C_4$ alkyl groups, $C_1$ - $C_4$ hydroxyalkyl groups, $C_1$ - $C_4$ alkoxy groups and halogen atoms or such a heterocyclic group having another ring fused thereto;
and pharmaceutically acceptable salts thereof.

**10.** Compounds according to any one of Claims 1 to 4 and 6 to 8, in which:
l is an integer of from 2 to 4;
A and B are the same or different and each represents an oxygen atom or a sulphur atom;
one of $R^1$ or $R^2$ represents an alkyl group containing from 10 to 22 carbon atoms, an aliphatic carboxylic acyl group containing from 10 to 22 carbon atoms or a group of formula (IIa):

$$-CONH-R^3 \qquad (IIa)$$

in which $R^3$ represents an alkyl group containing from 10 to 22 carbon atoms
and the other represents a group of formula (IIIc):

$$-E^{b}-\underset{R^{4c}}{\underset{|}{(CH)}}_{q}-(CH_2)_n-Q \qquad (IIIc)$$

in which $E^b$ represents a group of formula $-(CH_2)_{m'}-$ or a bivalent heterocyclic group;
m' is an integer from 1 to 3;
n is the cypher 0 or an integer from 1 to 10;
q is the cypher 0 or the integer 1;
$R^{4c}$ represents a hydroxy group, a $C_1$ - $C_4$ alkanoyloxy group, a $C_1$ - $C_4$ alkoxy group, a $C_7$ - $C_9$ aralkyloxy group, a carbamoyloxy group, an alkylcarbamoyloxy group in which the alkyl part is $C_1$ - $C_4$, a dialkylcarbamoyloxy group in which each alkyl part is $C_1$ - $C_4$, a mercapto group, a $C_1$ - $C_4$ alkanoylthio group, a $C_1$ - $C_4$ alkylthio group, a $C_7$ - $C_9$ aralkylthio group, a carbamoylthio group, an alkylcarbamoylthio group in which the alkyl part is $C_1$ - $C_4$ or a dialkylcarbamoylthio group in which each alkyl part is $C_1$ - $C_4$;
Q represents a group of formula (IV):

$$-N\genfrac{}{}{0pt}{}{/R^{7}}{\backslash R^{8}} \qquad (IV)$$

in which $R^7$ and $R^8$ are the same or different and each represents an hydrogen atoms and $C_1$ - $C_6$ alkyl groups,
or a monovalent heterocyclic group having from 5 to 7 ring atoms, of which from 1 to 4 atoms are nitrogen and/or oxygen and/or sulphur hetero-atoms, at least one of these being a nitrogen atom, and the heterocyclic groups represented by Q being unsubstituted or having at least one substituent selected from $C_1$ - $C_4$ alkyl groups, $C_1$ - $C_4$ hydroxyalkyl groups, $C_1$ - $C_4$ alkoxy groups, carbamoyl groups and halogen atoms or such a heterocyclic group having another ring fused thereto;

and pharmaceutically acceptable salts thereof.

**11.** Compounds according to any one of the preceding Claims, in which:
l is the integer 2 or 3.

**12.** Compounds according to Claim 11, in which:
l is the integer 3.

**13.** Compounds according to any one of the preceding Claims, in which:
A represents an oxygen or sulphur atom and B represents an oxygen atom.

**14.** Compounds according to any one of the preceding Claims, in which:
$R^1$ represents $C_8$ - $C_{22}$ alkyl group or a group of formula (II):

$$\begin{array}{l} -CON-R^3 \\ \quad\;\;| \\ \quad\;R^5 \end{array} \qquad (II)$$

in which $R^3$ and $R^5$ are as defined in Claim 1.

**15.** Compounds according to Claim 14, in which $R^1$ represents said group of formula (II).

**16.** Compounds according to Claim 14 or Claim 15, in which $R^5$ represents a hydrogen atom or a $C_2$ - $C_4$ alkanoyl group.

**17.** Compounds according to any one of Claims 14 to 16, in which $R^3$ represents a $C_{13}$ - $C_{20}$ alkyl group.

**18.** Compounds according to any one of the preceding Claims, in which n is an integer from 1 to 7.

**19.** Compounds according to any one of the preceding Claims, in which:
Q represents a thiazolyl, pyridyl, quinolyl, isoquinolyl or imidazolyl group or a thiazolyl, pyridyl, quinolyl, isoquinolyl or imidazolyl group containing at least one $C_1$ - $C_4$ alkyl substituent.

**20.** Compounds according to Claim 19, in which Q represents a thiazolyl or pyridyl group.

**21.** Compounds according to any one of the preceding Claims, in which E represents an isoxazolediyl or thiazolediyl group.

**22.** Compounds according to Claim 21, in which E represents a 3,5-isoxazolediyl group.

**23.** Compounds according to any one of the preceding Claims, in which:
$R^2$ represents a group of formula (III):

$$-E-(CH_2)_m-\underset{\displaystyle R^4}{\underset{|}{(CH)}}{}_q-(CH_2)_n-Q \qquad (III)$$

in which E, Q, $R^4$, m, n and q are as defined in Claim 1.

**24.** Compounds according to any one of the preceding Claims, in which:
in the group represented by $R^2$, the group of formula

$$-E-(CH_2)_m-\underset{\displaystyle R^4}{\underset{|}{(CH)}}{}_q-$$

is a group of formula:

$$-\underset{\underset{O}{\|}}{C}-\underset{\underset{R^6}{|}}{N}- \quad \text{or} \quad -CH_2-\underset{\underset{R^4}{|}}{CH}-$$

[in which $R^4$ and $R^6$ are as defined in Claim 1] or an isoxazolediyl group.

**25.** Compounds according to Claim 24, in which $R^6$ is a hydrogen atom or an acetyl group.

**26.** Compounds according to any one of the preceding Claims, in which:
$R^1$ represents a group of formula (II):

$$-CO\underset{\underset{R^5}{|}}{N}-R^3 \qquad (II)$$

in which $R^3$ and $R^5$ are as defined in Claim 1, and
$R^2$ represents a group of formula (III):

$$-E-(CH_2)_m-\underset{\underset{R^4}{|}}{(CH)}_q-(CH_2)_n-Q \qquad (III)$$

in which E, *m*, *n*, *q*, $R^4$ and Q are as defined in Claim 1.

**27.** Compounds according to Claim 26, in which $R^5$ represents a hydrogen atom or a $C_2$ - $C_4$ alkanoyl group.

**28.** Compounds according to Claim 26, in which:
the group of formula

$$-E-(CH_2)_m-\underset{\underset{R^4}{|}}{(CH)}_q-$$

in the group represented by $R^2$ is a group of formula:

$$-\underset{\underset{O}{\|}}{C}-\underset{\underset{R^6}{|}}{N}- \quad \text{or} \quad -CH_2-\underset{\underset{R^4}{|}}{CH}-$$

in which $R^6$ is as defined in Claim 1,
or an isoxazolediyl group.

**29.** Compounds according to Claim 28, in which $R^6$ represents a hydrogen atom or an acetyl group.

**30.** A 3-{6-ethoxycarbonyl-6-[(3-heptadecylcarbamoyloxytetrahydropyran-2-yl)methoxycarbonylamino]-hexyl}thiazolium salt.

**31.** A *dl*-3-{6-ethoxycarbonyl-6-[(*trans*-3-heptadecylcarbamoyloxytetrahydropyran-2-yl)-methoxycarbonylamino]hexyl}thiazolium salt.

**32.** A 3-{5-[(3-heptadecylcarbamoyloxytetrahydropyran-2-yl)methoxycarbonylamino]pentyl}thiazolium salt.

**33.** A *dl*-3-{5-[(*trans*-3-heptadecylcarbamoyloxytetrahydropyran-2-yl)methoxycarbonylamino]-pentyl}thiazolium salt.

**34.** A 1-ethyl-2-{N-acetyl-N-[3-(N-heptadecylcarbamoyloxy)tetrahydropyran-2-ylmethoxycarbonyl]-aminomethyl}pyridinium salt.

**35.** A dl-1-ethyl-2-{N-acetyl-N-[trans-3-(N-heptadecylcarbamoyloxy)tetrahydropyran-2-ylmethoxycarbonyl]-aminomethyl}pyridinium salt.

**36.** A 3-{7-acetoxy-8-[(3-heptadecylcarbamoyloxytetrahydropyran-2-yl)methoxy]octyl}thiazolium salt.

**37.** A 1-ethyl-2-{N-acetyl-N-[3-(N-acetyl-N-heptadecylcarbamoylthio)tetrahydropyran-2-ylmethoxycarbonyl]-aminomethyl}pyridinium salt.

**38.** A dl-1-ethyl-2-{N-acetyl-N-[cis-3-(N-acetyl-N-heptadecylcarbamoylthio)tetrahydropyran-2-ylmethoxycarbonyl]aminomethyl}pyridinium salt.

**39.** A 1-ethyl-2-{N-[3-(N-acetyl-N-heptadecylcarbamoylthio)tetrahydropyran-2-ylmethoxycarbonyl]-aminomethyl}pyridinium salt.

**40.** A dl-1-ethyl-2-{N-[cis-3-(N-acetyl-N-heptadecylcarbamoylthio)tetrahydropyran-2-ylmethoxycarbonyl]-aminomethyl}pyridinium salt.

**41.** A 3-{4-[3-(3-heptadecylcarbamoyloxytetrahydropyran-2-yl)methoxy-5-isoxazolyl]butyl}thiazolium salt.

**42.** A dl-3-{4-[3-(trans-3-heptadecylcarbamoyloxytetrahydropyran-2-yl)methoxy-5-isoxazolyl]-butyl}thiazolium salt.

**43.** A pharmaceutical composition for the treatment of inflammation or shock, comprising a PAF antagonist in combination with a pharmaceutically acceptable carrier or diluent, in which the PAF antagonist is at least one compound according to any one of the preceding Claims.

**44.** The use for the manufacture of a medicament for the treatment or prophylaxis of asthma, inflammation or shock of at least one compound of formula (I) and/or pharmaceutically acceptable salt or ester thereof, as claimed in any one of Claims 1 to 42.

**45.** A process for preparing a compound according to any one of Claims 1 to 42, which process comprises:
(i) reacting a compound of formula (D):

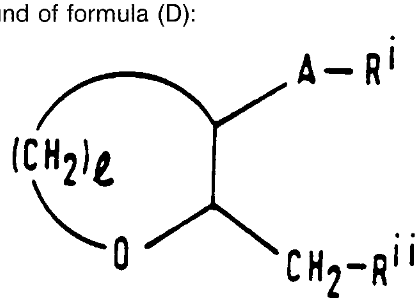

(D)

[in which:
(a) $R^i$ represents a group of formula $-A-R^{1x}$ and
$R^{ii}$ represents a group of formula -B-H; or
(g) $R^i$ represents a group of formula $-A-R^{10}$ and
$R^{ii}$ represents a group of formula -B-H;
in which:
A and B are as defined above;
$R^{1x}$ represents an alkyl group containing from 8 to 22 carbon atoms, an aliphatic carboxylic acyl group containing from 8 to 22 carbon atoms or a group of formula (II):

$$-\underset{\underset{R^5}{|}}{CON}-R^3 \qquad (II)$$

in which $R^3$ and $R^5$ are as defined above;
$R^{10}$ represents a hydroxy-protecting or mercapto-protecting group];
or a compound of formula (D) in which any active group is protected, with a compound of formula (VI):

$$Y-(CH_2)_m-\underset{\underset{R^{4'}}{|}}{(CH)}_q-(CH_2)_n-Q' \qquad (VI)$$

or of formula (VII):

$$HB-E^f-(CH_2)_m-\underset{\underset{R^{4'}}{|}}{(CH)}_q-(CH_2)_n-Q' \qquad (VII)$$

or of formula (XIX):

$$W-\underset{\underset{O}{\|}}{C}-(CH_2)_m-\underset{\underset{R^{4'}}{|}}{(CH)}_q-(CH_2)_n-Q'' \qquad (XIX)$$

or of formula (XX):

$$O=C=N-(CH_2)_m-\underset{\underset{R^{4'}}{|}}{(CH)}_q-(CH_2)_n-Q'' \qquad (XX)$$

or with a compound of formula (XXII) followed by a compound of formula (XXIV):

$$Y-\underset{\underset{O}{\|}}{C}-Y'' \qquad (XXII)$$

$$H_2N-(CH_2)_m-\underset{\underset{R^{4'}}{|}}{(CH)}_q-(CH_2)_n-Q' \qquad (XXIV)$$

or with said compound of formula (XXII) and then with a compound of formula (XXX):

$$HO-(CH_2)_m-\underset{\underset{R^{4'}}{|}}{(CH)}_q-(CH_2)_n-Q' \qquad (XXX)$$

<u>or in case (g)</u>
either with an allyl halide and then an oxidising agent or with an epihalohydrin and, in either case, then with a compound of formula $M-(CH_2)_{(n-1)}-Q'$,
[in which:
$R^{4'}$ represents any of the groups defined above for
$R^4$, but in which any reactive group is, if necessary, protected;
$E^f$ represents a heterocyclic group containing from 5 to 14 ring atoms, as defined above for E.
Q' represents a group having the formula $-O-R^{11}$, in which $R^{11}$ represents a hydroxy-protecting

group;
Q" represents a group of formula Y, defined above, or any one of the heterocyclic groups represented by Q, in which Q is as defined above;
W represents a residue of a reactive carboxylic acid, a lower aliphatic acyloxy group or an aromatic acyloxy group;
Y represents a halogen atom;
Y" represents a leaving group; and
M represents a metal atom];
(ii) where the product of step (i) contains a group of formula Q', converting said group to a group of formula Q"; and
(iii) optionally subjecting the product of step (i) or (ii) to one or more of the reactions amination, protection, deprotection, salification and esterification, in any order.

**46.** A process for preparing a compound according to any one of claims 1 to 42, which process comprises:
(i) reacting a compound of formula (D):

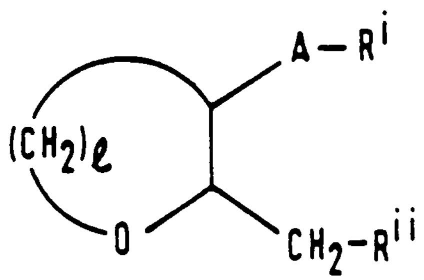

(D)

[in which:
(b) $R^i$ represents a group of formula $-A-R^{1x'}$ and
$R^{ii}$ represents a group of formula -Y; or
(c) $R^i$ represents a group of formula $-A-R^{10}$ and
$R^{ii}$ represents a group of formula -Y;
in which:
A, $R^{10}$ and Y are as defined in claim 45; and $R^{1x'}$ represents an alkyl group containing from 8 to 22 carbon atoms];
or a compound of formula (D) in which any active group is protected, with a compound of formula (XI):

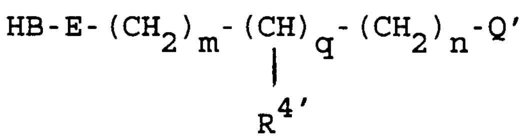

(XI)

[in which $R^{4'}$ and Q' are as defined in claim 45];
(ii) where the product of step (i) contains a group of formula Q', converting said group to a group of formula Q"; and
(iii) optionally subjecting the product of step (i) or step (ii) to one or more of the reactions amination protection, deprotection, salification and esterification, in any order.

**47.** A process for preparing a compound according to any one of claims 1 to 42, which process comprises:
(i) reacting a compound of formula (D):

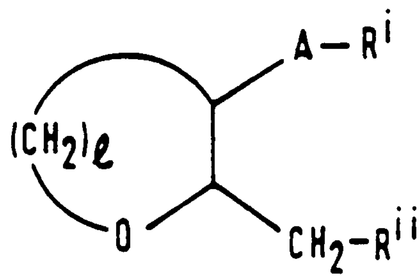

(D)

[in which

(d) $R^i$ represents a group of formula -A-H and
$R^{ii}$ represents a group of formula -B-$R^{1x}$; or

(h) $R^i$ represents a group of formula -A-H and
$R^{ii}$ represents a group of formula -B-$R^{10}$;

in which:

A, B, $R^{1x}$ and $R^{10}$ are as defined above]:

or a compound of formula (D) in which any active group is protected, with a compound of formula (VI), defined in claim 45, or a compound of formula (VII'):

$$HA-E^{f}-(CH_2)_m-\underset{\displaystyle R^{4'}}{\underset{|}{(CH)}}{}_q-(CH_2)_n-Q'$$

(VII')

or the compound of formula (XIX) or the compound of formula (XX) or with the compound of formula (XXII) followed by either said compound of formula (XXIV) or (XXX),all of (XIX), (XX), (XXII), (XXIV) and (XXX) being as defined in Claim 45;

[in which:

$R^{4'}$, $E^f$ and Q' are as defined above]

(ii) where the product of step (i) contains a group of formula Q', converting said group to a group of formula Q", and

(iii) optionally subjecting the product of step (i) or step (ii) to one or more of the reactions amination, protection, deprotection, salification and esterification, in any order.

**48.** A process for preparing a compound according to any one of claims 1 to 42, which process comprises:

(i) reacting a compound of formula (D):

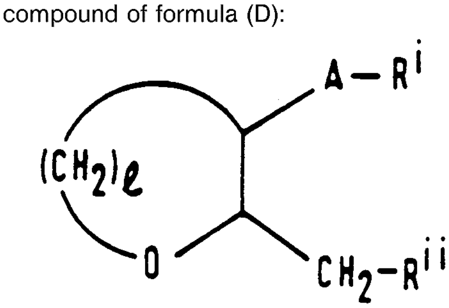

(D)

[in which:

(e) $R^i$ represents a group of formula -Y and $R^{ii}$ represents a group of formula -B-$R^{1x'}$; or

(f) $R^i$ represents a group of formula -Y and $R^{ii}$ represents a group of formula -B-$R^{10}$;

in which:

A, B, Y, $R^{1x'}$ and $R^{10}$ are as defined above]:

or a compound of formula (D) in which any active group is protected, with a compound of formula

(XI'):

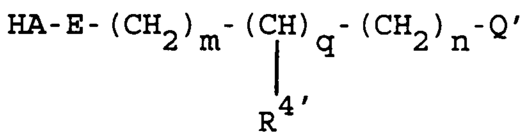

(XI')

[in which:
A, E, $R^{4'}$ and Q' are as defined above]:
(ii) where the product of step (i) contains a group of formula Q', converting said group to a group of formula Q"; and
(iii) optionally subjecting the product of step (i) or step (ii) to one or more of the reactions amination, protection, deprotection, salification and esterification, in any order.

**49.** A process for preparing a compound according to any one of claims 1 to 42, which process comprises:
(i) reacting a compound of formula (D):

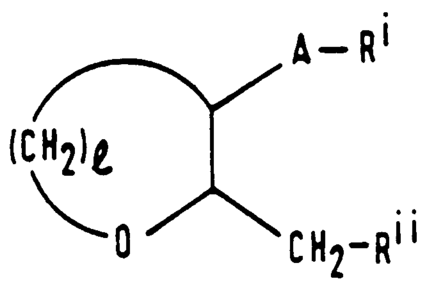

(D)

[in which:
(i) $R^i$ represents a double bond between the position $\alpha$ and $\beta$ to the other oxygen atom and $R^{ii}$ represents a group formula -Y;
in which:
Y is as defined above];
or a compound of formula (D) in which any active group is protected; with a compound of formula (XI)

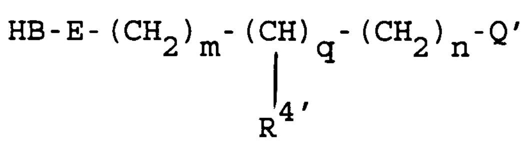

(XI)

[in which:
B, E, $R^{4'}$ and Q' are as defined above];
followed by hydroboration and alkylation, acylation or carbamation of the hydroxy group;
(ii) where the product of step (i) contains a group of formula Q', converting said group to a group of formula Q"; and
(iii) optionally subjecting the product of step (i) or step (ii) to one or more of the reactions amination, protection, deprotection, salification and esterification, in any order.

**Claims for the following Contracting States : AT, ES, GR**

**1.** A process for preparing a compound of formula (I):

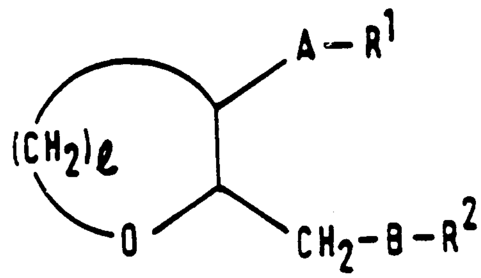

(I)

[in which:

l is an integer of from 2 to 4;

A and B are the same or different and each represents an oxygen atom or a sulphur atom;

one of $R^1$ and $R^2$ represents an alkyl group containing from 8 to 22 carbon atoms, an aliphatic carboxylic acyl group containing from 8 to 22 carbon atoms or a group of formula (II):

$$-CON(R^5)-R^3 \qquad (II)$$

in which $R^3$ represents an alkyl group containing from 8 to 22 carbon atoms, and

$R^5$ represents a hydrogen atom, a $C_1$ - $C_4$ alkyl group, a $C_1$ - $C_4$ alkanoyl group or an aralkyl group in which the alkyl part is $C_1$ - $C_4$,

and the other of $R^1$ and $R^2$ represents a group of formula (III):

$$-E-(CH_2)_m-(CH(R^4))_q-(CH_2)_n-Q \qquad (III)$$

in which E represents a single bond, a bivalent heterocyclic group or a group of formula

$$-C(=O)-, \quad -C(=O)-O- \quad \text{or} \quad -C(=O)-N(R^6)-$$

in which $R^6$ represents a hydrogen atom or an imino-protecting group;

m is the cypher 0 or an integer from 1 to 3;

n is the cypher 0 or an integer from 1 to 10;

q is the cypher 0 or the integer 1;

$R^4$ represents a hydroxy group, a $C_1$ - $C_4$ alkanoyloxy group, a $C_1$ - $C_4$ alkoxy group, a $C_7$ - $C_9$ aralkyloxy group, a carbamoyloxy group, an alkylcarbamoyloxy group in which the alkyl part is $C_1$ - $C_4$, a dialkylcarbamoyloxy group in which each alkyl part is $C_1$ - $C_4$, a mercapto group, a $C_1$ - $C_4$ alkylthio group, a $C_7$ - $C_9$ aralkylthio group,

a $C_1$ - $C_4$ alkanoylthio group, a carbamoylthio group, an alkylcarbamoylthio group in which the alkyl part is $C_1$ - $C_4$, a dialkylcarbamoylthio group in which each alkyl part is $C_1$ - $C_4$ or a carboxy group;

Q represents a group of formula (IV):

$$-N(R^7)(R^8) \qquad (IV)$$

in which $R^7$ and $R^8$ are the same or different and each represents a hydrogen atom or a $C_1$ - $C_6$ alkyl group,

or a monovalent heterocyclic group;

said heterocyclic groups having from 5 to 14 ring atoms, of which from 1 to 4 are nitrogen and/or

oxygen and/or sulphur hetero-atoms, at least one of these being a nitrogen atom, and said heterocyclic group being unsubstituted or having at least one of substituents (a) and/or substituents (b), defined below;

substituents (a):

oxygen atoms and $C_6$ - $C_{14}$ aryl groups;

substituents (b):

halogen atoms, hydroxy groups, $C_1$ - $C_4$ alkyl groups, $C_1$ - $C_4$ haloalkyl groups, $C_1$ - $C_4$ hydroxyalkyl groups, $C_1$ - $C_4$ alkoxy groups, $C_1$ - $C_6$ alkanoyl groups,

$C_3$ - $C_6$ alkenoyl groups, $C_7$ - $C_{15}$ aromatic carboxylic acyl groups, carbamoyl groups, $C_7$ - $C_{15}$ aralkyl groups, $C_2$ - $C_5$ alkoxycarbonyl groups, cyano groups, amino groups, alkylamino groups in which the alkyl part is $C_1$ - $C_4$, dialkylcarbamoyloxy groups in which each alkyl part is $C_1$ - $C_4$ and nitro groups;

said aryl groups and the aryl parts of aralkyl groups and aromatic acyl groups having from 6 to 14 ring atoms and being unsubstituted or having at least one of substituents (b), defined above];

and pharmaceutically acceptable salts and esters thereof, which process comprises:

(i) reacting a compound of formula (D):

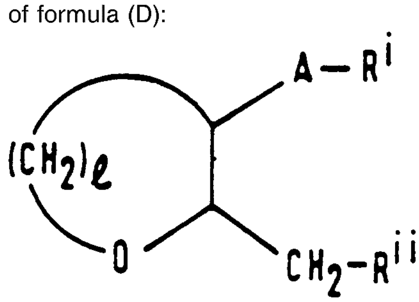

(D)

[in which:

(a) $R^i$ represents a group of formula $-A-R^{1x}$ and

$R^{ii}$ represents a group of formula -B-H; or

(g) $R^i$ represents a group of formula $-A-R^{10}$ and

$R^{ii}$ represents a group of formula -B-H;

in which:

A and B are as defined above;

$R^{1x}$ represents an alkyl group containing from 8 to 22 carbon atoms, an aliphatic carboxylic acyl group containing from 8 to 22 carbon atoms or a group of formula (II):

$$-CON(R^5)-R^3 \qquad (II)$$

in which $R^3$ and $R^5$ are as defined above;

$R^{10}$ represents a hydroxy-protecting or mercapto-protecting group];

or a compound of formula (D) in which any active group is protected, with:

a compound of formula (VI):

$$Y-(CH_2)_m-(CH(R^{4'}))_q-(CH_2)_n-Q' \qquad (VI)$$

or of formula (VII):

$$HB-E^f-(CH_2)_m-(CH(R^{4'}))_q-(CH_2)_n-Q' \qquad (VII)$$

or of formula (XIX):

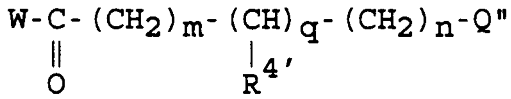
(XIX)

or of formula (XX):

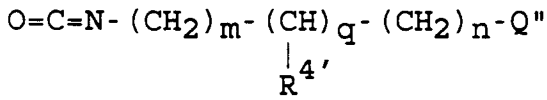
(XX)

or with a compound of formula (XXII) followed by a compound of formula (XXIV):

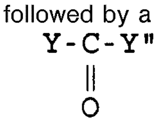
(XXII)

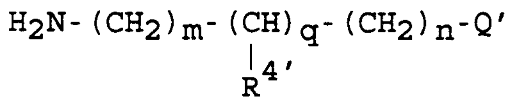
(XXIV)

or with said compound of formula (XXII) and then with a compound of formula (XXX):

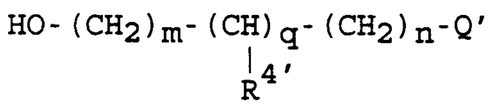
(XXX)

or in case (g)
either with an allyl halide and then an oxidising agent or with an epihalohydrin and, in either case, then with a compound of formula $M\text{-}(CH_2)_{(n-1)}\text{-}Q'$,
[in which:
$R^{4'}$ represents any of the groups defined above for $R^4$, but in which any reactive group is, if necessary, protected;
$E^f$ represents a heterocyclic group containing from 5 to 14 ring atoms, as defined above for E.
Q' represents a group having the formula $-O-R^{11}$, in which $R^{11}$ represents a hydroxy-protecting group;
Q" represents a group of formula Y, defined above, or any one of the heterocyclic groups represented by Q, in which Q is as defined above;
W represents a residue of a reactive carboxylic acid, a lower aliphatic acyloxy group or an aromatic acyloxy group;
Y represents a halogen atom;
Y" represents a leaving group; and
M represents a metal atom];
(ii) where the product of step (i) contains a group of formula Q', converting said group to a group of formula Q"; and
(iii) optionally subjecting the product of step (i) or step (ii) to one or more of the reactions amination, protection, deprotection, salification and esterification, in any order.

**2.** A process for preparing a compound of formula (I) as defined in according to claim 1, which process comprises:
(i) reacting a compound of formula (D):

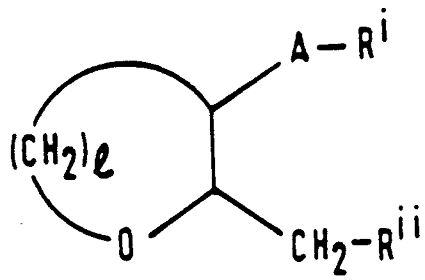

(D)

[in which:

(b) $R^i$ represents a group of formula $-A-R^{1x'}$ and

$R^{ii}$ represents a group of formula -Y; or

(c) $R^i$ represents a group of formula $-A-R^{10}$ and

$R^{11}$ represents a group of formula -Y;

in which:

A, $R^{10}$ and Y are as defined in claim 1; and $R^{1x'}$ represents an alkyl group containing from 8 to 22 carbon atoms];

or a compound of formula (D) in which any active group is protected, with a compound of formula (XI):

$$HB-E-(CH_2)_m-\underset{\displaystyle R^{4'}}{\underset{|}{(CH)}}{}_q-(CH_2)_n-Q' \qquad (XI)$$

[in which $R^{4'}$ and Q' are as defined in claim 1];

(ii) where the product of step (i) contains a group of formula Q', converting said group to a group of formula Q"; and

(iii) optionally subjecting the product of step (i) or step (ii) to one or more of the reactions amination protection, deprotection, salification and esterification, in any order.

**3.** A process for preparing a compound of formula (I) as defined in claim 1, which process comprises:

(i) reacting a compound of formula (D):

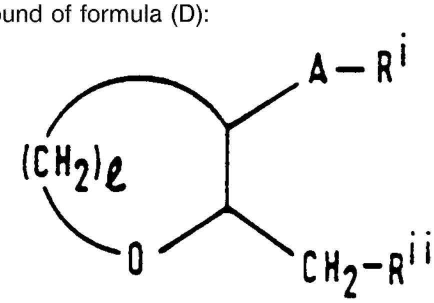

(D)

[in which

(d) $R^i$ represents a group of formula -A-H and

$R^{ii}$ represents a group of formula $-B-R^{1x}$; or

(h) $R^i$ represents a group of formula -A-H and

$R^{ii}$ represents a group of formula $-B-R^{10}$;

in which:

A, B, $R^{1x}$ and $R^{10}$ are as defined above]:

or a compound of formula (D) in which any active group is protected, with a compound of formula (VI), defined in claim 1, or a compound of formula (VII'):

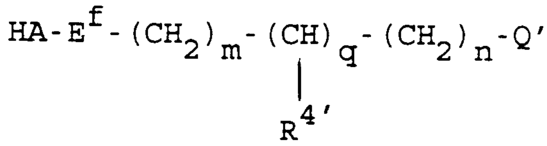

(VII')

or said compound of formula (XIX) or said compound of formula (XX) or with said compound of formula (XXII) followed by either said compound of formula (XXIV) or (XXX), all of compounds (XIX), (XX), (XXII), (XXIV) and (XXX) being defined in claim 1;

[in which:

$R^{4'}$, $E^f$ and Q' are as defined above]

(ii) where the product of step (i) contains a group of formula Q', converting said group to a group of formula Q", and

(iii) optionally subjecting the product of step (i) or step (ii) to one or more of the reactions amination, protection, deprotection, salification and esterification, in any order.

**4.** A process for preparing a compound of formula (I) as defined in claim 1, which process comprises:

(i) reacting a compound of formula (D):

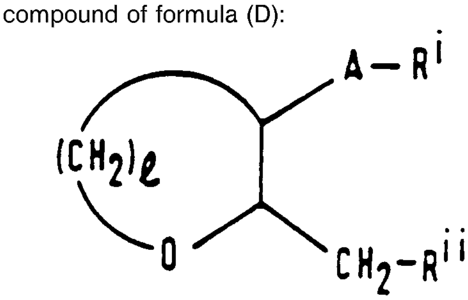

(D)

[in which:

(e) $R^i$ represents a group of formula -Y and $R^{ii}$ represents a group of formula $-B-R^{1x'}$; or

(f) $R^i$ represents a group of formula -Y and $R^{ii}$ represents a group of formula $-B-R^{10}$;

in which:

A, B, Y, $R^{1x'}$ and $R^{10}$ are as defined above]:

or a compound of formula (D) in which any active group is protected, with a compound of formula (XI'):

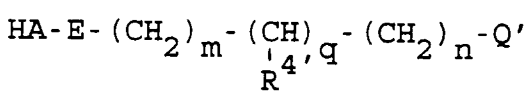

(XI')

[in which:

A, E, $R^{4'}$ and Q' are as defined above]:

(ii) where the product of step (i) contains a group of formula Q', converting said group to a group of formula Q"; and

(iii) optionally subjecting the product of step (i) or step (ii) to one or more of the reactions amination, protection, deprotection, salification and esterification, in any order.

**5.** A process for preparing a compound of formula (I) as defined in claim 1, which process comprises:

(i) reacting a compound of formula (D):

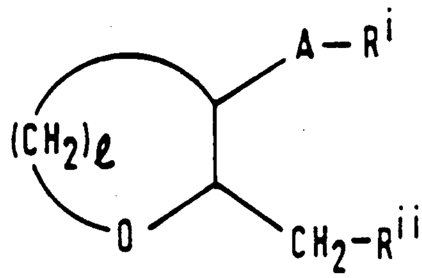

(D)

[in which:

(i) $R^i$ represents a double bond between the position $\alpha$ and $\beta$ to the other oxygen atom and $R^{ii}$ represents a group formula -Y;

in which:

Y is as defined above];

or a compound of formula (D) in which any active group is protected; with a compound of formula (XI);

$$HB\text{-}E\text{-}(CH_2)_m\text{-}\underset{\displaystyle R^{4'}}{\underset{|}{(CH)}}_q\text{-}(CH_2)_n\text{-}Q' \qquad (XI)$$

[in which:

B, E, $R^{4'}$ and Q' are as defined above];

followed by hydroboration and alkylation, acylation or carbamation of the hydroxy group;

(ii) where the product of step (i) contains a group of formula Q', converting said group to a group of formula Q"; and

(iii) optionally subjecting the product of step (i) or step (ii) to one or more of the reactions amination, protection, deprotection, salification and esterification, in any order.

**6.** A process as claimed in any one of Claims 1 to 5, in which the reagents and reaction conditions are so selected as to prepare a compound having the formula (I) as shown in Claim 1 in which one of $R^1$ and $R^2$ represents a group of formula (III'):

$$-E\text{-}(CH_2)_m\text{-}\underset{\displaystyle COO^-}{\underset{|}{CH}}\text{-}(CH_2)_n\text{-}Q^+ \qquad (III')$$

in which E, m and n are as defined in Claim 1 and $Q^+$ represents a quaternised heterocyclic group or a group of formula (IV'):

```
     R7
    /
-N+-R9          (IV')
    \
     R8
```

in which $R^7$, $R^8$ and $R^9$ are the same or different and each represents a hydrogen atom or a $C_1$ - $C_6$ alkyl group.

**7.** A process as claimed in any one of Claims 1 to 6, wherein said salt is in the form of a quaternary ammonium salt or an acid addition salt, in which Q represents a group of formula (IV"):

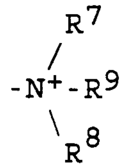

(IV")

in which $R^7$, $R^8$ are as defined in Claim 1, $R^9$ is as defined in Claim 6 and $Z^-$ represents a complementary pharmaceutically acceptable anion,
or a quaternised heterocyclic group together with a complementary pharmaceutically acceptable anion.

**8.** A process as claimed in Claim 7, wherein said complementary pharmaceutically acceptable anion is a halogen atom, an anionic residue of another mineral acid, a $C_1$ - $C_6$ alkylsulphonyloxy group, an arylsulphonyloxy group or an anion derived from an organic carboxylic acid or an amino acid.

**9.** A process as claimed in any one of the preceding Claims, in which the reagents and reaction conditions are so selected as to prepare a compound represented by the formula (Ia):

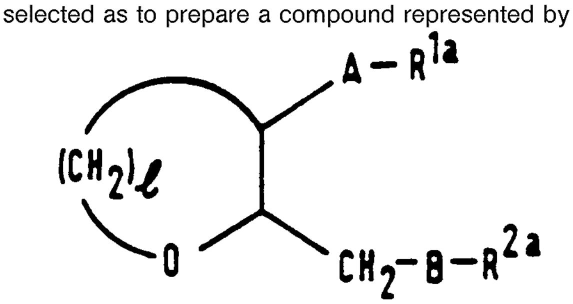

(Ia)

in which: l, A and B are as defined in Claim 1, and one of $R^{1a}$ and $R^{2a}$ represents an alkyl group containing from 8 to 22 carbon atoms, an aliphatic carboxylic acyl group containing from 8 to 22 carbon atoms or a group of formula (II), as defined in Claim 1, and the other of $R^{1a}$ and $R^{2a}$ represents a group of formula (IIIa):

$$-E-(CH_2)_m-\underset{\underset{R^{4a}}{|}}{(CH)}_q-(CH_2)_n-Q \qquad \text{(IIIa)}$$

in which m n, q and Q are as defined in Claim 1, and E represents a single bond, a bivalent heterocyclic group or a group of formula

$$-\underset{\underset{O}{\|}}{C}-, \quad -\underset{\underset{O}{\|}}{C}-O- \quad \text{or} \quad -\underset{\underset{O}{\|}}{C}-\underset{\underset{R^6}{|}}{N}-$$

in which $R^6$ is as defined in Claim 1;
$R^{4a}$ represents a hydroxy group, a $C_1$ - $C_4$ alkanoyloxy group, a $C_1$ - $C_4$ alkoxy group, a $C_7$ - $C_9$ aralkyloxy group, a carbamoyloxy group, an alkylcarbamoyloxy group in which the alkyl part is $C_1$ - $C_4$, a dialkylcarbamoyloxy group in which each alkyl part is $C_1$ - $C_4$, a mercapto group, a $C_1$ - $C_4$ alkylthio group, a $C_7$ - $C_9$ aralkylthio group, a $C_1$ - $C_4$ alkanoylthio group, a carbamoylthio group, an alkylcarbamoylthio group in which the alkyl part is $C_1$ - $C_4$, a dialkylcarbamoylthio group in which each alkyl part is $C_1$ - $C_4$, a carboxy group or an alkoxycarbonyl group in which the alkoxy part is $C_1$ - $C_4$;
said heterocyclic groups having from 5 to 10 ring atoms, of which from 1 to 4 are nitrogen and/or oxygen and/or sulphur hetero-atoms, the heterocyclic groups represented by E being unsubstituted and the heterocyclic groups represented by Q being unsubstituted or having at least one $C_1$ - $C_4$ alkyl substituent.

**10.** A process as claimed in any one of the preceding Claims, wherein the cyclic ether portion of the compound has the 3S configuration.

**11.** A process as claimed in Claim 10, wherein the cyclic ether portion of the compound has the (3S, 2R) configuration.

**12.** A process as claimed in Claim 10, wherein the cyclic ether portion of the compound has the (3S, 2S) configuration.

**13.** A process as claimed in any one of Claims 1 to 8 and 10 to 12, wherein:
l is an integer of from 2 to 4;
A and B are the same or different and each represents an oxygen atom or a sulphur atom;
one of $R^1$ or $R^2$ represents an alkyl group containing from 10 to 22 carbon atoms, an aliphatic carboxylic acyl group containing from 10 to 22 carbon atoms or a group of formula (IIa):

$-CONH-R^3$ (IIa)

in which $R^3$ represents an alkyl group containing from 10 to 22 carbon atoms,
and the other represents a group of formula (IIIb):

$$-E^{a}-(CH_2)_m-\underset{R^{4b}}{\underset{|}{CH}}-(CH_2)_n-Q \qquad (IIIb)$$

in which $E^a$ represents a single bond or a group of formula

$$-\underset{O}{\underset{||}{C}}-, \quad -\underset{O}{\underset{||}{C}}-O- \quad \text{or} \quad -\underset{O}{\underset{||}{C}}-\underset{R^6}{\underset{|}{N}}-$$

in which $R^6$ represents a hydrogen atom or an imino-protecting group;
m is the cypher 0 or an integer from 1 to 3;
n is the cypher 0 or an integer from 1 to 10;
$R^{4b}$ represents a hydrogen atom, a carboxy group or an alkoxycarbonyl group in which the alkoxy part is $C_1$ - $C_4$;
Q represents a group of formula (IV):

$$-N\begin{matrix} / R^7 \\ \backslash R^8 \end{matrix} \qquad (IV)$$

in which $R^7$ and $R^8$ are the same or different and each represents a hydrogen atom or a $C_1$ - $C_6$ alkyl group,
or a monovalent heterocyclic group having from 5 to 7 ring atoms, of which from 1 to 4 atoms are nitrogen and/or oxygen and/or sulphur hetero-atoms, at least one of these being a nitrogen atom, and the heterocyclic groups represented by Q being unsubstituted or having at least one substituent selected from $C_1$ - $C_4$ alkyl groups, $C_1$ - $C_4$ hydroxyalkyl groups, $C_1$ - $C_4$ alkoxy groups and halogen atoms or such a heterocyclic group having another ring fused thereto.

**14.** A process as claimed in any one of Claims 1 to 8 and 10 to 12, wherein:
l is an integer of from 2 to 4;
A and B are the same or different and each represents an oxygen atom or a sulphur atom;
one of $R^1$ or $R^2$ represents an alkyl group containing from 10 to 22 carbon atoms, an aliphatic carboxylic acyl group containing from 10 to 22 carbon atoms or a group of formula (IIa):

$-CONH-R^3$ (IIa)

in which $R^3$ represents an alkyl group containing from 10 to 22 carbon atoms
and the other represents a group of formula (IIIc):

$$-E^{b}-\underset{\displaystyle R^{4c}}{\underset{|}{(CH)}}{}_{q}-(CH_2)_n-Q \qquad (IIIc)$$

in which $E^b$ represents a group of formula $-(CH_2)_{m'}-$ or a bivalent heterocyclic group;
m' is an integer from 1 to 3;
n is the cypher 0 or an integer from 1 to 10;
q is the cypher 0 or the integer 1;
$R^{4c}$ represents a hydroxy group, a $C_1$ - $C_4$ alkanoyloxy group, a $C_1$ - $C_4$ alkoxy group, a $C_7$ - $C_9$ aralkyloxy group, a carbamoyloxy group, an alkylcarbamoyloxy group in which the alkyl part is $C_1$ - $C_4$, a dialkylcarbamoyloxy group in which each alkyl part is $C_1$ - $C_4$, a mercapto group, a$C_1$ - $C_4$ alkanoylthio group, a $C_1$ - $C_4$ alkylthio group, a $C_7$ - $C_9$ aralkylthio group, a carbamoylthio group, an alkylcarbamoylthio group in which the alkyl part is $C_1$ - $C_4$ or a dialkylcarbamoylthio group in which each alkyl part is $C_1$ - $C_4$;
Q represents a group of formula (IV):

$$-N\begin{matrix} R^7 \\ R^8 \end{matrix} \qquad (IV)$$

in which $R^7$ and $R^8$ are the same or different and each represents an hydrogen atoms and $C_1$ - $C_6$ alkyl groups,
or a monovalent heterocyclic group having from 5 to 7 ring atoms, of which from 1 to 4 atoms are nitrogen and/or oxygen and/or sulphur hetero-atoms, at least one of these being a nitrogen atom, and the heterocyclic groups represented by Q being unsubstituted or having at least one substituent selected from $C_1$ - $C_4$ alkyl groups, $C_1$ - $C_4$ hydroxyalkyl groups, $C_1$ - $C_4$ alkoxy groups, carbamoyl groups and halogen atoms or such a heterocyclic group having another ring fused thereto.

**15.** A process as claimed in any one of the preceding Claims, wherein:
l is the integer 2 or 3.

**16.** A process as claimed in Claim 15, wherein:
l is the integer 3.

**17.** A process as claimed in any one of the preceding Claims, wherein:
A represents an oxygen or sulphur atom and B represents an oxygen atom.

**18.** A process as claimed in any one of the preceding Claims, wherein:
$R^1$ represents $C_8$ - $C_{22}$ alkyl group or a group of formula (II):

$$-CON\underset{\displaystyle R^{5}}{\underset{|}{}}-R^{3} \qquad (II)$$

in which $R^3$ and $R^5$ are as defined in Claim 1.

**19.** A process as claimed in Claim 18, wherein $R^1$ represents said group of formula (II).

**20.** A process as claimed in Claim 18 or Claim 19, wherein $R^5$ represents a hydrogen atom or a $C_2$ - $C_4$ alkanoyl group.

**21.** A process as claimed in any one of Claims 18 to 20, wherein $R^3$ represents a $C_{13}$ - $C_{20}$ alkyl group.

**22.** A process as claimed in any one of the preceding Claims, wherein n is an integer from 1 to 7.

**23.** A process as claimed in any one of the preceding Claims, wherein:
Q represents a thiazolyl, pyridyl, quinolyl, isoquinolyl or imidazolyl group or a thiazolyl, pyridyl, quinolyl, isoquinolyl or imidazolyl group containing at least one substituent selected from the group consisting of $C_1$ - $C_4$ alkyl groups.

**24.** A process as claimed in Claim 23, wherein Q represents a thiazolyl or pyridyl group.

**25.** A process as claimed in any one of the preceding Claims, wherein E represents an isoxazolediyl or thiazolediyl group.

**26.** A process as claimed in Claim 25, wherein E represents a 3,5-isoxazolediyl group.

**27.** A process as claimed in any one of the preceding Claims, wherein:
$R^2$ represents a group of formula (III):

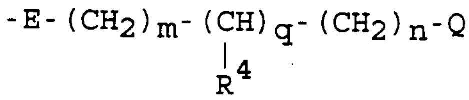

(III)

in which E, Q, $R^4$, m, n and q are as defined in Claim 1.

**28.** A process as claimed in any one of the preceding Claims, wherein:
in the group represented by $R^2$, the group of formula

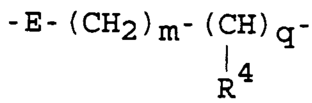

is a group of formula:

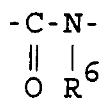 or 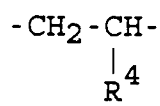

[in which $R^4$ and $R^6$ are as defined in Claim 1]
or an isoxazolediyl group.

**29.** A process as claimed in Claim 28, wherein $R^6$ is a hydrogen atom or an acetyl group.

**30.** A process as claimed in any one of the preceding Claims, wherein:
$R^1$ represents a group of formula (II):

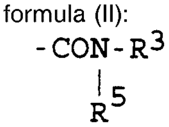

(II)

in which $R^3$ and $R^5$ are as defined in Claim 1, and
$R^2$ represents a group of formula (III):

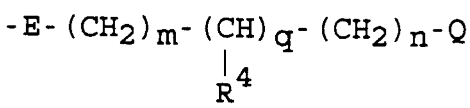

(III)

in which E, m, n, q, $R^4$ and Q are as defined in Claim 1.

**31.** A process as claimed in Claim 30, wherein $R^5$ represents a hydrogen atom or a $C_2$ - $C_4$ alkanoyl group.

**32.** A process as claimed in Claim 30, wherein: the group of formula

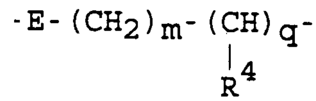

in the group represented by $R^2$ is a group of formula:

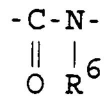 or 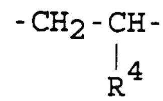

in which $R^6$ is as defined in Claim 1, or an isoxazolediyl group.

**33.** A process as claimed in Claim 32, wherein $R^6$ represents a hydrogen atom or an acetyl group.

**34.** A process as claimed in any one of Claims 1 to 5, in which the reagents and reaction conditions are so selected as to prepare a 3-{6-ethoxycarbonyl-6-[(3-heptadecylcarbamoyloxytetrahydropyran-2-yl)-methoxycarbonylamino]hexyl}thiazolium salt.

**35.** A process as claimed in any one of Claims 1 to 5, in which the reagents and reaction conditions are so selected as to prepare a dl-3-{6-ethoxycarbonyl-6-[(trans-3-heptadecylcarbamoyloxytetrahydropyran-2-yl)methoxycarbonylamino]hexyl}thiazolium salt.

**36.** A process as claimed in any one of Claims 1 to 5, in which the reagents and reaction conditions are so selected as to prepare a 3-{5-[(3-heptadecylcarbamoyloxytetrahydropyran-2-yl)methoxycarbonylamino]-pentyl}thiazolium salt.

**37.** A process as claimed in any one of Claims 1 to 5, in which the reagents and reaction conditions are so selected as to prepare a dl-3-{5-[(trans-3-heptadecylcarbamoyloxytetrahydropyran-2-yl)-methoxycarbonylamino]pentyl}thiazolium salt.

**38.** A process as claimed in any one of Claims 1 to 5, in which the reagents and reaction conditions are so selected as to prepare a 1-ethyl-2-{N-acetyl-N-[3-(N-heptadecylcarbamoyloxy)tetrahydropyran-2-ylmethoxycarbonyl]aminomethyl}pyridinium salt.

**39.** A process as claimed in any one of Claims 1 to 5, in which the reagents and reaction conditions are so selected as to prepare a dl-1-ethyl-2-{N-acetyl-N-[trans-3-(N-heptadecylcarbamoyloxy)tetrahydropyran-2-ylmethoxycarbonyl]aminomethyl}pyridinium salt.

**40.** A process as claimed in any one of Claims 1 to 5, in which the reagents and reaction conditions are so selected as to prepare a 3-{7-acetoxy-8-[(3-heptadecylcarbamoyloxytetrahydropyran-2-yl)methoxy]-octyl}thiazolium salt.

**41.** A process as claimed in any one of Claims 1 to 5, in which the reagents and reaction conditions are so selected as to prepare a 1-ethyl-2-{N-acetyl-N-[3-(N-acetyl-N-heptadecylcarbamoylthio)tetrahydropyran-2-ylmethoxycarbonyl]aminomethyl}pyridinium salt.

**42.** A process as claimed in any one of Claims 1 to 5, in which the reagents and reaction conditions are so selected as to prepare a dl-1-ethyl-2-{N-acetyl-N-[cis-3-(N-acetyl-N-heptadecylcarbamoylthio)-tetrahydropyran-2-ylmethoxycarbonyl]aminomethyl}pyridinium salt.

**43.** A process as claimed in any one of Claims 1 to 5, in which the reagents and reaction conditions are so selected as to prepare a 1-ethyl-2-{N-[3-(N-acetyl-N-heptadecylcarbamoylthio)tetrahydropyran-2-ylmethoxycarbonyl]aminomethyl}pyridinium salt.

**44.** A process as claimed in any one of Claims 1 to 5, in which the reagents and reaction conditions are so selected as to prepare a dl-1-ethyl-2-{N-[cis-3-(N-acetyl-N-heptadecylcarbamoylthio)tetrahydropyran-2-ylmethoxycarbonyl]aminomethyl}pyridinium salt.

**45.** A process as claimed in any one of Claims 1 to 5, in which the reagents and reaction conditions are so selected as to prepare a 3-{4-[3-(3-heptadecylcarbamoyloxytetrahydropyran-2-yl)methoxy-5-isoxazolyl]-butyl}thiazolium salt.

**46.** A process as claimed in any one of Claims 1 to 5, in which the reagents and reaction conditions are so selected as to prepare a dl-3-{4-[3-(trans-3-heptadecylcarbamoyloxytetrahydropyran-2-yl)methoxy-5-isoxazolyl]butyl}thiazolium salt.

**Patentansprüche**
**Patentansprüche für folgende Vertragsstaaten : BE, CH, DE, FR, GB, IT, LU, NL, SE**

**1.** Verbindungen der Formel (I):

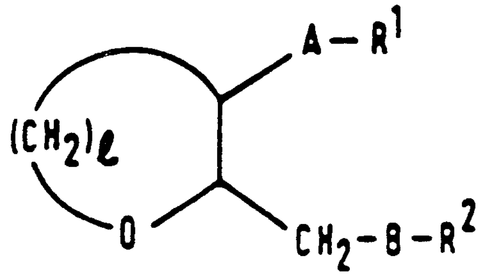

(I)

in der
l eine ganze Zahl von 2 bis 4 ist,
A und B gleich oder verschieden sind und jeweils ein Sauerstoffatom oder ein Schwefelatom bedeuten,
eines von $R^1$ und $R^2$ eine Alkylgruppe mit 8 bis 22 Kohlenstoffatomen, eine Acylgruppe einer aliphatischen Carbonsäure mit 8 bis 22 Kohlenstoffatomen oder eine Gruppe der Formel (II)

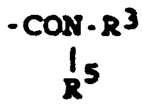

(II)

bedeutet,
in der $R^3$ eine Alkylgruppe mit 8 bis 22 Kohlenstoffatomen und
$R^5$ ein Wasserstoffatom, eine $C_1$ - $C_4$-Alkylgruppe, eine $C_1$ - $C_4$-Alkanoylgruppe oder eine Aralkylgruppe, deren Alkylteil $C_1$ - $C_4$ umfaßt, bedeutet
und das andere von $R^1$ und $R^2$ eine Gruppe der Formel (III) darstellt,

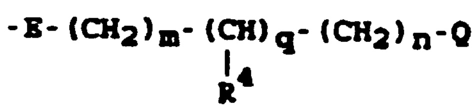

(III)

in der E eine Einfachbindung, eine zweiwertige heterocyclische Gruppe oder eine Gruppe der Formel

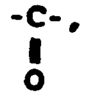 , 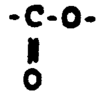 oder 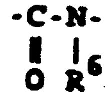

darstellt,
worin $R^6$ ein Wasserstoffatom oder eine Imino-Schutzgruppe bedeutet,
m die Ziffer 0 oder eine ganze Zahl von 1 bis 3 ist,
n die Ziffer 0 oder eine ganze Zahl von 1 bis 10 ist,
q die Ziffer 0 oder die ganze Zahl 1 ist,

$R^4$ eine Hydroxygruppe, eine $C_1$ - $C_4$-Alkanoyloxygruppe, eine $C_1$ - $C_4$-Alkoxygruppe, eine $C_7$- $C_9$-Aralkyloxygruppe, eine Carbamoyloxygruppe, eine Alkylcarbamoyloxygruppe, deren Alkylteil $C_1$ - $C_4$ umfaßt, eine Dialkylcarbamoyloxygruppe, in der Jeder Alkylteil $C_1$ - $C_4$ umfaßt, eine Mercaptogruppe, eine $C_1$ - $C_4$-Alkylthiogruppe, eine $C_7$ - $C_9$-Aralkylthiogruppe, eine $C_1$ - $C_4$-Alkanoylthiogruppe, eine Carbamoylthiogruppe, eine Alkylcarbamoylthiogruppe, deren Alkylteil $C_1$ - $C_4$ umfaßt, eine Dialkylcarbamoylthiogruppe, in der jeder Alkylteil $C_1$ - $C_4$ umfaßt, oder eine Carboxygruppe bedeutet,
Q eine Gruppe der Formel (IV)

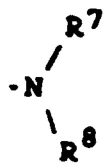

(IV)

in der $R^7$ und $R^8$ gleich oder verschieden sind und jeweils ein Wasserstoffatom oder eine $C_1$ - $C_6$-Alkylgruppebedeuten,
oder eine einwertige heterocyclische Gruppe bedeutet,
wobei die heterocyclischen Gruppen 5 bis 14 Ringatome aufweisen, wovon 1 bis 4 Stickstoff- und/oder Sauerstoff- und/oder Schwefel-Heteroatome sind, mindestens eines dieser ein Stickstoffatom ist und die heterocyclische Gruppe unsubstituiert ist oder mindestens einen der nachstehend definierten Substituenten (a) und/oder Substituenten (b) aufweist:
Substituenten (a):
Sauerstoffatome und $C_6$ - $C_{14}$-Arylgruppen,
Substituenten (b):
Halogenatome, Hydroxygruppen, $C_1$ - $C_4$-Alkylgruppen, $C_1$ - $C_4$-Halogenalkylgruppen, $C_1$- $C_4$-Hydroxyalkylgruppen, $C_1$ - $C_4$-Alkoxygruppen, $C_1$ - $C_6$-Alkanoylgruppen, $C_3$ - $C_6$-Alkenoylgruppen, Acylgruppen von $C_7$ - $C_{15}$-aromatischen Carbonsäuren, Carbamoylgruppen, $C_7$ - $C_{15}$-Aralkylgruppen, $C_2$ - $C_5$-Alkoxycarbonylgruppen, Cyangruppen, Aminogruppen, Alkylaminogruppen, in denen der Alkylteil $C_1$ - $C_4$ umfaßt, Dialkylcarbamoyloxygruppen, in denen jeder Alkylteil $C_1$ - $C_4$ umfaßt, und Nitrogruppen,
wobei die Arylgruppen und die Arylteile von Aralkylgruppen und aromatischen Acylgruppen 6 bis 14 Ringatome haben und unsubstituiert sind oder mindestens einen der oben definierten Substituenten (b) aufweisen,
und pharmazeutisch verträgliche Salze und Ester davon.

2. Verbindungen gemäß Anspruch 1 der in Anspruch 1 gezeigten Formel (I), in der eines von $R^1$ und $R^2$ eine Gruppe der Formel (III') bedeutet:

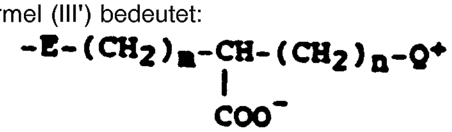

(III')

in der E, m und n wie in Anspruch 1 definiert sind, und $Q^+$ eine quaternisierte heterocyclische Gruppe oder eine Gruppe der Formel (IV') bedeutet:

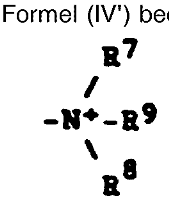

(IV')

worin $R^7$, $R^8$ und $R^9$ gleich oder verschieden sind und jeweils ein Wasserstoffatom oder eine $C_1$ - $C_6$-Alkylgruppebedeuten.

3. Verbindungen gemäß Anspruch 1 oder Anspruch 2, wobei das Salz in Form eines quaternären Ammoniumsalzes oder eines Säure-Additionssalzes vorliegt, worin Q eine Gruppe der Formel (IV"):

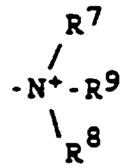 $Z^-$ (IV")

in der $R^7$ und $R^8$ wie in Anspruch 1 definiert sind, $R^9$ wie in Anspruch 2 definiert ist und $Z^-$ ein pharmazeutisch verträgliches Komplementäranion bedeutet,
oder eine quaternisierte heterocyclische Gruppe zusammen mit einem pharmazeutisch verträglichen Komplementäranion darstellt.

4. Verbindungen gemäß Anspruch 3, worin das pharmazeutisch verträgliche Komplementäranion ein Halogenatom, der anionische Rest einer anderen Mineralsäure, eine $C_1$ - $C_6$-Alkylsulfonyloxygruppe, eine Arylsulfonyloxygruppe oder ein von einer organischen Carbonsäure oder einer Aminosäure abgeleitetes Anion bedeutet.

5. Verbindungen gemäß einem der vorhergehenden Patentansprüche, die durch die Formel (Ia) dargestellt sind:

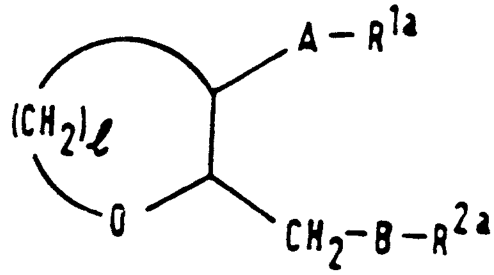 (Ia)

in der l, A und B wie in Anspruch 1 definiert sind, und
eines von $R^{1a}$ und $R^{2a}$ eine Alkylgruppe mit 8 bis 22 Kohlenstoffatomen, eine Acylgruppe einer aliphatischen Carbonsäure mit 8 bis 22 Kohlenstoffatomen oder eine Gruppe der Formel (II) gemäß der Definition in Anspruch 1 bedeutet, und das andere von $R^{1a}$ und $R^{2a}$ eine Gruppe der Formel (IIIa) darstellt:

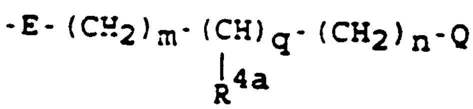 (IIIa)

worin m, n, q und Q wie in Anspruch 1 definiert sind, und
E eine Einfachbindung, eine zweiwertige heterocyclische Gruppe oder eine Gruppe der Formel

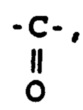 , 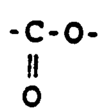 oder 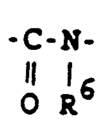

darstellt,
worin $R^6$ wie in Anspruch 1 definiert ist,
$R^{4a}$ eine Hydroxygruppe, eine $C_1$ - $C_4$- Alkanoyloxygruppe, eine $C_1$ - $C_4$-Alkoxygruppe, eine $C_7$ - $C_9$-Aralkyloxygruppe, eine Carbamoyloxygruppe, eine Alkylcarbamoyloxygruppe, in der der Alkylteil $C_1$ - $C_4$ umfaßt, eine Dialkylcarbamoyloxygruppe, in der jeder Alkylteil $C_1$ - $C_4$ umfaßt, eine Mercaptogruppe, eine $C_1$ - $C_4$-Alkylthiogruppe, eine $C_7$ - $C_9$-Aralkylthiogruppe, eine $C_1$ - $C_4$-Alkanoylthiogruppe, eine Carbamoylthiogruppe, eine Alkylcarbamoylthiogruppe, in der der Alkylteil $C_1$ - $C_4$ umfaßt, eine Dialkylcarbamoylthiogruppe, in der jeder Alkylteil $C_1$ - $C_4$ umfaßt, eine Carboxygruppe oder eine Alkoxycarbonylgruppe, in der der Alkoxyteil $C_1$ - $C_4$ umfaßt, bedeutet,
wobei die heterocyclischen Gruppen 5 bis 10 Ringatome haben, von denen 1 bis 4 Stickstoff- und/oder Sauerstoff- und/oder Schwefel-Heteroatome sind, die durch E dargestellten heterocyclischen Gruppen

unsubstituiert sind, und die durch Q dargestellten heterocyclischen Gruppen unsubstituiert sind oder mindestens einen $C_1$ - $C_4$-Alkylsubstituenten haben.

**6.** Verbindungen gemäß einem der vorhergehenden Patentansprüche, in denen der cyclische Etherteil der Verbindung die 3S-Konfiguration hat.

**7.** Verbindungen gemäß Anspruch 6, in denen der cyclische Etherteil der Verbindung die (3S, 2R)-Konfiguration hat.

**8.** Verbindungen gemäß Anspruch 6, in denen der cyclische Etherteil der Verbindung die (3S, 2S)-Konfiguration hat.

**9.** Verbindungen gemäß einem der Ansprüche 1 bis 4 und 6 bis 8, worin
l eine ganze Zahl von 2 bis 4 ist,
A und B gleich oder verschieden sind und jeweils ein Sauerstoffatom oder ein Schwefelatom bedeuten,
eines von $R^1$ oder $R^2$ eine Alkylgruppe mit 10 bis 22 Kohlenstoffatomen, eine Acylgruppe einer aliphatischen Carbonsäüre mit 10 bis 22 Kohlenstoffatomen oder eine Gruppe der Formel (IIa) bedeutet:

$-CONH-R^3$ (IIa)

worin $R^3$ eine Alkylgruppe mit 10 bis 22 Kohlenstoffatomen bedeutet,
und das andere eine Gruppe der Formel (IIIb) darstellt:

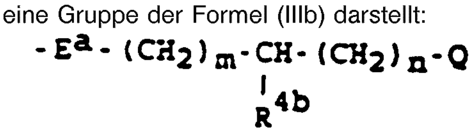 (IIIb)

in der $E^a$ eine Einfachbindung oder eine Gruppe der Formel

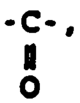 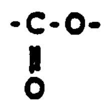 oder 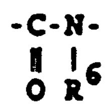

worin $R^6$ ein Wasserstoffatom oder eine Imino-Schutzgruppe darstellt, bedeutet,
m die Ziffer 0 oder eine ganze Zahl von 1 bis 3 ist,
n die Ziffer 0 oder eine ganze Zahl von 1 bis 10 ist,
$R^{4b}$ ein Wasserstoffatom, eine Carboxygruppe oder eine Alkoxycarbonylgruppe, in der der Alkoxyteil $C_1$ - $C_4$ bedeutet,
Q eine Gruppe der Formel (IV) darstellt:

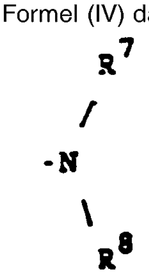 (IV)

worin $R^7$ und $R^8$ gleich oder verschieden sind und jeweils ein Wasserstoffatom oder eine $C_1$ - $C_6$-Alkylgruppebedeuten,
oder eine einwertige heterocyclische Gruppe mit 5 bis 7 Ringatomen, wovon 1 bis 4 Atome Stickstoff- und/oder Sauerstoff- und/oder Schwefel-Heteroatome sind und mindestens eines dieser Atome ein Stickstoffatom ist, bedeutet, und die durch Q dargestellten heterocyclischen Gruppen unsubstituiert sind oder mindestens einen Substituenten aufweisen, der unter $C_1$ - $C_4$-Alkylgruppen, $C_1$ - $C_4$-Hydroxyalkylgruppen, $C_1$ - $C_4$-Alkoxygruppen und Halogenatomen ausgewählt ist, oder eine solche heterocyclische Gruppe einen ankondensierten anderen Ring aufweist,
und pharmazeutisch verträgliche Salze davon.

**10.** Verbindungen gemäß einem der Ansprüche 1 bis 4 und 6 bis 8, worin
l eine ganze Zahl von 2 bis 4 ist,
A und B gleich oder verschieden sind und jeweils ein Sauerstoffatom oder ein Schwefelatom bedeuten,
eines von $R^1$ oder $R^2$ eine Alkylgruppe mit 10 bis 22 Kohlenstoffatomen, eine Acylgruppe einer aliphatischen Carbonsäure mit 10 bis 22 Kohlenstoffatomen oder eine Gruppe der Formel (IIa) bedeutet:

$-CONH-R^3$ (IIa)

worin $R^3$ eine Alkylgruppe mit 10 bis 22 Kohlenstoffatomen bedeutet,
und das andere eine Gruppe der Formel (IIIc) darstellt:

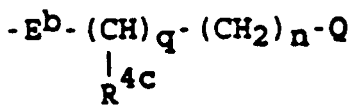

(IIIc)

worin $E^b$ eine Gruppe der Formel $-(CH_2)_{m'}-$ oder eine zweiwertige heterocyclische Gruppe bedeutet,
m' eine ganze Zahl von 1 bis 3 ist,
n die Ziffer 0 oder eine ganze Zahl von 1 bis 10 ist,
q die Ziffer 0 oder die ganze Zahl 1 ist,
$R^{4C}$ eine Hydroxygruppe, eine $C_1$ - $C_4$-Alkanoyloxygruppe, eine $C_1$ - $C_4$-Alkoxygruppe, eine $C_7$ - $C_9$-Aralkyloxygruppe, eine Carbamoyloxygruppe, eine Alkylcarbamoyloxygruppe, in der der Alkylteil $C_1$ - $C_4$ umfaßt, eine Dialkylcarbamoyloxygruppe, in der jeder Alkylteil $C_1$ - $C_4$ umfaßt, eine Mercaptogruppe, eine $C_1$ - $C_4$-Alkanoylthiogruppe, eine $C_1$ - $C_4$-Alkylthiogruppe, eine $C_7$ - $C_9$-Aralkylthiogruppe, eine Carbamoylthiogruppe, eine Alkylcarbamoylthiogruppe, in der der Alkylteil $C_1$ - $C_4$ umfaßt, oder eine Dialkylcarbamoylthiogruppe, in der jeder Alkylteil $C_1$ - $C_4$ umfaßt, bedeutet,
Q eine Gruppe der Formel (IV):

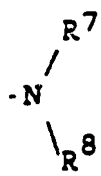

(IV)

in der $R^7$ oder $R^8$ gleich oder verschieden sind und jeweils ein Wasserstoffatom und $C_1$ - $C_6$-Alkylgruppen bedeuten,
oder eine einwertige heterocyclische Gruppe mit 5 bis 7 Ringatomen bedeutet, von denen 1 bis 4 Atome Stickstoff- und/oder Sauerstoff- und/oder Schwefel-Heteroatome sind, mindestens eines dieser ein Stickstoffatom ist, und die durch Q dargestellten heterocyclischen Gruppen unsubstituiert sind oder mindestens einen Substituenten haben, der unter $C_1$ - $C_4$-Alkylgruppen, $C_1$ - $C_4$-Hydroxyalkylgruppen, $C_1$ - $C_4$-Alkoxygruppen, Carbamoylgruppen und Halogenatomen ausgewählt ist, oder eine solche heterocyclische Gruppe einen anderen ankondensierten Ring aufweist,
und pharmazeutisch verträgliche Salze davon.

**11.** Verbindungen gemäß einem der vorhergehenden Patentansprüche, worin
l die ganze Zahl 2 oder 3 ist.

**12.** Verbindungen gemäß Anspruch 11, worin
l die ganze Zahl 3 ist.

**13.** Verbindungen gemäß einem der vorhergehenden Patentansprüche, worin
A ein Sauerstoff- oder Schwefelatom bedeutet und B ein Sauerstoffatom bedeutet.

**14.** Verbindungen gemäß einem der vorhergehenden Patentansprüche, worin
$R^1$ eine $C_8$ - $C_{22}$-Alkylgruppe oder eine Gruppe der Formel (II) bedeutet:

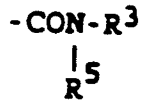 (II)

in der $R^3$ und $R^5$ wie in Anspruch 1 definiert sind.

**15.** Verbindungen gemäß Anspruch 14, worin $R^1$ die Gruppe der Formel (II) bedeutet.

**16.** Verbindungen gemäß Anspruch 14 oder Anspruch 15, worin $R^5$ ein Wasserstoffatom oder eine $C_2$ - $C_4$-Alkanoylgruppe bedeutet.

**17.** Verbindungen gemäß einem der Ansprüche 14 bis 16, worin $R^3$ eine $C_{13}$ - $C_{20}$-Alkylgruppe bedeutet.

**18.** Verbindungen gemäß einem der vorhergehenden Patentansprüche, worin n eine ganze Zahl von 1 bis 7 ist.

**19.** Verbindungen gemäß einem der vorhergehenden Patentansprüche, worin
Q eine Thiazolyl-, Pyridyl-, Chinolyl-, Isochinolyl- oder Imidazolylgruppe oder eine Thiazolyl-, Pyridyl-, Chinolyl-, Isochinolyl- oder Imidazolylgruppe, die mindestens einen $C_1$ -$C_4$-Alkylsubstituenten aufweist, bedeutet.

**20.** Verbindungen gemäß Anspruch 19, worin Q eine Thiazolyl- oder Pyridylgruppe bedeutet.

**21.** Verbindungen gemäß einem der vorhergehenden Patentansprüche, worin E eine Isoxazoldiyl- oder Thiazoldiylgruppe bedeutet.

**22.** Verbindungen gemäß Anspruch 21, worin E eine 3,5-Isoxazoldiylgruppe bedeutet.

**23.** Verbindungen gemäß einem der vorhergehenden Patentansprüche, worin
$R^2$ eine Gruppe der Formel (III) bedeutet:

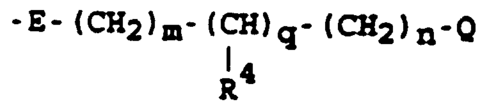 (III)

in der E, Q, $R^4$, m, n und q wie in Anspruch 1 definiert sind.

**24.** Verbindungen gemäß einem der vorhergehenden Patentansprüche, worin
die Gruppe der Formel

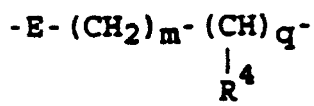

in der durch $R^2$ dargestellten Gruppe eine Gruppe der Formel

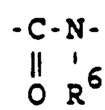 oder 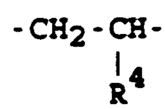

(in der $R^4$ und $R^6$ wie in Anspruch 1 definiert sind)
oder eine Isoxazoldiylgruppe darstellt.

**25.** Verbindungen gemäß Anspruch 24, worin $R^6$ ein Wasserstoffatom oder eine Acetylgruppe ist.

**26.** Verbindungen gemäß einem der vorhergehenden Patentansprüche, worin
$R^1$ eine Gruppe der Formel (II) bedeutet:

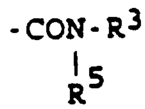

(II)

worin $R^3$ und $R^5$ wie in Anspruch 1 definiert sind, und
$R^2$ eine Gruppe der Formel (III) bedeutet:

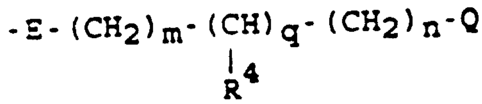

(III)

in der E, m, n, q, $R^4$ und Q wie in Anspruch 1 definiert sind.

**27.** Verbindungen gemäß Anspruch 26, worin $R^5$ ein Wasserstoffatom oder eine $C_2$ - $C_4$-Alkanoylgruppe bedeutet.

**28.** Verbindungen gemäß Anspruch 26, worin
die Gruppe der Formel

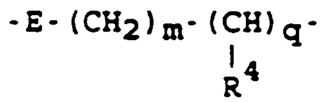

in der durch $R^2$ dargestellten Gruppe eine Gruppe der Formel

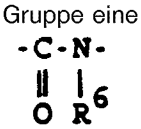

oder

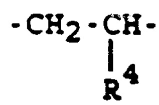

worin $R^6$ wie in Anspruch 1 definiert ist, oder
eine Isoxazoldiylgruppe darstellt.

**29.** Verbindungen gemäß Anspruch 28, worin $R^6$ ein Wasserstoffatom oder eine Acetylgruppe bedeutet.

**30.** A 3-{6-Ethoxycarbonyl-6-[(3-heptadecylcarbamoyloxytetrahydropyran-2-yl)methoxycarbonylamino]-hexyl}thiazoliumsalz.

**31.** A dl-3-{6-Ethoxycarbonyl-6-[(trans-3-heptadecylcarbamaoyloxytetrahydropyran-2-yl)-methoxycarbonylaminolhexyl}thiazoliumsalz.

**32.** A 3-{5-[(3-Heptadecylcarbamoyloxytetrahydropyran-2-yl)methoxycarbonylamino]pentyl}thiazoliumsalz.

**33.** A dl-3-{5-[(trans-3-Heptadecylcarbamoyloxytetrahydropyran-2-yl)methoxycarbonylamino]-pentyl}thiazoliumsalz.

**34.** A 1-Ethyl-2-{N-acetyl-N-[3-(N-heptadecylcarbamoyloxy)tetrahydropyran-2-ylmethoxycarbonyl]-aminomethyl}pyridiniumsalz.

**35.** A dl-1-Ethyl-2-{N-acetyl-N-[trans-3-(N-hepcadecylcarbamoyloxy)tecrahydropyran-2-ylmethoxycarbonyl]-aminomethyl}pyridiniumsalz.

**36.** A 3-{7-Acetoxy-8-[(3-heptadecylcarbamoyloxytetrahydropyran-2-yl)methoxy]octyl}thiazoliumsalz.

**37.** A 1-Ethyl-2-{N-acetyl-N-[3-(N-acetyl-N-hepcadecylcarbamoylthio)tetrahydropyran-2-ylmethoxycarbonyl]aminomethyl}pyridiniumsalz.

**38.** A dl-1-Ethyl-2-{N-acetyl-N-[cis-3-(N-acetyl-N-heptadecylcarbamoylthio)tetrahydropyran-2-ylmethoxycarbonyl]aminomethyl}pyridiniumsalz.

**39.** A 1-Ethyl-2-{N-[3-(N-acetyl-N-heptadecylcarbamoylthio)tetrahydropyran-2-ylmethoxycarbonyl]-aminomethyl}pyridiniumsalz.

**40.** A dl-1-Ethyl-2-{N-[cis-3-(N-acetyl-N-heptadecylcarbamoylthio)tecrahydropyran-2-ylmethoxycarbonyl]-aminomethyl}pyridiniumsalz.

**41.** A 3-{4-[3-(3-Hepcadecylcarbamoyloxytetrahydropyran-2-yl)methoxy-5-isoxazolyl]butyl}thiazoliumsalz.

**42.** A dl-3-{4-[3-(trans-3-Heptadecylcarbamoyloxytetrahydropyran-2-yl)methoxy-5-isoxazolyl]-butyl}thiazoliumsalz.

**43.** Arzneimittelzusammensetzung zur Behandlung von Entzündungen oder Schock, die einen PAF-Antagonisten in Kombination mit einem pharmazeutisch verträglichen Trägermaterial oder Verdünnungsmittel enthält, worin der PAF-Antagonist mindestens eine Verbindung gemäß einem der vorhergehenden Patentansprüche ist.

**44.** Verwendung mindestens einer Verbindung der Formel (I) und/oder eines pharmazeutisch verträglichen Salzes oder Esters davon, gemäß einem der Ansprüche 1 bis 42, zur Herstellung eines Arzneimittels zur Behandlung oder Prophylaxe von Asthma, Entzündungen oder Schock.

**45.** Verfahren zur Herstellung einer Verbindung gemäß einem der Ansprüche 1 bis 42, welches darin besteht, daß

(i) eine Verbindung der Formel (D)

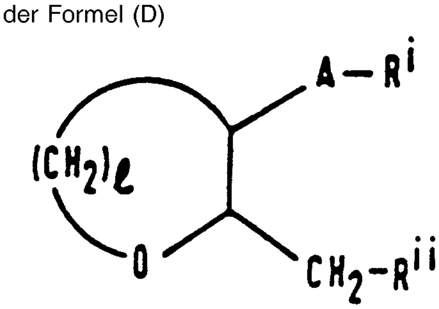

(D)

[worin:

(a) $R^i$ eine Gruppe der Formel -A-$R^{1x}$ bedeutet und
$R^{ii}$ eine Gruppe der Formel -B-H bedeutet, oder

(g) $R^i$ eine Gruppe der Formel -A-$R^{10}$ bedeutet und
$R^{ii}$ eine Gruppe der Formel -B-H bedeutet,

worin

A und B wie oben definiert sind,

$R^{1x}$ eine Alkylgruppe mit 8 bis 22 Kohlenstoffatomen, eine Acylgruppe einer aliphatischen Carbonsäure mit 8 bis 22 Kohlenstoffatomen oder eine Gruppe der Formel (II) darstellt:

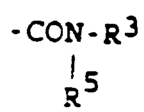

(II)

worin $R^3$ und $R^5$ wie oben definiert sind,

$R^{10}$ eine Hydroxy-Schutzgruppe oder eine Mercapto-Schutzgruppe darstellt],

oder eine Verbindung der Formel (D), in der eine aktive Gruppe geschützt ist, mit einer Verbindung der Formel (VI):

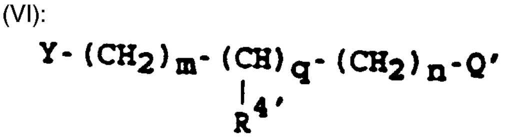

(VI)

oder der Formel (VII):

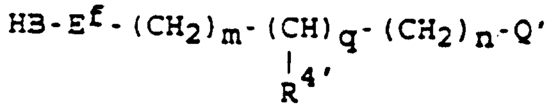

(VII)

oder der Formel (XIX):

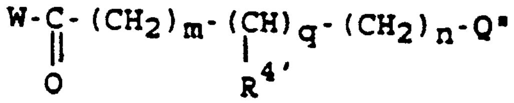

(XIX)

oder der Formel (XX):

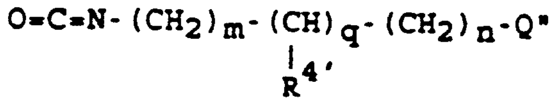

(XX)

oder mit einer Verbindung der Formel (XXII), und danach mit einer Verbindung der Formel (XXIV):

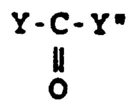

(XXII)

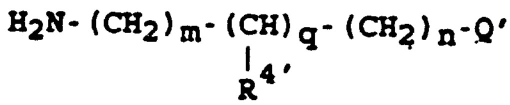

(XXIV)

oder mit der Verbindung der Formel (XXII) und danach mit einer Verbindung der Formel (XXX):

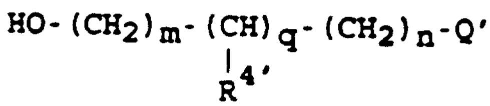

(XXX)

umgesetzt wird,
oder im Fall (g)
entweder mit einem Allylhalogenid und danach einem Oxidationsmittel oder mit einem Epihalogenhydrin und, in beiden Fällen, danach mit einer Verbindung der Formel $M-(CH_2)_{(n-1)}-Q'$,
umgesetzt wird,
[worin
$R^{4'}$ irgend eine der vorstehend für $R^4$ definierten Gruppen bedeutet, worin jedoch eine reaktive Gruppe erforderlichenfalls geschützt ist,
$E^f$ eine heterocyclische Gruppe mit 5 bis 14 Ringatomen darstellt, wie sie vorstehend für E definiert ist,
Q' eine Gruppe der Formel $-O-R^{11}$ bedeutet, in der $R^{11}$ eine Hydroxy-Schutzgruppe darstellt,
Q" eine Gruppe der oben definierten Formel Y oder irgend eine der durch Q dargestellten heterocyclischen Gruppen bedeutet, wobei Q wie oben definiert ist,
W den Rest einer reaktiven Carbonsäure, eine niedere aliphatische Acyloxygruppe oder eine aromatische Acyloxygruppe darstellt,
Y ein Halogenatom bedeutet,
Y" eine austretende Gruppe bedeutet, und
M ein Metallatom bedeutet],
(ii) dann, wenn das Produkt der Stufe (i) eine Gruppe der Formel Q' enthält, diese Gruppe in eine Gruppe der Formel Q" umgewandelt wird, und
(iii) gegebenenfalls das Produkt der Stufe (i) oder (ii) einer oder mehreren der nachstehenden Reaktionen in beliebiger Reihenfolge unterworfen wird: Aminierung, Schützen, Entfernen der Schutzgruppe, Salzbildung und Veresterung.

**46.** Verfahren zur Herstellung einer Verbindung gemäß einem der Ansprüche 1 bis 42, das darin besteht, daß

(i) eine Verbindung der Formel (D):

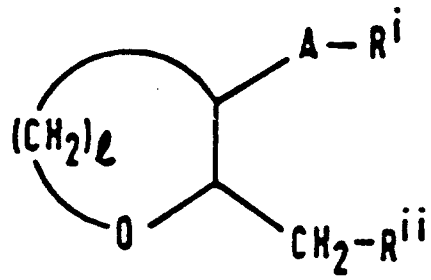

(D)

[worin

(b) $R^{i}$ eine Gruppe der Formel $-A-R^{1x'}$ bedeutet und
$R^{ii}$ eine Gruppe der Formel -Y bedeutet, oder
(c) $R^{i}$ eine Gruppe der Formel $-A-R^{10}$ bedeutet und
$R^{ii}$ eine Gruppe der Formel -Y bedeutet,

worin

A, $R^{10}$ und Y wie in Anspruch 45 definiert sind und $R^{1x'}$ eine Alkylgruppe mit 8 bis 22 Kohlenstoffatomen bedeutet],

oder eine Verbindung der Formel (D), in der eine aktive Gruppe geschützt ist, mit einer Verbindung der Formel (XI):

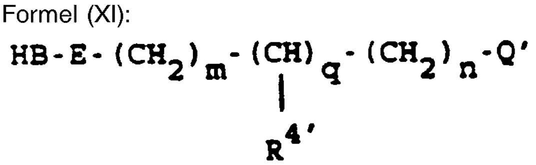

(XI)

[worin $R^{4'}$ und Q' wie in Anspruch 45 definiert sind], umgesetzt wird,

(ii) dann, wenn das Produkt der Stufe (i) eine Gruppe der Formel Q' enthält, diese Gruppe in eine Gruppe der Formel Q" umgewandelt wird, und

(iii) gegebenenfalls das Produkt der Stufe (i) oder der Stufe (ii) einer oder mehreren der nachstehenden Reaktionen in beliebiger Reihenfolge unterworfen wird: Aminierung, Schützen, Entfernen der Schutzgruppe, Salzbildung und Veresterung.

**47.** Verfahren zur Herstellung einer Verbindung gemäß einem der Ansprüche 1 bis 42, das darin besteht, daß

(i) eine Verbindung der Formel (D):

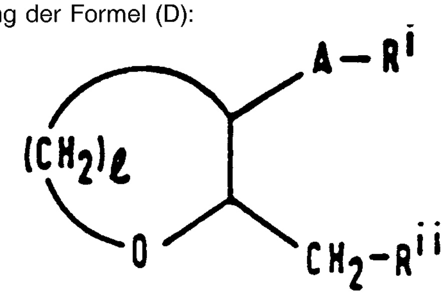

(D)

[worin

(d) $R^i$ eine Gruppe der Formel -A-H bedeutet und
$R^{ii}$ eine Gruppe der Formel -B-$R^{1x}$ bedeutet, oder
(h) $R^i$ eine Gruppe der Formel -A-H bedeutet und
$R^{ii}$ eine Gruppe der Formel -B-$R^{10}$ bedeutet,
worin
A, B, $R^{1x}$ und $R^{10}$ wie oben definiert sind],
oder eine Verbindung der Formel (D), in der eine aktive Gruppe geschützt ist, mit einer in Anspruch 45 definierten Verbindung der Formel (VI) oder einer Verbindung der Formel (VII'):

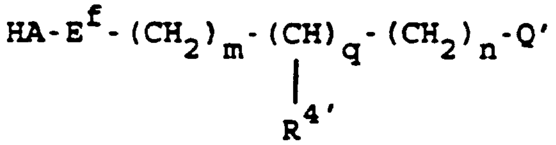

(VII')

oder der Verbindung der Formel (XIX) oder der Verbindung der Formel (XX) oder mit der Verbindung der Formel (XXII) und danach entweder mit der Verbindung der Formel (XXIV) oder (XXX), umgesetzt wird, wobei alle der Verbindungen (XIX), (XX), (XXII), (XXIV) und (XXX) wie in Anspruch 45 definiert sind,
wobei
$R^{4'}$, $E^f$ und Q' wie oben definiert sind]
umgesetzt wird,
(ii) dann, wenn das Produkt der Stufe (i) eine Gruppe der Formel Q' enthält, diese Gruppe in eine Gruppe der Formel Q'' umgewandelt wird, und
(iii) gegebenenfalls das Produkt der Stufe (i) oder der Stufe (ii) einer oder mehreren der nachstehenden Reaktionen in beliebiger Reihenfolge unterworfen wird: Aminierung, Schützen, Entfernen der Schutzgruppe, Salzbildung und Veresterung.

**48.** Verfahren zur Herstellung einer Verbindung gemäß einem der Ansprüche 1 bis 42, welches darin besteht, daß
(i) eine Verbindung der Formel (D):

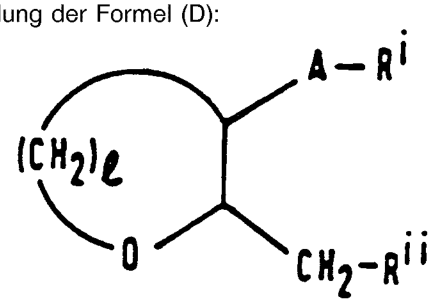

(D)

[in der
(e) $R^i$ eine Gruppe der Formel -Y und $R^{ii}$ eine Gruppe der Formel -B-$R^{1x'}$ bedeuten, oder
(f) $R^i$ eine Gruppe der Formel -Y und $R^{ii}$ eine Gruppe der Formel -B-$R^{10}$ bedeuten,
worin
A, B, Y, $R^{1x'}$ und $R^{10}$ wie oben definiert sind]
oder eine Verbindung der Formel (D), in der eine aktive Gruppe geschützt ist,
mit einer Verbindung der Formel (XI'):

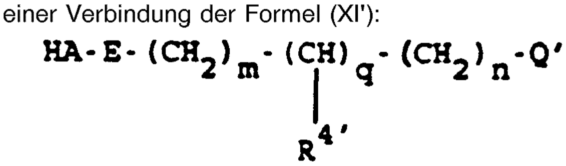

(XI')

[worin

A, E, $R^{4'}$ und Q' wie oben definiert sind],
umgesetzt wird,
(ii) dann, wenn das Produkt der Stufe (i) eine Gruppe der Formel Q' enthält, diese Gruppe in eine Gruppe der Formel Q" umgewandelt wird, und
(iii) gegebenenfalls das Produkt der Stufe (i) oder der Stufe (ii) einer oder mehreren der nachstehenden Reaktionen in beliebiger Reihenfolge unterworfen wird: Aminierung, Schützen, Entfernen der Schutzgruppe, Salzbildung und Veresterung.

**49.** Verfahren zur Herstellung einer Verbindung gemäß einem der Ansprüche 1 bis 42, das darin besteht, daß
(i) eine Verbindung der Formel (D):

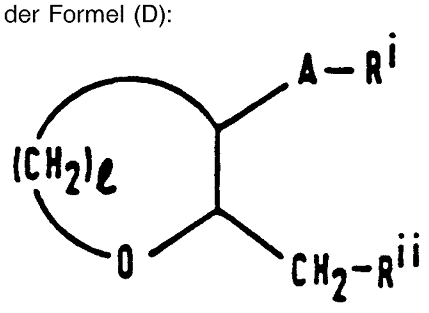

(D)

[worin
(i) $R^i$ eine Doppelbindung zwischen der $\alpha$- und der $\beta$-Stellung zu dem anderen Sauerstoffatom und $R^{ii}$ eine Gruppe der Formel -Y bedeuten,
worin
Y wie oben definiert ist],
oder eine Verbindung der Formel (D), in der eine aktive Gruppe geschützt ist,
mit einer Verbindung der Formel (XI):

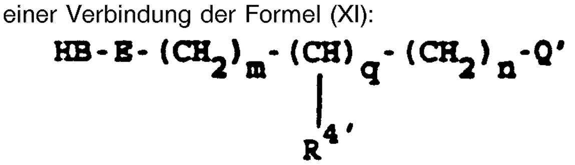

(XI)

[worin
B, E, $R^{4'}$ und Q' wie oben definiert sind],
umgesetzt wird,
wonach die Hydroborierung und Alkylierung, Acylierung oder Carbamatierung der Hydroxygruppe erfolgt,
(ii) dann, wenn das Produkt der Stufe (i) eine Gruppe der Formel Q' enthält, diese Gruppe zu einer Gruppe der Formel Q" umgewandelt wird, und
(iii) gegebenenfalls das Produkt der Stufe (i) oder der Stufe (ii) einer oder mehreren der nachstehenden Reaktionen in beliebiger Reihenfolge unterworfen wird: Aminierung, Schützen, Entfernen der Schutzgruppe, Salzbildung und Veresterung.

**Patentansprüche für folgende Vertragsstaaten : AT, ES, GR**

**1.** Verfahren zur Herstellung einer Verbindung der Formel (I):

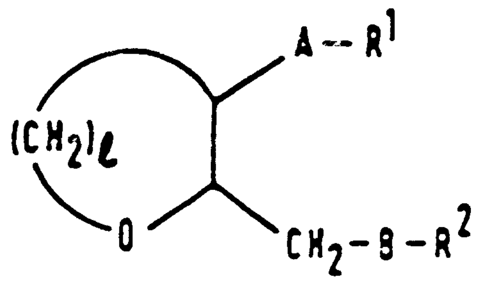

(I)

[in der

l eine ganze Zahl von 2 bis 4 ist,

A und B gleich oder verschieden sind und jeweils ein Sauerstoffatom oder ein Schwefelatom bedeuten,

eines von $R^1$ und $R^2$ eine Alkylgruppe mit 8 bis 22 Kohlenstoffatomen, eine Acylgruppe einer aliphatischen Carbonsäure mit 8 bis 22 Kohlenstoffatomen oder eine Gruppe der Formel (II)

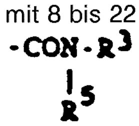

(II)

bedeutet,

in der $R^3$ eine Alkylgruppe mit 8 bis 22 Kohlenstoffatomen und

$R^5$ ein Wasserstoffatom, eine $C_1$ - $C_4$-Alkylgruppe, eine $C_1$ - $C_4$-Alkanoylgruppe oder eine Aralkylgruppe, deren Alkylteil $C_1$ - $C_4$ umfaßt, bedeutet,

und das andere von $R^1$ und $R^2$ eine Gruppe der Formel (III) darstellt,

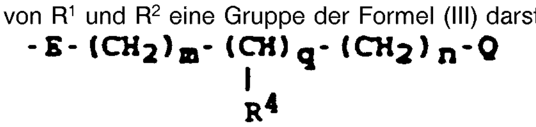

(III)

in der E eine Einfachbindung, eine zweiwertige heterocyclische Gruppe oder eine Gruppe der Formel

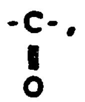 , 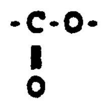 oder 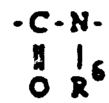

darstellt,

worin $R^6$ ein Wasserstoffatom oder eine Imino-Schutzgruppe bedeutet,

m die Ziffer 0 oder eine ganze Zahl von 1 bis 3 ist,

n die Ziffer 0 oder eine ganze Zahl von 1 bis 10 ist,

q die Ziffer 0 oder die ganze Zahl 1 ist,

$R^4$ eine Hydroxygruppe, eine $C_1$ - $C_4$-Alkanoyloxygruppe, eine $C_1$ - $C_4$-Alkoxygruppe, eine $C_7$- $C_9$-Aralkyloxygruppe, eine Carbamoyloxygruppe, eine Alkylcarbamoyloxygruppe, deren Alkylteil $C_1$ - $C_4$ umfaßt, eine Dialkylcarbamoyloxygruppe, in der jeder Alkylteil $C_1$ - $C_4$ umfaßt, eine Mercaptogruppe, eine $C_1$ - $C_4$-Alkylthiogruppe, eine $C_7$ - $C_9$-Aralkylthiogruppe, eine $C_1$ - $C_4$-Alkanoylthiogruppe, eine Carbamoylthiogruppe, eine Alkylcarbamoylthiogruppe, deren Alkylteil $C_1$ - $C_4$ umfaßt, eine Dialkylcarbamoylthiogruppe, in der jeder Alkylteil $C_1$ - $C_4$ umfaßt, oder eine Carboxygruppe bedeutet,

Q eine Gruppe der Formel (IV)

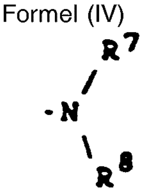

(IV)

in der $R^7$ und $R^8$ gleich oder verschieden sind und jeweils ein Wasserstoffatom oder eine $C_1$ - $C_6$-Alkylgruppebedeuten,

oder eine einwertige heterocyclische Gruppe bedeutet,

wobei die heterocyclischen Gruppen 5 bis 14 Ringatome aufweisen, wovon 1 bis 4 Stickstoff- und/oder Sauerstoff- und/oder Schwefel-Heteroatome sind, mindestens eines dieser ein Stickstoffatom ist und die heterocyclische Gruppe unsubstituiert ist oder mindestens einen der nachstehend definierten Substituenten (a) und/oder Substituenten (b) aufweist:

Substituenten (a):

Sauerstoffatome und $C_6$ - $C_{14}$-Arylgruppen,

Substituenten (b):

Halogenatome, Hydroxygruppen, $C_1$ - $C_4$-Alkylgruppen, $C_1$ - $C_4$-Halogenalkylgruppen, $C_1$- $C_4$-Hydroxyalkylgruppen, $C_1$ - $C_4$-Alkoxygruppen, $C_1$ - $C_6$-Alkanoylgruppen, $C_3$ - $C_6$-Alkenoylgruppen, Acylgruppen von $C_7$ - $C_{15}$-aromatischen Carbonsäuren, Carbamoylgruppen, $C_7$ - $C_{15}$-Aralkylgruppen, $C_2$ - $C_5$-Alkoxycarbonylgruppen, Cyangruppen, Aminogruppen, Alkylaminogruppen, in denen der Alkylteil $C_1$ - $C_4$ umfaßt, Dialkylcarbamoyloxygruppen, in denen jeder Alkylteil $C_1$ - $C_4$ umfaßt, und Nitrogruppen,

wobei die Arylgruppen und die Arylteile von Aralkylgruppen und aromatischen Acylgruppen 6 bis 14 Ringatome haben und unsubstituiert sind oder mindestens einen der oben definierten Substituenten (b) aufweisen],

und von pharmazeutisch verträglichen Salzen und Estern dieser, welches darin besteht, daß

(i) eine Verbindung der Formel (D)

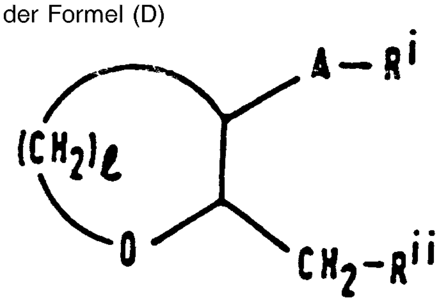

(D)

[worin:

(a) $R^i$ eine Gruppe der Formel $-A-R^{1x}$ bedeutet und

$R^{ii}$ eine Gruppe der Formel -B-H bedeutet, oder

(g) $R^i$ eine Gruppe der Formel $-A-R^{10}$ bedeutet und

$R^{ii}$ eine Gruppe der Formel -B-H bedeutet,

worin

A und B wie oben definiert sind,

$R^{1x}$ eine Alkylgruppe mit 8 bis 22 Kohlenstoffatomen, eine Acylgruppe einer aliphatischen Carbonsäure mit 8 bis 22 Kohlenstoffatomen oder eine Gruppe der Formel (II) darstellt:

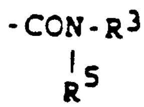

(II)

worin $R^3$ und $R^5$ wie oben definiert sind,

$R^{10}$ eine Hydroxy-Schutzgruppe oder eine Mercapto-Schutzgruppe darstellt],

oder eine Verbindung der Formel (D), in der eine aktive Gruppe geschützt ist, mit einer Verbindung der Formel (VI):

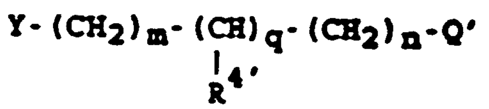

(VI)

oder der Formel (VII):

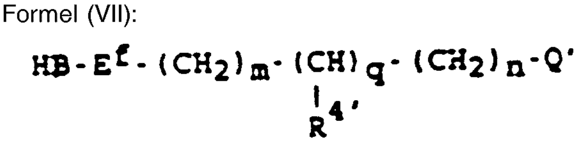

(VII)

oder der Formel (XIX):

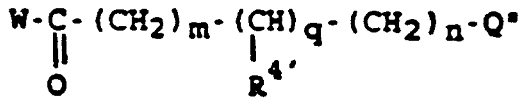

(XIX)

oder der Formel (XX):

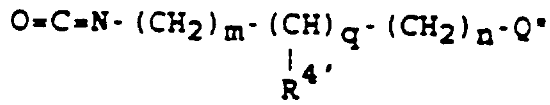

(XX)

oder mit einer Verbindung der Formel (XXII), und danach mit einer Verbindung der Formel (XXIV):

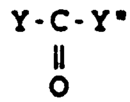

(XXII)

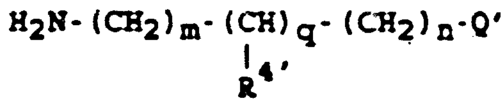

(XXIV)

oder mit der Verbindung der Formel (XXII) und danach mit einer Verbindung der Formel (XXX):

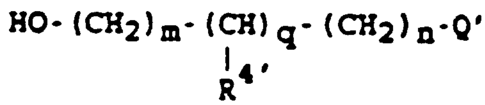

(XXX)

umgesetzt wird,
oder im Fall (g)
entweder mit einem Allylhalogenid und danach einem Oxidationsmtttel oder mit einem Epihalogenhydrin und, in beiden Fällen, danach mit einer Verbindung der Formel M-$(CH_2)_{(n-1)}$-Q',
umgesetzt wird,
[worin
$R^{4'}$ irgend eine der vorstehend für $R^4$ definierten Gruppen bedeutet, worin jedoch eine reaktive Gruppe erforderlichenfalls geschützt ist,
$E^f$ eine heterocyclische Gruppe mit 5 bis 14 Ringatomen darstellt, wie sie vorstehend für E definiert ist,
Q' eine Gruppe der Formel -O-$R^{11}$ bedeutet, in der $R^{11}$ eine Hydroxy-Schutzgruppe darstellt,
Q" eine Gruppe der oben definierten Formel Y oder irgend eine der durch Q dargestellten heterocyclischen Gruppen bedeutet, wobei Q wie oben definiert ist,
W den Rest einer reaktiven Carbonsäure, eine niedere aliphatische Acyloxygruppe oder eine aromatische Acyloxygruppe darstellt,
Y ein Halogenatom bedeutet,
Y" eine austretende Gruppe bedeutet, und
M ein Metallatom bedeutet],
(ii) dann, wenn das Produkt der Stufe (i) eine Gruppe der Formel Q' enthält, diese Gruppe in eine Gruppe der Formel Q" umgewandelt wird, und
(iii) gegebenenfalls das Produkt der Stufe (i) oder (ii) einer oder mehreren der nachstehenden Reaktionen in beliebiger Reihenfolge unterworfen wird: Aminierung, Schützen, Entfernen der Schutzgruppe, Salzbildung und Veresterung.

**2.** Verfahren zur Herstellung einer Verbindung der Formel (I) gemäß der Definition in Anspruch 1, das darin besteht, daß
(i) eine Verbindung der Formel (D):

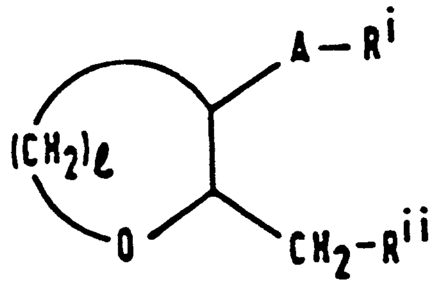

(D)

[worin

(b) $R^i$ eine Gruppe der Formel -A-$R^{1x'}$ bedeutet und

$R^{ii}$ eine Gruppe der Formel -Y bedeutet, oder

(c) $R^i$ eine Gruppe der Formel -A-$R^{10}$ bedeutet und

$R^{ii}$ eine Gruppe der Formel -Y bedeutet,

worin

A, $R^{10}$ und Y wie in Anspruch 1 definiert sind und $R^{1x'}$ eine Alkylgruppe mit 8 bis 22 Kohlenstoffatomen bedeutet],

oder eine Verbindung der Formel (D), in der eine aktive Gruppe geschützt ist, mit einer Verbindung der Formel (XI):

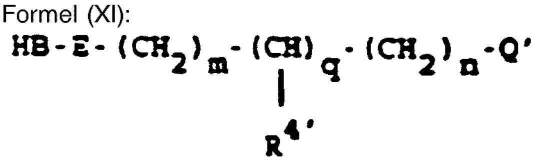

(XI)

[worin $R^{4'}$ und Q' wie in Anspruch 1 definiert sind], umgesetzt wird,

(ii) dann, wenn das Produkt der Stufe (i) eine Gruppe der Formel Q' enthält, diese Gruppe in eine Gruppe der Formel Q" umgewandelt wird, und

(iii) gegebenenfalls das Produkt der Stufe (i) oder der Stufe (ii) einer oder mehreren der nachstehenden Reaktionen in beliebiger Reihenfolge unterworfen wird: Aminierung, Schützen, Entfernen der Schutzgruppe, Salzbildung und Veresterung.

**3.** Verfahren zur Herstellung einer Verbindung der Formel (I) gemäß der Definition in Anspruch 1, das darin besteht, daß

(i) eine Verbindung der Formel (D):

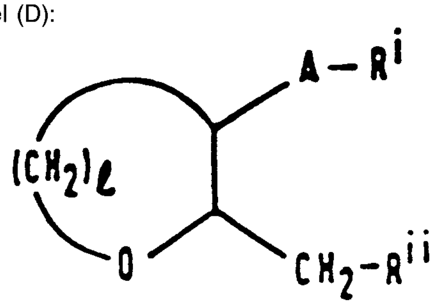

[worin

(d) $R^i$ eine Gruppe der Formel -A-H bedeutet und

$R^{ii}$ eine Gruppe der Formel -B-$R^{1x}$ bedeutet, oder

(h) $R^i$ eine Gruppe der Formel -A-H bedeutet und

$R^{ii}$ eine Gruppe der Formel -B-$R^{10}$ bedeutet,

worin

A, B, $R^{1x}$ und $R^{10}$ wie oben definiert sind],

oder eine Verbindung der Formel (D), in der eine aktive Gruppe geschützt ist, mit einer in Anspruch 1 definierten Verbindung der Formel (VI) oder einer Verbindung der Formel (VII'):

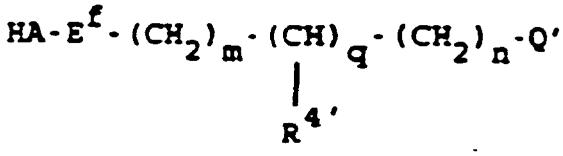

(VII')

oder der Verbindung der Formel (XIX) oder der Verbindung der Formel (XX) oder mit der Verbindung der Formel (XXII) und danach entweder mit der Verbindung der Formel (XXIV) oder (XXX), umgesetzt wird, wobei alle der Verbindungen (XIX), (XX), (XXII), (XXIV) und (XXX) wie in Anspruch 1 definiert sind,
wobei
$R^{4'}$, $E^{f}$ und Q' wie oben definiert sind]
umgesetzt wird,
(ii) dann, wenn das Produkt der Stufe (i) eine Gruppe der Formel Q' enthält, diese Gruppe in eine Gruppe der Formel Q" umgewandelt wird, und
(iii) gegebenenfalls das Produkt der Stufe (i) oder der Stufe (ii) einer oder mehreren der nachstehenden Reaktionen in beliebiger Reihenfolge unterworfen wird: Aminierung, Schützen, Entfernen der Schutzgruppe, Salzbildung und Veresterung.

4. Verfahren zur Herstellung einer Verbindung der Formel (I) gemäß der Definition in Anspruch 1, welches darin besteht, daß
(i) eine Verbindung der Formel (D):

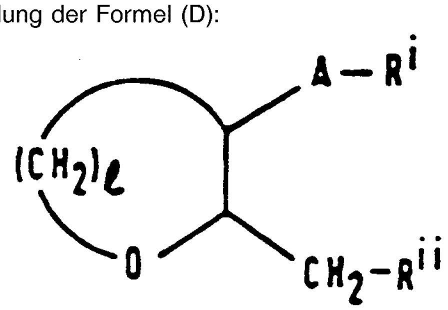

(D)

[in der
(e) $R^{i}$ eine Gruppe der Formel -Y und $R^{ii}$ eine Gruppe der Formel $-B-R^{1x'}$ bedeuten, oder
(f) $R^{i}$ eine Gruppe der Formel -Y und $R^{ii}$ eine Gruppe der Formel $-B-R^{10}$ bedeuten,
worin
A, B, Y, $R^{1x'}$ und $R^{10}$ wie oben definiert sind]
oder eine Verbindung der Formel (D), in der eine aktive Gruppe geschützt ist,
mit einer Verbindung der Formel (XI'):

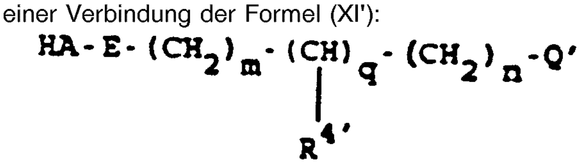

(XI')

[worin A, E, $R^{4'}$ und Q' wie oben definiert sind],
umgesetzt wird,
(ii) dann, wenn das Produkt der Stufe (i) eine Gruppe der Formel Q' enthält, diese Gruppe in eine Gruppe der Formel Q" umgewandelt wird, und
(iii) gegebenenfalls das Produkt der Stufe (i) oder der Stufe (ii) einer oder mehreren der nachstehenden Reaktionen in beliebiger Reihenfolge unterworfen wird: Aminierung, Schützen, Entfernen der Schutzgruppe, Salzbildung und Veresterung.

5. Verfahren zur Herstellung einer Verbindung der Formel (I) gemäß der Definition in Anspruch 1, das darin besteht, daß

(i) eine Verbindung der Formel (D):

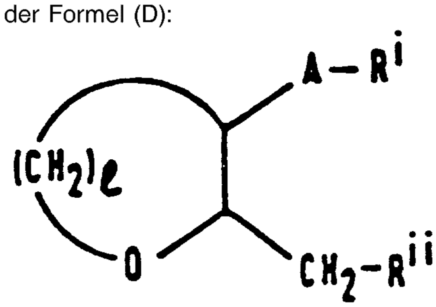

(D)

[worin

(i) $R^i$ eine Doppelbindung zwischen der α- und der β-Stellung zu dem anderen Sauerstoffatom und $R^{ii}$ eine Gruppe der Formel -Y bedeuten,

worin

Y wie oben definiert ist],

oder eine Verbindung der Formel (D), in der eine aktive Gruppe geschützt ist,

mit einer Verbindung der Formel (XI):

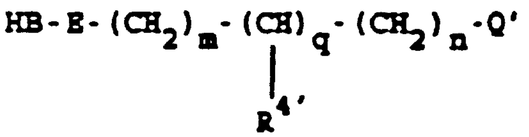

(XI)

[worin

B, E, $R^{4'}$ und Q' wie oben definiert sind],

umgesetzt wird,

wonach die Hydroborierung und Alkylierung, Acylierung oder Carbamatierung der Hydroxygruppe erfolgt,

(ii) dann, wenn das Produkt der Stufe (i) eine Gruppe der Formel Q' enthält, diese Gruppe zu einer Gruppe der Formel Q" umgewandelt wird, und

(iii) gegebenenfalls das Produkt der Stufe (i) oder der Stufe (ii) einer oder mehreren der nachstehenden Reaktionen in beliebiger Reihenfolge unterworfen wird: Aminierung, Schützen, Entfernen der Schutzgruppe, Salzbildung und Veresterung.

**6.** Verfahren gemäß einem der Ansprüche 1 bis 5, bei dem die Reaktanten und Reaktionsbedingungen so gewählt werden, daß eine Verbindung der in Anspruch 1 gezeigten Formel (I) hergestellt wird, in der eines von $R^1$ und $R^2$ eine Gruppe der Formel (III') bedeutet:

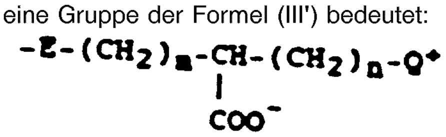

(III')

in der E, m und n wie in Anspruch 1 definiert sind, und $Q^+$ eine quaternisierte heterocyclische Gruppe oder eine Gruppe der Formel (IV') bedeutet:

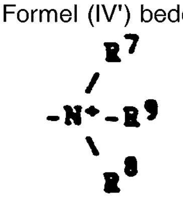

(IV')

worin $R^7$, $R^8$ und $R^9$ gleich oder verschieden sind und jeweils ein Wasserstoffatom oder eine $C_1$ - $C_6$-Alkylgruppebedeuten.

7. Verfahren gemäß einem der Ansprüche 1 bis 6, wobei das Salz in Form eines quaternären Ammoniumsalzes oder eines Säure-Additionssalzes vorliegt, worin Q eine Gruppe der Formel (IV"):

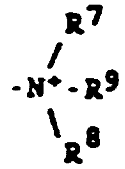 $Z^-$ (IV")

in der $R^7$ und $R^8$ wie in Anspruch 1 definiert sind, $R^9$ wie in Anspruch 6 definiert ist und $Z^-$ ein pharmazeutisch verträgliches Komplementäranion bedeutet,
oder eine quaternisierte heterocyclische Gruppe zusammen mit einem pharmazeutisch verträglichen Komplementäranion darstellt.

8. Verfahren gemäß Anspruch 7, worin das pharmazeutisch verträgliche Komplementäranion ein Halogenatom, der anionische Rest einer anderen Mineralsäure, eine $C_1$ - $C_6$-Alkylsulfonyloxygruppe, eine Arylsulfonyloxygruppe oder ein von einer organischen Carbonsäure oder einer Aminosäure abgeleitetes Anion bedeutet.

9. Verfahren gemäß einem der vorhergehenden Patentansprüche, bei dem die Reaktanten und Reaktionsbedingungen so gewählt werden, daß eine Verbindung hergestellt wird, die durch die Formel (Ia) dargestellt ist:

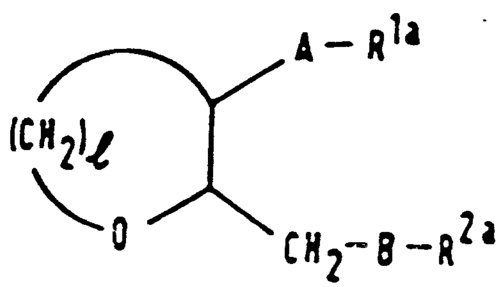 (Ia)

in der l, A und B wie in Anspruch 1 definiert sind, und eines von $R^{1a}$ und $R^{2a}$ eine Alkylgruppe mit 8 bis 22 Kohlenstoffatomen, eine Acylgruppe einer aliphatischen Carbonsäure mit 8 bis 22 Kohlenstoffatomen oder eine Gruppe der Formel (II) gemäß der Definition in Anspruch 1 bedeutet, und das andere von $R^{1a}$ und $R^{2a}$ eine Gruppe der Formel (IIIa) darstellt:

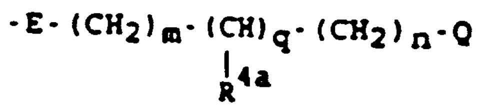 (IIIa)

worin m, n, q und Q wie in Anspruch 1 definiert sind, und E eine Einfachbindung, eine zweiwertige heterocyclische Gruppe oder eine Gruppe der Formel

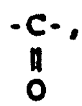, 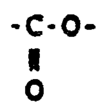 oder 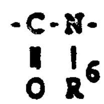

darstellt,
worin $R^6$ wie in Anspruch 1 definiert ist,
$R^{4a}$ eine Hydroxygruppe, eine $C_1$ - $C_4$- Alkanoyloxygruppe, eine $C_1$ - $C_4$-Alkoxygruppe, eine $C_7$ - $C_9$-Aralkyloxygruppe, eine Carbamoyloxygruppe, eine Alkylcarbamoyloxygruppe, in der der Alkylteil $C_1$ - $C_4$ umfaßt, eine Dialkylcarbamoyloxygruppe, in der jeder Alkylteil $C_1$ - $C_4$ umfaßt, eine Mercaptogruppe, eine $C_1$ - $C_4$-Alkylthiogruppe, eine $C_7$ - $C_9$-Aralkylthiogruppe, eine $C_1$ - $C_4$-Alkanoylthiogruppe, eine Carbamoylthiogruppe, eine Alkylcarbamoylthiogruppe, in der der Alkylteil $C_1$ - $C_4$ umfaßt, eine Dialkylcarbamoylthiogruppe, in der jeder Alkylteil $C_1$ - $C_4$ umfaßt, eine Carboxygruppe oder eine Alkoxycarbonylgruppe, in der der Alkoxyteil $C_1$ - $C_4$ umfaßt, bedeutet,

wobei die heterocyclischen Gruppen 5 bis 10 Ringatome haben, von denen 1 bis 4 Stickstoff- und/oder Sauerstoff- und/oder Schwefel-Heteroatome sind, die durch E dargestellten heterocyclischen Gruppen unsubstituiert sind, und die durch Q dargestellten heterocyclischen Gruppen unsubstituiert sind oder mindestens einen $C_1$ - $C_4$-Alkylsubstituenten haben.

**10.** Verfahren gemäß einem der vorhergehenden Patentansprüche, wobei der cyclische Etherteil der Verbindung die 3S-Konfiguration hat.

**11.** Verfahren gemäß Anspruch 10, wobei der cyclische Etherteil der Verbindung die (3S, 2R)-Konfiguration hat.

**12.** Verfahren gemaß Anspruch 10, wobei der cyclische Etherteil der Verbindung die (3S, 2S)-Konfiguration hat.

**13.** Verfahren gemäß einem der Ansprüche 1 bis 8 und 10 bis 12, worin
l eine ganze Zahl von 2 bis 4 ist,
A und B gleich oder verschieden sind und jeweils ein Sauerstoffatom oder ein Schwefelatom bedeuten,
eines von $R^1$ oder $R^2$ eine Alkylgruppe mit 10 bis 22 Kohlenstoffatomen, eine Acylgruppe einer aliphatischen Carbonsäure mit 10 bis 22 Kohlenstoffatomen oder eine Gruppe der Formel (IIa) bedeutet:

$-CONH-R^3$ (IIa)

worin $R^3$ eine Alkylgruppe mit 10 bis 22 Kohlenstoffatomen bedeutet,
und das andere eine Gruppe der Formel (IIIb) darstellt:

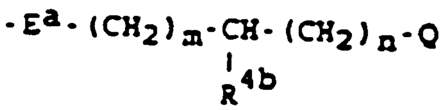 (IIIb)

in der $E^a$ eine Einfachbindung oder eine Gruppe der Formel

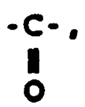, 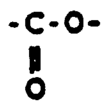 oder 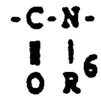

worin $R^6$ ein Wasserstoffatom oder eine Imino-Schutzgruppe darstellt, bedeutet,
m die Ziffer 0 oder eine ganze Zahl von 1 bis 3 ist,
n die Ziffer 0 oder eine ganze Zahl von 1 bis 10 ist,
$R^{4b}$ ein Wasserstoffatom, eine Carboxygruppe oder eine Alkoxycarbonylgruppe, in der der Alkoxyteil $C_1$ - $C_4$ umfaßt, bedeutet,
Q eine Gruppe der Formel (IV):

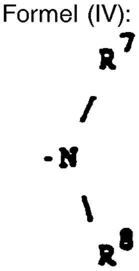 (IV)

worin $R^7$ und $R^8$ gleich oder verschieden sind und jeweils ein Wasserstoffatom oder eine $C_1$ - $C_6$-Alkylgruppebedeuten,
oder eine einwertige heterocyclische Gruppe mit 5 bis 7 Ringatomen, wovon 1 bis 4 Atome Stickstoff- und/oder Sauerstoff- und/oder Schwefel-Heteroatome sind und mindestens eines dieser Atome ein Stickstoffatom ist, bedeutet, und die durch Q dargestellten heterocyclischen Gruppen unsubstituiert

sind oder mindestens einen Substituenten aufweisen, der unter $C_1$ - $C_4$-Alkylgruppen, $C_1$ - $C_4$-Hydroxyalkylgruppen, $C_1$ - $C_4$-Alkoxygruppen und Halogenatomen ausgewählt ist, oder eine solche heterocyclische Gruppe einen ankondensierten anderen Ring aufweist,
und pharmazeutisch verträgliche Salze davon.

**14.** Verfahren gemäß einem der Ansprüche 1 bis 8 und 10 bis 12, worin
l eine ganze Zahl von 2 bis 4 ist,
A und B gleich oder verschieden sind und jeweils ein Sauerstoffatom oder ein Schwefelatom bedeuten,
eines von $R^1$ oder $R^2$ eine Alkylgruppe mit 10 bis 22 Kohlenstoffatomen, eine Acylgruppe einer aliphatischen Carbonsäure mit 10 bis 22 Kohlenstoffatomen oder eine Gruppe der Formel (IIa) bedeutet:

$-CONH-R^3$ (IIa)

worin $R^3$ eine Alkylgruppe mit 10 bis 22 Kohlenstoffatomen bedeutet,
und das andere eine Gruppe der Formel (IIIc) darstellt:

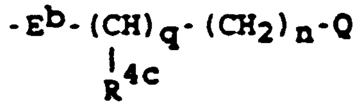

(IIIc)

worin $E^b$ eine Gruppe der Formel $-(CH_2)_{m'}-$ oder eine zweiwertige heterocyclische Gruppe bedeutet,
m' eine ganze Zahl von 1 bis 3 ist,
n die Ziffer 0 oder eine ganze Zahl von 1 bis 10 ist,
q die Ziffer 0 oder die ganze Zahl 1 ist,
$R^{4C}$ eine Hydroxygruppe, eine $C_1$ - $C_4$-Alkanoyloxygruppe, eine $C_1$ - $C_4$-Alkoxygruppe, eine $C_7$ - $C_9$-Aralkyloxygruppe, eine Carbamoyloxygruppe, eine Alkylcarbamoyloxygruppe, in der der Alkylteil $C_1$ - $C_4$ umfaßt, eine Dialkylcarbamoyloxygruppe, in der jeder Alkylteil $C_1$ - $C_4$ umfaßt, eine Mercaptogruppe, eine $C_1$ - $C_4$-Alkanoylthiogruppe, eine $C_1$ - $C_4$-Alkylthiogruppe, eine $C_7$ - $C_9$-Aralkylthiogruppe, eine Carbamoylthiogruppe, eine Alkylcarbamoylthiogruppe, in der der Alkylteil $C_1$ - $C_4$ umfaßt, oder eine Dialkylcarbamoylthiogruppe, in der jeder Alkylteil $C_1$ - $C_4$ umfaßt, bedeutet,
Q eine Gruppe der Formel (IV):

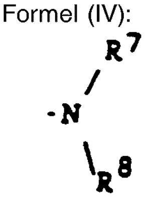

(IV)

in der $R^7$ oder $R^8$ gleich oder verschieden sind und jeweils ein Wasserstoffatom und $C_1$ - $C_6$-Alkylgruppen bedeuten,
oder eine einwertige heterocyclische Gruppe mit 5 bis 7 Ringatomen bedeutet, von denen 1 bis 4 Atome Stickstoff- und/oder Sauerstoff- und/oder Schwefel-Heteroatome sind, mindestens eines dieser ein Stickstoffatom ist, und die durch Q dargestellten heterocyclischen Gruppen unsubstituiert sind oder mindestens einen Substituenten haben, der unter $C_1$ - $C_4$-Alkylgruppen, $C_1$ - $C_4$-Hydroxyalkylgruppen, $C_1$ - $C_4$-Alkoxygruppen, Carbamoylgruppen und Halogenatomen ausgewählt ist, oder eine solche heterocyclische Gruppe einen anderen ankondensierten Ring aufweist,
und pharmazeutisch verträgliche Salze davon.

**15.** Verfahren gemäß einem der vorhergehenden Patentansprüche, worin
l die ganze Zahl 2 oder 3 ist.

**16.** Verfahren gemäß Anspruch 15, worin
l die ganze Zahl 3 ist.

**17.** Verfahren gemäß einem der vorhergehenden Patentansprüche, worin
A ein Sauerstoff- oder Schwefelatom bedeutet und B ein Sauerstoffatom bedeutet.

**18.** Verfahren gemäß einem der vorhergehenden Patentansprüche, worin $R^1$ eine $C_8$ - $C_{22}$-Alkylgruppe oder eine Gruppe der Formel (II) bedeutet:

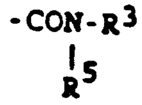

(II)

in der $R^3$ und $R^5$ wie in Anspruch 1 definiert sind.

**19.** Verfahren gemäß Anspruch 18, worin $R^1$ die Gruppe der Formel (II) bedeutet.

**20.** Verfahren gemäß Anspruch 18 oder Anspruch 19, worin $R^5$ ein Wasserstoffatom oder eine $C_2$ - $C_4$-Alkanoylgruppe bedeutet.

**21.** Verfahren gemäß einem der Ansprüche 18 bis 20, worin $R^3$ eine $C_{13}$ - $C_{20}$-Alkylgruppe bedeutet.

**22.** Verfahren gemäß einem der vorhergehenden Patentansprüche, worin n eine ganze Zahl von 1 bis 7 ist.

**23.** Verfahren gemäß einem der vorhergehenden Patentansprüche, worin Q eine Thiazolyl-, Pyridyl-, Chinolyl-, Isochinolyl- oder Imidazolylgruppe oder eine Thiazolyl-, Pyridyl-, Chinolyl-, Isochinolyl- oder Imidazolylgruppe, die mindestens einen unter $C_1$ - $C_4$-Alkylgruppen ausgewählten Substituenten aufweist, bedeutet.

**24.** Verfahren gemäß Anspruch 23, worin Q eine Thiazolyl- oder Pyridylgruppe bedeutet.

**25.** Verfahren gemäß einem der vorhergehenden Patentansprüche, worin E eine Isoxazoldiyl- oder Thiazoldiylgruppe bedeutet.

**26.** Verfahren gemäß Anspruch 25, worin E eine 3,5-Isoxazoldiylgruppe bedeutet.

**27.** Verfahren gemäß einem der vorhergehenden Patentansprüche, worin $R^2$ eine Gruppe der Formel (III) bedeutet:

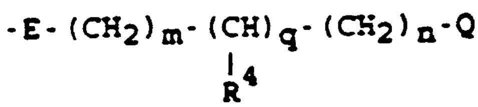

(III)

in der E, Q, $R^4$, m, n und q wie in Anspruch 1 definiert sind.

**28.** Verfahren gemäß einem der vorhergehenden Patentansprüche, worin die Gruppe der Formel

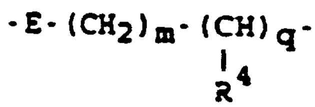

in der durch $R^2$ dargestellten Gruppe eine Gruppe der Formel

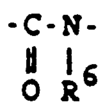

oder

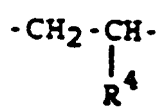

(in der $R^4$ und $R^6$ wie in Anspruch 1 definiert sind) oder eine Isoxazoldiylgruppe darstellt.

**29.** Verfahren gemäß Anspruch 28, worin $R^6$ ein Wasserstoffatom oder eine Acetylgruppe ist.

**30.** Verfahren gemäß einem der vorhergehenden Patentansprüche, worin $R^1$ eine Gruppe der Formel (II) bedeutet:

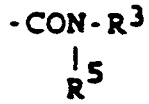

(II)

worin $R^3$ und $R^5$ wie in Anspruch 1 definiert sind, und $R^2$ eine Gruppe der Formel (III) bedeutet:

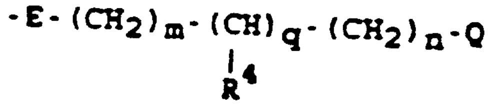

(III)

in der E, m, n, q, $R^4$ und Q wie in Anspruch 1 definiert sind.

**31.** Verfahren gemaß Anspruch 30, worin $R^5$ ein Wasserstoffatom oder eine $C_2$ - $C_4$-Alkanoylgruppe bedeutet.

**32.** Verfahren gemäß Anspruch 30, worin die Gruppe der Formel

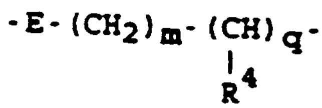

in der durch $R^2$ dargestellten Gruppe eine Gruppe der Formel

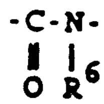

oder

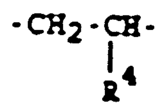

worin $R^6$ wie in Anspruch 1 definiert ist, oder eine Isoxazoldiylgruppe darstellt.

**33.** Verfahren gemäß Anspruch 32, worin $R^6$ ein Wasserstoffatom oder eine Acetylgruppe bedeutet.

**34.** Verfahren gemäß einem der Ansprüche 1 bis 5, bei dem die Reaktanten und Reaktionsbedingungen so gewählt werden, daß ein 3-{6-Ethoxycarbonyl-6-[(3-heptadecylcarbamoyloxytetrahydropyran-2-yl)-methoxycarbonylamino]hexyl}thiazoliumsalz hergestellt wird.

**35.** Verfahren gemäß einem der Ansprüche 1 bis 5, bei dem die Reaktanten und Reaktionsbedingungen so gewählt werden, daß ein dl-3-{6-Ethoxycarbonyl-6-[(trans-3-heptadecylcarbamoyloxytetrahydropyran-2-yl)methoxycarbonylamino]hexyl}thiazoliumsalz hergestellt wird.

**36.** Verfahren gemäß einem der Ansprüche 1 bis 5, bei dem die Reaktanten und Reaktionsbedingungen so gewählt werden, daß ein 3-{5-[(3-Heptadecylcarbamoyloxytetrahydropyran-2-yl)-methoxycarbonylamino]pentyl}thiazoliumsalz hergestellt wird.

**37.** Verfahren gemäß einem der Ansprüche 1 bis 5, bei dem die Reaktanten und Reaktionsbedingungen so gewählt werden, daß ein dl-3-{5-[(trans-3-Heptadecylcarbamoyloxytetrahydropyran-2-yl)-methoxycarbonylamino]pentyl}thiazoliumsalz hergestellt wird.

**38.** Verfahren gemäß einem der Ansprüche 1 bis 5, bei dem die Reaktanten und Reaktionsbedingungen so gewählt werden, daß ein 1-Ethyl-2-{N-acetyl-N-[3-(N-heptadecylcarbamoyloxy)tetrahydropyran-2-ylmethoxycarbonyl]aminomethyl}pyridiniumsalz hergestellt wird.

**39.** Verfahren gemäß einem der Ansprüche 1 bis 5, bei dem die Reaktanten und Reaktionsbedingungen so gewählt werden, daß ein dl-1-Ethyl-2-{N-acetyl-N-[trans-3-(N-heptadecylcarbamoyloxy)tetrahydropyran-2-ylmethoxycarbonyl]aminomethyl}pyridiniumsalz hergestellt wird.

**40.** Verfahren gemäß einem der Ansprüche 1 bis 5, bei dem die Reaktanten und Reaktionsbedingungen so gewählt werden, daß ein 3-{7-Acetoxy-8-[(3-heptadecylcarbamoyloxytetrahydropyran-2-yl)methoxy]-octyl}thiazoliumsalz hergestellt wird.

**41.** Verfahren gemäß einem der Ansprüche 1 bis 5, bei dem die Reaktanten und Reaktionsbedingungen so gewählt werden, daß ein 1-Ethyl-2-{N-acetyl-N-[3-(N-acecyl-N-heptadecylcarbamoylthio)-tetrahydropyran-2-ylmethoxycarbonyl]aminomethyl}pyridiniumsalz hergestellt wird.

**42.** Verfahren gemäß einem der Ansprüche 1 bis 5, bei dem die Reaktanten und Reaktionsbedingungen so gewählt werden, daß ein dl-1-Ethyl-2-{N-acetyl-N-[cis-3-(N-acetyl-N-heptadecylcarbamoylthio)-tetrahydropyran-2-ylmethoxycarbonyl]aminomethyl}pyridiniumsalz hergestellt wird.

**43.** Verfahren gemäß einem der Ansprüche 1 bis 5, bei dem die Reaktanten und Reaktionsbedingungen so gewählt werden, daß ein 1-Ethyl-2-{N-[3-(N-acetyl-N-heptadecylcarbamoylthio)tetrahydropyran-2-ylmethoxycarbonyl]aminomethyl}pyridiniumsalz hergestellt wird.

**44.** Verfahren gemäß einem der Ansprüche 1 bis 5, bei dem die Reaktanten und Reaktionsbedingungen so gewählt werden, daß ein dl-1-Ethyl-2-{N-[cis-3-(N-acetyl-N-heptadecylcarbamoylthio)tetrahydropyran-2-ylmethoxycarbonyl]aminomethyl}pyridiniumsalz hergestellt wird.

**45.** Verfahren gemäß einem der Ansprüche 1 bis 5, bei dem die Reaktanten und Reaktionsbedingungen so gewählt werden, daß ein 3-{4-[3-(3-Heptadecylcarbamoyloxytetrahydropyran-2-yl)methoxy-5-isoxazoyl]-butyl}thiazoliusalz hergestellt wird.

**46.** Verfahren gemäß einem der Ansprüche 1 bis 5, bei dem die Reaktanten und Reaktionsbedingungen so gewählt werden, daß ein dl-3-{4-[3-(trans-3-Heptadecylcarbamoyloxytetrahydropyran-2-yl)methoxy-5-isoxazoyl]butyl}thiazoliumsalz hergestellt wird.

**Revendications**
**Revendications pour les Etats contractants suivants : BE, CH, DE, FR, GB, IT, LU, NL, SE**

**1.** Composés de formule (I)

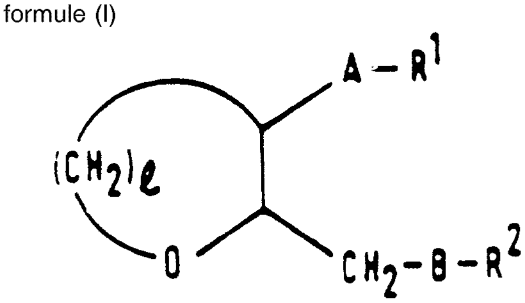

(I)

dans laquelle

ℓ est un nombre entier allant de 2 à 4;

A et B sont identiques ou différents et représentent chacun un atome d'oxygène ou un atome de soufre;

l'un des radicaux $R^1$ et $R^2$ représente un groupe alkyle contenant de 8 à 22 atomes de carbone, un groupe acyle carboxylique aliphatique contenant de 8 à 22 atomes de carbone ou un groupe de formule (II):

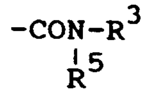

(II)

dans laquelle

$R^3$ représente un groupe alkyle contenant de 8 à 22 atomes de carbone, et

$R^5$ représente un atome d'hydrogène, un groupe alkyle en $C_1$-$C_4$, un groupe alcanoyle en $C_1$-$C_4$ ou un groupe aralkyle dans lequel le fragment alkyle est en $C_1$-$C_4$,

et l'autre des radicaux $R^1$ et $R^2$ représente un groupe de formule (III):

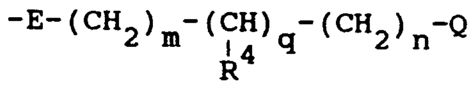

(III)

dans laquelle E représente une simple liaison, un groupe hétérocyclique bivalent ou un groupe de formule

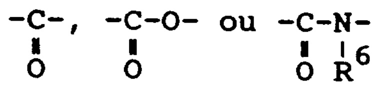

dans laquelle $R^6$ représente un atome d'hydrogène ou un groupe protecteur de fonction imino;

m est le chiffre 0 ou un nombre entier allant de 1 à 3;

n est le chiffre 0 ou un nombre entier allant de 1 à 10;

q est le chiffre 0 ou le nombre entier 1;

$R^4$ représente le groupe hydroxy, un groupe alcanoyloxy en $C_1$-$C_4$, un groupe alcoxy en $C_1$-$C_4$, un groupe aralkyloxy en $C_7$-$C_9$, un groupe carbamoyloxy, un groupe alkylcarbamoyloxy dans lequel le fragment alkyle est en $C_1$-$C_4$, un groupe dialkylcarbamoyloxy dans lequel chaque fragment alkyle est en $C_1$-$C_4$, le groupe mercapto, un groupe alkyl($C_1$-$C_4$)-thio, un groupe aralkyl($C_7$-$C_9$)-thio, un groupe alcanoyl-($C_1$-$C_4$)-thio, le groupe carbamoylthio, un groupe alkylcarbamoylthio dans lequel le fragment alkyle est en $C_1$-$C_4$, un groupe dialkylcarbamoylthio dans lequel chaque fragment alkyle est en $C_1$-$C_4$, ou le groupe carboxy;

Q représente un groupe de formule (IV):

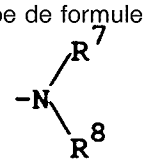

(IV)

dans laquelle $R^7$ et $R^8$ sont identiques ou différents et représentent chacun un atome d'hydrogène ou un groupe alkyle en $C_1$-$C_6$,

ou un groupe hétérocyclique monovalent;

lesdits groupes hétérocycliques ayant de 5 à 14 atomes formant le cycle, dont de 1 à 4 sont des hétéroatomes d'azote et/ou d'oxygène et/ou de soufre, au moins l'un de ceux-ci étant un atome d'azote, et ledit groupe hétérocyclique étant non substitué ou comportant au moins l'un des substituants (a) et/ou des substituants (b) définis ci-dessous:

substituants (a):

des atomes d'oxygène et des groupes aryle en $C_6$-$C_{14}$;

substituants (b):

des atomes d'halogène, le groupe hydroxy, des groupes alkyle en $C_1$-$C_4$, halogénoalkyle en $C_1$-$C_4$, hydroxyalkyle en $C_1$-$C_4$, alcoxy en $C_1$-$C_4$, alcanoyle en $C_1$-$C_6$, alcénoyle en $C_3$-$C_6$, des groupes acyle carboxyliques aromatiques en $C_7$-$C_{15}$, le groupe carbamoyle, des groupes aralkyle en $C_7$-$C_{15}$, alcoxy-($C_2$-$C_5$)-carbonyle, le groupe cyano, le groupe amino, des groupes alkylamino dans lesquels le fragment alkyle est en $C_1$-$C_4$, des groupes dialkylcarbamoyloxy dans lesquels chaque fragment alkyle est en $C_1$-$C_4$, et le groupe nitro;

lesdits groupes aryle et les fragments aryle des groupes aralkyle et des groupes acyle aromatiques ayant de 6 à 14 atomes formant le cycle et n'étant pas substitués ou comportant au moins l'un des substituants (b) définis plus haut;

et sels et esters pharmaceutiquement acceptables de ceux-ci.

2. Composés selon la revendication 1, de formule (I) telle qu'indiquée dans la revendication 1, dans lesquels l'un des radicaux $R^1$ et $R^2$ représente un groupe de formule (III'):

$$-E-(CH_2)_m-\underset{COO^-}{CH}-(CH_2)_n-Q^+ \qquad (III')$$

dans laquelle E, m et n sont tels que définis dans la revendication 1, et $Q^+$ représente un groupe hétérocyclique rendu quaternaire ou un groupe de formule (IV'):

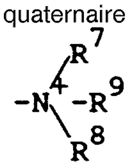

(IV')

dans laquelle $R^7$, $R^8$ et $R^9$ sont identiques ou différents et représentent chacun un atome d'hydrogène ou un groupe alkyle en $C_1$-$C_6$.

3. Composés selon la revendication 1 ou 2, dans lesquels ledit sel est sous la forme d'un sel d'ammonium quaternaire ou d'un sel d'addition avec un acide, dans lesquels Q représente un groupe de formule (IV")

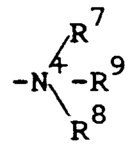

$Z^-$ (IV")

dans laquelle $R^7$, $R^8$ sont tels que définis dans la revendication 1, $R^9$ est tel que défini dans la revendication 2 et $Z^-$ représente un anion complémentaire pharmaceutiquement acceptable,
ou un groupe hétérocyclique rendu quaternaire, conjointement avec un anion complémentaire pharmaceutiquement acceptable.

4. Composés selon la revendication 3, dans lesquels ledit anion complémentaire pharmaceutiquement acceptable est un halogène, un reste anionique d'un autre acide minéral, un groupe alkyl($C_1$-$C_6$)-sulfonyloxy, un groupe arylsulfonyloxy ou un anion dérivé d'un acide carboxylique organique ou d'un aminoacide.

5. Composés selon l'une quelconque des revendications précédentes, qui sont représentés par la formule (Ia):

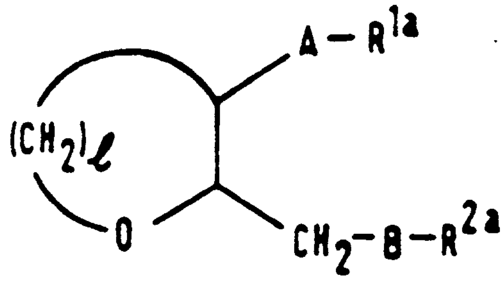

(Ia)

dans laquelle $\ell$, A et B sont tels que définis dans la revendication 1, et
l'un des radicaux $R^{1a}$ et $R^{2a}$ représente un groupe alkyle contenant de 8 à 22 atomes de carbone, un groupe acyle carboxylique aliphatique contenant de 8 à 22 atomes de carbone ou un groupe de formule (II), telle que définie dans la revendication 1, et l'autre des radicaux $R^{1a}$ et $R^{2a}$ représente un groupe de formule (IIa):

$$-E-(CH_2)_m-(\underset{R^{4a}}{CH})_q-(CH_2)_n-Q \qquad (IIIa)$$

dans laquelle m, n, q et Q sont tels que définis dans la revendication 1, et

E représente une simple liaison, un groupe hétérocyclique bivalent ou un groupe de formule

$$-\underset{\underset{O}{\|}}{C}-,\quad -\underset{\underset{O}{\|}}{C}-O-\quad \text{ou}\quad -\underset{\underset{O}{\|}}{C}-\underset{\underset{R^6}{|}}{N}-$$

dans laquelle $R^6$ est tel que défini dans la revendication 1;

$R^{4a}$ représente le groupe hydroxy, un groupe alcanoyloxy en $C_1$-$C_4$, un groupe alcoxy en $C_1$-$C_4$, un groupe aralkyloxy en $C_7$-$C_9$, le groupe carbamoyloxy, un groupe alkylcarbamoyloxy dans lequel le fragment alkyle est en $C_1$-$C_4$, un groupe dialkylcarbamoyloxy dans lequel chaque fragment alkyle est en $C_1$-$C_4$, le groupe mercapto, un groupe alkyl($C_1$-$C_4$)-thio, un groupe aralkyl($C_7$-$C_9$)-thio, un groupe alcanoyl($C_1$-$C_4$)-thio, le groupe carbamoylthio, un groupe alkylcarbamoylthio dans lequel le fragment alkyle est en $C_1$-$C_4$, un groupe dialkylcarbamoylthio dans lequel chaque fragment alkyle est en $C_1$-$C_4$, le groupe carboxy ou un groupe alcoxycarbonyle dont le fragment alcoxy est en $C_1$-$C_4$;

lesdits groupes hétérocycliques ayant de 5 à 10 atomes formant le cycle, dont de 1 à 4 sont des hétéroatomes d'azote et/ou d'oxygène et/ou de soufre, les groupes hétérocycliques représentés par E n'étant pas substitués et les groupes hétérocycliques représentés par Q n'étant pas substitués ou comportant au moins un substituant alkyle en $C_1$-$C_4$.

**6.** Composés selon l'une quelconque des revendications précédentes, dans lesquels le fragment éther cyclique du composé a la configuration 3S.

**7.** Composés selon la revendication 6, dans lesquels le fragment éther cyclique du composé a la configuration (3S,2R).

**8.** Composés selon la revendication 6, dans lesquels le fragment éther cyclique du composé a la configuration (3S,2S).

**9.** Composés selon l'une quelconque des revendications 1 à 4 et 6 à 8, dans lesquels:

$\ell$ est un nombre entier allant de 2 à 4;

A et B sont identiques ou différents et représentent chacun un atome d'oxygène ou un atome de soufre;

l'un des radicaux $R^1$ et $R^2$ représente un groupe alkyle contenant de 10 à 22 atomes de carbone, un groupe acyle carboxylique aliphatique contenant de 10 à 22 atomes de carbone ou un groupe de formule (IIa):

$$-CONH-R^3 \qquad \text{(IIa)}$$

dans laquelle $R^3$ représente un groupe alkyle contenant de 10 à 22 atomes de carbone,

et l'autre représente un groupe de formule (IIIb):

$$-E^a-(CH_2)_m-\underset{\underset{R^{4b}}{|}}{CH}-(CH_2)_n-Q \qquad \text{(IIIb)}$$

dans laquelle $E^a$ représente une simple liaison ou un groupe de formule

$$-\underset{\underset{O}{\|}}{C}-,\quad -\underset{\underset{O}{\|}}{C}-O-\quad \text{ou}\quad -\underset{\underset{O}{\|}}{C}-\underset{\underset{R^6}{|}}{N}-$$

dans laquelle $R^6$ représente un atome d'hydrogène ou un groupe protecteur de fonction imino;

m est le chiffre 0 ou un nombre entier allant de 1 à 3;

n est le chiffre 0 ou un nombre entier allant de 1 à 10;

$R^{4b}$ représente un atome d'hydrogène, le groupe carboxy ou un groupe alcoxycarbonyle dans lequel le fragment alcoxy est en $C_1$-$C_4$;

Q représente un groupe de formule (IV):

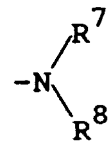

(IV)

dans laquelle $R^7$ et $R^8$ sont identiques ou différents et représentent chacun un atome d'hydrogène ou un groupe alkyle en $C_1$-$C_6$,

ou un groupe hétérocyclique monovalent ayant de 5 7 atomes formant le cycle, dont de 1 à 4 atomes sont des hétéroatomes d'azote et/ou d'oxygène et/ou de soufre, au moins l'un de ceux-ci étant un atome d'azote, et le groupe hétérocyclique représenté par Q n'étant pas substitué ou comportant au moins un substituant choisi parmi des groupes alkyle en $C_1$-$C_4$, hydroxyalkyle en $C_1$-$C_4$, alcoxy en $C_1$-$C_4$ et des atomes d'halogène, ou un tel groupe hétérocyclique comportant un autre cycle soudé à celui-ci;

et sels pharmaceutiquement acceptables de ceux-ci.

**10.** Composés selon l'une quelconque des revendications 1 à 4 et 6 à 8, dans lesquels:

ℓ est un nombre entier allant de 2 à 4;

A et B sont identiques ou différents et représentent chacun un atome d'oxygène ou un atome de soufre;

l'un des radicaux $R^1$ et $R^2$ représente un groupe alkyle contenant de 10 à 22 atomes de carbone, un groupe acyle carboxylique aliphatique contenant de 10 à 22 atomes de carbone ou un groupe de formule (IIa):

$-CONH-R^3$ (IIa)

dans laquelle $R^3$ représente un groupe alkyle contenant de 10 à 22 atomes de carbone,

et l'autre représente un groupe de formule (IIIc):

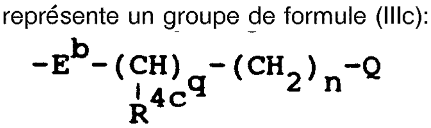

(IIIc)

dans laquelle

- $E^b$ représente un groupe de formule $-(CH_2)_{m'}-$ ou un groupe hétérocyclique bivalent;
- m' est un nombre entier allant de 1 à 3;
- n est le chiffre 0 ou un nombre entier allant de 1 à 10;
- q est le chiffre 0 ou le nombre entier 1;
- $R^{4c}$ représente le groupe hydroxy, un groupe alcanoyloxy en $C_1$-$C_4$, un groupe alcoxy en $C_1$-$C_4$, un groupe aralkyloxy en $C_7$-$C_9$, le groupe carbamoyloxy, un groupe alkylcarbamoyloxy dans lequel le fragment alkyle est en $C_1$-$C_4$, un groupe dialkylcarbamoyloxy dans lequel chaque fragment alkyle est en $C_1$-$C_4$, le groupe mercapto, un groupe alcanoyl($C_1$-$C_4$)-thio, un groupe alkyl($C_1$-$C_4$)-thio, un groupe aralkyl($C_7$-$C_9$)-thio, le groupe carbamoylthio, un groupe alkylcarbamoylthio dans lequel le fragment alkyle est en $C_1$-$C_4$, ou un groupe dialkylcarbamoylthio dans lequel chaque fragment alkyle est en $C_1$-$C_4$;
- Q représente un groupe de formule (IV):

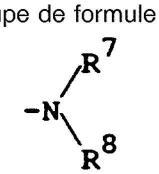

(IV)

dans laquelle $R^7$ et $R^8$ sont identiques ou différents et représentent chacun un atome d'hydrogène ou un groupe alkyle en $C_1$-$C_6$,

ou un groupe hétérocyclique monovalent ayant de 5 7 atomes formant le cycle, dont de 1 à 4 atomes sont des hétéroatomes d'azote et/ou d'oxygène et/ou de soufre, au moins l'un de ceux-ci étant un atome d'azote, et les groupes hétérocycliques représentés par Q étant non substitués ou comportant au moins un substituant choisi parmi des groupes alkyle en $C_1$-$C_4$,

hydroxyalkyle en $C_1$-$C_4$, alcoxy en $C_1$-$C_4$, carbamoyle et des atomes d'halogène, ou un tel groupe hétérocyclique comportant un autre cycle soudé à celui-ci;
et sels pharmaceutiquement acceptables de ceux-ci.

**11.** Composés selon l'une quelconque des revendications précédentes, dans lesquels:
$\ell$ est le nombre entier 2 ou 3.

**12.** Composés selon la revendication 11, dans lesquels:
$\ell$ est le nombre entier 3.

**13.** Composés selon l'une quelconque des revendications précédentes, dans lesquels:
A représente un atome d'oxygène ou de soufre et
B représente un atome d'oxygène.

**14.** Composés selon l'une quelconque des revendications précédentes, dans lesquels:
$R^1$ représente un groupe alkyle en $C_8$-$C_{22}$ ou un groupe de formule (II):

$$-\underset{\substack{|\\ R^5}}{\mathrm{CON}}-R^3 \qquad \text{(II)}$$

dans laquelle $R^3$ et $R^5$ sont tels que définis dans la revendication 1.

**15.** Composés selon la revendication 14, dans lesquels $R^1$ représente ledit groupe de formule (II).

**16.** Composés selon la revendication 14 ou 15, dans lesquels $R^5$ représente un atome d'hydrogène ou un groupe alcanoyle en $C_2$-$C_4$.

**17.** Composés selon l'une quelconque des revendications 14 à 16, dans lesquels $R^3$ représente un groupe alkyle en $C_{13}$-$C_{20}$.

**18.** Composés selon l'une quelconque des revendications précédentes, dans lesquels n est un nombre entier allant de 1 à 7.

**19.** Composés selon l'une quelconque des revendications précédentes dans lesquels:
Q représente le groupe thiazolyle, pyridyle, quinolyle, isoquinolyle ou imidazolyle, ou un groupe thiazolyle, pyridyle, quinolyle, isoquinolyle ou imidazolyle contenant au moins un substituant alkyle en $C_1$-$C_4$.

**20.** Composés selon la revendication 19, dans lesquels Q représente le groupe thiazolyle ou pyridyle.

**21.** Composés selon l'une quelconque des revendications précédentes, dans lesquels E représente un groupe isoxazolediyle ou thiazolediyle.

**22.** Composés selon la revendication 21, dans lesquels E représente le groupe 3,5-isoxazolediyle.

**23.** Composés selon l'une quelconque des revendications précédentes, dans lesquels:
$R^2$ représente un groupe de formule (III):

$$-E-(CH_2)_m-\underset{\substack{|\\ R^4}}{(CH)}_q-(CH_2)_n-Q \qquad \text{(III)}$$

dans laquelle E, Q, $R^4$, m, n et q sont tels que définis dans la revendication 1.

**24.** Composés selon l'une quelconque des revendications précédentes, dans lesquels:
dans le groupe représenté par $R^2$, le groupe de formule

$$-E-(CH_2)_m-\underset{R^4}{(\overset{}{CH})}_q-$$

est un groupe de formule

$$-\underset{O}{\overset{}{C}}\!\!\!\!\!\!\overset{}{}-\underset{R^6}{N}- \quad \text{ou} \quad -CH_2-\underset{R^4}{CH}-$$

(formules dans lesquelles $R^4$ et $R^6$ sont tels que définis dans la revendication 1) ou un groupe isoxazolediyle.

**25.** Composés selon la revendication 24, dans lesquels $R^6$ est un atome d'hydrogène ou le groupe acétyle.

**26.** Composés selon l'une quelconque des revendications précédentes, dans lesquels:
$R^1$ représente un groupe de formule (II):

$$-CO\underset{R^5}{N}-R^3 \qquad (II)$$

dans laquelle $R^3$ et $R^5$ sont tels que définis dans la revendication 1, et
$R^2$ représente un groupe de formule (III):

$$-E-(CH_2)_m-\underset{R^4}{(CH)}_q-(CH_2)_n-Q \qquad (III)$$

dans laquelle E, m, n, Q, $R^4$ et q sont tels que définis dans la revendication 1.

**27.** Composés selon la revendication 26, dans lesquels $R^5$ représente un atome d'hydrogène ou un groupe alcanoyle en $C_2$-$C_4$.

**28.** Composés selon la revendication 26, dans lesquels:
le groupe de formule

$$-E-(CH_2)_m-\underset{R^4}{(CH)}_q-$$

dans le groupe représenté par $R^2$ est un groupe de formule:

$$-\underset{O}{\overset{\|}{C}}-\underset{R^6}{N}- \quad \text{ou} \quad -CH_2-\underset{R^4}{CH}-$$

dans laquelle $R^6$ est tel que défini dans la revendication 1, ou un groupe isoxazolediyle.

**29.** Composés selon la revendication 28, dans lesquels $R^6$ représente un atome d'hydrogène ou le groupe acétyle.

**30.** Sel de 3-{6-éthoxycarbonyl-6-[(3-heptadécylcarbamoyloxytétrahydropyranne-2-yl)méthoxycarbonylamino]hexyl}-thiazolium.

**31.** Sel de *dl*-3-{6-éthoxycarbonyl-6-((*trans*-3-heptadécylcarbamoyloxytétrahydropyranne-2-yl)méthoxycarbonylamino]hexyl}thiazolium.

**32.** Sel de 3-{5-[(3-heptadécylcarbamoyloxytétrahydropyranne-2-yl)méthoxycarbonylamino]-pentyl}thiazolium.

**33.** Sel de *dl*-3-{5-[(*trans*-3-heptadécylcarbamoyloxytétrahydropyranne-2-yl)méthoxycarbonylamino]-pentyl}thiazolium.

**34.** Sel de 1-éthyl-2-{N-acétyl-N-[3-(N-hepadécylcarbamoyloxy)tétrahydropyranne-2-ylméthoxycarbonyl]-aminométhyl}pyridinium.

**35.** Sel de *dl*-1-éthyl-2-{N-acétyl-N-[*trans*-3-(N-heptadécylcarbamoyloxy)tétrahydropyranne-2-ylméthoxy-carbonyl]aminométhyl}pyridinium.

**36.** Sel de 3-{7-acétoxy-8-[(3-heptadécylcarbamoyloxytétrahydropyranne-2-yl)méthoxy]octyl}thiazolium.

**37.** Sel de 1-éthyl-2-{N-acétyl-N-[3-(N-acétyl-N-heptadécylcarbamoylthio)tétrahydropyranne-2-ylméthoxycarbonyl]aminométhyl}pyridinium.

**38.** Sel de *dl*-1-éthyl-2-{N-acétyl-N-[*cis*-3-(N-acétyl-N-heptadécylcarbamoylthio)tétrahydropyranne-2-ylméthoxycarbonyl]aminométhyl}pyridinium.

**39.** Sel de 1-éthyl-2-{N-[3-(N-acétyl-N-heptadécylcarbamoylthio)tétrahydropyranne-2-ylméthoxycarbonyl]-aminométhyl}pyridinium.

**40.** Sel de *dl*-1-éthyl-2-{N-[*cis*-3-(N-acétyl-N-heptadécylcarbamoylthio)tétrahydropyranne-2-ylméthoxy-carbonyl]aminométhyl}pyridinium.

**41.** Sel de 3-{4-[3-(3-heptadécylcarbamoyloxytétrahydropyranne-2-yl)méthoxy-5-isoxazolyl]butyl}thiazolium.

**42.** Sel de *dl*-3-{4-[3-(*trans*-3-heptadécylcarbamoyloxytétrahydropyranne-2-yl)méthoxy-5-isoxazolyl]-butyl}thiazolium.

**43.** Composition pharmaceutique pour le traitement de l'inflammation ou de l'état de choc, comprenant un antagoniste du facteur d'activation des plaquettes (PAF) en association avec un véhicule ou diluant pharmaceutiquement acceptable, dans laquelle l'antagoniste de PAF est au moins un composé selon l'une quelconque des revendications précédentes.

**44.** Utilisation d'au moins un composé de formule (I) et/ou d'un sel ou ester pharmaceutiquement acceptable de celui-ci, selon l'une quelconque des revendications 1 à 42, pour la fabrication d'un médicament pour le traitement ou la prophylaxie de l'asthme, de l'inflammation ou de l'état de choc.

**45.** Procédé pour la préparation d'un composé selon l'une quelconque des revendications 1 à 42, lequel procédé comprend:

(I) la mise en réaction d'un composé de formule (D)

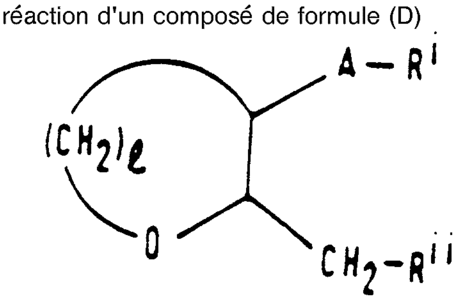

(D)

dans laquelle

(a) $R^i$ représente un groupe de formule $-A-R^{1x}$ et

$R^{ii}$ représente un groupe de formule -B-H; ou

(g) $R^i$ représente un groupe de formule $-A-R^{10}$ et

$R^{ii}$ représente un groupe de formule -B-H;

formules dans lesquelles

A et B sont tels que définis plus haut;

$R^{1x}$ représente un groupe alkyle contenant de 8 à 22 atomes de carbone, un groupe acyle

carboxylique aliphatique contenant de 8 à 22 atomes de carbone ou un groupe de formule (II):

$$-CON(R^5)-R^3 \qquad (II)$$

dans laquelle $R^3$ et $R^5$ sont tels que définis plus haut;

$R^{10}$ représente un groupe protecteur de fonction hydroxy ou protecteur de fonction mercapto;

ou un composé de formule (D) dans lequel tout groupe actif est protégé, avec un composé de formule (VI):

$$Y-(CH_2)_m-(\underset{R^{4'}}{CH})_q-(CH_2)_n-Q' \qquad (VI)$$

ou de formule (VII):

$$HB-E^f-(CH_2)_m-(\underset{R^{4'}}{CH})_q-(CH_2)_n-Q' \qquad (VII)$$

ou de formule (XIX):

$$W-\underset{O}{\overset{\|}{C}}-(CH_2)_m-(\underset{R^{4'}}{CH})_q-(CH_2)_n-Q'' \qquad (XIX)$$

ou de formule (XX):

$$O=C=N-(CH_2)_m-(\underset{R^{4'}}{CH})_q-(CH_2)_n-Q'' \qquad (XX)$$

ou avec un composé de formule (XXII) suivi d'un composé de formule (XXIV):

$$Y-\underset{O}{\overset{\|}{C}}-Y'' \qquad (XXII)$$

$$H_2N-(CH_2)_m-(\underset{R^{4'}}{CH})_q-(CH_2)_n-Q' \qquad (XXIV)$$

ou avec ledit composé de formule (XXII) et ensuite avec un composé de formule (XXX):

$$HO-(CH_2)_m-(\underset{R^{4'}}{CH})_q-(CH_2)_n-Q' \qquad (XXX)$$

ou dans le cas (g)

soit avec un halogénure d'allyle et ensuite un oxydant, soit avec une épihalogénohydrine et, dans l'un ou l'autre cas, ensuite avec un composé de formule

$M-(CH_2)_{(n-1)}-Q'$,

formules dans lesquelles

$R^{4'}$ représente l'un quelconque des groupes définis plus haut pour $R^4$, mais dans lequel tout groupe réactif est, si nécessaire, protégé;

$E^f$ représente un groupe hétérocyclique contenant de 5 à 14 atomes formant le cycle, comme défini plus haut pour E;

Q' représente un groupe de formule $-O-R^{11}$, dans laquelle $R^{11}$ représente un groupe protecteur de fonction hydroxy;

Q" représente un groupe de formule Y, défini plus haut ou l'un quelconque des groupes hétérocycliques représentés par Q, Q étant tel que défini plus haut;
W représente un reste d'un acide carboxylique réactif, un groupe acyloxy aliphatique inférieur ou un groupe acyloxy aromatique;
Y représente un atome d'halogène;
Y" représente un groupe séparable; et
M représente un atome métallique;

(II) lorsque le produit de l'étape (I) contient un groupe de formule Q', la conversion dudit groupe en un groupe de formule Q"; et

(III) éventuellement la soumission du produit de l'étape (I) ou (II) à une ou plusieurs des réactions amination, protection, élimination des groupes protecteurs, salification et estérification, dans un ordre quelconque.

**46.** Procédé pour la préparation d'un composé selon l'une quelconque des revendications 1 à 42, lequel procédé comprend:

(I) la mise en réaction d'un composé de formule (D)

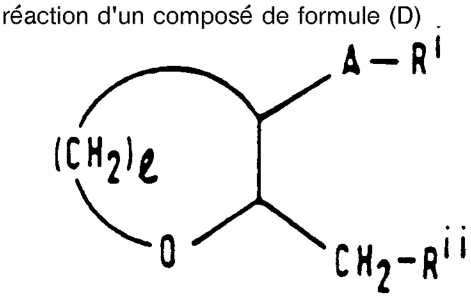

(D)

dans laquelle

(b) $R^i$ représente un groupe de formule $-A-R^{1x'}$ et
$R^{ii}$ représente un groupe de formule -Y; ou
(c) $R^i$ représente un groupe de formule $-A-R^{10}$ et
$R^{ii}$ représente un groupe de formule -Y;

formules dans lesquelles
A, $R^{10}$ et Y sont tels que définis dans la revendication 45; et $R^{1x'}$ représente un groupe alkyle contenant de 8 à 22 atomes de carbone;
ou d'un composé de formule (D) dans laquelle tout groupe actif est protégé, avec un composé de formule (XI):

$$HB-E-(CH_2)_m-\underset{R^{4'}}{(CH)}_q-(CH_2)_n-Q' \qquad (XI)$$

dans laquelle $R^{4'}$ et Q' sont tels que définis dans la revendication 45;

(II) lorsque le produit de l'étape (I) contient un groupe de formule Q', la conversion dudit groupe en un groupe de formule Q"; et

(III) éventuellement la soumission du produit de l'étape (I) ou de l'étape (II) à une ou plusieurs des réactions amination, protection, élimination des groupes protecteurs, salification et estérification, dans un ordre quelconque.

**47.** Procédé pour la préparation d'un composé selon l'une quelconque des revendications 1 à 42, lequel procédé comprend:

(I) la mise en réaction d'un composé de formule (D):

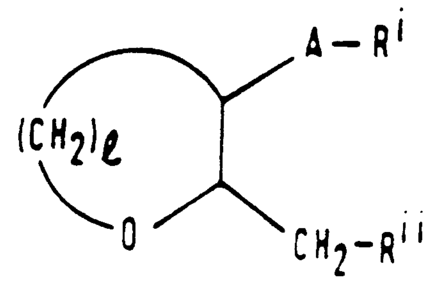

(D)

dans laquelle

(d) $R^i$ représente un groupe de formule -A-H et $R^{ii}$ représente un groupe de formule $-B-R^{1x}$; ou

(h) $R^i$ représente un groupe de formule -A-H et $R^{ii}$ représente un groupe de formule $-B-R^{10}$;

formules dans lesquelles:

A, B, $R^{1x}$ et $R^{10}$ sont tels que définis plus haut; ou d'un composé de formule (D) dans lequel tout groupe actif est protégé,

avec un composé de formule (VI), défini dans la revendication 45, ou un composé de formule (VII'):

$$HA-E^{f}-(CH_2)_m-\underset{R^{4'}}{(CH)}_q-(CH_2)_n-Q' \qquad (VII')$$

ou un composé de formule (XIX) ou un composé de formule (XX) ou avec un composé de formule (XXII), suivi dudit composé de formule (XXIV) ou (XXX), toutes les formules (XIX), (XX), (XXII), (XXIV) et (XXX) étant telles que définis dans la revendication 45;

dans lesquelles

$R^{4'}$, $E^f$ et Q' sont tels que définis plus haut;

(II) lorsque le produit de l'étape (I) contient un groupe de formule Q', la conversion dudit groupe en un groupe de formule Q", et

(III) éventuellement la soumission du produit de l'étape (I) ou de l'étape (II) à une ou plusieurs des réactions amination, protection, élimination des groupes protecteurs, salification et estérification, dans un ordre quelconque.

**48.** Procédé pour la préparation d'un composé selon l'une quelconque des revendications 1 à 42, lequel procédé comprend:

(I) la mise en réaction d'un composé de formule (D)

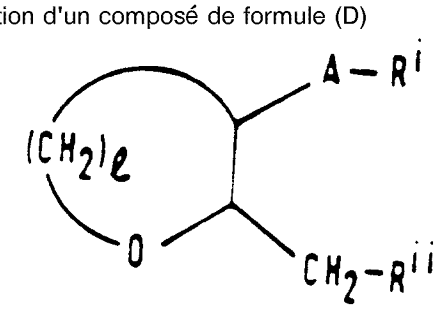

(D)

dans laquelle

(e) $R^i$ représente un groupe de formule -Y et $R^{ii}$ représente un groupe de formule $-B-R^{1x'}$; ou

(f) $R^i$ représente un groupe de formule -Y et $R^{ii}$ représente un groupe de formule $-B-R^{10}$;

formules dans lesquelles

A, B, Y, $R^{1x'}$ et $R^{10}$ sont tels que définis plus haut; ou d'un composé de formule (D) dans lequel tout groupe actif est protégé, avec un composé de formule (XI'):

$$HA\text{-}E\text{-}(CH_2)_m\text{-}(\underset{R^{4'}}{CH})_q\text{-}(CH_2)_n\text{-}Q' \qquad (XI')$$

dans laquelle A, E, $R^{4'}$ et Q' sont tels que définis plus haut;

(II) lorsque le produit de l'étape (I) contient un groupe de formule Q', la conversion dudit groupe en un groupe de formule Q"; et

(III) éventuellement la soumission du produit de l'étape (I) ou de l'étape (II) à une ou plusieurs des réactions amination, protection, élimination des groupes protecteurs, salification et estérification, dans un ordre quelconque.

**49.** Procédé pour la préparation d'un composé selon l'une quelconque des revendications 1 à 42, lequel procédé comprend:

(I) la mise en réaction d'un composé de formule (D)

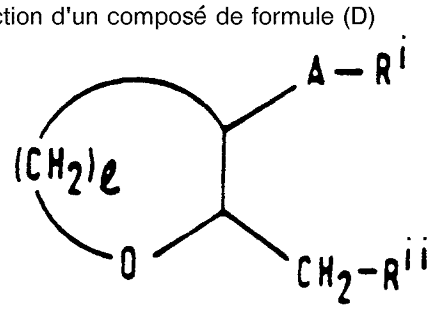

(D)

dans laquelle

(i) $R^i$ représente une double liaison entre les positions $\alpha$ et $\beta$ par rapport à l'autre atome d'oxygène, et $R^{ii}$ représente un groupe -Y;

Y étant tel que défini plus haut;

ou d'un composé de formule (D) dans lequel tout groupe actif est protégé;

avec un composé de formule (XI):

$$HB\text{-}E\text{-}(CH_2)_m\text{-}(\underset{R^{4'}}{CH})_q\text{-}(CH_2)_n\text{-}Q' \qquad (XI)$$

dans laquelle B, E, $R^{4'}$ et Q' sont tels que définis plus haut; suivie d'une hydroboration et d'une alkylation, d'une acylation ou d'une carbamation du groupe hydroxy;

(II) lorsque le produit de l'étape (I) contient un groupe de formule Q', la conversion dudit groupe en un groupe de formule Q"; et

(III) éventuellement la soumission du produit de l'étape (I) ou de l'étape (II) à une ou plusieurs des réactions amination, protection, élimination des groupes protecteurs, salification et estérification, dans un ordre quelconque.

**Revendications pour les Etats contractants suivants : AT, ES, GR**

**1.** Procédé pour la préparation d'un composé de formule (I)

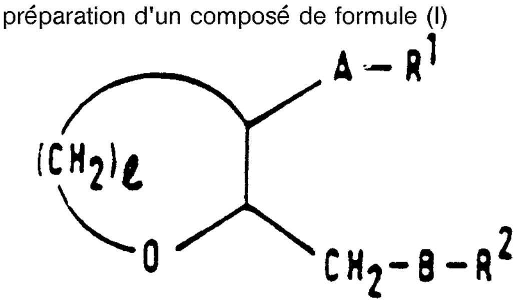

(I)

dans laquelle

ℓ est un nombre entier allant de 2 à 4;

A et B sont identiques ou différents et représentent chacun un atome d'oxygène ou un atome de

soufre;

l'un des radicaux $R^1$ et $R^2$ représente un groupe alkyle contenant de 8 à 22 atomes de carbone, un groupe acyle carboxylique aliphatique contenant de 8 à 22 atomes de carbone ou un groupe de formule (II):

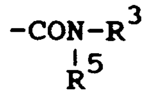

(II)

dans laquelle

$R^3$ représente un groupe alkyle contenant de 8 à 22 atomes de carbone, et

$R^5$ représente un atome d'hydrogène, un groupe alkyle en $C_1$-$C_4$, un groupe alcanoyle en $C_1$-$C_4$ ou un groupe aralkyle dans lequel le fragment alkyle est en $C_1$-$C_4$,

et l'autre des radicaux $R^1$ et $R^2$ représente un groupe de formule (III):

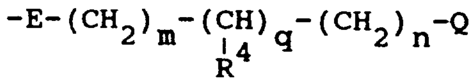

(III)

dans laquelle E représente une simple liaison, un groupe hétérocyclique bivalent ou un groupe de formule

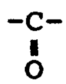 , 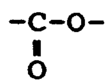 ou 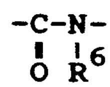

dans laquelle $R^6$ représente un atome d'hydrogène ou un groupe protecteur de fonction imino;

m est le chiffre 0 ou un nombre entier allant de 1 à 3;

n est le chiffre 0 ou un nombre entier allant de 1 à 10;

q est le chiffre 0 ou le nombre entier 1;

$R^4$ représente le groupe hydroxy, un groupe alcanoyloxy en $C_1$-$C_4$, un groupe alcoxy en $C_1$-$C_4$, un groupe aralkyloxy en $C_7$-$C_9$, un groupe carbamoyloxy, un groupe alkylcarbamoyloxy dans lequel le fragment alkyle est en $C_1$-$C_4$, un groupe dialkylcarbamoyloxy dans lequel chaque fragment alkyle est en $C_1$-$C_4$, le groupe mercapto, un groupe alkyl($C_1$-$C_4$)-thio, un groupe aralkyl($C_7$-$C_9$)-thio, un groupe alcanoyl-($C_1$-$C_4$)-thio, le groupe carbamoylthio, un groupe alkylcarbamoylthio dans lequel le fragment alkyle est en $C_1$-$C_4$, un groupe dialkylcarbamoylthio dans lequel chaque fragment alkyle est en $C_1$-$C_4$, ou le groupe carboxy;

Q représente un groupe de formule (IV):

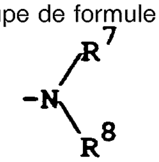

(IV)

dans laquelle $R^7$ et $R^8$ sont identiques ou différents et représentent chacun un atome d'hydrogène ou un groupe alkyle en $C_1$-$C_6$,

ou un groupe hétérocyclique monovalent;

lesdits groupes hétérocycliques ayant de 5 à 14 atomes formant le cycle, dont de 1 à 4 sont des hétéroatomes d'azote et/ou d'oxygène et/ou de soufre, au moins l'un de ceux-ci étant un atome d'azote, et ledit groupe hétérocyclique étant non substitué ou comportant au moins l'un des substituants (a) et/ou des substituants (b) définis ci-dessous:

substituants (a):

des atomes d'oxygène et des groupes aryle en $C_6$-$C_{14}$;

substituants (b):

des atomes d'halogène, le groupe hydroxy, des groupes alkyle en $C_1$-$C_4$, halogénoalkyle en $C_1$-$C_4$, hydroxyalkyle en $C_1$-$C_4$, alcoxy en $C_1$-$C_4$, alcanoyle en $C_1$-$C_6$, alcénoyle en $C_3$-$C_6$, des groupes acyle carboxyliques aromatiques en $C_7$-$C_{15}$, le groupe carbamoyle, des groupes aralkyle en $C_7$-$C_{15}$, alcoxy-($C_2$-$C_5$)-carbonyle, le groupe cyano, le groupe amino, des groupes alkylamino dans lesquels le fragment alkyle est en $C_1$-$C_4$, des groupes dialkylcarbamoyloxy dans lesquels chaque fragment alkyle est en $C_1$-$C_4$, et le groupe nitro;

lesdits groupes aryle et les fragments aryle des groupes aralkyle et des groupes acyle aromatiques ayant de 6 à 14 atomes formant le cycle et n'étant pas substitués ou comportant au moins l'un des substituants (b) définis plus haut;

et de ses sels et esters pharmaceutiquement acceptables, lequel procédé comprend:

(I) la mise en réaction d'un composé de formule (D)

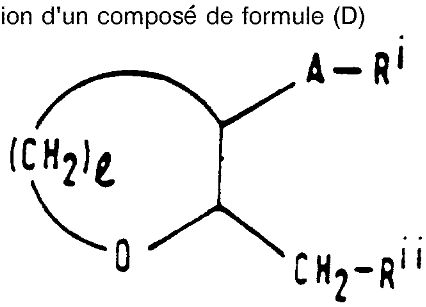

(D)

dans laquelle

(a) $R^i$ représente un groupe de formule -A-$R^{1x}$ et

$R^{ii}$ représente un groupe de formule -B-H; ou

(g) $R^i$ représente un groupe de formule -A-$R^{10}$ et

$R^{ii}$ représente un groupe de formule -B-H;

formules dans lesquelles

A et B sont tels que définis plus haut;

$R^{1x}$ représente un groupe alkyle contenant de 8 à 22 atomes de carbone, un groupe acyle carboxylique aliphatique contenant de 8 à 22 atomes de carbone ou un groupe de formule (II):

$$-CO\underset{\displaystyle R^5}{\underset{|}{N}}-R^3 \qquad (II)$$

dans laquelle $R^3$ et $R^5$ sont tels que définis plus haut;

$R^{10}$ représente un groupe protecteur de fonction hydroxy ou protecteur de fonction mercapto;

ou un composé de formule (D) dans lequel tout groupe actif est protégé,

avec un composé de formule (VI):

$$Y-(CH_2)_m-(\underset{\displaystyle R^{4'}}{\underset{|}{CH}})_q-(CH_2)_n-Q' \qquad (VI)$$

ou de formule (VII):

$$HB-E^f-(CH_2)_m-(\underset{\displaystyle R^{4'}}{\underset{|}{CH}})_q-(CH_2)_n-Q' \qquad (VII)$$

ou de formule (XIX):

$$W-\underset{\displaystyle O}{\underset{\|}{C}}-(CH_2)_m-(\underset{\displaystyle R^{4'}}{\underset{|}{CH}})_q-(CH_2)_n-Q'' \qquad (XIX)$$

ou de formule (XX):

$$O=C=N-(CH_2)_m-(\underset{\displaystyle R^{4'}}{\underset{|}{CH}})_q-(CH_2)_n-Q'' \qquad (XX)$$

ou avec un composé de formule (XXII) suivi d'un composé de formule (XXIV):

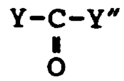 (XXII)

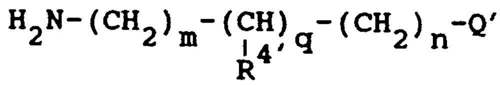 (XXIV)

ou avec ledit composé de formule (XXII) et ensuite avec un composé de formule (XXX):

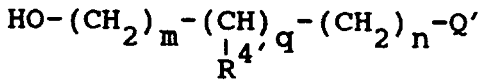 (XXX)

ou dans le cas (g)

soit avec un halogénure d'allyle et ensuite un oxydant, soit avec une épihalogénohydrine et, dans l'un ou l'autre cas, ensuite avec un composé de formule

$M-(CH_2)_{(n-1)}-Q'$,

formules dans lesquelles

- $R^{4'}$ représente l'un quelconque des groupes définis plus haut pour $R^4$, mais dans lequel tout groupe réactif est, si nécessaire, protégé;
- $E^f$ représente un groupe hétérocyclique contenant de 5 à 14 atomes formant le cycle, comme défini plus haut pour E;
- Q' représente un groupe de formule $-O-R^{11}$, dans laquelle $R^{11}$ représente un groupe protecteur de fonction hydroxy;
- Q" représente un groupe de formule Y, défini plus haut ou l'un quelconque des groupes hétérocycliques représentés par Q, Q étant tel que défini plus haut;
- W représente un reste d'un acide carboxylique réactif, un groupe acyloxy aliphatique inférieur ou un groupe acyloxy aromatique;
- Y représente un atome d'halogène;
- Y" représente un groupe séparable; et
- M représente un atome métallique;

(II) lorsque le produit de l'étape (I) contient un groupe de formule Q', la conversion dudit groupe en un groupe de formule Q"; et

(III) éventuellement la soumission du produit de l'étape (I) ou (II) à une ou plusieurs des réactions amination, protection, élimination des groupes protecteurs, salification et estérification, dans un ordre quelconque.

**2.** Procédé pour la préparation d'un composé de formule (I) tel que défini selon la revendication 1, lequel procédé comprend:

(I) la mise en réaction d'un composé de formule (D)

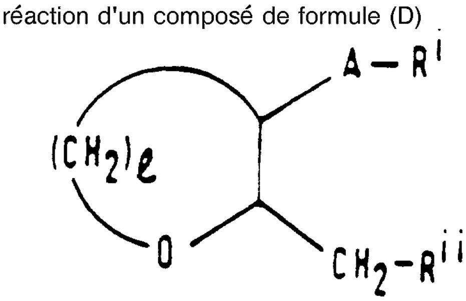 (D)

dans laquelle

(b) $R^i$ représente un groupe de formule $-A-R^{1x'}$ et

$R^{ii}$ représente un groupe de formule -Y; ou

(c) $R^i$ représente un groupe de formule $-A-R^{10}$ et

$R^{ii}$ représente un groupe de formule -Y;

formules dans lesquelles

A, $R^{10}$ et Y sont tels que définis dans la revendication 1; et $R^{1x'}$ représente un groupe alkyle

contenant de 8 à 22 atomes de carbone; ou d'un composé de formule (D) dans laquelle tout groupe actif est protégé, avec un composé de formule (XI):

$$HB-E-(CH_2)_m-\underset{R^{4'}}{\underset{|}{(CH)}}_q-(CH_2)_n-Q' \qquad (XI)$$

dans laquelle $R^{4'}$ et Q' sont tels que définis dans la revendication 1;
(II) lorsque le produit de l'étape (I) contient un groupe de formule Q', la conversion dudit groupe en un groupe de formule Q"; et
(III) éventuellement la soumission du produit de l'étape (I) ou de l'étape (II) à une ou plusieurs des réactions amination, protection, élimination des groupes protecteurs, salification et estérification, dans un ordre quelconque.

3. Procédé pour la préparation d'un composé de formule (I) telle que définie dans la revendication 1, lequel procédé comprend:
(I) la mise en réaction d'un composé de formule (D):

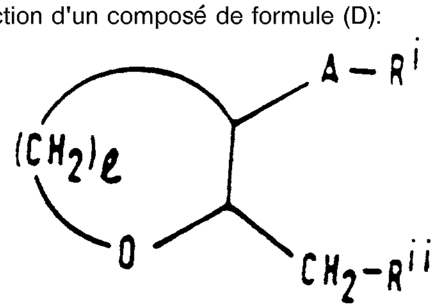

(D)

dans laquelle
(d) $R^i$ représente un groupe de formule -A-H et $R^{ii}$ représente un groupe de formule $-B-R^{1x}$; ou
(h) $R^i$ représente un groupe de formule -A-H et $R^{ii}$ représente un groupe de formule $-B-R^{10}$;
formules dans lesquelles:
A, B, $R^{1x}$ et $R^{10}$ sont tels que définis plus haut; ou d'un composé de formule (D) dans lequel tout groupe actif est protégé,
avec un composé de formule (VI), défini dans la revendication 1, ou un composé de formule (VII'):

$$HA-E^f-(CH_2)_m-\underset{R^{4'}}{\underset{|}{(CH)}}_q-(CH_2)_n-Q' \qquad (VII')$$

ou un composé de formule (XIX) ou un composé de formule (XX) ou avec un composé de formule (XXII), suivi dudit composé de formule (XXIV) ou (XXX), toutes les formules (XIX), (XX), (XXII), (XXIV) et (XXX) étant telles que définis dans la revendication 1;
dans lesquelles
$R^{4'}$, $E^f$ et Q' sont tels que définis plus haut;
(II) lorsque le produit de l'étape (I) contient un groupe de formule Q', la conversion dudit groupe en un groupe de formule Q", et
(III) éventuellement la soumission du produit de l'étape (I) ou de l'étape (II) à une ou plusieurs des réactions amination, protection, élimination des groupes protecteurs, salification et estérification, dans un ordre quelconque.

4. Procédé pour la préparation d'un composé de formule (I) telle que définie dans la revendication 1, lequel procédé comprend:
(I) la mise en réaction d'un composé de formule (D)

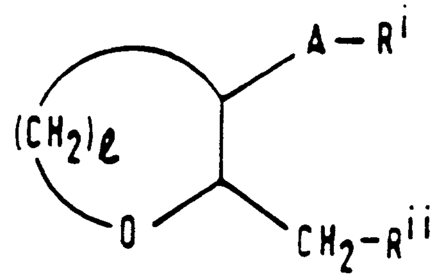

(D)

dans laquelle

(e) $R^i$ représente un groupe de formule -Y et $R^{ii}$ représente un groupe de formule -B-$R^{1x'}$; ou

(f) $R^i$ représente un groupe de formule -Y et $R^{ii}$ représente un groupe de formule -B-$R^{10}$;

formules dans lesquelles

A, B, Y, $R^{1x'}$ et $R^{10}$ sont tels que définis plus haut; ou d'un composé de formule (D) dans lequel tout groupe actif est protégé, avec un composé de formule (XI'):

$$HA-E-(CH_2)_m-\underset{R^{4'}}{(CH)}_q-(CH_2)_n-Q' \qquad (XI')$$

dans laquelle A, E, $R^{4'}$ et Q' sont tels que définis plus haut;

(II) lorsque le produit de l'étape (I) contient un groupe de formule Q', la conversion dudit groupe en un groupe de formule Q"; et

(III) éventuellement la soumission du produit de l'étape (I) ou de l'étape (II) à une ou plusieurs des réactions amination, protection, élimination des groupes protecteurs, salification et estérification, dans un ordre quelconque.

5. Procédé pour la préparation d'un composé de formule (I) telle que définie dans la revendication 1, lequel procédé comprend:

(I) la mise en réaction d'un composé de formule (D)

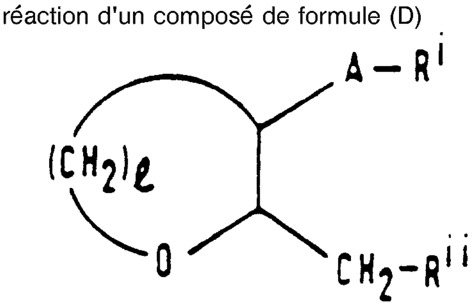

(D)

dans laquelle

(i) $R^i$ représente une double liaison entre les positions $\alpha$ et $\beta$ par rapport à l'autre atome d'oxygène, et $R^{ii}$ représente un groupe -Y;

Y étant tel que défini plus haut;

ou d'un composé de formule (D) dans lequel tout groupe actif est protégé;

avec un composé de formule (XI):

$$HB-E-(CH_2)_m-\underset{R^{4'}}{(CH)}_q-(CH_2)_n-Q' \qquad (XI)$$

dans laquelle B, E, $R^{4'}$ et Q' sont tels que définis plus haut; suivie d'une hydroboration et d'une alkylation, d'une acylation ou d'une carbamation du groupe hydroxy;

(II) lorsque le produit de l'étape (I) contient un groupe de formule Q', la conversion dudit groupe en un groupe de formule Q"; et

(III) éventuellement la soumission du produit de l'étape (I) ou de l'étape (II) à une ou plusieurs des réactions amination, protection, élimination des groupes protecteurs, salification et estérification,

dans un ordre quelconque.

**6.** Procédé selon l'une quelconque des revendications 1 à 5, dans lequel on choisit les réactifs et conditions réactionnelles de manière à préparer un composé de formule (I) telle qu'indiquée dans la revendication 1, dans laquelle l'un des radicaux $R^1$ et $R^2$ représente un groupe de formule (III'):

$$-E-(CH_2)_m-\underset{COO^-}{\underset{|}{CH}}-(CH_2)_n-Q^+ \qquad (III')$$

dans laquelle E, m et n sont tels que définis dans la revendication 1, et $Q^+$ représente un groupe hétérocyclique rendu quaternaire ou un groupe de formule (IV'):

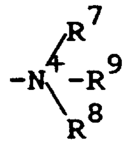

(IV')

dans laquelle $R^7$, $R^8$ et $R^9$ sont identiques ou différents et représentent chacun un atome d'hydrogène ou un groupe alkyle en $C_1$-$C_6$.

**7.** Procédé selon l'une quelconque des revendications 1 à 6, dans lequel ledit sel est sous la forme d'un sel d'ammonium quaternaire ou d'un sel d'addition avec un acide, dans lesquels Q représente un groupe de formule (IV")

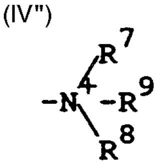

$Z^-$ (IV")

dans laquelle $R^7$, $R^8$ sont tels que définis dans la revendication 1, $R^9$ est tel que défini dans la revendication 6 et $Z^-$ représente un anion complémentaire pharmaceutiquement acceptable,
ou un groupe hétérocyclique rendu quaternaire, conjointement avec un anion complémentaire pharmaceutiquement acceptable.

**8.** Procédé selon la revendication 7, dans lequel ledit anion complémentaire pharmaceutiquement acceptable est un halogène, un reste anionique d'un autre acide minéral, un groupe alkyl($C_1$-$C_6$)-sulfonyloxy, un groupe arylsulfonyloxy ou un anion dérivé d'un acide carboxylique organique ou d'un aminoacide.

**9.** Procédé selon l'une quelconque des revendications précédentes, dans lequel les réactifs et conditions réactionnelles sont choisis de manière à préparer un composé représenté par la formule (Ia):

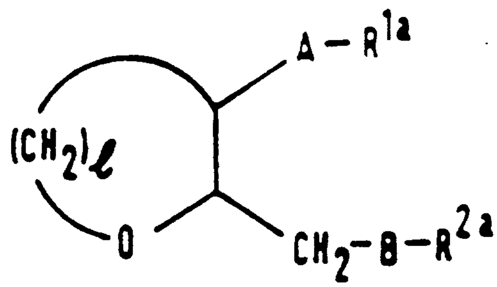

(Ia)

dans laquelle ℓ, A et B sont tels que définis dans la revendication 1, et
l'un des radicaux $R^{1a}$ et $R^{2a}$ représente un groupe alkyle contenant de 8 à 22 atomes de carbone, un groupe acyle carboxylique aliphatique contenant de 8 à 22 atomes de carbone ou un groupe de formule (II), telle que définie dans la revendication 1, et l'autre des radicaux $R^{1a}$ et $R^{2a}$ représente un groupe de formule (IIa):

$$-E-(CH_2)_m-\underset{R^{4a}}{\underset{|}{(CH)}}_q-(CH_2)_n-Q \qquad \text{(IIIa)}$$

dans laquelle m, n, q et Q sont tels que définis dans la revendication 1, et

E représente une simple liaison, un groupe hétérocyclique bivalent ou un groupe de formule

$$\underset{O}{\overset{}{\underset{\|}{-C-}}},\quad \underset{O}{\underset{\|}{-C}}-O-\quad ou \quad \underset{O}{\underset{\|}{-C}}-\underset{R^6}{\underset{|}{N}}-$$

dans laquelle $R^6$ est tel que défini dans la revendication 1;

$R^{4a}$ représente le groupe hydroxy, un groupe alcanoyloxy en $C_1$-$C_4$, un groupe alcoxy en $C_1$-$C_4$, un groupe aralkyloxy en $C_7$-$C_9$, le groupe carbamoyloxy, un groupe alkylcarbamoyloxy dans lequel le fragment alkyle est en $C_1$-$C_4$, un groupe dialkylcarbamoyloxy dans lequel chaque fragment alkyle est en $C_1$-$C_4$, le groupe mercapto, un groupe alkyl($C_1$-$C_4$)-thio, un groupe aralkyl($C_7$-$C_9$)-thio, un groupe alcanoyl($C_1$-$C_4$)-thio, le groupe carbamoylthio, un groupe alkylcarbamoylthio dans lequel le fragment alkyle est en $C_1$-$C_4$, un groupe dialkylcarbamoylthio dans lequel chaque fragment alkyle est en $C_1$-$C_4$, le groupe carboxy ou un groupe alcoxycarbonyle dont le fragment alcoxy est en $C_1$-$C_4$;

lesdits groupes hétérocycliques ayant de 5 à 10 atomes formant le cycle, dont de 1 à 4 sont des hétéroatomes d'azote et/ou d'oxygène et/ou de soufre, les groupes hétérocycliques représentés par E n'étant pas substitués et les groupes hétérocycliques représentés par Q n'étant pas substitués ou comportant au moins un substituant alkyle en $C_1$-$C_4$.

**10.** Procédé selon l'une quelconque des revendications précédentes, dans lequel le fragment éther cyclique du composé a la configuration 3S.

**11.** Procédé selon la revendication 10, dans lequel le fragment éther cyclique du composé a la configuration (3S,2R).

**12.** Procédé selon la revendication 10, dans lequel le fragment éther cyclique du composé a la configuration (3S,2S).

**13.** Procédé selon l'une quelconque des revendications 1 à 8 et 10 à 12, dans lequel on prépare un composé dans lequel:

ℓ est un nombre entier allant de 2 à 4;

A et B sont identiques ou différents et représentent chacun un atome d'oxygène ou un atome de soufre;

l'un des radicaux $R^1$ et $R^2$ représente un groupe alkyle contenant de 10 à 22 atomes de carbone, un groupe acyle carboxylique aliphatique contenant de 10 à 22 atomes de carbone ou un groupe de formule (IIa):

$-CONH-R^3$ (IIa)

dans laquelle $R^3$ représente un groupe alkyle contenant de 10 à 22 atomes de carbone,

et l'autre représente un groupe de formule (IIIb):

$$-E^a-(CH_2)_m-\underset{R^{4b}}{\underset{|}{CH}}-(CH_2)_n-Q \qquad \text{(IIIb)}$$

dans laquelle $E^a$ représente une simple liaison ou un groupe de formule

$$\underset{O}{\underset{\|}{-C-}},\quad \underset{O}{\underset{\|}{-C}}-O-\quad ou \quad \underset{O}{\underset{\|}{-C}}-\underset{R^6}{\underset{|}{N}}-$$

dans laquelle $R^6$ représente un atome d'hydrogène ou un groupe protecteur de fonction imino;

m est le chiffre 0 ou un nombre entier allant de 1 à 3;

n est le chiffre 0 ou un nombre entier allant de 1 à 10;

$R^{4b}$ représente un atome d'hydrogène, le groupe carboxy ou un groupe alcoxycarbonyle dans lequel le fragment alcoxy est en $C_1$-$C_4$;

Q représente un groupe de formule (IV):

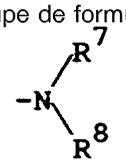

(IV)

dans laquelle $R^7$ et $R^8$ sont identiques ou différents et représentent chacun un atome d'hydrogène ou un groupe alkyle en $C_1$-$C_6$,

ou un groupe hétérocyclique monovalent ayant de 5 7 atomes formant le cycle, dont de 1 à 4 atomes sont des hétéroatomes d'azote et/ou d'oxygène et/ou de soufre, au moins l'un de ceux-ci étant un atome d'azote, et le groupe hétérocyclique représenté par Q n'étant pas substitué ou comportant au moins un substituant choisi parmi des groupes alkyle en $C_1$-$C_4$, hydroxyalkyle en $C_1$-$C_4$, alcoxy en $C_1$-$C_4$ et des atomes d'halogène, ou un tel groupe hétérocyclique comportant un autre cycle soudé à celui-ci.

**14.** Procédé selon l'une quelconque des revendications 1 à 8 et 10 à 12, dans lequel on prépare un composé dans lequel:

ℓ est un nombre entier allant de 2 à 4;

A et B sont identiques ou différents et représentent chacun un atome d'oxygène ou un atome de soufre;

l'un des radicaux $R^1$ et $R^2$ représente un groupe alkyle contenant de 10 à 22 atomes de carbone, un groupe acyle carboxylique aliphatique contenant de 10 à 22 atomes de carbone ou un groupe de formule (IIa):

$-CONH-R^3$ (IIa)

dans laquelle $R^3$ représente un groupe alkyle contenant de 10 à 22 atomes de carbone,

et l'autre représente un groupe de formule (IIIc):

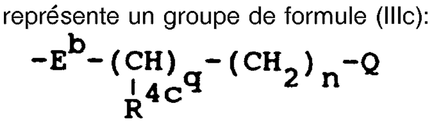

(IIIc)

dans laquelle

$E^b$ représente un groupe de formule $-(CH_2)_{m'}-$ ou un groupe hétérocyclique bivalent;

m' est un nombre entier allant de 1 à 3;

n est le chiffre 0 ou un nombre entier allant de 1 à 10;

q est le chiffre 0 ou le nombre entier 1;

$R^{4c}$ représente le groupe hydroxy, un groupe alcanoyloxy en $C_1$-$C_4$, un groupe alcoxy en $C_1$-$C_4$, un groupe aralkyloxy en $C_7$-$C_9$, le groupe carbamoyloxy, un groupe alkylcarbamoyloxy dans lequel le fragment alkyle est en $C_1$-$C_4$, un groupe dialkylcarbamoyloxy dans lequel chaque fragment alkyle est en $C_1$-$C_4$, le groupe mercapto, un groupe alcanoyl($C_1$-$C_4$)-thio, un groupe alkyl($C_1$-$C_4$)-thio, un groupe aralkyl($C_7$-$C_9$)-thio, le groupe carbamoylthio, un groupe alkylcarbamoylthio dans lequel le fragment alkyle est en $C_1$-$C_4$, ou un groupe dialkylcarbamoylthio dans lequel chaque fragment alkyle est en $C_1$-$C_4$;

Q représente un groupe de formule (IV):

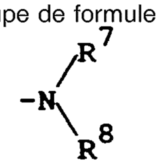

(IV)

dans laquelle $R^7$ et $R^8$ sont identiques ou différents et représentent chacun un atome d'hydrogène ou un groupe alkyle en $C_1$-$C_6$,

ou un groupe hétérocyclique monovalent ayant de 5 7 atomes formant le cycle, dont de 1 à 4 atomes sont des hétéroatomes d'azote et/ou d'oxygène et/ou de soufre, au moins l'un de ceux-ci étant un atome d'azote, et les groupes hétérocycliques représentés par Q étant non substitués ou comportant au moins un substituant choisi parmi des groupes alkyle en $C_1$-$C_4$, hydroxyalkyle en $C_1$-$C_4$, alcoxy en $C_1$-$C_4$, carbamoyle et des atomes d'halogène, ou un tel groupe hétérocyclique comportant un autre cycle soudé à celui-ci.

**15.** Procédé selon l'une quelconque des revendications précédentes, dans lequel:
$\ell$ est le nombre entier 2 ou 3.

**16.** Procédé selon la revendication 15, dans lequel:
$\ell$ est le nombre entier 3.

**17.** Procédé selon l'une quelconque des revendications précédentes, dans lequel:
A représente un atome d'oxygène ou de soufre et
B représente un atome d'oxygène.

**18.** Procédé selon l'une quelconque des revendications précédentes, dans lequel:
$R^1$ représente un groupe alkyle en $C_8$-$C_{22}$ ou un groupe de formule (II):

$$-\underset{\displaystyle R^5}{\underset{|}{CON}}-R^3 \qquad (II)$$

dans laquelle $R^3$ et $R^5$ sont tels que définis dans la revendication 1.

**19.** Procédé selon la revendication 18, dans lequel $R^1$ représente ledit groupe de formule (II).

**20.** Procédé selon la revendication 18 ou 19, dans lequel $R^5$ représente un atome d'hydrogène ou un groupe alcanoyle en $C_2$-$C_4$.

**21.** Procédé selon l'une quelconque des revendications 18 à 20, dans lequel $R^3$ représente un groupe alkyle en $C_{13}$-$C_{20}$.

**22.** Procédé selon l'une quelconque des revendications précédentes, dans lequel n est un nombre entier allant de 1 à 7.

**23.** Procédé selon l'une quelconque des revendications précédentes dans lequel:
Q représente le groupe thiazolyle, pyridyle, quinolyle, isoquinolyle ou imidazolyle, ou un groupe thiazolyle, pyridyle, quinolyle, isoquinolyle ou imidazolyle contenant au moins un substituant alkyle en $C_1$-$C_4$.

**24.** Procédé selon la revendication 23, dans lequel Q représente le groupe thiazolyle ou pyridyle.

**25.** Procédé selon l'une quelconque des revendications précédentes, dans lequel E représente un groupe isoxazolediyle ou thiazolediyle.

**26.** Procédé selon la revendication 25, dans lequel E représente le groupe 3,5-isoxazolediyle.

**27.** Procédé selon l'une quelconque des revendications précédentes, dans lequel:
$R^2$ représente un groupe de formule (III):

$$-E-(CH_2)_m-(\underset{\displaystyle R^4}{\underset{|}{CH}})_q-(CH_2)_n-Q \qquad (III)$$

dans laquelle E, Q, $R^4$, m, n et q sont tels que définis dans la revendication 1.

**28.** Procédé selon l'une quelconque des revendications précédentes, dans lequel: dans le groupe représenté par $R^2$, le groupe de formule

$$-E-(CH_2)_m-(\underset{R^4}{\underset{|}{CH}})_q-$$

est un groupe de formule

$$-\underset{O}{\underset{\|}{C}}-\underset{R^6}{\underset{|}{N}}- \quad \text{ou} \quad -CH_2-\underset{R^4}{\underset{|}{CH}}-$$

(formules dans lesquelles $R^4$ et $R^6$ sont tels que définis dans la revendication 1) ou un groupe isoxazolediyle.

**29.** Procédé selon la revendication 28, dans lequel $R^6$ est un atome d'hydrogène ou le groupe acétyle.

**30.** Procédé selon l'une quelconque des revendications précédentes, dans lequel:

$R^1$ représente un groupe de formule (II):

$$-CON\underset{R^5}{\underset{|}{}}-R^3 \qquad (II)$$

dans laquelle $R^3$ et $R^5$ sont tels que définis dans la revendication 1, et

$R^2$ représente un groupe de formule (III):

$$-E-(CH_2)_m-(\underset{R^4}{\underset{|}{CH}})_q-(CH_2)_n-Q \qquad (III)$$

dans laquelle E, m, n, Q, $R^4$ et q sont tels que définis dans la revendication 1.

**31.** Procédé selon la revendication 30, dans lequel $R^5$ représente un atome d'hydrogène ou un groupe alcanoyle en $C_2$-$C_4$.

**32.** Procédé selon la revendication 30, dans lequel: le groupe de formule

$$-E-(CH_2)_m-(\underset{R^4}{\underset{|}{CH}})_q-$$

dans le groupe représenté par $R^2$ est un groupe de formule:

$$-\underset{O}{\underset{\|}{C}}-\underset{R^6}{\underset{|}{N}}- \quad \text{ou} \quad -CH_2-\underset{R^4}{\underset{|}{CH}}-$$

dans laquelle $R^6$ est tel que défini dans la revendication 1, ou un groupe isoxazolediyle.

**33.** Procédé selon la revendication 32, dans lequel $R^6$ représente un atome d'hydrogène ou le groupe acétyle.

**34.** Procédé selon l'une quelconque des revendications 1 à 5, dans lequel on choisit les réactifs et conditions réactionnelles de manière à préparer le sel de 3-{6-éthoxycarbonyl-6-[(3-heptadécylcarbamoyloxytétrahydropyranne-2-yl)méthoxycarbonylamino]hexyl}-thiazolium.

**35.** Procédé selon l'une quelconque des revendications 1 à 5, dans lequel on choisit les réactifs et conditions réactionnelles de manière à préparer le sel de *dl*-3-{6-éthoxycarbonyl-6-[(*trans*-3-heptadécylcarbamoyloxytétrahydropyranne-2-yl)methoxycarbonylamino]hexyl}thiazolium.

**36.** Procédé selon l'une quelconque des revendications 1 à 5, dans lequel on choisit les réactifs et conditions réactionnelles de manière à préparer le sel de 3-{5-[(3-

heptadécylcarbamoyloxytétrahydropyranne-2-yl)méthoxycarbonylamino]pentyl}thiazolium.

**37.** Procédé selon l'une quelconque des revendications 1 à 5, dans lequel on choisit les réactifs et conditions réactionnelles de manière à préparer le sel de *dl*-3-{5-[(*trans*-3-heptadécylcarbamoyloxytétrahydropyranne-2-yl)- méthoxycarbonylamino]pentyl}thiazolium.

**38.** Procédé selon l'une quelconque des revendications 1 à 5, dans lequel on choisit les réactifs et conditions réactionnelles de manière à préparer le sel de 1-éthyl-2-{N-acétyl-N-[3-(N-heptadécylcarbamoyloxy)tétrahydropyranne-2-ylméthoxycarbonyl]aminométhyl}pyridinium.

**39.** Procédé selon l'une quelconque des revendications 1 à 5, dans lequel on choisit les réactifs et conditions réactionnelles de manière à préparer le sel de *dl*-1-éthyl-2-{N-acétyl-N-[*trans*-3-(N-heptadécylcarbamoyloxy)tétrahydropyranne-2-ylméthoxycarbonyl]aminométhyl}pyridinium.

**40.** Procédé selon l'une quelconque des revendications 1 à 5, dans lequel on choisit les réactifs et conditions réactionnelles de manière à préparer le sel de 3-{7-acétoxy-8-[(3-heptadécylcarbamoyloxytétrahydropyranne-2-yl)méthoxy]octyl}thiazolium.

**41.** Procédé selon l'une quelconque des revendications 1 à 5, dans lequel on choisit les réactifs et conditions réactionnelles de manière à préparer le sel de 1-éthyl-2-{N-acétyl-N-[3-(N-acétyl-N-heptadécylcarbamoylthio)tétrahydropyranne-2-ylméthoxycarbony]aminométhyl} pyridinium.

**42.** Procédé selon l'une quelconque des revendications 1 à 5, dans lequel on choisit les réactifs et conditions réactionnelles de manière à préparer le sel de *dl*-1-éthyl-2-{N-acétyl-N-[*cis*-3-(N-acétyl-N-heptadécylcarbamoylthio)tétrahydropyranne-2-ylméthoxycarbonyl]aminométhyl}pyridinium.

**43.** Procédé selon l'une quelconque des revendications 1 à 5, dans lequel on choisit les réactifs et conditions réactionnelles de manière à préparer le sel de 1-éthyl-2-{N-[3-(N-acétyl-N-heptadécylcarbamoylthio)tétrahydropyranne-2-ylméthoxycarbonyl]aminométhyl}pyridinium.

**44.** Procédé selon l'une quelconque des revendications 1 à 5, dans lequel on choisit les réactifs et conditions réactionnelles de manière à préparer le sel de *dl*-1-éthyl-2-{N-[*cis*-3-(N-acétyl-N-heptadécylcarbamoylthio)tétrahydropyranne-2-ylméthoxycarbonyl]aminométhyl}pyridinium.

**45.** Procédé selon l'une quelconque des revendications 1 à 5, dans lequel on choisit les réactifs et conditions réactionnelles de manière à préparer le sel de 3-{4-[3-(3-heptadécylcarbamoyloxytétrahydropyranne-2-yl)méthoxy-5-isoxazolyl]butyl}thiazolium.

**46.** Procédé selon l'une quelconque des revendications 1 à 5, dans lequel on choisit les réactifs et conditions réactionnelles de manière à préparer le sel de *dl*-3-{4-[3-(*trans*-3-heptadécylcarbamoyloxytétrahydropyranne-2-yl)méthoxy-5-isoxazolyl]butyl}thiazolium.